# EUROPEAN PATENT SPECIFICATION

(11) **EP 1 123 931 B1**
(45) Date of publication and mention of the grant of the patent: **01.06.2005**
(21) Application number: 01109408.3
(22) Date of filing: 12.10.1994
(51) Int. Cl.: C07D 401/04, C07D 221/16, C07D 401/14, A61K 31/55, C07D 401/12

(54) **Tricylic amide and urea compounds useful for inhibition of G-protein function and for treatment of proliferative diseases**
Tricyclische Amide und Harnstoffderivate zur Inhibierung der G-Protein Funktion und für die Behandlung von proliferativen Behandlungen
Composés tricycliques à base d'amides et d'urée utiles pour inhiber la fonction de la protéine G et au traitement de maladies prolifératives

(30) Priority: 15.10.1993 US 137862
(43) Date of publication of application: 16.08.2001
(62) Divisional of application: 94930650.0
(73) Proprietor: SCHERING CORPORATION, Kenilworth New Jersey 07033 (US)
(72) Inventor: Bishop, Robert W., Pompton Plains, NJ 07444 (US); Mallams, Alan K., Hackettstown, NJ 07840 (US); Petrin, Joanne M., Cedar Grove, NJ 07009 (US); Doll, Ronald J., Maplewood, NJ 07040 (US); Njoroge, George F., Union, NJ 07083 (US); Piwinski, John J., Lebanon, NJ 08833 (US); Remiszewski, Stacy W., Township of Washington, NJ 07675 (US); Wolin, Ronald L., Westfield, NJ 07090 (US); Taveras, Arthur G., Rockaway, NJ 07866 (US)
(74) Representative: Ritter, Stephen David

(56) References cited:
- EP-A- 0 270 818
- EP-A- 0 396 083
- EP-A- 0 495 484
- WO-A-92/11034
- FRANK J. VILLANI ET AL: "Derivatives of 10,11-Dihydro-5H-dibenzo[a,d]cycloheptene and related compounds.6.Aminoalkyl derivatives of the aza isosteres" JOURNAL OF MEDICINAL CHEMISTRY, vol. 15, no. 7, 1972, pages 750-754, XP000578364 WASHINGTON US
- M.MOTASIM BILLAH ET AL: "Discovery and preliminary pharmacology of SCH 37370,adual antagonist ofPAF and histamine" LIPIDS, vol. 26, no. 2, 1991, pages 1172-1174, XP002169639
- JOHN J. PIWINSKI ET AL: "Dual antagonists of platelet activating factor and histamine.Identification of structural requirements for dual activity of N-acyl-4-(5,6-dihydro-11H-benzo[5,6]cycloh epta-[1,2-b]pyridin-11-ylidene)piperidines " JOURNAL OF MEDICINAL CHEMISTRY, vol. 34, no. 1, 1991, pages 457-461, XP002169640 WASHINGTON US

## Description

### BACKGROUND

International Publication Number WO92/11034, published July 9, 1992, discloses a method of increasing the sensitivity of a tumor to an antineoplastic agent, which tumor is resistant to the antineoplastic agent, by the concurrent administration of the antineoplastic agent and a potentiating agent of the formula: 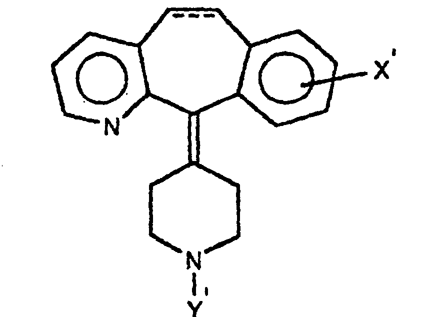 wherein the dotted line represents an optional double bond, X' is hydrogen or halo, and Y' is hydrogen, substituted carboxylate or substituted sulfonyl. For example, Y' can be, amongst others, -COOR' wherein R' is C1 to C6 alkyl or substituted alkyl, phenyl, substituted phenyl, C7 to C12 aralkyl or substituted aralkyl or -2, -3, or -4 piperidyl or N-substituted piperidyl. Y' can also be, amongst others, SO₂R' wherein R' is C1 to C6 alkyl, phenyl, substituted phenyl, C7 to C12 aralkyl or substituted aralkyl. Examples of such potentiating agents include 11-(4-piperidylidene)-5H-benzo[5,6]cyclohepta[1,2-b]pyridines such as Loratadine.

.Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

To acquire transforming potential, the precursor of the Ras oncoprotein must undergo farnesylation of the cysteine residue located in a carboxyl-terminal tetrapeptide. Inhibitors of the enzyme that catalyzes this modification, farnesyl protein transferase, have therefore been suggested as anticancer agents for tumors in which Ras contributes to transformation. Mutated, oncogenic forms of ras are frequently found in many human cancers, most notably in more than 50% of colon and pancreatic carcinomas (Kohl et al., Science, Vol. 260, 1834 to 1837, 1993).

In view of the current interest in inhibitors of famesyl protein transferase, a welcome contribution to the art would be compounds useful for the inhibition of farnesyl protein transferase. Such a contribution is provided by this invention.

### SUMMARY OF THE INVENTION

Inhibition of farnesyl protein transferase by tricyclic compounds of this invention has not been reported previously. Thus, this invention provides a method for inhibiting farnesyl protein transferase using tricyclic compounds of this invention which: (i) potently inhibit farnesyl protein transferase, but not geranylgeranyl protein transferase I, in vitro: (ii) block the phenotypic change induced by a form of transforming Ras which is a farnesyl acceptor but not by a form of transforming Ras engineered to be a geranylgeranyl acceptor; (iii) block intracellular processing of Ras which is a farnesyl acceptor but not of Ras engineered to be a geranylgeranyl acceptor; and (iv) block abnormal cell growth in culture induced by transforming Ras. Several compounds of this invention have been demonstrated to have anti-tumor activity in animal models.

This invention provides a method for inhibiting the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of:. (1).tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

Compounds useful in the claimed methods are represented by Formula 1.0: 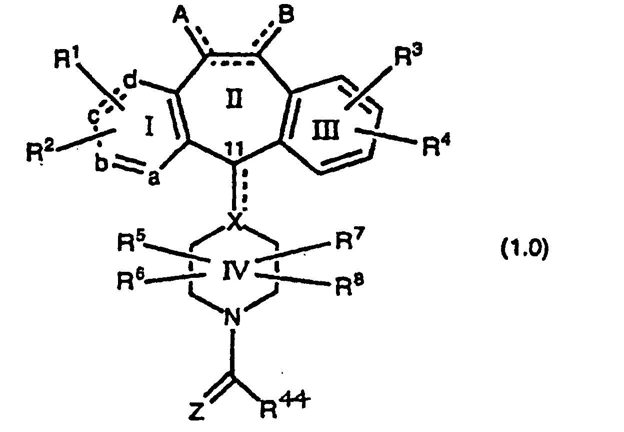 or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰ (e.g., -OCH₃), -COR¹⁰, -SR¹⁰ (e.g., -SCH₃ and -SCH₂C₆H₅), -S(O)ₜR¹¹ (wherein t is 0, 1 or 2, e.g., -SOCH₃ and -SO₂CH₃), -N(R¹⁰)₂, -NO₂, -OC(O)R¹⁰, -CO₂R¹⁰, -OCO₂R¹¹, -CN, -NR¹⁰COOR¹¹, -SR¹¹C(O)OR¹¹ (e.g., -SCH₂CO₂CH₃), -SR¹¹N(R⁷⁵)₂ wherein each R⁷⁵ is independently selected from H and -C(O)OR¹¹ (e.g., -S(CH₂)₂NHC(O)O-t-butyl and -S(CH₂)₂NH₂), benzotriazol-1-yloxy, tetrazol-5-ylthio, or substituted tetrazol-5-ylthio (e.g., alkyl substituted tetrazol5-ylthio such as 1-methyl-tetrazol-5-ylthio), alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R4 taken together represent a saturated or unsaturated C₅-C₇ fused ring to the benzene ring (Ring III);
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶, R⁷ and R⁸ can be taken in combination with R⁴⁰ as defined below to represent -(CH₂)ᵣ- wherein r is 1 to 4 which can be substituted with lower alkyl, lower alkoxy, -CF₃ or aryl, or R⁵ is combined with R⁶ to represent =O or =S and/or R⁷ is combined with R⁸ to represent =O or =S;
R¹⁰ represents H, alkyl, aryl, or aralkyl (e.g., benzyl);
R¹¹ represents alkyl or aryl;
X represents N, CH or C, which C may contain an optional double bond (represented by the dotted line) to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)ₚ-O- wherein p is 2, 3 or 4;
R⁴⁴ represents 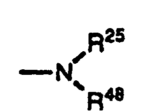 wherein R²⁵ represents heteroaryl (e.g., pyridyl or pyridyl N-oxide) or aryl (e.g., phenyl and substituted phenyl); and R⁴⁸ represents H or alkyl (e.g., methyl); and
Z represents O or S such that R can be taken in combination with R⁵, R⁶, R⁷ or R⁸ as defined above, or R represents R⁴⁴.

Examples of R²⁵ groups include: 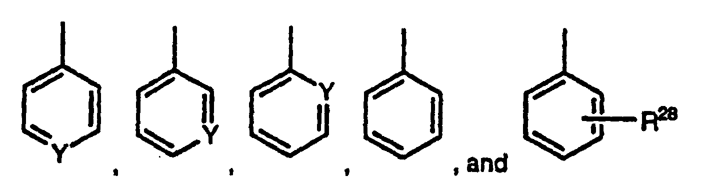 wherein Y represents N or NO, R²⁸ is selected from the group consisting of: C₁ to C₄ alkyl, halo, hydroxy, NO₂, amino (-NH₂), -NHR³⁰, and -N(R³⁰)₂ wherein R³⁰ represents C₁ to C₆ alkyl.

Tricyclic compounds useful in the methods of this invention are described in: (1) U.S. 5,151,423; (2) U.S. 4,826,853; (3) U.S. 5,089,496; (4) WO 88/03138 published on May 5, 1988 (PCT/US87/02777); and (5) U.S. 5,104,876; the disclosures of each being incorporated herein by reference thereto. Those compounds within the scope of this invention which are not described in these documents are described herein.

This invention further provides novel compounds of Formula 1.0 having the formula: 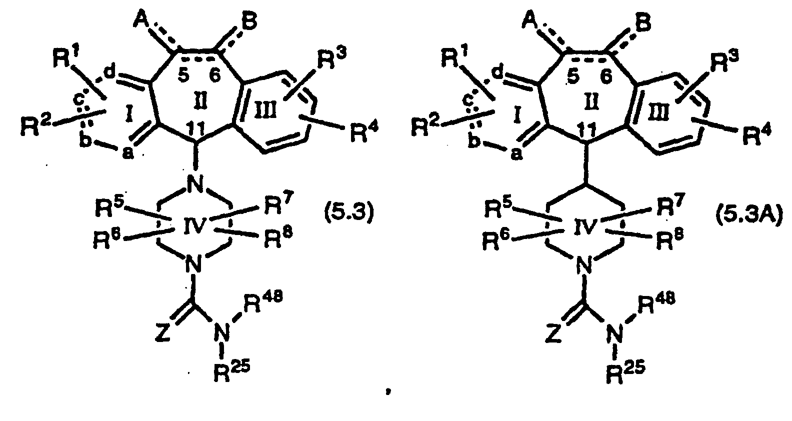 or 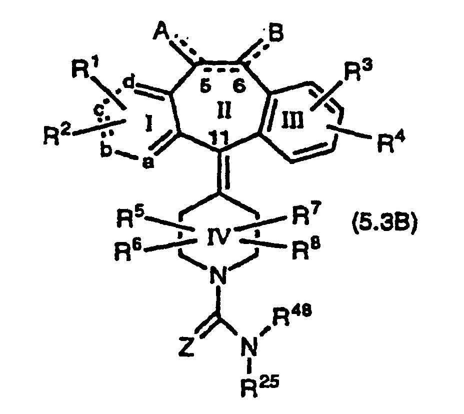 wherein all the substituents are as defined for Formula 1.0. Preferably R²⁵ represents heteroaryl.

This invention also provides the use of a compound of Formula (1.0) for the monufacture of a medicament for inhibiting tumor growth. In particular, this invention provides the use of a compound of Formula (1.0) for the manufacture of a mediment for inhibiting the growth of tumors expressing an activated Ras oncogene. Examples of tumors which may be inhibited include, but are not limited to, lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma and epidermal carcinoma.

It is believed that this invention also provides the use of a compound of Formula (1.0) for the manufacture of a medicament for inhibiting proliferative diseases, both benign and malignant, wherein Ras proteins are aberrantly activated as a result of oncogenic mutation in other genes-i.e., the Ras gene itself is not activated by mutation to an oncogenic form. For example, the benign proliferative disorder neurofibromatosis, or tumors in which Ras is activated due to mutation or overexpression of tyrosine kinase oncogenes (e.g., neu, src, abl, Ick, and fyn), may be inhibited by the tricyclic compounds described herein.

The compounds of this invention inhibit farnesyl protein transferase and the farnesylation of the oncogene protein Ras. This invention further provides the use of a compound of Formula (1.0) for the manufacture of a medicament for inhibitin ras farnesyl protein transferase, in mammals, especially humans. The administration of the compounds of this invention to patients, to inhibit farnesyl protein transferase, is useful in the treatment of the cancers described above.

. The tricyclic compounds useful in the methods of this invention inhibit the abnormal growth of cells. Without wishing to be bound by theory, it is believed that these compounds may function through the inhibition of G-protein function, such as ras p21, by blocking G-protein isoprenylation, thus making them useful in the treatment of proliferative diseases such as tumor growth and cancer. Without wishing to be bound by theory, it is believed that these compounds inhibit ras farnesyl protein transferase, and thus show antiproliferative activity against ras transformed cells.

This invention also provides a process for producing 3-nitro substituted compounds useful as intermediates for compounds of Formula (1.0). The process comprises reacting one molar equivalent of a compound: 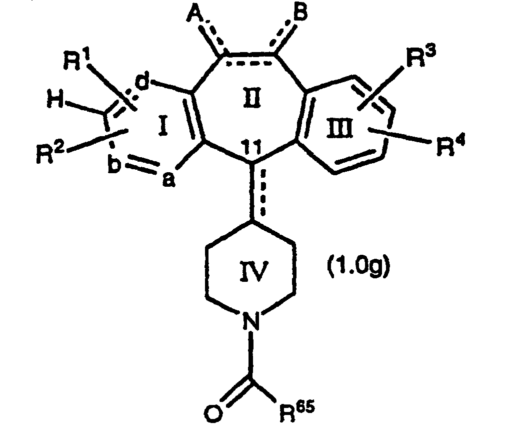 wherein R¹, R², R³, R⁴, A, B, a, b, d, and the dotted lines are as defined for Formula 1.0; and R⁶⁵ represents H or -OR⁶⁶ wherein R⁶⁶ represents alkyl (e.g., C₁ to C₄ alkyl, preferably ethyl); with one molar equivalent of a nitrating reagent, said nitrating reagent being preformed (i.e., prepared first) by mixing, at cold temperature (e.g., at 0°C) equimolar amounts of tetrabutyl ammonium nitrate with trifluoroacetic anhydride; the reaction of the nitrating reagent with the compound of Formula 1.0g taking place in a suitable aprotic solvent (e.g., methylene chloride, chloroform, toluene or tetrahydrofuran); said reaction with said nitrating reagent being conducted at a temperature and for a period of time sufficient to allow the reaction to proceed at a reasonable rate to produce the desired final 3-nitro compound of Formula 1.0h (described below)--i.e., the reaction of the compound of Formula 1.0g with said nitrating reagent is conducted at an intial temperature of 0°C, and said reaction temperature is thereafter allowed to rise to about 25°C during the reaction time period. The reaction usually proceeds overnight to completion, i.e., the reaction usually proceeds for about 16 hours. The reaction can be conducted within a temperature of 0°C to about 25°C during a time period of about 10 to about 24 hours. Preferably the reaction is initially conducted at 0°C and the temperature is allowed to warm up to 25°C. The reaction produces the 3-nitro compound:

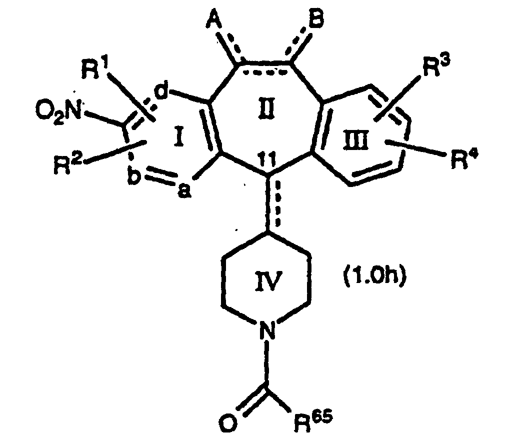

The compound of Formula 1.0h can then be converted to other 3-substituted products by methods well known to those skilled in the art. For example, the 3-nitro compounds can be converted to 3-amino, 3-halo, 3-cyano, 3-alkyl, 3-aryl, 3-thio, 3-arylalkyl, 3-hydroxyl, and 3-OR⁶⁷ wherein R⁶⁷ is alkyl or aryl. The 3-substituted compounds can then be converted to final products (wherein R⁶⁵ is R⁴² or R⁴⁴) by the procedures described herein.

This invention also provides a process for producing 3-nitro compounds of the formula (1.0i) useful as intermediates for compounds of Formula (1.0): 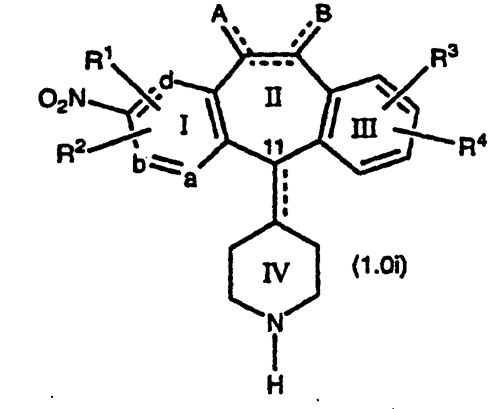 by producing a compound of Formula 1.0h from 1.0g as described above; and then hydrolyzing the compound of Formula 1.0h by dissolving the compound of Formula 1.0h in a sufficient amount of concentrated acid (e.g., concentrated HCI or aqueous sulfuric acid), and heating the resulting mixture to a temperature sufficient to remove (hydrolyze) the -C(O)R⁶⁵ substituent, for example, heating to reflux or to a temperature of about 100°C. This hydrolysis process is exemplified in Preparative Example 28.

The compound of Formula 1.0i can then be converted to other 3-substituted compounds as discussed above for the compounds of Formula 1.0h. The compounds of Formula 1.0i can then be converted to compounds of this invention by the methods described herein.

This invention also provides a process for producing compounds of the formula (1.0j) which are useful as intermediates for preparing compounds of Formula (1.0): 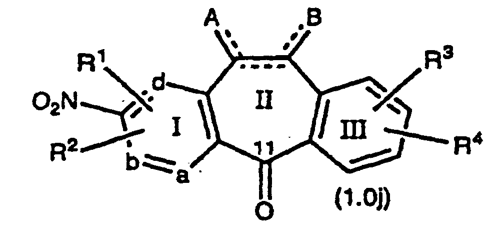 by reacting one molar equivalent a compound of formula: 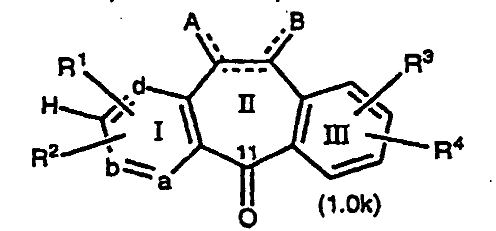 with one molar equivalent of a nitrating reagent, said nitrating reagent being preformed (i.e., prepared first) by mixing, at cold temperature (e.g., at 0°C) equimolar amounts of tetrabutyl ammonium nitrate with trifluoroacetic anhydride; the reaction of the nitrating reagent with the compound of Formula 1.0k taking place in a suitable aprotic solvent (e.g., methylene chloride, chloroform, toluene or tetrahydrofuran); said reaction with said nitrating reagent being conducted at a temperature and for a period of time sufficient to allow the reaction to proceed at a reasonable rate to produce the desired final 3-nitro compound of Formula 1.0j--i.e., the reaction of the compound of Formula 1.0k with said nitrating reagent is conducted at an intial temperature of 0°C, and said reaction temperature is thereafter allowed to rise to about 25°C during the reaction time period. The reaction usually proceeds overnight to completion, i.e., the reaction usually proceeds for about 16 hours. The reaction can be conducted within a temperature of 0°C to about 25°C during a time period of about 10 to about 24 hours. Preferably the reaction is initially conducted at 0°C and the temperature is allowed to warm up to 25°C. In Formulas 1.0j and 1.0k, R¹, R², R³, R⁴, A, B, a, b, d, and the dotted lines are as defined for Formula 1.0

The compounds of Formula 1.0j can be converted to compounds of Formula 1.0h by methods described below. Also, as discussed above for the compounds of Formula 1.0h, the compounds of Formula 1.0j can be converted to other 3-substituted compounds wherein the substituents are those discussed above for Formula 1.0h.

The compounds of Formula 1.0j can be converted to compounds of Formula 1.0m which are useful as intermediates for compounds of Formula (1.0): 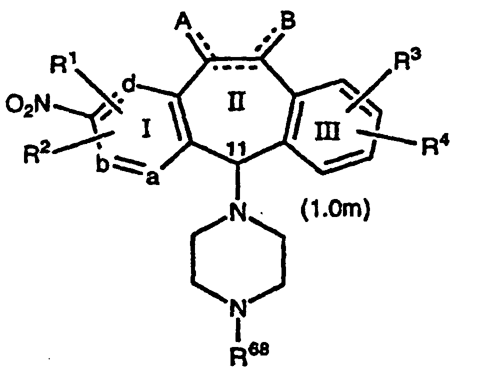 wherein R⁶⁸ is H or -COOR^{a} wherein R^{a} is a C₁ to C₃ alkyl group (preferably F⁶⁸ is H), by reducing a compound of Formula 1.0j with a suitable reducing agent (such as sodium borohydride) in a suitable solvent (such as ethanol or methanol) at a suitable temperature to allow the reaction to proceed at a reasonable rate (e.g., 0 to about 25°C); reacting the resulting product (Formula 1.0j wherein the =O has been reduced to a -OH) with a chlorinating agent (e.g., thionyl chloride) in an suitable organic solvent (e.g., benzene, toluene or pyridine) at a suitable temperature to allow the reaction to proceed at a reasonable rate (e.g., about -20 to about 20°C, preferably at -15°C, see, for example Preparative Example 7) to produce a compound of Formula 1.0n: 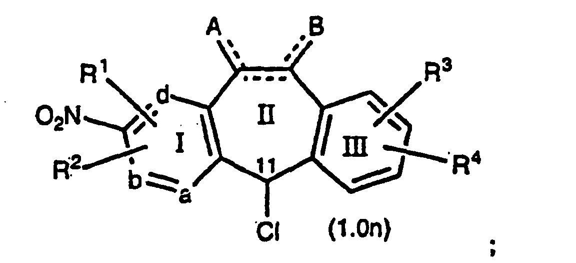 and reacting a compound of Formula 1.0n with a compound of the formula: 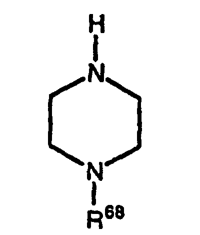 wherein R⁶⁸ is as previously defined, and is preferably H, in a suitable organic solvent (such as tetrahydrofuran or toluene) containing a suitable base (such as triethylamine or N-methylmorpholine) at a suitable temperature to allow the reaction to proceed at a reasonable rate (e.g., 25 to about 120°C).

Compounds of Formula 1.0m can be converted to compounds of this invention by the methods disclosed herein. Also, as discussed above for the compounds of Formula 1.0h, the compounds of Formula 1.0m can be converted to other 3-substituted compounds wherein the substituents are those discussed above for Formula 1.0h.

This invention also provides novel compounds (produced in the above described processes as intermediates to the compounds of this invention) having the formulas: 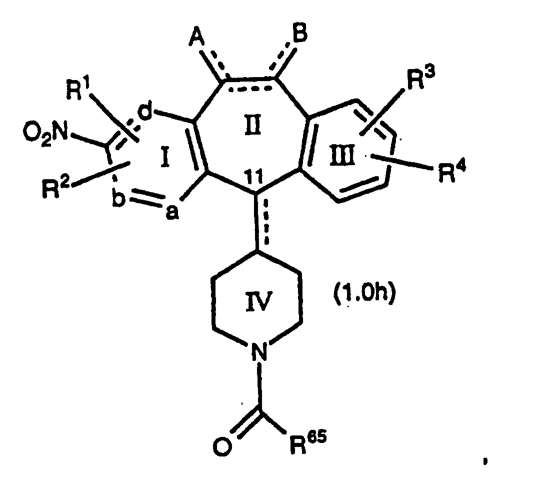 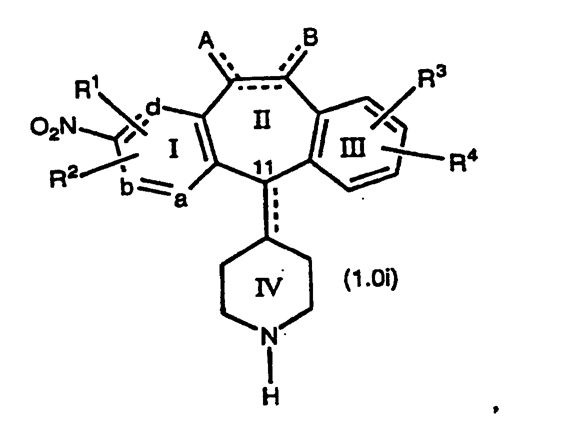 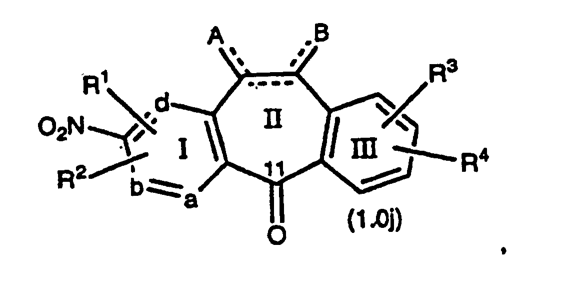 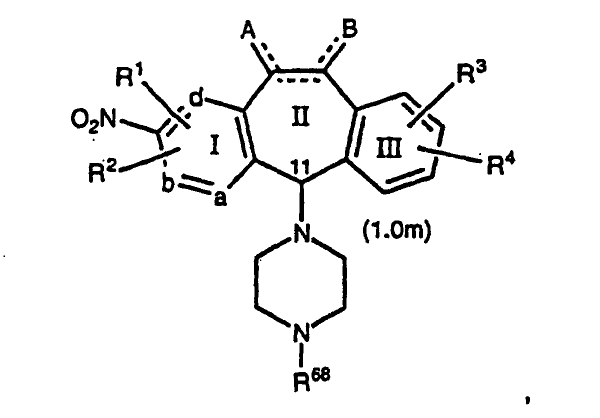 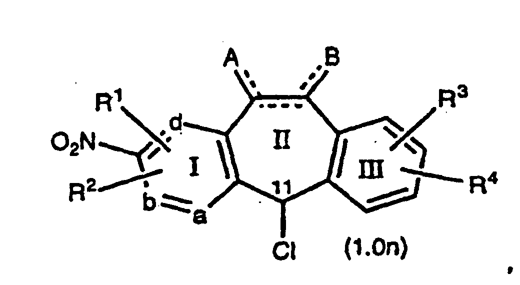 and 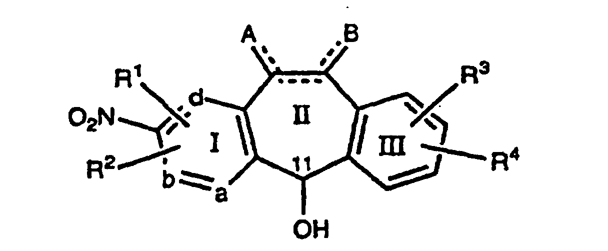 wherein all substituents are as defined herein.

Preferably, for the intermediate compounds of the processes of this invention, R¹ and R² are H; R³ is halo, most preferably Cl, in the C-8 position; R⁴ is H; and A and B are H when the double between C-5 and C-6 is present, and A and B are H₂ when the bond between C-5 and C-6 is a single bond (most preferably the bond between C-5 and C-6 is a single bond). Those skilled in the art will appreciate that Rings I, II, and/or III can be further substituted, as described herein, to produce the desired compounds of the invention.

Examples of such novel intermediate compounds include: 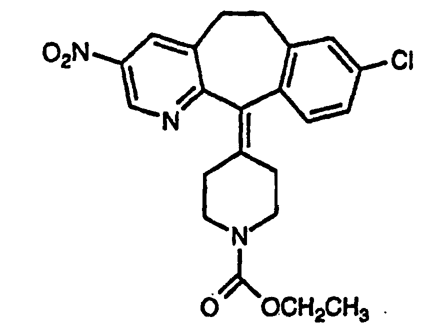 and

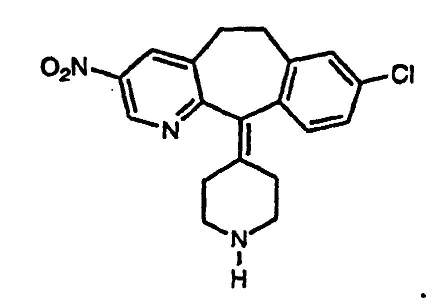

### DETAILED DESCRIPTION OF THE INVENTION

As used herein, the following terms are used as defined below unless otherwise indicated:
M⁺-represents the molecular ion of the molecule in the mass spectrum;
MH⁺-represents the molecular ion plus hydrogen of the molecule in the mass spectrum;
Bu-represents butyl;
Et-represents ethyl;
Me-represents methyl;
Ph-represents phenyl;
benzotriazol-1-yloxy represents 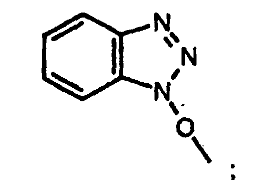
1-methyl-tetrazol-5-ylthio represents 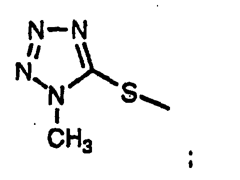
alkyl-(including the alkyl portions of alkoxy, alkylamino and dialkylamino)-represents straight and branched carbon chains and contains from one to twenty carbon atoms, preferably one to six carbon atoms;
alkanediyl-represents a divalent, straight or branched hydrocarbon chain having from 1 to 20 carbon atoms, preferably 1 to 6 carbon atoms, the two available bonds being from the same or different carbon atoms thereof, e.g., methylene, ethylene, ethylidene, -CH₂CH₂CH₂-, -CH₂CHCH₃, -CHCH₂CH₃, etc.
cycloalkyl-represents saturated carbocyclic rings branched or unbranched of from 3 to 20 carbon atoms, preferably 3 to 7 carbon atoms;
heterocycloalkyl-represents a saturated, branched or unbranched carbocylic ring containing from 3 to 15 carbon atoms, preferably from 4 to 6 carbon atoms, which carbocyclic ring is interrupted by 1 to 3 hetero groups selected from -O-, -S- or- NR¹⁰-(suitable heterocycloalkyl groups including 2- or 3-tetrahydrofuranyl, 2- or 3- tetrahydrothienyl, 2-, 3- or 4-piperidinyl, 2- or 3-pyrrolidinyl, 2- or 3-piperizinyl, 2- or 4-dioxanyl, etc.);
alkenyl-represents straight and branched carbon chains having at least one carbon to carbon double bond and containing from 2 to 12 carbon atoms, preferably from 2 to 6 carbon atoms and most preferably from 3 to 6 carbon atoms;
alkynyl-represents straight and branched carbon chains having at least one carbon to carbon triple bond and containing from 2 to 12 carbon atoms, preferably from 2 to 6 carbon atoms;
aryl (including the aryl portion of aryloxy and aralkyl)-represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, CF₃, amino, alkylamino, dialkylamino, -COOR¹⁰ or -NO₂; and
halo-represents fluoro, chloro, bromo and iodo; and
heteroaryl-represents cyclic groups, optionally substituted with R³ and R⁴, having at least one heteroatom selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms, e.g., 2-, 3- or 4-pyridyl or pyridyl N-oxide (optionally substituted with R³ and R⁴),wherein pyridyl N-oxide can be represented as:

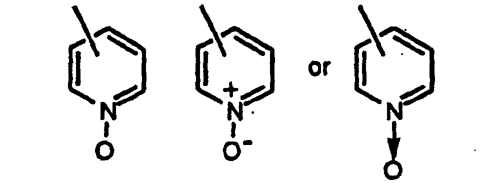

Reference to the position of the substituents R¹, R², R³, and R⁴ is based on the numbered ring structure: 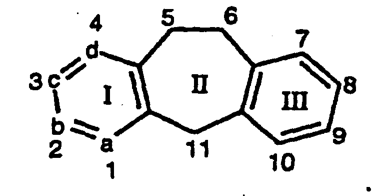 For example, R¹ can be at the C-4 position and R² can be at the C-2 or C-3 position. Also, for example, R³ can be at the C-8 position and R⁴ can be at the C-9 position.

Representative structures of Formula 1.0 include but are not limited to:

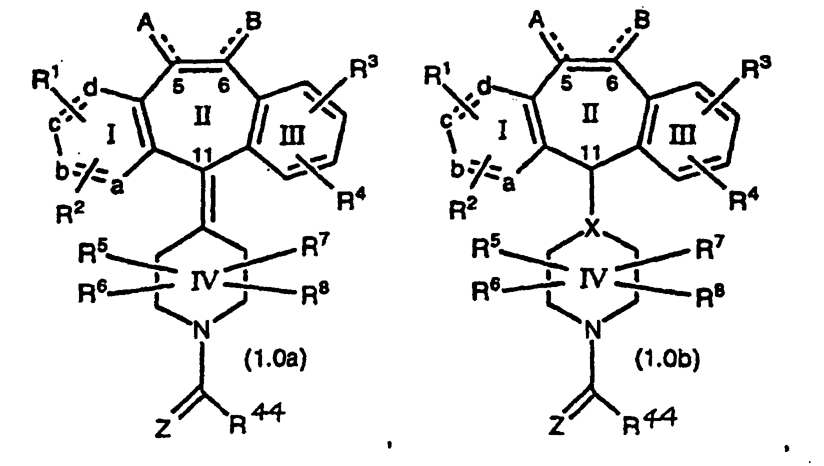

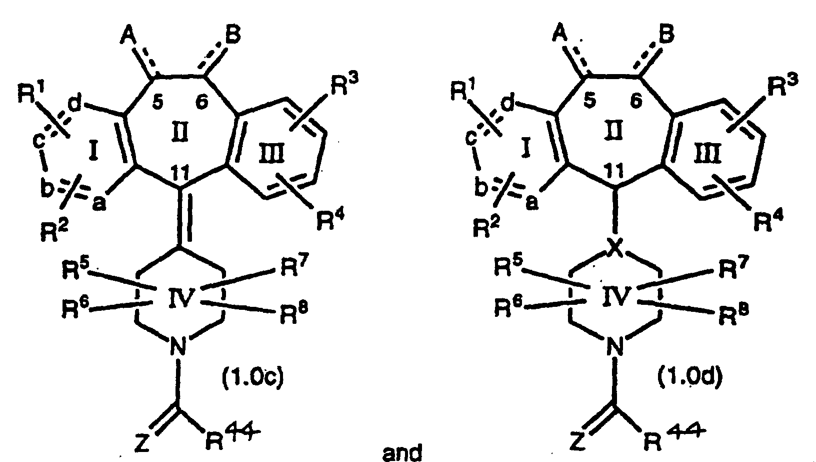

Preferably, for the compounds of Formula 1.0 (including 1.0a to 1.0d):
each of a, b, c, and d are C (carbon); or
one of a, b, c and d (most preferably a) represents N or NO, most preferably N, and the remaining a, b, c and d groups represent CR¹ or CR²;
each R¹ and each R² is independently selected from H, halo (e.g., Cl, Br and F), -CF₃, -OR¹⁰ (e.g., hydroxy and alkoxy (e.g., -OCH₃)), alkyl (e.g., methyl and t-butyl, said alkyl group being optionally substituted with halo), benzotriazol-1-yloxy, -S(O)ₜR¹¹ (e.g., -SCH₂CH₃), -SR¹¹C(O)OR¹¹ (e.g., -SCH₂CO₂CH₃), -SR¹⁰ (e.g., R¹⁰ represents -CH₂C₆H₅) and 1-methyl-tetrazol-5-ylthio; most preferably R¹ and R² are independently H, halo, -CF₃, lower alkyl (e.g., C₁ to C₄, more preferably methyl) or benzotriazol-1-yloxy; more preferably R¹ is Cl or H, and R² is H, Cl or Br; still more preferably R¹ is at the C-4 position, and R² is at the C-3 position; even more preferably R² is Br or H;
R³ and R⁴ are the same or different and each independently represents H, halo, -CF₃, -OR¹⁰, -COR¹⁰, -SR¹⁰, -S(O)ₜR¹¹ (wherein t is 0, 1 or 2), -N(R¹⁰)₂, -NO₂, -OC(O)R¹⁰, -CO₂R¹⁰, -OCO₂R¹¹, -CN, -NR¹⁰COOR¹¹, alkynyl, alkenyl or alkyl, said alkyl or alkenyl group optionally being substituted with halo, -OR¹⁰ or -CO₂R¹⁰; most preferably R³ and R⁴ independently represent H, halo, -CF₃, -OR¹⁰ or alkyl (said alkyl group being optionally substituted with halo); more preferably R³ and R⁴ independently represent H or halo (e.g., Cl, Br, or F); even more preferably R³ is at the C-8 position and R⁴ is at the C-9 positon; still more preferably R³ is Cl at theC-8 position and R⁴ is H at the C-9 position;
R⁵, R⁶, R⁷ and R⁸ each independently represents H, -CF₃ or alkyl (said alkyl optionally being substituted with -OR¹⁰); most preferably R⁵, R⁶, R⁷ and R⁸ independently represent H and alkyl, and more preferably H;
when the optional double bond between carbon atoms 5 and 6 is present, A and B independently represent H, -R¹⁰ or -OR¹⁰, most preferably H, lower alkyl (C₁ to C₄) and alkyloxy (i.e., R¹⁰ represents alkyl), more preferably H and -OH, and still more preferably H; and when no double bond is present between carbon atoms 5 and 6, A and B each independently represent H₂, -(OR¹⁰)₂, alkyl and H, (alkyl)₂, - H and -OR¹⁰ or =O, most preferably H₂, -H and -OH, or =O, and more preferably A represents H₂ and B represents H₂ or =O; and
Z represents O or S, and most preferably O.

The invention further includes compounds of Formula 5.3 having the following structures:

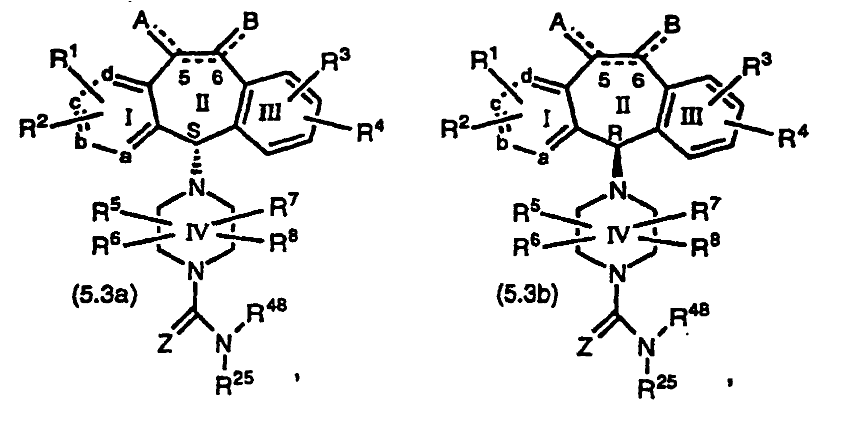

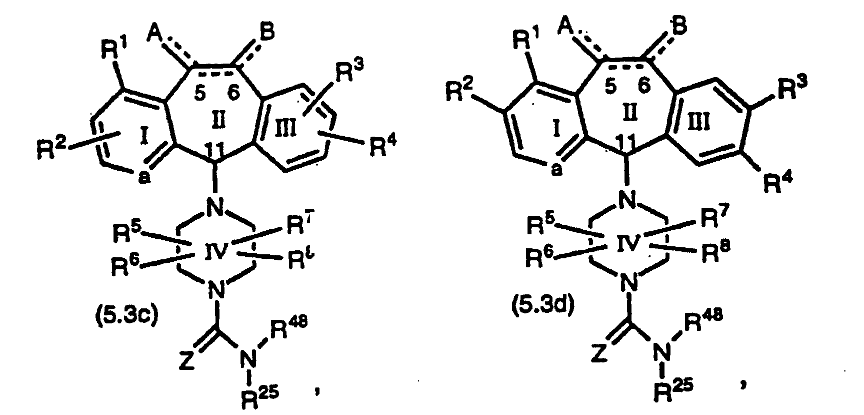

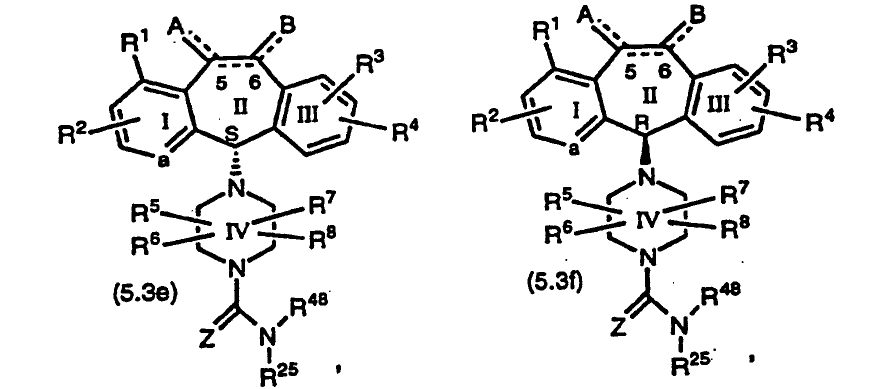

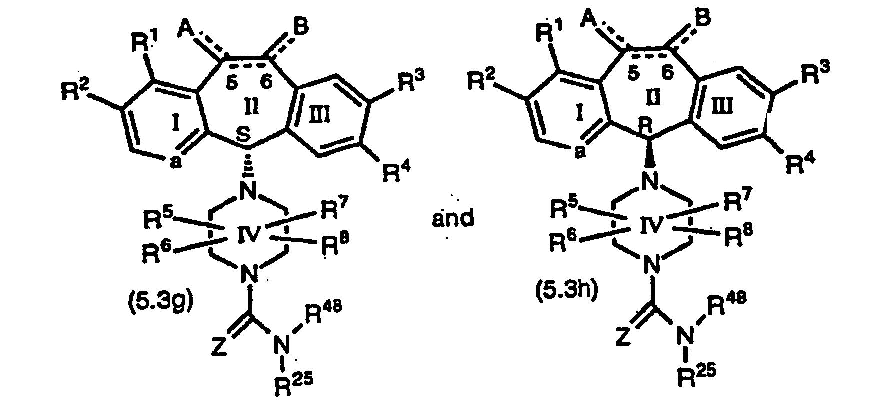

Compounds of formula 5.3A include:

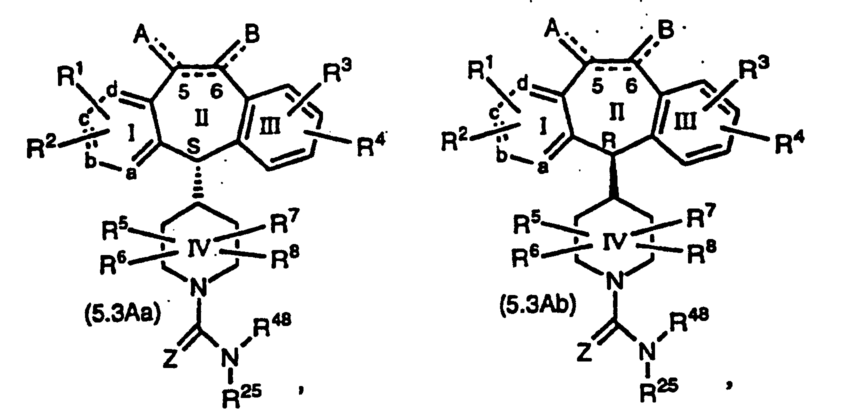

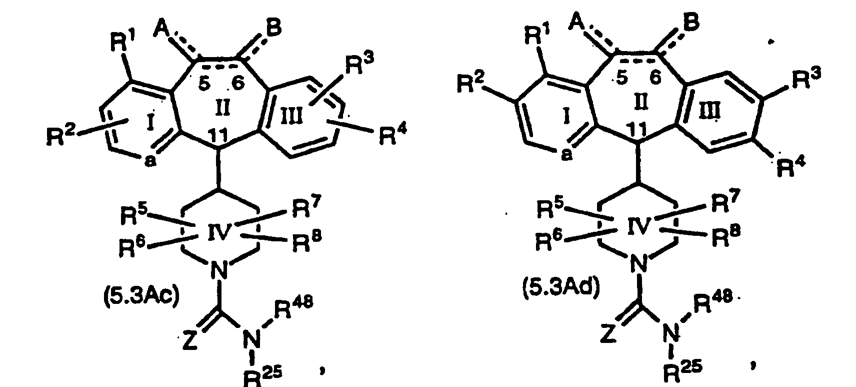

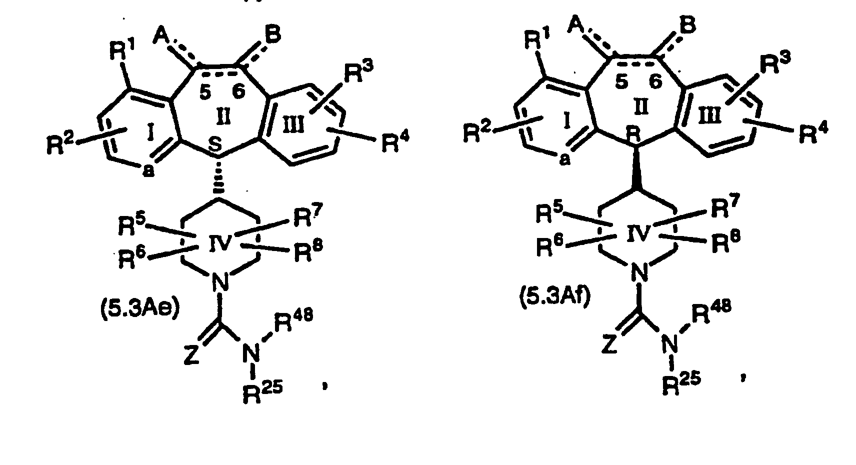

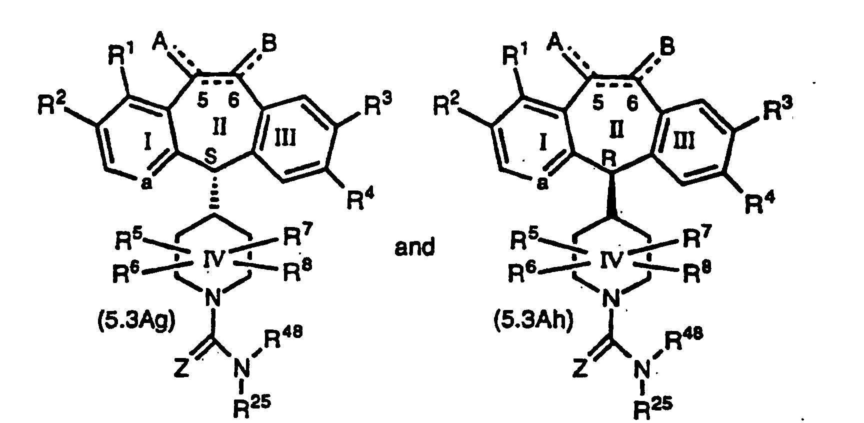

For the compounds of Formulas 5.3, 5.3a-5.3g, 5.3A, 5.3Aa-5.3Ag, and 5.3B, the definitions of the substituents are as defined for Formula 1.0.

Preferably for the compounds of Formulas 5.3, 5.3a-5.3g, 5.3A, 5.3Aa-5.3Ag, and 5.3B, R²⁵ represents phenyl, 2-pyridyl, 3-pyridyl or 4-pyridyl, and most preferably 3-pyridyl. More preferably, R⁴⁸ represents H or methyl and still more preferably H.

Representative compounds of the invention include: 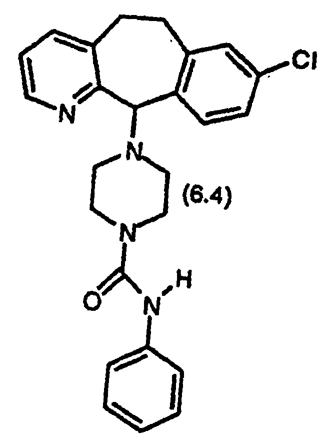 and

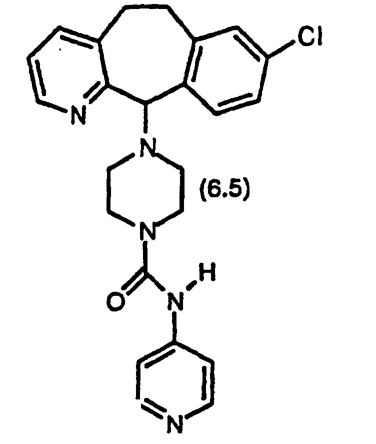

Preferred compounds of this invention are selected from the group consisting of compounds 6.4, 6.5, 6.7, 6.8, 6.9, 6.10, 6.11, 6.12, 6.13, 6.14, 6.15, 6.17, 6.19, 6.20, Example 354, Example 291, Example 292, Example 293, Example 294, Example 312, Example 313, Example 314, Example 365, Example 366, and Example 367.

More preferred compounds of this invention are selected from the group consisting of compounds of Examples: 183, 187 (6.7 and 6.8), 291, 292, 312, 313, 314, and 354.

Even more preferred compounds of this invention are selected from the group consisting of compounds of Examples: 312, and 354.

Lines drawn into the ring systems indicate that the indicated bond may be attached to any of the substitutable ring carbon atoms.

Certain compounds of the invention may exist in different isomeric (e.g., enantiomers and diastereoisomers) forms. The invention contemplates all such isomers both in pure form and in admixture, including racemic mixtures. Enol forms are also included.

Certain tricyclic compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

Compounds of Formula 1.0 wherein R is -N(R¹⁰)₂, and compounds of Formulas 5.3, 5.3A and 5.3B can be prepared by reacting compound 405.00 (described below) with an isocyanate (R¹⁰-N=C=O) in a solvent such as DMF, dichloromethane or THF in accordance with methods known in the art.

The following processes may be employed to produce compounds and intermediates suitable for preparing compounds of the invention. For purposes of describing the processes, the compounds are represented by Formula 400.00: 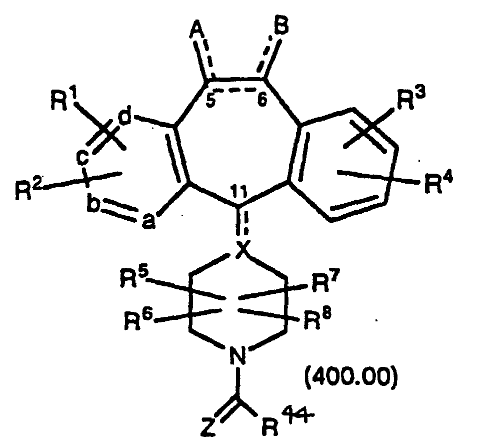 wherein and all substitutents are as described herein.

A. A compound of Formula 405.00 may be coupled with a compound of the formula RCOOH in the presence of coupling agent such as 1-(3-dimethylaminopropyl)-3-ethyl carbodiimde hydrochloride (DEC), N,N'-dicyclohexylcarbodiimide (DCC) or N,N'-carbonyl-dfimidazole (CDI) to produce comoounds of Formula 400.00: 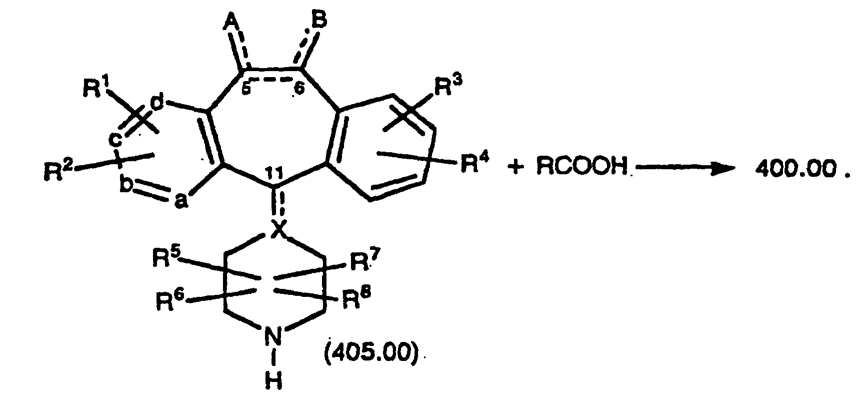 The reaction is usually conducted in an inert solvent such as tetrahydrofuran, DMF or methylene chloride at a temperature between about 0°C and reflux, preferably at about room temperature. When the coupling agent is DCC or DEC, the reaction is preferably run in the presence of 1-hydroxybenzotriazole (HOBT). Method A is the method of choice for preparing compounds of this invention.

B. A compound of Formula 405.00 may also be reacted with a compound of Formula 410.00 in the presence of base to produce compounds of Formula 400.00:

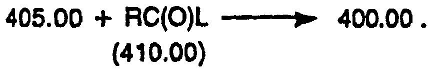

Representative examples of appropriate bases are pyridine and triethylamine. L designates a suitable leaving group. For example, a compound of compound 410.00 may be an acyl halide (e.g., L represents halo) or an acyl anhydride, (e.g., L is -O-C(O)-R). The leaving group may also be alkoxy, in which case the compounds of Formula 400.00 may be produced by refluxing a compound of Formula 405.00 with an excess of a compound of Formula 410.00.

Compounds of Formula 405.00 may be prepared by cleaving the group COOR^{a} from the corresponding carbamates 415.00, for example, via acid hydrolysis (e.g., HCl) or base hydrolysis (e.g., KOH): 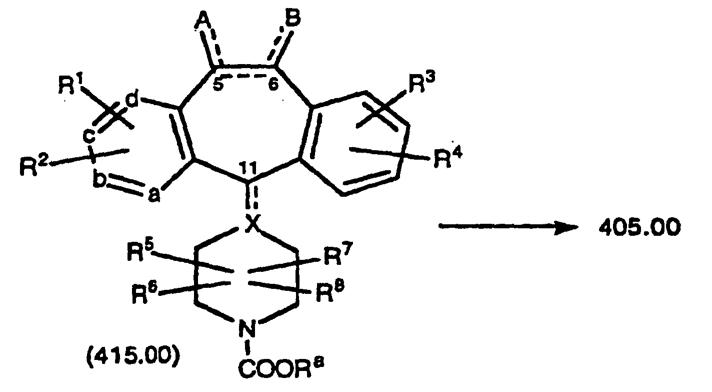 wherein R^{a} is a group which does not prevent the cleavage reaction, e.g., R^{a} is an optionally substituted alkyl such as ethyl.

Alternatively, depending upon the nature of R^{a}, as determined by one skilled in the art, Compound 415.00 may be treated with an organometallic reagent (e.g., CH₃Li), a reductive reagent (e.g., Zn in acid), etc., to form compounds of Formula 405.00.

Compound 415.00 may be prepared from the N-alkyl compound shown as Formula 420.00 below, in the manner disclosed in U.S. Patents 4,282,233 and 4,335,036.

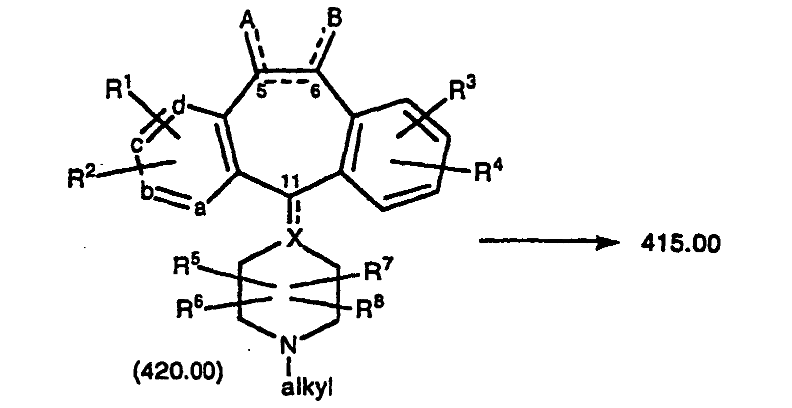

It also will be apparent to one skilled in the art that there are other methods for converting Compound 420.00 to Compound 405.00. For example, treatment of Compound 420.00 with BrCN via von Braun reaction conditions would provide nitrile 420.00a. Subsequent hydrolysis of the nitrile under either aqueous basic or acidic conditions would produce Compound 405.00. This method is preferable when there is substitution on the piperidine or piperazine ring.

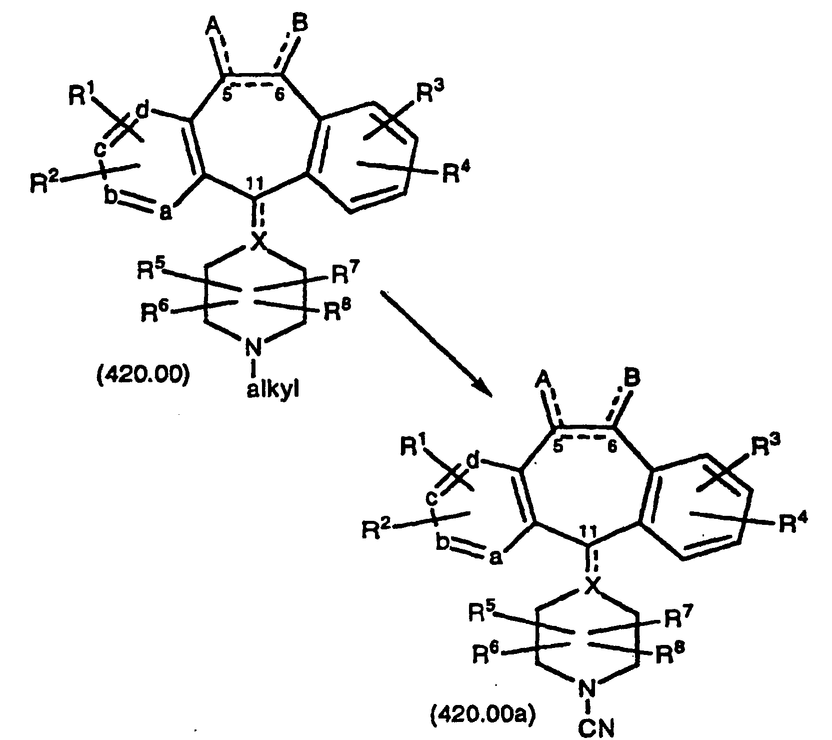

C. The compounds of Formula 400.00 wherein Z is O or S may be made by an alternative process using direct conversion of the N-alkyl compound 420.00 with an appropriate compound of Formula 410.00 such as an acyl halide or acyl anhydride. Preferably the reaction is run in the presence of an appropriate nucleophile (e.g. LiI, etc.) and solvent (e.g., toluene, dioxane or xylenes). An appropriate base, may be added, and heating may be required. Typically, a temperature ranging from 50-150°C (preferably 100-120°C) is utilized. 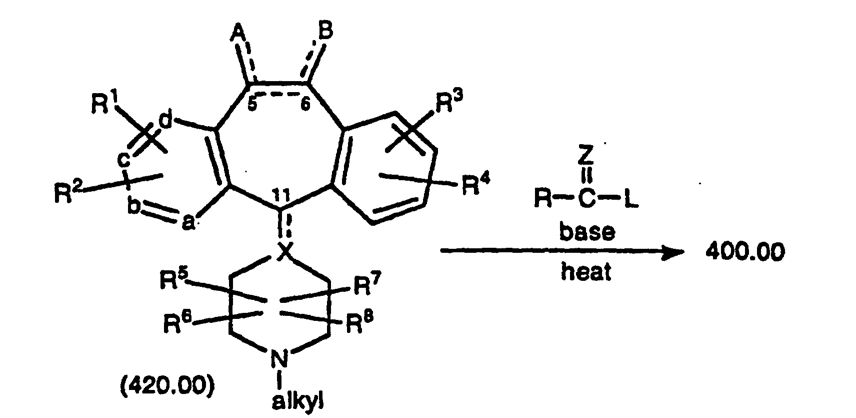 . Compound 420.00 is prepared as described in part B above.

### PREPARATION OF SINGLE BOND COMPOUNDS

Compounds of Formula 400.00, wherein X is carbon and the bond to carbon 11 (C-11) is a single bond, can be prepared by reducing compounds of Formula 405.00, wherein X is carbon and the bond to C-11 is a double bond, with lithium aluminum hydride in tetrahydrofuran. Conversion to final products can be done following the process described above for conversion of compounds of Formula 405.00 to compounds of Formula 400.00.

### PREPARATION OF DOUBLE BOND COMPOUNDS

Compounds of Formula 400.00, wherein X is a carbon atom having an exocyclic double bond to carbon 11, may be prepared from compound 420.00 as described above. Compounds of Formula 420.00 may be produced by the methods disclosed generally in U.S. Patent 3,326,924 or alternatively may be prepared by a ring closure reaction, wherein the desired cycloheptene ring is formed by treating compound 425.00 with a super acid. Suitable super acids for this purpose include, for example, HF/BF₃, CF₃SO₃H (triflic acid), CH₃SO₃H/BF₃, etc. The reaction can be performed in the absence of, or with, an inert co-solvent such as CH₂Cl₂. The temperature and time of the reaction vary with the acid employed. For example, with HF/BF₃ as the super acid system the temperature may be controlled so as to minimize side reactions, such as HF addition to the exocyclic double bond. For this purpose, the temperature is generally in the range of from about +5°C to -50°C. With CF₃SO₃H as the super acid system, the reaction may be run at elevated temperatures, e.g., from about 25°C to about 150°C and at lower temperatures but the reaction then takes longer to complete.

Generally the super acid is employed in excess, preferably in amounts of from about 1.5 to about 30 equivalents.

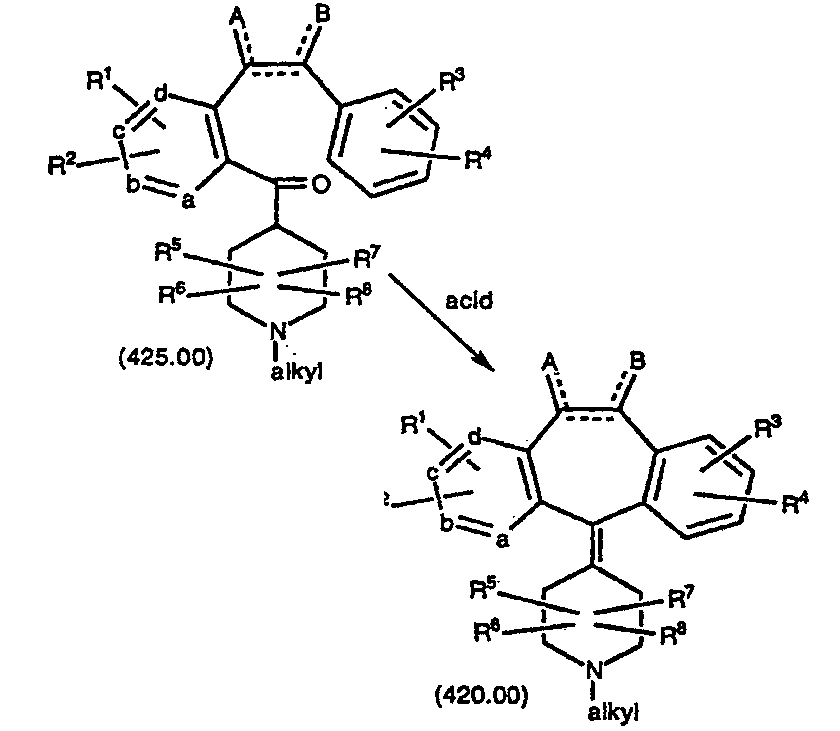

A ketone compound of Formula 425.00 may be formed by hydrolysis of 430.00, e.g., such as by reacting a Grignard intermediate of Formula 430.00 with an aqueous acid (e.g., aqueous HCl). I^{a} in Formula 430.00 represents chloro, bromo or iodo.

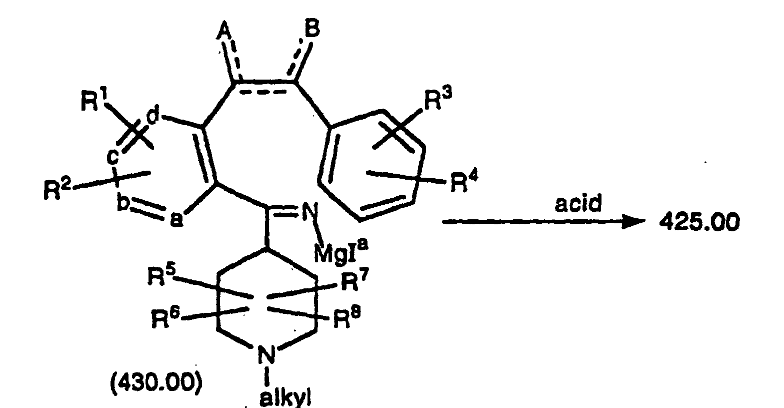

The Grignard intermediate 430.00 is formed by the reaction of the cyano compound 435.00 with an appropriate Grignard reagent 440.00 prepared from 1-alkyl-4halopiperidine. The reaction is generally performed in an inert solvent, such as ether, toluene, or tetrahydrofuran, under general Grignard conditions e.g., temperature of from about 0°C to about 75°C. Alternatively, other organometallic derivatives of the 1alkyl-4-halo piperidine can be employed.

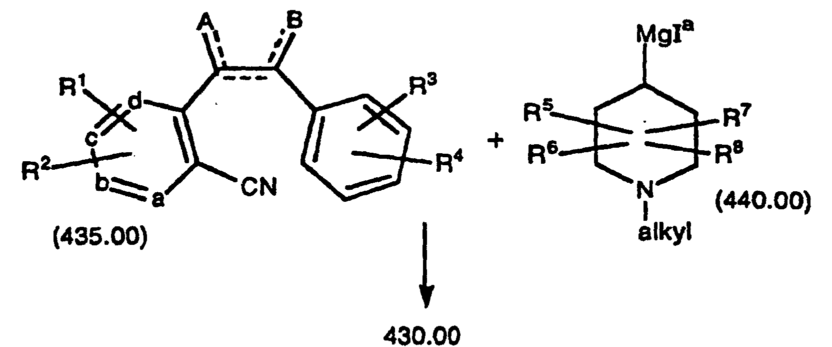

The cyano compound of Formula 435.00 is produced by converting the tertiary butyl amide of Formula 445.00 with a suitable dehydrating agent, such as POCl₃, SOCl₂, P₂O₅, toluene sulfonyl chloride in pyridine, oxalyl chloride in pyridine, etc. This reaction can be performed in the absence of or with a co-solvent, such as xylene.

The dehydrating agent such as POCl₃ is employed in equivalent amounts or greater and preferably in amounts of from about 2 to about 15 equivalents. Any suitable temperature and time can be employed for performing the reaction, but generally heat is added to accelerate the reaction. Preferably the reaction is performed at or near reflux.

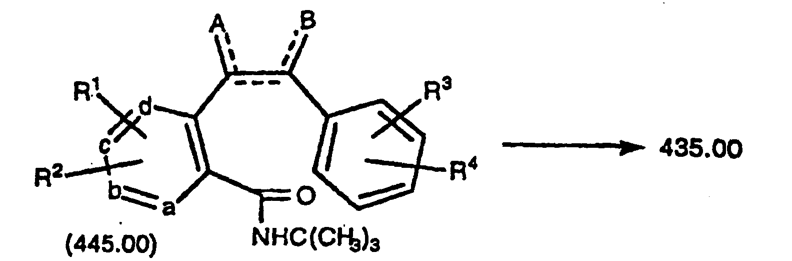

The tert-butylamide of Formula 445.00 may be produced by reaction of a compound of Formula 450.00a and 450.00b, in the presence of base, wherein G is chloro, bromo or iodo.

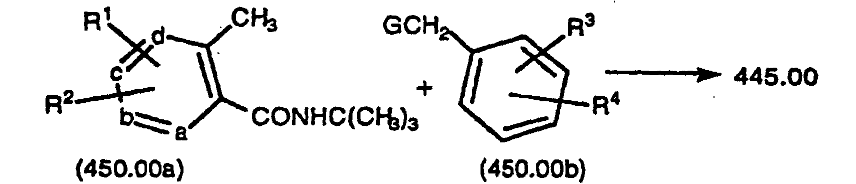

The compound of Formula 450.00a may be formed by hydrolysis of the corresponding nitrile wherein the appropriate cyanomethyl pyridine, such as 2-cyano-3-pyridine, is reacted with a tertiary butyl compound in acid, such as concentrated sulfuric acid or concentrated sulfuric acid in glacial acetic acid. Suitable tertiary butyl compounds include, but are not limited to, t-butyl alcohol, t-butyl chloride, t-butyl bromide, t-butyl iodide, isobutylene or any other compound which under hydrolytic conditions forms t-butyl carboxamides with cyano compounds. The temperature of the reaction will vary depending upon the reactants, but generally the reaction is conducted in the range of from about 50°C to about 100°C with t-butyl alcohol. The reaction may be performed with inert solvents, but is usually run neat.

An alternative process for the formation of compounds of Formula 400.00a may involve direct cyclization of Compound 455.00 as shown below.

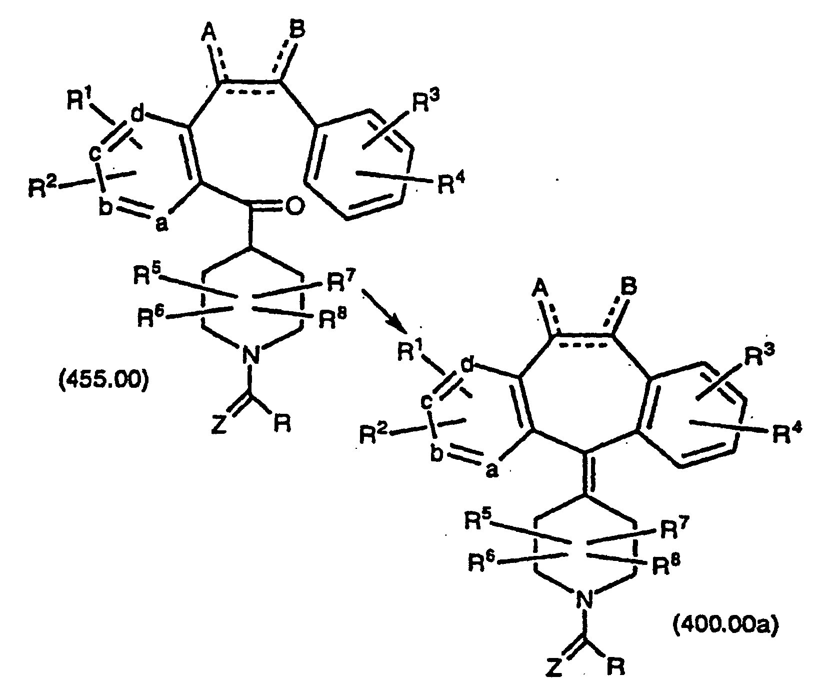

Cyclization to form the cycloheptene ring may be accomplished with a strong acid (e.g., triflic, polyphosphoric, HF/BF₃), and may be performed in an inert solvent, such as ether, toluene or THF. The temperature and time may vary with the acid employed, as described in process A above.

Compounds of Formula 455.00 wherein Z = O or S may be prepared by treating a compound of Formula 425.00 with an appropriate acyl halide or acyl anhydride of formula 410.00. Most preferably this reaction is run in the presence of a good nucleophile, such as LiI, in the appropriate solvent, such as toluene, dioxane or xylene, and at a temperature ranging from 50-150°C, preferably 100-120°C

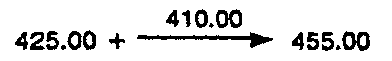

A second method of preparing compounds of Formula 455.00 involves reacting an unsubstituted piperidylidene compound of Formula 460.00 with the appropriate acyl halide or acyl anhydride of Formula 410.00 in the presence of base, such as pyridine or triethylamine. Alternatively, if L = OH in compound 410.00, then coupling of compound 460.00 with compound 410.00 may require use of a conventional coupling reagent, such as DCC or CDI.

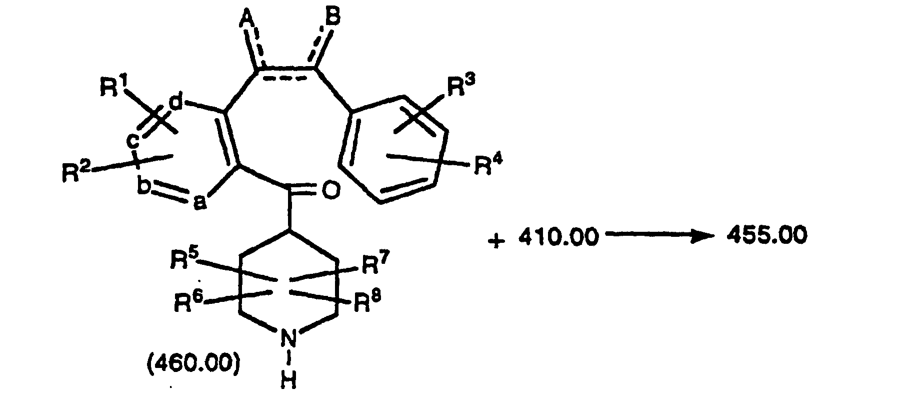

Compounds of Formula 460.00 may be produced from the corresponding carbamates of Formula 465.00, via acid hydrolysis, using for example, aqueous hydrochloric acid, or base hydrolysis using for example, potassium hydroxide. Alternatively, some compounds can be prepared by treating the carbamate, Formula 465.00, with an organometallic reagent, such as methyl lithium or a reductive reagent, such as zinc in acid, etc., depending upon the nature of the R^{a} group. For example, if R^{a} is a simple alkyl group, CO₂R^{a} may be cleaved by alkaline hydrolysis at 100°C.

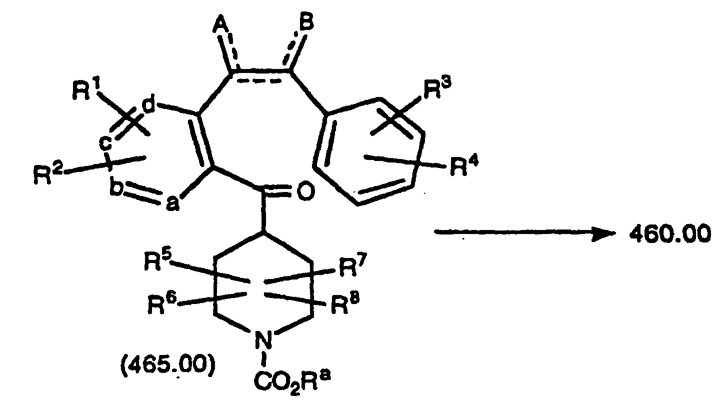

The carbamate compounds of Formula 465.00 may be prepared from the appropriate alkyl compound of Formula 425.00 by treatment with a chloroformate, preferably in an inert solvent, such as toluene, with warming to approximately 80°C. Other alternative methods are available for the conversion of 425.00 to 455.00 as previously described (e.g. Von Braun reaction conditions). Compounds of Formula 425.00 may be prepared as described above.

### SUBSTITUTION ON THE PYRIDINE RING

Various methods can be used as described in WO 88/03138 to provide compounds which are substituted on the pyridine ring, i.e., in positions 2-, 3- and or 4- positions of the tricyclic ring system. For example, the cyclization methods described on pages 20-30 of WO 88/03138 can already have the appropriate substituents on the pyridine ring in place. A variety of substituted pyridines are known in the literature and can be employed in these syntheses. Alternatively, the azaketone of Formula XIX (from page 27 of WO 88/03138) 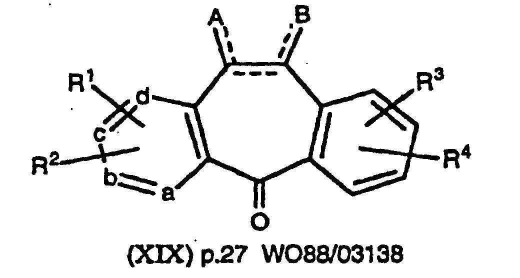 wherein R¹ and R² are both H can be converted to the appropriately substituted azaketone wherein R¹ and R² are non-H substitutents. If both R¹ and R² are desired to be non-H substitutents the procedure would be repeated.

. The azaketone is thus reacted with an oxidizing agent such as meta-chloroperoxybenzoic acid (MCPBA) or hydrogen peroxide to produce the corresponding compound in which the nitrogen of the pyridine ring is as an N-oxide: 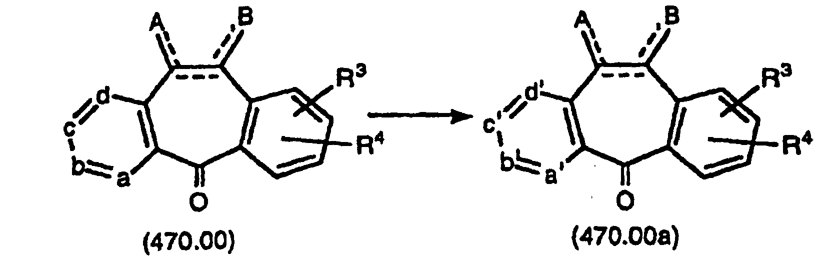 wherein one of a', b', c' or d' is N→O and the others are CH or CR¹ or CR². This reaction is normally run at temperatures from -15°C to reflux, more typically at about 0°C. The reaction is preferably conducted in an inert solvent such as methylene chloride for MCPBA or acetic acid for hydrogen peroxide.

The azaketone N-oxide of Formula 470.00a can then be reacted with a chlorinating agent such as SO₂Cl₂ or SOCl₂ to form a compound of Formula 470.00b. Typically, this reaction results in monosubstitution of Cl in the ortho or para-position relative to the N atom of the ring.

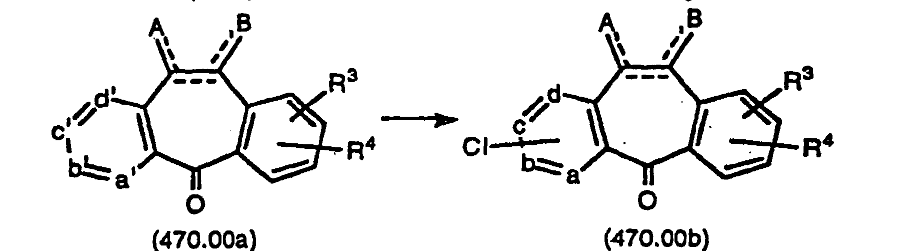

To provide the disubstituted products, steps 1 and 2 above are repeated. 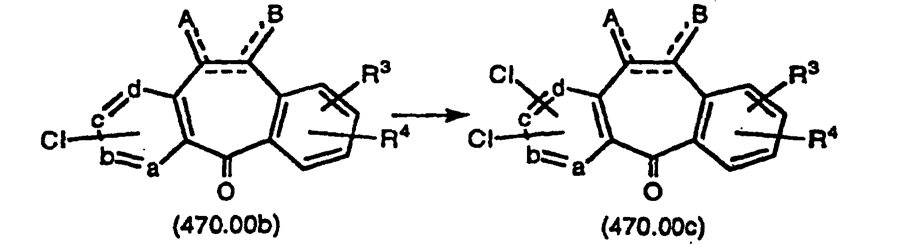 Typically, the resulting disubstituted compounds have Cl ortho and para relative to the N atom of the pyridine ring.

The mono or disubstituted compounds of Formulas 470.00b and 470.00c above can be reacted with various nucleophiles such as alkoxides, amines, thiols, etc. This will result in compounds where one or both of the Cl substituents are replaced by the nucleophile to provide a compound of Formula 470.00d or a compound easily converted to Formula 470.00d.

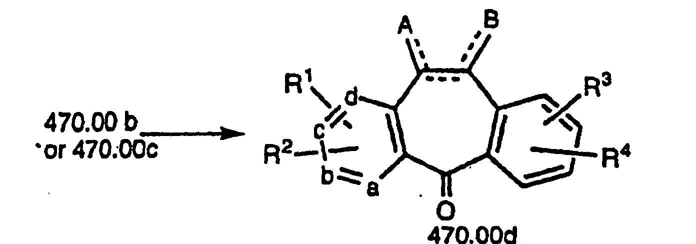

The substituted ketone of Formula 470.00 can then be converted to the desired compound by the methods described above and in WO 88/03138 and in U.S. Patent No. 3,326,924.

Formula 405.00, wherein R¹ or R² are chlorine, can be made by the following alternate process.

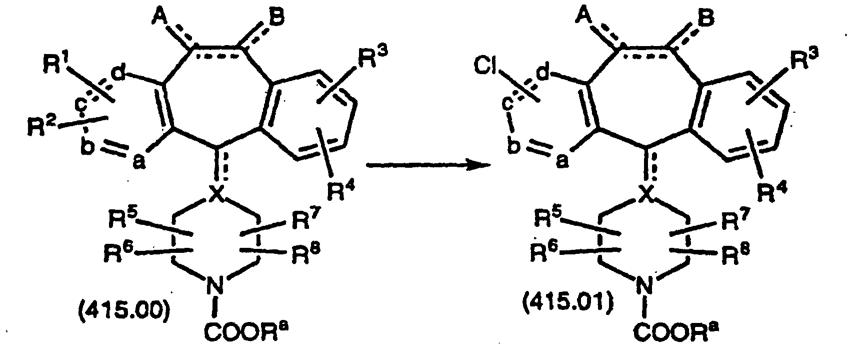

The N-oxide of Formula 415.00 can be treated with POCI₃ to form a compound of Formula 415.01. Typically, this reaction results in monosubstitution of Cl in the ortho or para position relative to the N atom of the ring.

Alternatively, the Cl substituted azaketones of Formula 470.00b or 470.00c above can be converted to the corresponding derivatives of Formula 405.00 above Wherein R¹ and/or R² is Cl by methods analogous to those described above. At this point the Cl substituent(s) can be displaced by an appropriate nucleophile to provide the desired substituent. Suitable nucleophiles include alkoxide, amines, thiols, etc. This reaction usually requires higher tempertures (e.g., from about 100° to about 200°C) than the displacement reaction to produce ketone 470.00d above. It is also usually conducted in a sealed vessel in an inert solvent. The compound of Formula 405.00 is then converted to a compound of Formula 400.00 as described above.

Various electrophilic species can also be added to the pyridine ring from the corresponding halo-substituted pyridine (Formula 405.00 wherein R¹ is halo, preferably bromo or iodo). Transmetallation of the halo derivative using an alkyl lithium (e.g. n-BuLi) provides the lithio derivative, which can then be quenched with the appropriate electrophile (e.g. R¹L, etc.).

An alternative process for introducing substituents at the C-3 position of pyridine Ring I of Formula 1.0, involves nitrating a compound of Formula 415.00 (except wherein X is nitrogen) or a compound of Formula 470.00d with tetrabutylammonium nitrate - trifluoroacetic anhydride in methylene chloride at a temperature of 0°C to room temperature (about 25°C). The nitro group may then be reduced to the corresponding amine using iron filings in ethanol, or powdered zinc - acetic acid in aqueous THF. By methods know to those skilled in the art, the amine group can be converted to a variety of substituents, such as, halo, cyano, thio, hydroxyl, alkyl, alkenyl, alkynyl and haloalkyl.

Wherein Z represents suifur, a compound of Formula 400.00 wherein Z is oxygen is reacted with P₂S₅, Lawesson's reagent, or another reagent capable of introducing sulfur in place of oxygen. The reaction may take place at elevated temperature in pyridine, toluene or other suitable solvents. In this and other reactions, numerous conversions of a compound of Formula 400.00 (Z = 0) to another compound of Formula 400.00 (Z = S) are possible.

### PREPARATION OF C5-C6-ENE DERIVATIVES

Compounds of formula 400.00 with a double bond between C-5 and C-6 can be prepared by heating a compound of Formula 470.00h in acetic acid with SeO₂ to produce a compound of Formula 470.00i. Compounds of Formula 470.00i can be converted to final products according to methods already described.

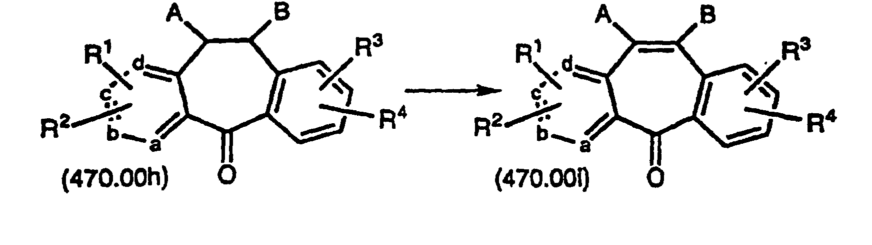

### PREPARATION OF PIPERAZINE ANALOGS

Compounds having a piperazine ring bound to the C-11 of the tricyclic nucleus, i.e., Formula 1.0 wherein X is N, are best prepared via alkylation of the appropriately substituted piperazine compound of Formula 700.00 with a compound of Formula 705.00. Compounds of Formula 705.00 contain the appropriately substituted halide (such as Cl, Br, or I) or other similar leaving group (e.g., tosyloxy or mesyloxy). The reaction is usually conducted in an inert solvent, such as THF or toluene, optionally with a base such as triethylamine or potassium carbonate, and typically at a temperature range of ambient to reflux to produce a compound of Formula 710.00. 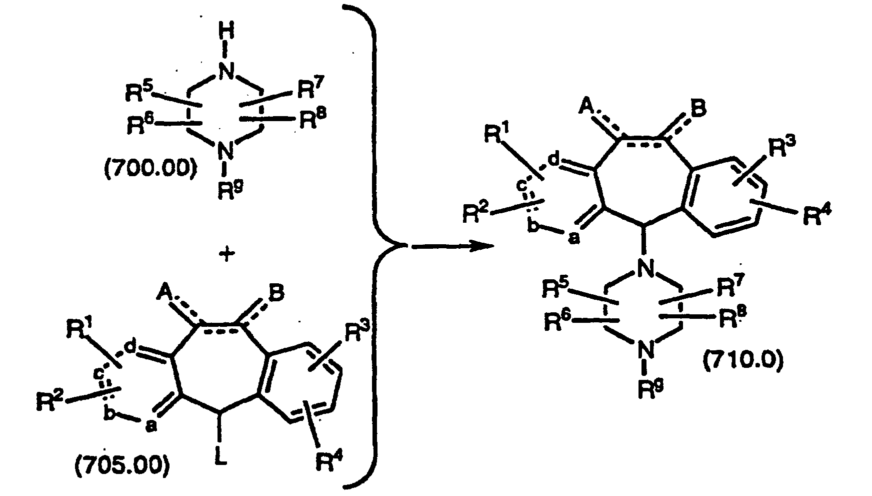 In this reaction R⁹ is H, CO₂R^{a} (wherein R^{a} is a C₁ to C₄ alkyl group) or C(Z)R. The preparation of compound 705.00 wherein L is Cl is analogous to the procedure described in U.S. 3, 409,621. One skilled In the art can prepare other derivatives of 705.00 (e.g., L is Br, I, mesyloxy, or tosyloxy). When R⁹ is H, or CO₂R^{a}, these are converted to compounds of the invention by processes known in the art.

An alternate route for generating the compound of Formula 710.00 is by reductive amination of the aza ketone 715.00 with the piperazine 700.00.

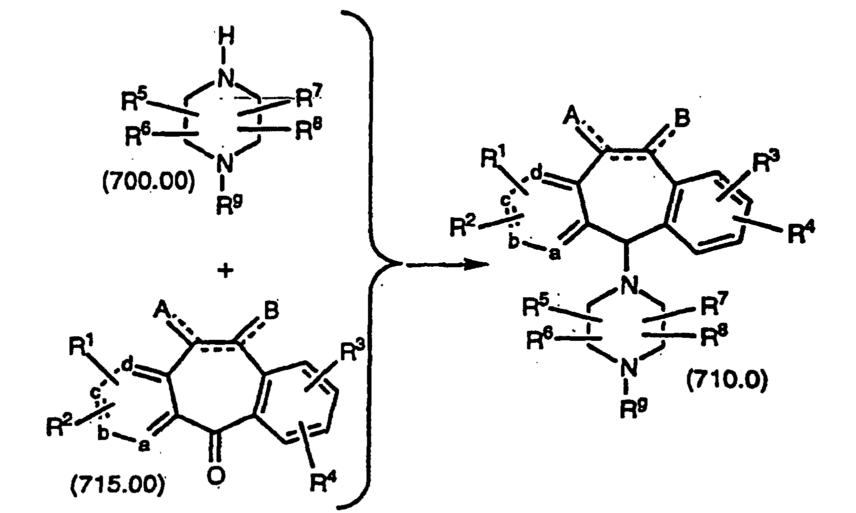

The reaction is typically carried out in a polar solvent, such as methanol or ethanol, optionally in the presence of a dehydrating agent, such as 3Å molecular sieves. The intermediate Schiff base can be reduced to the compound of Formula 710.00 by employing a variety of reducing agents, such as NaCNBH₃, or catalytic hydrogenation, for example, hydrogen over Pd/C.

When R⁹ is C(Z)R, these are the compounds of the invention. When R⁹ is H or CO₂R^{a}, these are converted to compounds of the invention as described herein.

Compounds of Formulas 5.3A and 5.3B, wherein R²⁵ represents a pyridyl N-oxide, can be produced by reacting compounds of Formulas 5.3A and 5.3B, wherein R²⁵ is pyridyl, with a one molar equivalent of an oxidizing agent (such as oxone).

Compounds of Formulas 5.3, 5.3A and 5.3B, wherein R²⁵ represents a pyridyl N-oxide, can be produced by reacting the product of Preparative Example 12 with a peroxyacid (such as m-chloroperbenzoic acid) to give the corresponding N-oxide intermediate. The desired N-oxide product may be obtained from the N-oxide intermediate by following the procedure of Example 183.

In the above processes, it is sometimes desirable and/or necessary to protect certain R¹, R², R³ and R⁴ etc., groups during the reactions. Conventional protecting groups are operable as described in Greene, T.W., "Protective Groups In Organic Synthesis," John Wiley & Sons, New York, 1981. For example, the groups listed in column 1 of Table 1 may be protected as indicated in column 2 of the table:

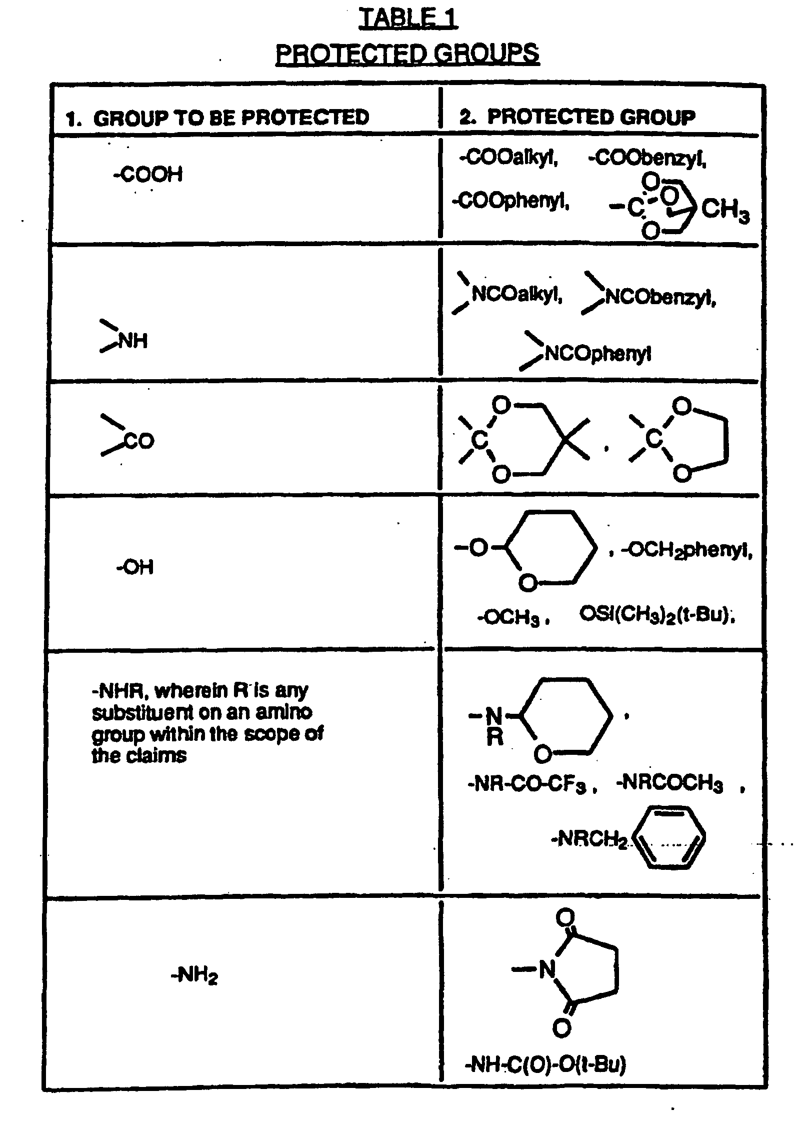

Other protecting groups well known in the art also may be used. After the reaction or reactions, the protecting groups may be removed by standard procedures.

Compounds useful in this invention are exemplified by the following preparative examples. The following preparative examples are provided to show how intermediates for preparing compounds of the present invention may be made.

### PREPARATIVE EXAMPLE 1

### A. N-(1,1-DIMETHYLETHYL)-3-METHYL-2-PYRIDINE CARBOXAMIDE

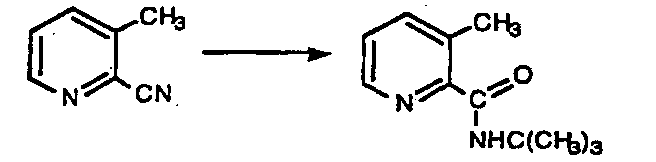

Suspend 2-cyano-3-methyl pyridine (400 g) in t-butanol (800 mL) and heat to 70°C. Add concentrated sulphuric acid (400 mL) dropwise over 45 minutes. Maintain the temperature at 75°C, until the reaction is complete, and for an additional 30 minutes. Dilute the mixture with water (400 mL), charge with toluene (600 mL) and bring to pH 10 with concentrated aqueous ammonia. Maintain the temperature at 50-55°C during the work up. Separate the toluene phase, and reextract the aqueous layer. Combine toluene phases and wash with water. Remove the toluene to yield the title compound N-(1,1-dimethylethyl)-3-methyl-2-pyridine carboxamide, as an oil, from which solid product is crystallized. (Yield 97%, as determined by an internal standard assay with gas chromatography).

### B. 3-[2-(3-CHLOROPHENYL)ETHYL]-N-(1,1-DIMETHYL-ETHYL)-2-PYRIDINE CARBOXAMIDE

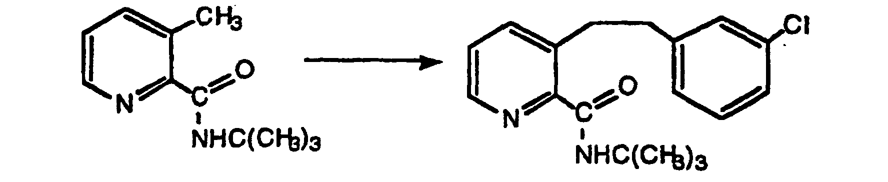

Dissolve the title compound of Preparative Example 1A, N-(1,1-dimethylethyl)-3-methyl-2-pyridine carboxamide (31.5 g.) in tetrahydrofuran (600 mL) and cool the resulting solution to -40°C. Add n-butyllithium (2 eq.) in hexane while maintaining the temperature at - 40°C. The solution turns deep purple-red. Add sodium bromide (1.6 g) and stir the mixture. Add solution of m-chlorobenzylchloride (26.5 g., 0.174 mole) in tetrahydrofuran (125 mL) while maintaining the temperature at -40°C. Stir the reaction mixture until the reaction is complete as determined by thin layer chromatography. Add water to the reaction until the color is dissipated. Extract the reaction mixture with ethyl acetate, wash with water, and concentrate to a residue which is the title compound. (Yield 92% as shown by chromatography).

### C. 3-[2-(3-CHLOROPHENYL)ETHYL]-2-PYRIDINE-CARBO-NITRILE

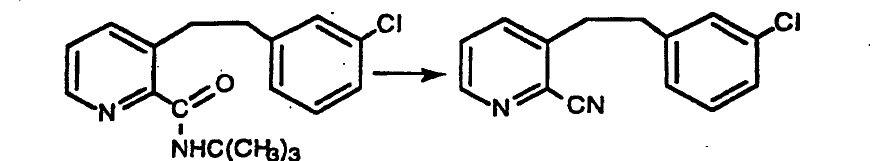

Heat a solution of the title compound of Preparative Example 1B, 3-[2-(3-chlorophenyl)ethyl]-N-(1,1-dimethylethyl)-2-pyridine carboxamide (175 g, 0.554 mole) in phosphorous oxychloride (525 mL, 863 g, 5.63 mole) and reflux for 3 hours. Determine completion of the reaction by thin layer chromatography. Remove any excess phosphorous oxychloride by distillation at reduced pressure and quench the reaction in a mixture of water and isopropanol. Bring to pH 5-7 by adding 50% aqueous sodium hydroxide solution while maintaining the temperature below 30°C. Filter the crystalline slurry of crude product and wash with water. Purify the crude product by slurrying the wet cake in hot isopropanol, and cool to 0-5°C. Filter the product, wash with hexane and dry at a temperature below 50°C to yield the title compound. (Yield: 118g (HPLC purity 95.7%), m.p. 72°C-73°C, 89.4% of theory).

### D. 1-(METHYL-4-PIPERIDINYL)[3-(2-(3-CHLOROPHENYL)ETHYL)-2-PYRIDINYL]METHANONE HYDROCHLORIDE

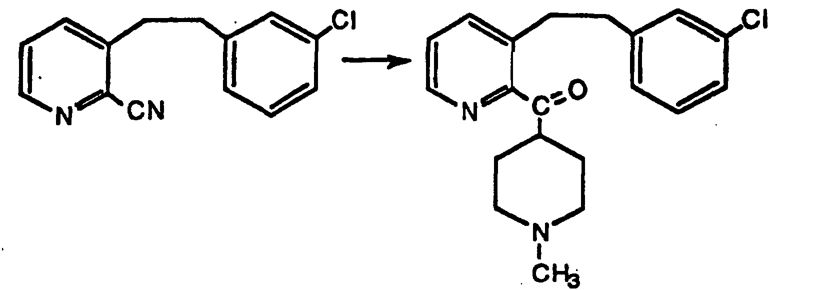

Dissolve the title compound of Preparative Example 1C, (118 g, 0.487 mole) in dry tetrahydrofuran (1.2L) and add N-methyl-piperidyl magnesium chloride (395 mL, 2.48 mole/liter, 0.585 mole, 1.2 eq.) over 15 minutes. Maintain the temperature at 40°C-50°C by cooling with water as necessary, for 30 minutes. Determine completion of the reaction by thin layer chromatography. Quench the reaction by reducing the pH to below 2 with 2N HCl and stir the resulting solution at 25°C for 1 hour. Remove the bulk of the tetrahydrofuran by distillation and adjust the resulting solution to pH 3.5 by addition of aqueous sodium hydroxide. Cool to 0 to 5°C and filter off the crystalline hydrochloride salt product. Wash with ice cold water and dry to constant weight at 60°C to yield the title compound. (Yield: 168.2 g (HPLC purity 94%), m.p. 183°-185°C, 89% of theory).

### E. 8-CHLORO-11-(1-METHYL-4-PIPERIDYLIDENE)-6,11-DIHYDRO-5H-BENZO[5,6]CYCLOHEPTA[1,2-b]PYRIDINE

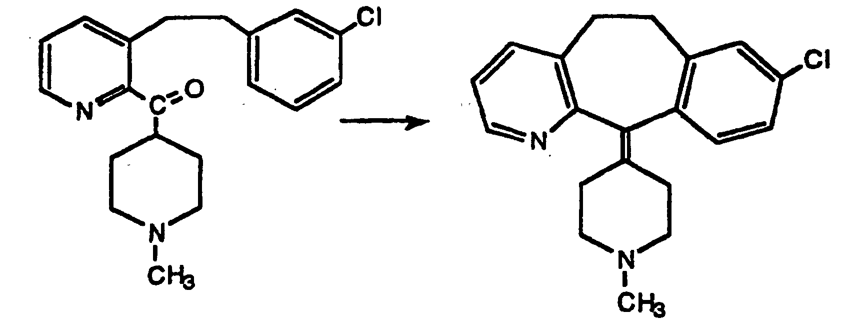

Dissolve the title compound of Preparative Example 1 D above (59 g, 0.15 mole) in hydrofluoric acid (120 mL, 120 g, 6.0 mole) at -35°C and add boron trifluoride (44.3 g, 0.66 mole) over 1 hour. Determine completeness of the reaction by thin layer chromatography. Quench the reaction using ice, water and potassium hydroxide bringing the solution to a final pH of 10. Extract the product with toluene and wash with water and brine. Concentrate the toluene solution to a residue, and dissolve in hot hexane. Remove the insolubles by filtration and concentrate the filtrate to yield the title compound as an off-white powder. (Yield: 45.7 g (HPLC purity: 95%), 92% of theory).

### Alternative Step E: 8-CHLORO-11-(1-METHYL-4-PIPERIDYLIDENE)-6,11-DIHYDRO-5H-BENZO[5,6]CYCLOHEPTA[1,2-b]PYRIDINE

React the title compound of Preparative Example 1D above (177 g, 0.49 mole) in trifluoromethanesulfonic acid (480 ml, 814.1 g, 5.31 mole) at 90-95°C for 18 hours under nitrogen. Determine the completeness of the reaction by thin layer chromatography. Cool the reaction and quench the reaction with ice-water and adjust the pH to 6 with barium carbonate. Extract the product with methylene chloride, and concentrate under reduced pressure to about 1 liter. Wash with water, and extract the product into 1 N HCI which is treated with 30 g of activated charcoal, and filter through celite. Adjust the pH of the filtrate to 10 with aqueous sodium hydroxide (50%), extract the product into methylene chloride, and remove under reduced pressure to form a residue. Dissolve the residue in hot hexane, and filter to remove insolubles. Concentrate the filtrate to yield the title compound as a beige powder. (Yield: 126 g (HPLC purity 80%), 65% of theory).

### F. 8-CHLORO-11-(1-ETHOXYCARBONYL-4-PIPERIDYLIDENE)-6,11-DIHYDRO-5H-BENZO[5,6]CYCLOHEPTA[1,2-b]PYRIDINE

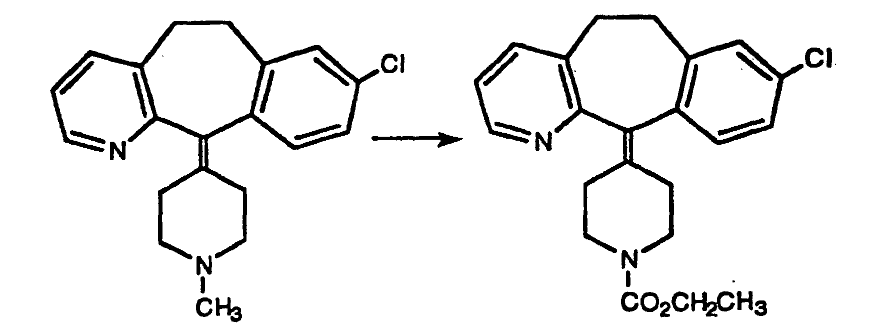

Dissolve the title compound of Preparative Example 1E above (45.6 g, 0.141 mole) in toluene (320 mL) at 80°C and to it gradually add ethyl chloroformate (40.4 mL, 45.9 g, 0.423 mole). Following complete addition, maintain the temperature at 80°C for 1 hour, then add diisopropylethylamine (2.7 mL, 2.00 g, 0.016 mole) and additional ethyl chloroformate (4.1 mL, 4.65 g, 0.0429 mole). Monitor completeness of the reaction by thin layer chromatography. Upon completion, cool the reaction mixture to ambient temperature, and wash the toluene solution with water. Concentrate the organic layer to a residue and dissolve in hot acetonitrile (320 mL). Decolorize the solution with 14 g of activated charcoal. Remove the activated charcoal by filtration and concentrate the filtrate to a crystalline slurry. Cool the mixture to 0-5°C, and isolate the product by filtration. Wash with cold acetonitrile and dry the product at below 70°C to yield the title compound. (Yield: 42.4 g (HPLC purity 97.4%), 80% of theory).

### G. 8-CHLORO-11-(4-PIPERIDYLIDENE)-6,11-DIHYDRO-5H-BENZO[5,6]CYCLOHEPTA[1,2-B]PYRIDINE

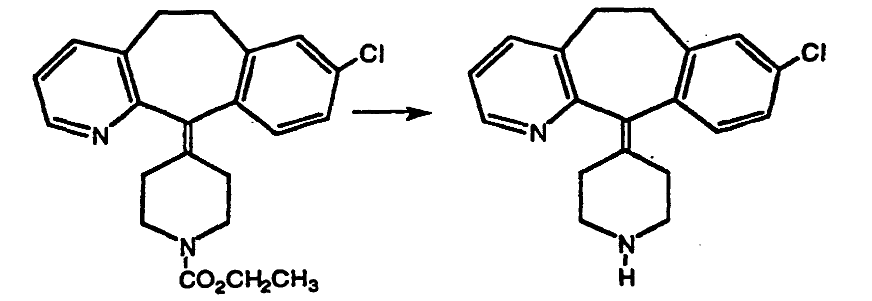

Hydrolize the title compound of Preparative Example 1F, 8-chloro-11-(1-ethoxycarbonyl-4-piperidylidene)-6,11-dihydro-5H-benzo[5,6]cyclohepta[1,2-b]pyridine (39 g, 0.101 mole) with KOH (50 g) in ethanol (305 mL) and water (270 mL) at reflux under an argon atmosphere for 64 hours. Partially distill off the ethanol and dilute the residue with brine, and extract with ethyl acetate (3x). Wash the combined organic phases with water and dry with Na₂SO₄. Remove the solvent to give a solid which can be recrystallized from toluene to give the title compound as a white solid. (Yield: 24.5 g, 77%, melting point 154-155°C).

### H. By substituting in step 1 B above, the benzylic halide:

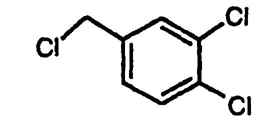

for meta-chlorobenzylchloride, and employing basically the same methods as steps C through G, the compound 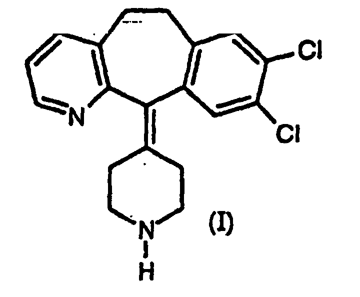 is prepared. Dichloro compound (I) is recrystallized from toluene and has a melting point of 150-152°C. Reaction times are determined by TLC or HPLC. In some instances purification of the product by chromatography is necessary.

### PREPARATIVE EXAMPLE 2

### A. N-(1,1-DIMETHYLETHYL)-3-[2-(4-FLUOROPHENYL)-ETHYL]-2-PYRIDINE CARBOXAMIDE

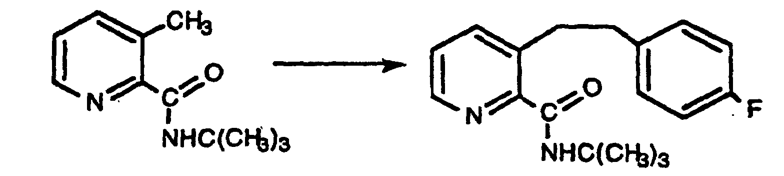

Cool a solution of N-(1,1-dimethylethyl)-3-methyl-2-pyridine-carboxamide (38.4 g, 0.2 mole) in dry THF (250 mL) to -40°C and add n-butyl lithium (185 mL, 0.44 mole). Add sodium bromide (1.9 g, 18 mmol.) and stir for 15 minutes. Add 4-fluorobenzylchloride (31.8 g, 0.22 mole) and stir for 2.5 hours while warming to -5°C. Quench the reaction with water and extract the product twice with ethyl acetate, then wash with brine (2X). Dry the organic phase over Na₂SO₄, filter and remove the solvent to give the title compound. (60.0 g, Yield 99%, m.p. 59-61°C.)

### B. 3-[2-(4-FLUOROPHENYL)ETHYL]-2-PYRIDINE CARBONITRILE

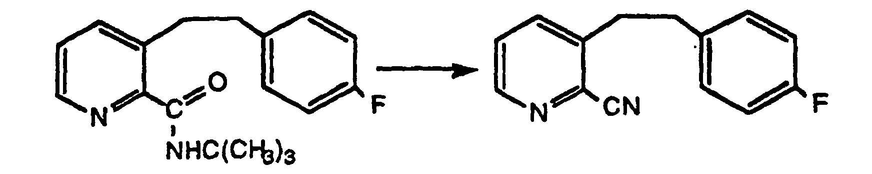

Heat the title compound of Preparative Example 2A above (60.0 g, 0.2 mole) in POCl₃ (200 mL) to 110°C under an argon atmosphere for 3.5 hours. Pour the reaction mixture onto ice-and basify with NaOH (50%) solution. Extract the mixture with ethyl acetate (3x) and wash with water. Wash with brine and dry over Na₂SO₄. Remove the solvent and pass the residue through a coarse SiO₂ (60-200 mesh) column to give the title compound as a white solid (40 g, Yield 88%, m.p. 48- 49°C.).

### C. 9-FLUORO-5,6-DIHYDRO-11H-BENZO[5,6]CYCLOHEPTA[1,2-b]PYRIDIN-11-ONE

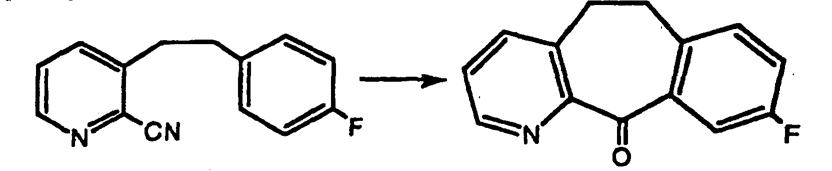

Cyclize the title compound of Preparative Example 2B above (31.5 g, 139 mmol) in polyphosphoric acid (1.24 kg) at 200°C for 5.5 hours. Pour onto ice and basify with NaOH solution (50%). Extract the product with chloroform (3x) and wash with brine. Dry the organic phase with Na₂SO₄, filter and remove the solvent to give the title compound (20.4 g, yield 64%, m.p. 78-81°C after recrystallization from diisopropyl ether).

### D. 9-FLUORO-11-(1-METHYL-4-PIPERIDINYL)-6,11-DIHYDRO-5H-BENZO[5,6]CYCLOPHEPTA[1,2-b]PYRIDIN-11-OL

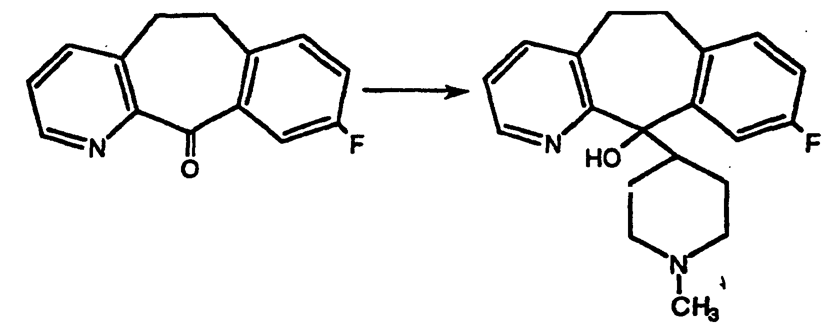

Dissolve the title compound of Preparative Example 2C above (10.0 g, 44 mmol) in THF (100 mL) and add slowly to a cooled (-40°C) solution of the Grignard reagent prepared from N-methyl-4-chloropiperidine (57.9 mL, 88 mmol) and magnesium in THF (70 mL). Stir the mixture for about 1 hour while warming up to 0°C. Quench the reaction with NH₄Cl solution and extract with ethyl acetate (2X). Wash the organic phase with brine and dry over Na₂SO₄, filter and remove the solvent. Purify the residue with flash chromatography and elute with methanol (5%) in CHCl₃ to give the title compound as white granular crystals. (10.1 g, Yield 70%, m.p. 126-127°C after recrystallization from diisopropyl ether.)

### E. 9-FLUORO-11-(1-METHYL-4-PIPERIDYLENE)-6,11-DIHYDRO-5H-BENZO[5,6]CYCLOHEPTA[1,2-b]PYRIDINE

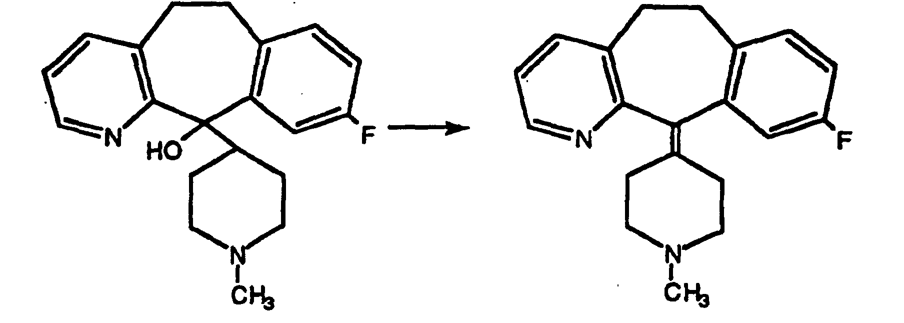

Add the title compound of Preparative Example 2D above (7.3 g, 22.3 mmol) to a mixture of cooled H₂SO₄ and CF₃SO₃H (1:1), (146 mL). Stir the reaction mixture for 0.5 hours at ice bath temperature and then at room temperature for 1.5 hours. Pour the reaction mixture onto ice and basify with NaOH (50%) solution. Extract the product with ethyl acetate (3X) and wash with brine. Dry the organic phase over Na₂SO₄, filter and remove the solvent to give a crude oil. Charcoal the oil and recrystallize from ethyl acetate and isopropyl ether to give the title compound. (5.6 g, Yield 82%, m.p. 134.5-135.5°C.).

### F. 9-FLUORO-11-(1-ETHOXYCARBONYL-4-PIPERIDYLIDENE)-6,11-DIHYDRO-5H-BENZO[5,6]CYCLOHEPTA[1,2-b]PYRIDINE

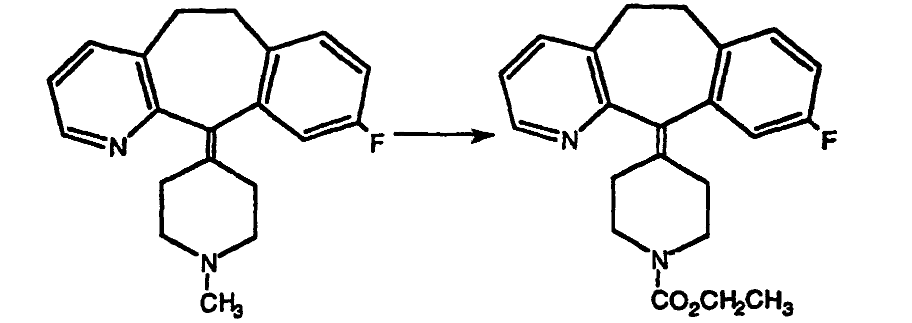

Stir a solution of the title compound of Preparative Example 2E above (5.0 g, 16.2 mmol) and triethylamine (2.6 g, 26 mmol) in dry toluene (60 mL) at 80°C under an argon atmosphere, and add ethyl chloroformate (9.8 g, 90 mmol) via a syringe. Stir the reaction at this temperature for 30 minutes and at room temperature for one hour. Filter the reaction and remove the solvent. Pass the residue through a coarse SiO₂ column (60-200 mesh), and elute with CHCl₃ to yield the title compound as a white solid. (4.5 g, Yield 76%, m.p. 112-114°C after trituration with pentane).

### G. 9-FLUORO-11-(4-PIPERIDYLIDENE)-6,11-DIHYDRO-5H-BENZO[5,6]CYCLOHEPTA[1,2-b]PYRIDINE

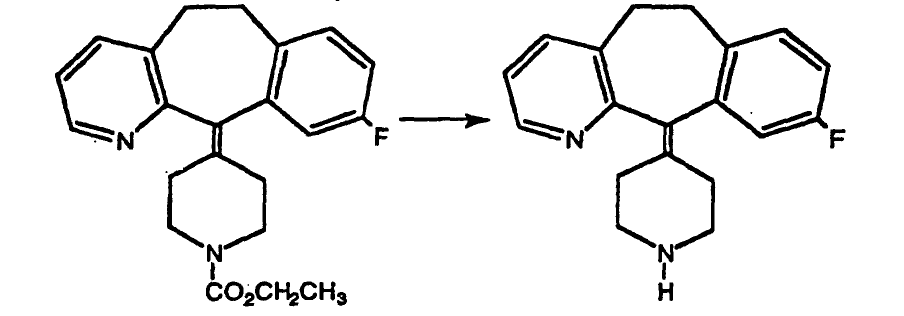

Reflux the title compound of Preparative Example 2F above (3.83 g, 10.4 mmol) with KOH (4.6 g) in 50 mL of ethanol/H₂O (1:1) for 4 hours under an argon atmosphere. Pour the reaction mixture into a brine solution and extract with ethyl acetate (2X), dry over Na₂SO₄ and filter. Remove the solvent to give the title compound (2.86 g, Yield 90%, m.p. 138-140°C.).

### . H. By employing the benzyl halide

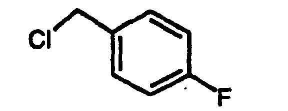

in place of 4-fluorobenzyl chloride in step 2A above, the product 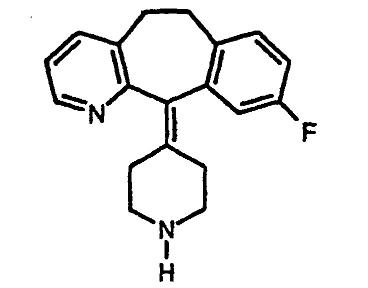 is prepared (m.p. 138-140°C, triturated with pentane) by employing basically the same process as described in steps 2A-2G. Workup time is determined by either TLC or HPLC. In some instances purification of the product by chromatography is necessary.

### PREPARATIVE EXAMPLE 3

### A. 3.5-DIMETHYLPYRIDINIUM N-OXIDE

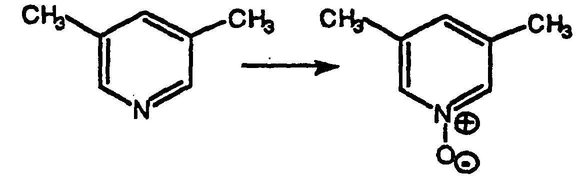

A solution of 285 mL (1.31 mol) of 35% peracetic acid was slowly added to a stirred solution of 149 g (1.39 mol) of 3,5-dimethylpyridine during which the temperature rose to 85°C and was maintained at this temperature during addition. After the temperature of the mixture dropped to about 35°C the reaction was stored at 5°C overnight.

After partial removal of 185 ml of acetic acid via distillation under vacuum, the reaction was washed with NaHSO₄ solution and then neutralized with 10% NaOH solution to pH of about 7. The product was extracted with CH₂Cl₂ to give the title compound as a white solid (yield 142 g, 83%).

### B. 1-METHOXY-3,5-DIMETHYLPYRIDINIUM METHYL SULFATE

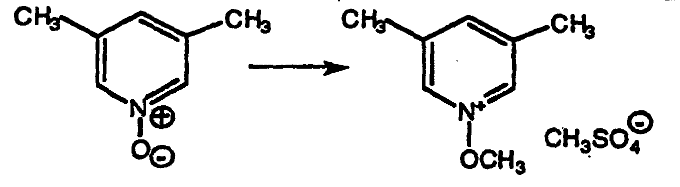

Dimethylsulfate (42.0 g, 0.33 mol) was slowly added to 41.0 g (0.33 mol) of 3,5-dimethylpyridinium N-oxide with mechanical stirring. The mixture was then heated on a steam bath for 1 hr. Then vacuum was applied while cooling to give a brownish solid of the title compound in quantitative yield.

### C. 2-CYANO-3,5-DIMETHYLPYRIDINE

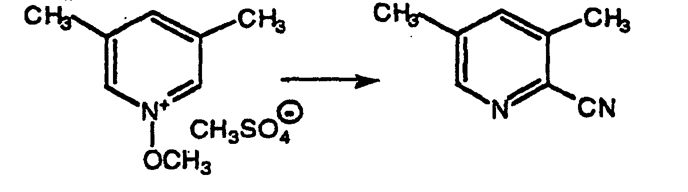

To a cooled (0°C) solution of sodium cyanide (49.0 g, 0.999 mol, 3.0 eq.) in 135 mL of water (air free) was dripped 1-methoxy-3,5-dimethyl pyridinium methyl sulfate (83.0g, 0.33 mol) in 100 mL water (air free) in 1.25 hr., keeping the temperature below 3°C. The reaction mixture was stored at about 3°C overnight. The mixture was filtered and washed with water to give 40g of the title compound. An analytical sample was recrystallized from isopropyl ether and pentane (4:1) (m.p.: 61-62°C).

### D. N-(1,1-DIMETHYLETHYL)-3,5-DIMETHYL-2-PYRIDINE CARBOXAMIDE

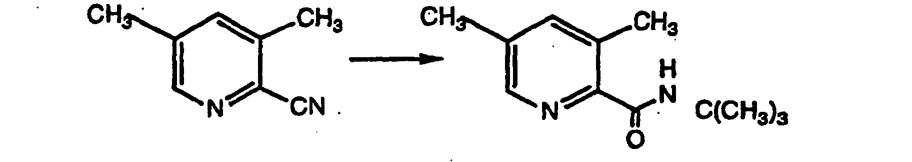

To a stirred solution of 20.3 g (0.153 mol) of 2-cyano-3,5-dimethylpyridine in 100 mL of 20 mL of conc. sulfuric acid within 10 minutes, followed by 20 mL of t-butanol over an additional 15 minutes. The solution was warmed at 75°C for 30 minutes after which it was cooled to room temperature and basified with 25% NaOH. The product was extracted 3X with EtOAc (600 mL), which was combined and washed 1X with brine, dried (Na₂SO₄), filtered and concentrated in vacuo to give the title compound (31.26 g) as a yellowish oil.

### E. 8-CHLORO-3-METHYL-11-(4-PIPERIDYLIDENE)-6,11-DIHYDRO-5H-BENZO[5,6]CYCLOHEPTA[1,2-b]PYRIDINE

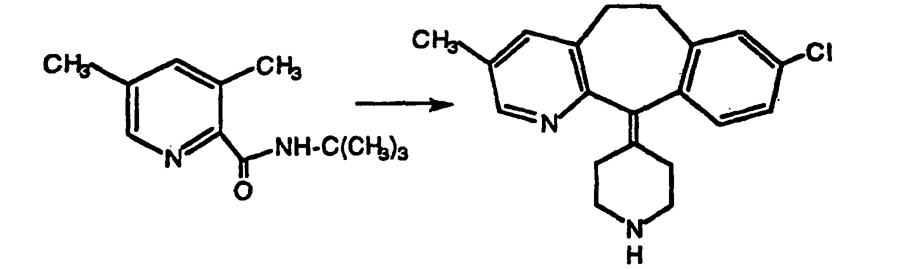

By substituting in step 1B above N-(1,1-dimethylethyl)-3,5-dimethyl-2-pyridine carboxamide for N-(1,1-dimethylethyl)-3-methyl-2-pyridine carboxamide and employing basically the same methods as steps B through G of Preparative Example 1, one obtains 8-chloro-3-methyl-11-(4-piperidylidene)-6,11-dihydro-5H-benzo[5,6]cyclohepta[1,2-b]pyridine. Reaction times are determined by TLC or HPLC.

### PREPARATIVE EXAMPLE 4

By substituting 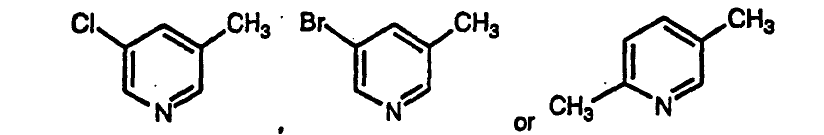 for 3,5-dimethylpyridine in Preparative Example 3 above and following basically the same procedure (steps A-E), the compounds 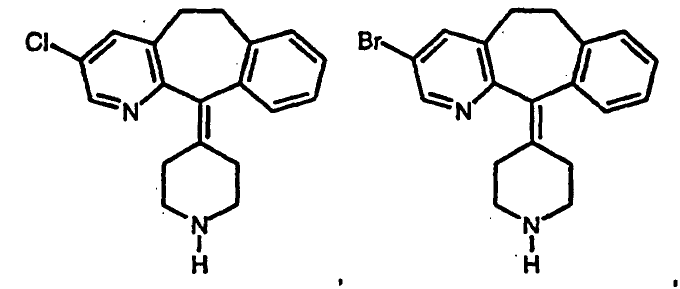 or 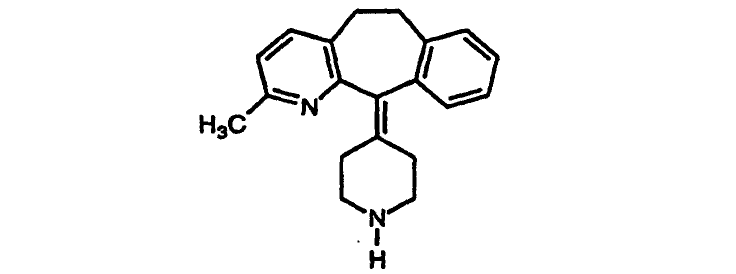 respectively, can be prepared. Note that the addition of the nitrile group to the pyridine in Step C of Preparative Example 3 can result in the formation of other undesirable isomers which can be removed via flash chromatography.

### PREPARATIVE EXAMPLE 5

### A. 8-CHLORO-5,6-DIHYDRO-11H-BENZO[5,6]CYCLOHEPTA-[1,2-b]PYRIDIN-11-ONE N-OXIDE

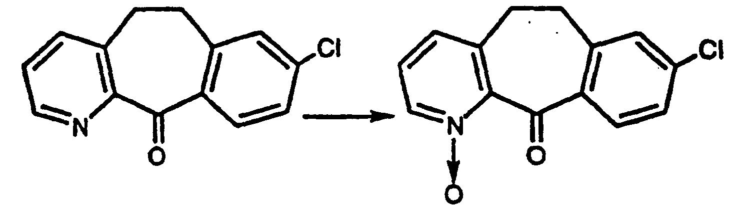

To a mixture of 25.1 grams (0.103 mole) of 8-chloro-5,6-dihydro-11H-benzo[5,6]cyclohepta[1,2-b]pyridin-11-one in 175 ml of dry methylene chloride at 0°C under an argon atmosphere was added dropwise over 70 minutes a solution of 24.12 grams of 3-chloroperoxybenzoic acid in 150 ml of methylene chloride. After the addition the solution was stirred for 1/2 hour after which the ice bath was removed. After two days the reaction was poured into 1.0 N aqueous sodium hydroxide and extracted with methylene chloride. The organic portions were combined, washed once with water, dried over magnesium sulfate, filtered and concentrated in vacuo. The resultant product was triturated with isopropyl ether and filtered to provide 25.8 grams (96%) yield of the title compound.

### B. 2,8-DICHLORO-5,6-DIHYDRO-11H-BENZO[5,6]-CYCLOHEPTA[1,2-b]PYRIDIN-11-ONE AND 4,8-DICHLORO-5,6-DIHYDRO-11H-BENZO[5,6]CYCLOHEPTA[1,2-b]PYRIDIN-11-ONE

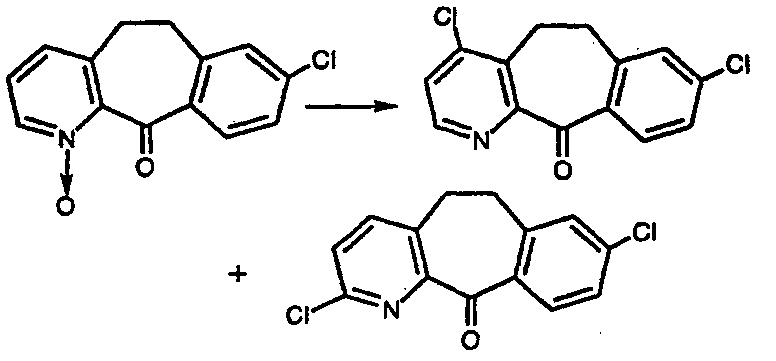

To a mixture of 29.13 grams (112.2 mmol) of the title compound from Preparative Example 5A above, in 40 ml of dry methylene chloride at 0°C and under argon atmosphere was added 500 ml of 1.0 M SO₂Cl₂ dropwise over 1 hour. The ice bath was then removed and the reaction stirred at room temperature for 1 hr and then refluxed for seven hours. The mixture was poured into 1.0 N aqueous NaOH and extracted three times with CH₂Cl₂. The organic portions were combined, dried over MgSO₄, filtered and concentrated in vacuo to yield a product which was purified and separated via flash chromatography to yield the two title compounds.

### C. 4-(2,8-DICHLORO-5,6-DIHYDRO-11H-BENZO[5,6]-CYCLOHEPTA[1,2-b]PYRIDIN-11-YLIDENE)PIPERIDINE AND 4-(4,8-DICHLORO-5,6-DIHYDRO-11H-BENZO[5,6]CYCLOHEPTA-[1,2-b]-PYRIDIN-11-YLIDENE)PIPERIDINE

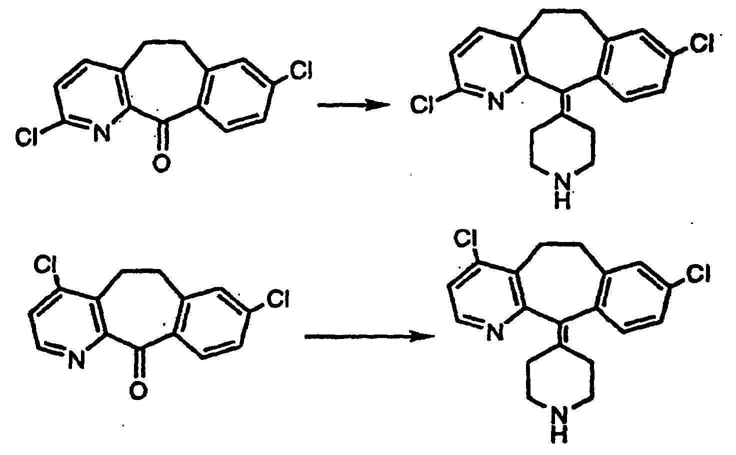

By following essentially the same procedure as that described in parts D-G of Preparative Example 2 above, the 2,8-dichloro and 4,8-dichloro products of Preparative Example 5B above were converted to the corresponding title compounds.

### PREPARATIVE EXAMPLE 6

### A. 3-(1,1-DIMETHYL-1-ETHYL)-8-CHLORO-5,6-DIHYDRO-11H-BENZO[5,6]CYCLOHEPTA[1,2-b]PYRIDIN-11-ONE

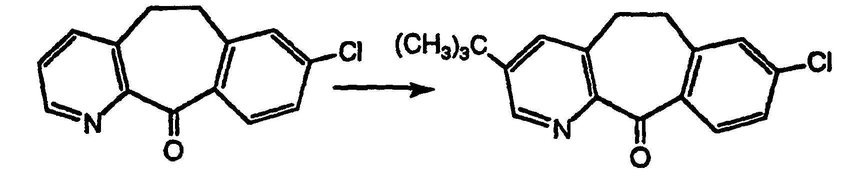

To a mixture of 20.05 grams (82.28 mmol) of 8-chloro-5,6-dihydro-11H-benzo[5,6]cyclohepta[1,2-b]pyridin-11-one in 400 ml of dry tetrahydrofuran at -72°C and under an atmosphere of nitrogen was added dropwise over 40 minutes 66.0 ml of 2.7 M t-butyl magnesium chloride in tetrahydrofuran. The reaction mixture was slowly warmed to room temperature and stirred overnight. The mixture was then poured into 10% aqueous ammonium chloride and extracted four times with methylene chloride. The combined organic portions were dried over magnesium sulfate, filtered, and concentrated in vacuo to give the title compound, along with 8-chloro-11-(1,1-dimethyl-1-ethyl)-6,11-dihydro-5H-benzo[5,6]cyclohepta[1,2-b]pyridin-11-ol. These compounds were separated via flash chromatography to give the title compound, which was recrystallized from isopropyl ether to give 4.37 grams (18%) of the title compound as a white solid.

### B. 4-[3-(1,1-DIMETHYL-1-ETHYL)-8-CHLORO-5,6-DIHYDRO-11H-BENZO[5,6]CYCLOHEPTA[1,2-b]PYRIDIN-11-YLIDENE]PIPERIDINE

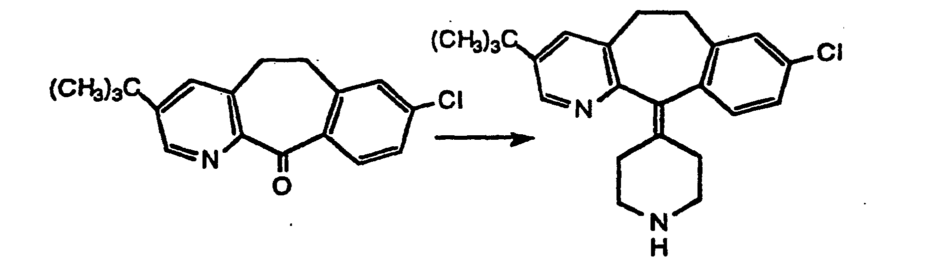

By using the title compound of Part A above and applying essentially the same procedure described in parts D-G of Preparative Example 2 above, one can obtain the title compound.

### PREPARATIVE EXAMPLE 7

### A. 8-CHLORO-6,11-DIHYDRO-11-HYDROXY-5H-BENZO[5,6]-CYCLOHEPTA[1,2-b]PYRIDINE

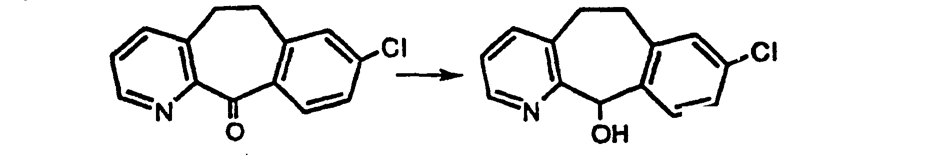

To a mixture of 25.03 g (103 mmol) of 8-chloro-5,6-dihydro-11H-benzo[5,6]cyclohepta[1,2-b]pyridin-11-one in 200 mL of methanol at room temperature and under a nitrogen atmosphere was added portionwise over a period of about 1 hour 4.82 g (124 mmol) of sodium borohydride. Occasional cooling with an ice bath was necessary at times during the addition in order to avoid excessive reflux. After 1.6 hours the mixture was poured into ice cold water and then extracted with ethyl acetate (3X). The combined organic portions were washed with brine, dried over magnesium sulfate, filtered, and concentrated in vacuo. The residue was recrystallized from hot isopropyl ether. The remaining filtrate was purified via flash chromatography (20% ethyl acetate in hexanes) to yield more product which solidified on standing. Both batches were combined to yield 20.41 g of the title compound as a white solid.

### B. 8,11-DICHLORO-6,11-DIHYDRO-5H-BENZO[5,6]CYCLO-HEPTA[1,2-b]PYRIDINE

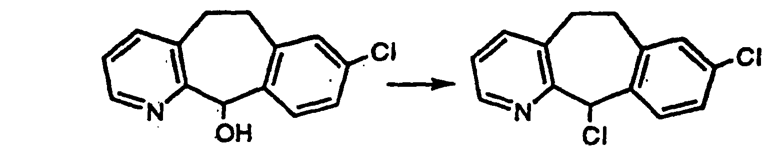

To a mixture of 13.3 g (54 mmol) of 8-chloro-6,11-dihydro-11-hydroxy-5H-benzo[5,6]cyclohepta[1,2-b]pyridine in 290 mL of toluene at -15°C and under an atmosphere of nitrogen was added via syringe pump over a period of 1 hour 6.20 mL (85.7 mmol) of thionyl chloride.The extent of reaction was monitored by TLC (50% ethyl acetate in hexanes). When completed the mixture was poured into 300 mL of 1.0 N aqueous sodium hydroxide and extracted with ethyl acetate (5X). The combined organic portions were washed with brine, dried over sodium sulfate, filtered, and concentrated in vacuo. The residue was taken up in ethyl acetate, quickly filtered through basic alumina, and concentrated again to yield a product which was triturated with pentane to yield 10.22 g of the title compound as a tan solid.

### C. 8-CHLORO-11-(1-PIPERAZINYL)-6,11-DIHYDRO-5H-BENZO[5,6]CYCLOHEPTA[1,2-b]PYRIDINE

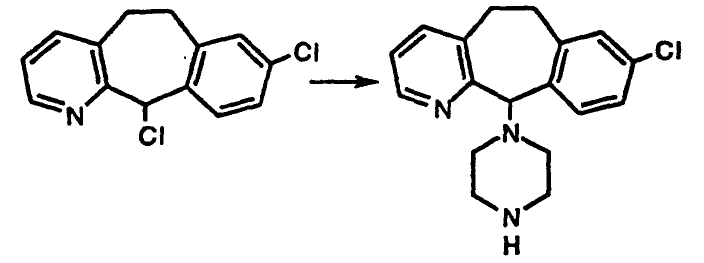

To a mixture of 10.0 g (37.9 mmol) of 8,11-dichloro-6,11-dihydro-5H-benzo[5,6]cyclohepta[1,2-b]pyridine and 1.0 mL of triethylamine in 200 mL of dry tetrahydrofuran at room temperature and under a nitrogen atmosphere was added 33.0 g of piperazine. The mixture was stirred at room temperature for 22.5 hours and then refluxed for 5.5 hours. It was then cooled to room temperature, poured into 250 mL of 5% aqueous sodium hydroxide, and extracted with methylene chloride (3X). The combined organic portions were washed with brine, dried over magnesium sulfate, filtered, and concentrated in vacuo. The residue was purified via flash chromatography (2→5% methanol saturated with ammonia in methylene chloride) to yield the title compound as a glass.

### PREPARATIVE EXAMPLE 8

### A. ETHYL 3-PYRIDYLACETIC ACID 1-N-OXIDE

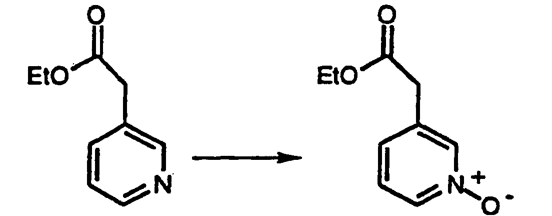

Ethyl 3-pyridylacetic acid (10grams) (60.6 mmoles) was dissolved in dry dichloromethane (120ml) and the solution was stirred at -18°C for 30 minutes. 3-Chloroperbenzoic acid (31.34 grams) (181.6 mmoles) was added and the mixture was stirred at -18°C for 1 hour and then at 25°C for 87 hours. The reaction mixture was diluted with dichloromethane and washed with saturated aqueous sodium bicarbonate and then water. The dichloromethane was then dried (magnesium sulphate), filtered and evaporated to dryness. The residue was chromatographed on silica gel using 3% (10% concentrated ammonium hydroxide in methanol)-dichloromethane as the eluant to give the title compound (Yield: 8.45 grams, 77%, MH⁺ 182).

### B. 3-PYRIDYLACETIC ACID 1-N-OXIDE

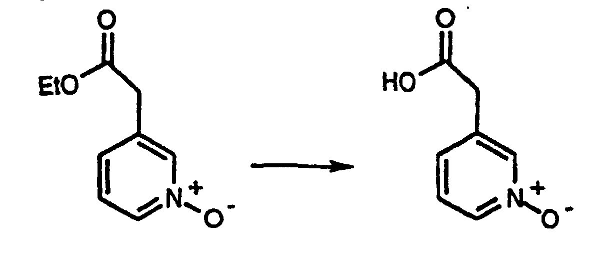

3-Pyridylacetic acid (0.2747 grams) (1.5 mmoles) was dissolved in ethanol (200 proof) (1.22 ml.) and a 1M solution of lithium hydroxide in water (3.64 ml.) (3.0 mmoles) was added and the mixture was stirred at 25°C for 4 hours. 1N Hydrochloric acid (4.28 ml.) was added and the mixture was pumped down to dryness on a rotary evaporator to give the title compound (Yleld: 0.2931 grams, 100%).

### PREPARATIVE EXAMPLE 9

### A. ETHYL α-METHYL-3-PYRIDYLACETIC ACID.

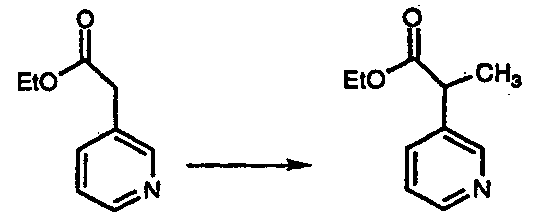

To ethyl 3-pyridylacetic acid (10.86 grams) ( 65.7 mmoles) was added a 2.0M solution of lithium diisopropylamide in THF / heptane / ethyl benzene (32.87 ml.) (65.8 mmoles) at -30°C. The semi-solid mixture was agitated and sonicated for 1 hour. The mixture was allowed to remain at 25°C for 1 hour, whereupon methyl iodide (4.09 ml.) (65.7 mmoles) was added. After 1 hour at 25°C the mixture was taken up in dichloromethane and washed with saturated aqueous sodium bicarbonate and water. The dichloromethane was dried (magnesium sulphate), filtered and evaporated to dryness. The residue was chromatographed on silica gel using 10% ethyl acetate in hexane as the eluant to give the title compound (Yield: 3.48 grams, 30%, MH⁺ 180).

### B. α-METHYL-3-PYRIDYLACETIC ACID.

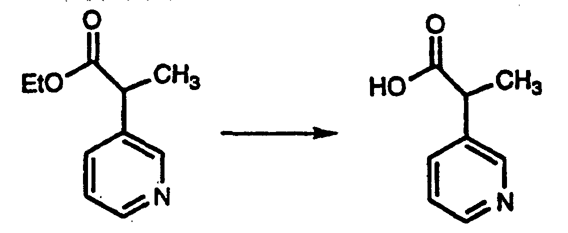

The title compound from Preparative Example 9A above (2.16 grams) (12.05 mmoles) was dissolved in ethanol (10 ml.) and 1.0M lithium hydroxide in water (29.15 ml.) (29.2 mmoles) was added. The mixture was stirred at 25°C for 4 hours, whereupon 1N hydrochloric acid (34.27 ml.) (34.2 mmoles) was added and the solution was evaporated to dryness to give the title compound (Yield 2.33 grams, 100%).

### PREPARATIVE EXAMPLE 10

### α,α-DIMETHYL-3-PYRIDYLACETIC ACID.

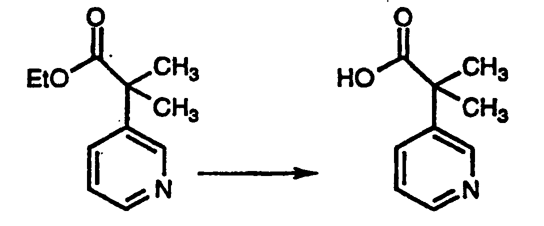

Ethyl α,α-dimethyl-3-pyridylacetate (disclosed in EP Application 0 288 279, published October 26, 1988) (2.67 grams, 13.8 mmoles) was dissolved in ethanol (11.1 ml.) and a 1.0M lithium hydroxide in water (33.3 ml.) (33.4 mmoles) was added. The mixture was stirred at 25°C for 4 hours. 1N Hydrochloric acid (38.73 ml.) was added and after 5 minutes the mixture was evaporated to dryness to give the titlle compound (Yield: 100%).

### PREPARATIVE EXAMPLE 11

### A. 8-CHLORO-6,11-DIHYDRO-11-(1-PIPERAZINYL)-5H-BENZO[5,6]CYCLOHEPTA[1,2-b]PYRIDINE 1-N-OXIDE

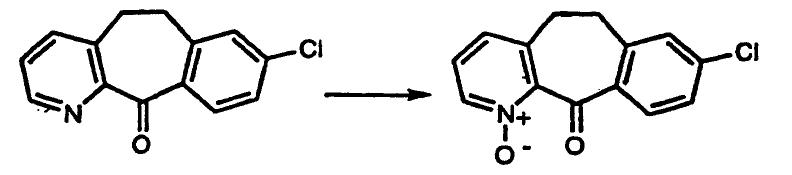

To a mixture of 8-chloro-5,6-dihydro-11H-benzo[5,6]cyclohepta[1,2-b]pyridin-11-one (5 grams) (20.6 mmoles) in dry dichloromethane (35 ml) was added dropwise 3-chloroperbenzoic acid (4.7 grams) (27.3 mmoles) in dry dichloromethane (75 ml) at 0-25°C over 1 hour. The mixture was diluted with dichloromethane and washed with saturated aqueous sodium bicarbonate and water. The dichloromethane was dried (magnesium sulphate), filtered and evaporated to dryness. The residue was chromatographed on silica gel using 1% (10% saturated ammonium hydroxide in methanol)-dichloromethane as the eluant to give the title compound (Yield: 2.81 grams, 53%, MH⁺ 260).

### B. 8-CHLORO-6,11-DIHYDRO-5H-BENZO[5,6]CYCLOHEPTA[1,2-b]PYRIDIN-11-OL 1-N-OXIDE

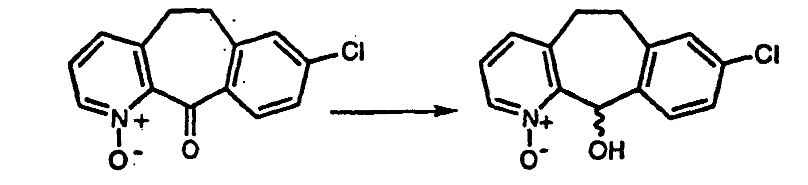

By using the title compound (8.6 grams) from Preparative Example 11A and reducing it by the procedure described in Preparative Example 7A above the title alcohol was obtained (Yield: 7.03 grams, 81%, MH⁺ 262).

### C. 8,11-DICHLORO-6,11-DIHYDRO-5H-BENZO[5,6]CYCLOHEPTA[1,2-b]PYRIDINE 1-N-OXIDE

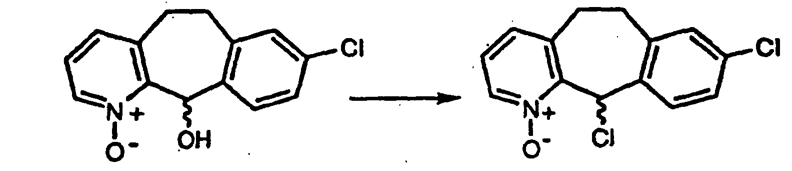

The title compound from Preparative Example 11 B (6.2 grams) (23.7 mmoles) was reacted with thlonyl chloride as described in Preparative Example 7B to give the title compound.

### D. 8-CHLORO-6,11-DIHYDRO-11-(1-PIPERAZINYL)-5H-BENZO[5,6]CYCLOHEPTA[1,2-b]PYRIDINE 1-N-OXIDE

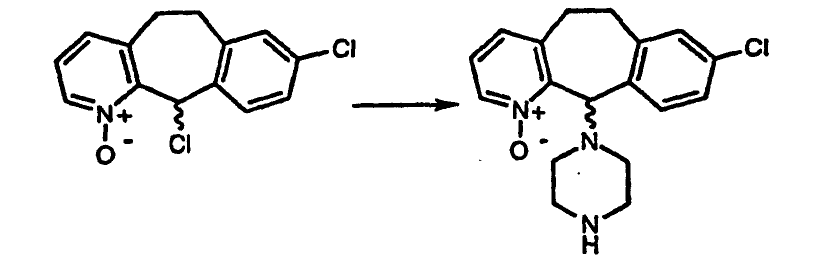

The title compound from Preparativ eExample11C above was reacted with piperazine (9.9 grams) (115.0 mmoles) as described in Preparative Example 7C to give the title compound (Yield: 6.78 grams, 87%, MH⁺ 330).

### PREPARATIVE EXAMPLE 12

### 4-ETHOXYCARBONYLAMINOPYRIDINE

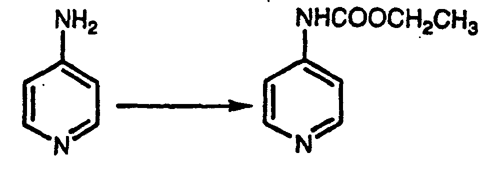

4-Aminopyridine (17.34 grams) (184.3) was dissolved in dry pyridine (217 ml.) and cooled to 0°C over 30 minutes. Ethyl chloroformate (17.2 ml.) (180.7 mmoles) was addedand the solution was stirred at 0°C for 1 hour and then at 25°C for 40 hours. The mixture was diluted with dichloromethane and washed with saturated aqueous sodium bicarbonate and water. The dichloromethane was dried (magnesium sulphate), filtered and evaporated to dryness. The residue was chromatographed on silica gel using 2%(10% saturated ammonium hydroxide in methanol)-dichloromethane to give the title compound (Yield: 10 grams, 33%, M⁺ 166).

By using essentially the same procedure, with the exception that 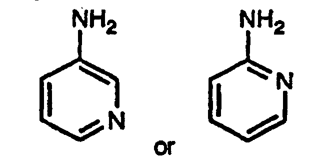 was used instead of 4-aminopyridine, the compound 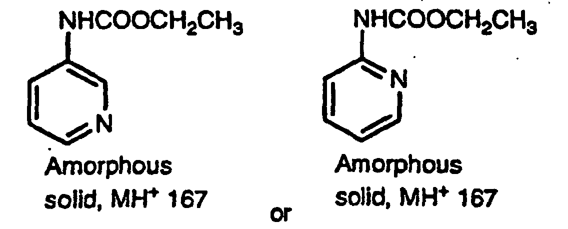 was obtained, respectively.

### PREPARATIVE EXAMPLE 13

### A. N-ACETYLISONIPECOTIC ACID

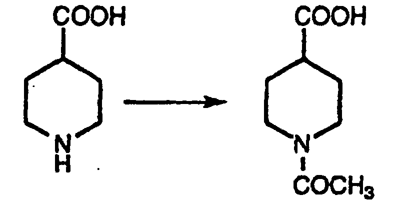

Isonipecotic acid (10 grams) (77.5 mmoles) and acetic anhydride (23.7 grams) (232.5 mmoles) were dissolved in methanol (100 ml.) and the mixture was stirred at 25°C for 24 hours. The mixture was evaporated to dryness and the residue was azeotroped with toluene to give the title compound (Yield: 12.8 grams, 97%, MH⁺ 172).

### B. 1-N-tert-BUTOXYCARBONYLISONIPECOTIC ACID

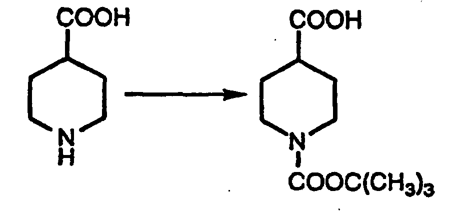

Isonipecotic acid (20 grams) (155.0 mmoles) was dissolved in THF-water (1:1) (400 ml) and sodium hydroxide (6.2 grams) (155.0 mmoles) and di-tert-butyldicarbonate (37.2 grams) (170.5 mmoles) were added. The mixture was stirred at 25°C for 72 hours. The solution was then eluted through a bed of washed BioRad 50WX4 (RSO3H resin) (150 ml bed) and the resin was eluted with a 1:1 mixture of THF and water. The eluate was evaporated to dryness to give the title compound (Yield: 33.78 grams, 90%).

### PREPARATIVE EXAMPLE 14

### 1-N-ACETYLNIPECOTIC ACID

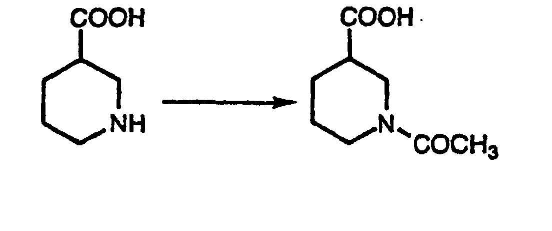

Nipecotic acid (3.87 grams) (30.0 mmoles) was reacted with acetic anhydride (9.17 grams) (90 mmoles) as described in Preparative Example 13A to give the title compound (Yield: 5.0 grams, 97%, MH⁺ 172).

### PREPARATIVE EXAMPLE 15

### 1-N-METHYLNIPECOTIC ACID

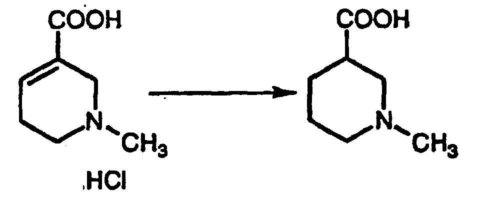

Arecaidine hydrochloride (4 grams) (22.6 mmoles) was hydrogenated in water (100 ml) using 10% Pd-C at 40 psi at 25°C for 24 hours. The catalyst was filtered off and washed with water. The aqueous solution was shaken with BioRad AG1X8 resin (OH⁻ form) (23 ml bed) and after 5 minutes the resin was filtered off and washed with water. The aqueous solution was evaporated to give the title compound (Yield: 2.95 grams, 92%).

### PREPARATIVE EXAMPLE 16

### 1-N-ACETYL D,L-PIPECOLINIC ACID

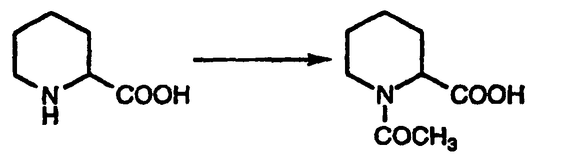

D,L-Pipecolinic acid (10 grams) (77.5 mmoles) and acetic anhydride (23.7 grams) (232.5 mmoles) were reacted as described in Preparative Example 13A above to give the title compound (Yield: 12.94 grams, 98%, MH⁺ 172).

### PREPARATIVE EXAMPLE 17

### A. PIPERIDINE-4-ACETIC ACID

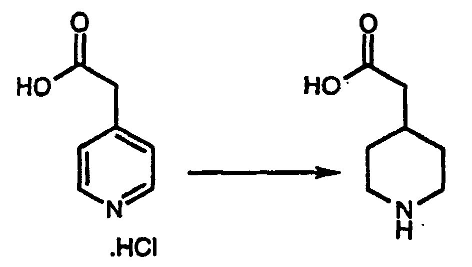

4-Pyridylacetic acid (7 grams) (40.4 mmoles) was hydrogenated as described in Preparative Example 15 to give the title compound (Yield: 5.2 grams, 90%, MH⁺ 144).

### B. 1-N-ACETYL-4-PIPERIDINYLACETlC ACID

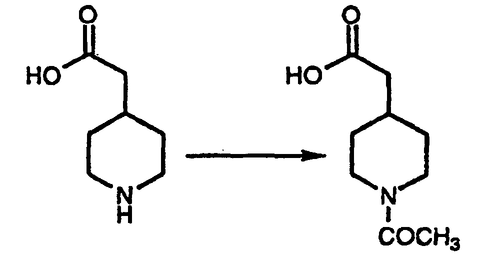

4-Piperidinylacetic acid (5 grams) (35.0 mmoles) was reacted with acetic anhydride (10.7 grams) (105.0 mmoles) as described in Preparative Example 13A to give the title compound (Yield: 6.4 grams, 99%, MH⁺ 185).

### C. 1-N-METHYL-4-PIPERIDINYLACETIC ACID

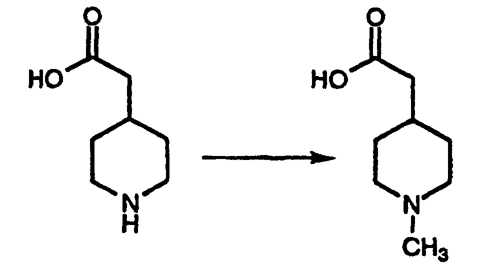

4-Piperidinylacetic acid (4 grams) (28.0 mmoles) from Preparative Example 17A was dissolved in water (50 ml) and 37% formalin (2.72 ml) (33.6 mmoles) was added. The mixture was hydrogenated over 10% Pd-C at 55psi at 25°C for 68 hours. The catalyst was filtered off and washed with water. The combined filtrates were evaporated to dryness to give the title compound (MH⁺158).

### D. 1-N-tert-BUTOXYCARBONYLPIPERIDINYL-4-ACETIC ACID

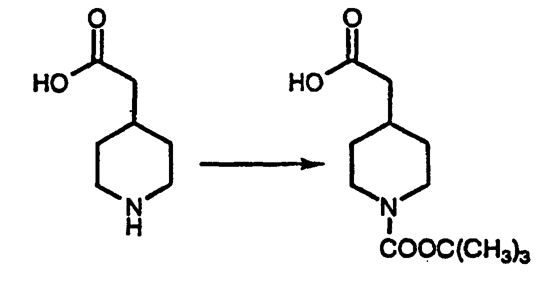

4-Piperidinylacetic acid (41.24 grams) (288.4 mmoles) from Preparative Example 17A was reacted with di-tert-butyldicarbonate (69.14 grams) (317.3 mmoles) and sodium hydroxide (11.52 grams) (288.4 mmoles) as described in Preparative Example 13B above to give the title compound (Yield: 53.0 grams, 76%).

### PREPARATIVE EXAMPLE 18

### A 3-PIPERIDINYLACETIC ACID

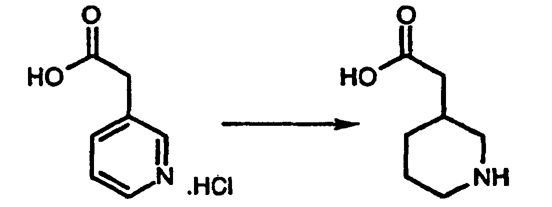

3-Pyridylacetic acid hydrochloride (13 grams) (74.9 mmoles) was hydrogenated as described in Preparative Example 15 to give a mixture of unreacted 3-pyridylacetic acid and the title compound (76:24) (8.63 grams, MH⁺ 144).

### B. 1-N-ACETYL-3-PIPERIDINYLACETIC ACID

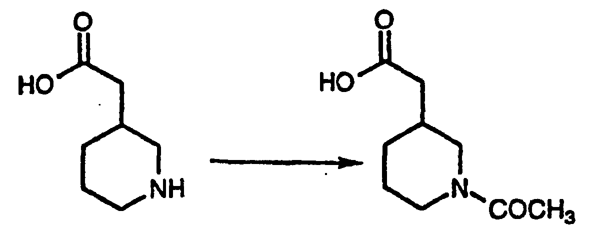

The mixture of compounds from Preparative Example 18A (8.56 grams) were reacted with acetic anhydride (8.56 grams) as described in Preparative Example 13A and the crude mixture of products was taken up in methanol (60 ml) and passed over a bed of BioRad AG50WX4 resin (RSO₃H) and the latter was eluted with methanol. The eluates were evaporated to dryness to give the title compound (Yield: 1.23 grams, MH⁺ 186).

### C. 1-N-METHYL-3-PIPERIDINYLACETIC ACID

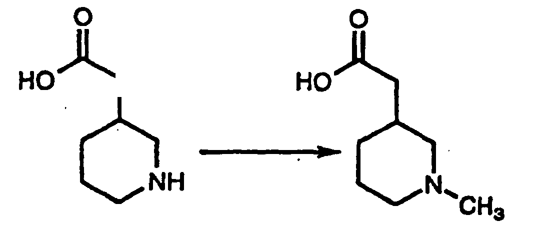

The mixture of compounds from Preparative Example 18A (4 grams) and 37% formalin (2.72 ml.) were hydrogenated as described in Preparative Example 17C to give the title compound (MH⁺ 158).

### PREPARATIVE EXAMPLE 19

### PREPARATION OF THE R(+) AND S(-) DIASTEREOISOMERS

The racemic 8-chloro-11-(1-piperazinyl)-6,11-dihydro-5H-benzo[5,6]cyclohepta[1,2-b]pyridine prepared in Preparative Example 7C above. was resolved by the method described in Preparative Example 15 A-C, pages 116-118, of WO 92/00293, published January 9, 1992, to give the R(+) and S(-) diastereoisomers:

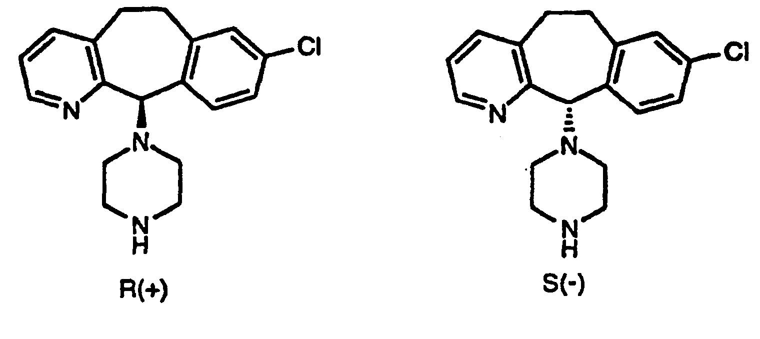

### PREPARATIVE EXAMPLE 20

### A. 3-BROMO-8-CHLORO-5,6-DIHYDRO-11H-BENZO[5,6]-CYCLOHEPTA[1,2-b]PYRIDIN-11-ONE

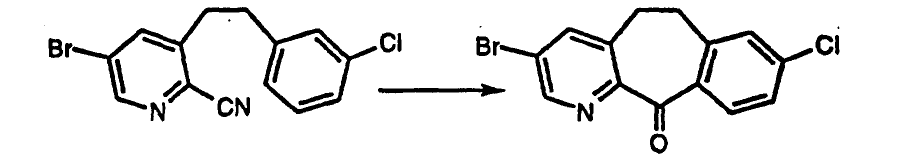

Cyclize 3-[2-(3-chlorophenyl)ethyl]-4-bromo-2-pyridine carbonitrile (10.7g, 32.8 mmol) in triflic acid (82 mL) at 60°C for 2 hours and then at room temperature for 2 hours. Add 80 mL of 5N HCI carefully, then reflux in an oil bath (120°C) for 30 minutes. Cool the solution and pour into ice and basify with 25% NaOH solution. Extract the product with CH₂Cl₂ and wash with brine. Dry the organic layer with Na₂SO₄,filter and remove the solvent to give crude product (10.4g). Purify the crude product with flash chromatography on silica gel and elute with 15% ethyl acetate-hexane to give the title compound as a white solid (9g ,27.95 mmol, Yield 85.2% MH⁺ 322).

### B. 8-CHLORO-3-METHOXY-5,6-DIHYDRO-11H-BENZO[5,6]-CYCLOHEPTA[1,2-b]PYRIDIN-11-ONE

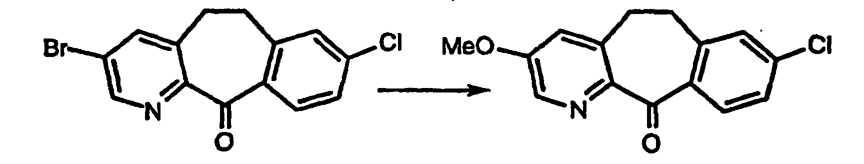

Dissolve the title compound of Preparative Example 20A (2.37g, 7.4 mmol) in dry methanol and add sodium metal (3.37g, 180 mmol). the reaction is stirred overnight at room temperature. Reflux the reaction for 3 hours, cool to room temperature and extract with dichloromethane-water. Dry the CH₂Cl₂ fraction and chromatograph on silica gel eluting with 50% EtOAc-hexanes to give the title compound as a light yellow solid(1.5g, Yield 72% MH⁺ 274).

### C. 8-CHLORO-3-METHOXY-11-(4-PIPERIDYLIDENE)-6,11-DIHYDRO-5H-BENZO[5,6]-CYCLOHEPTA[1,2-b]PYRIDINE

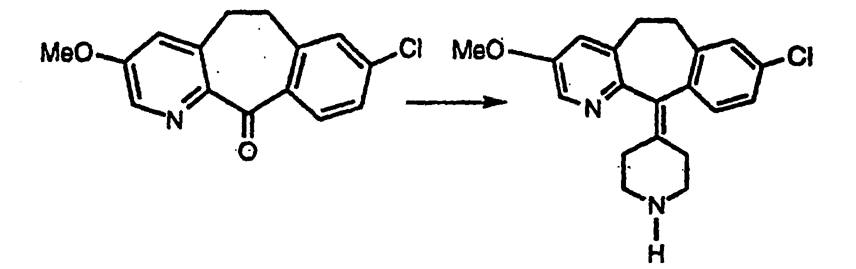

By substituting in Preparative Example 2 step D, 8-chloro-3-methoxy-5,6-dihydro-11H-benzo[5,6]-cyclohepta[1,2-b]pyrldin-11-one for 9-fluoro-5,6-dihydro-11H-benzo[5,6]-cyclohepta[1,2-b]pyridin-11-one and employing basically the same methods as steps D through H of Preparative Example 2, one obtains 8-chloro-3-methoxy- 11-(4-piperidylidene)-6,11-dihydro-5H-benzo[5,6]-cyclohepta[1,2-b]pyridine as a white solid (MH⁺ 340).

### PREPARATIVE EXAMPLE 25

### A. ETHYL α-METHYL-4-PYRIDYL ACETIC ACID

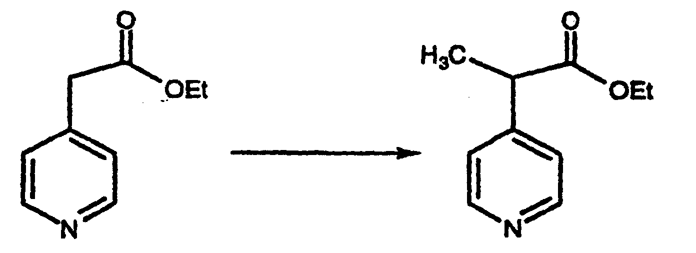

To dry THF at -78°C was added diisopropylamine(5.05g 48 mmol, 7mL) and then n-butyl lithium. The reaction mixture was stirred for 0.5 h and then ethyl 4-pyridyl acetic acid (7.85g, 46 mmol) was added, and after sirring for 0.5 h at that -78°C the reaction temperature was raised to room temperature. DMF (20 mL was added and the reaction mixture cooled to -78°C again. Methyl iodide(7.07g, 50.2 mmol, 3.15 mL) was added and the reaction mixture stirred at that temperature for 1h and then at room temperature overnight. All the volatiles were then stripped off and the reaction mixture was partitioned between water-CH₂Cl₂. The aqueous phase was washed twice with CH₂Cl₂. The combined CH₂Cl₂ phases were dried and evaporated. The crude product was chromatographed on silica gel eluting with 80% ethyl acetate hexane to give the title compound (7.88g, MH⁺ 179).

### B. α-METHYL-4-PYRIDYL ACETIC ACID

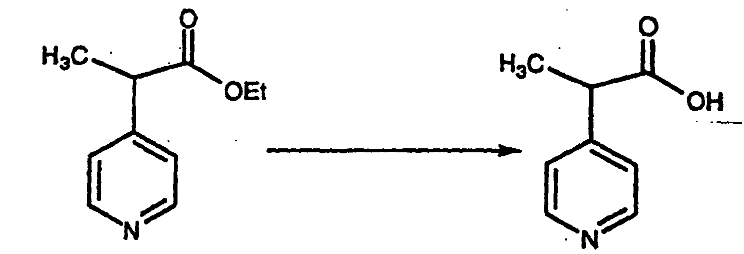

The compound from Preparative Example 25A was hydrolysed in a similar manner to Preparative Example 9B to give the title compound (MH⁺ 152).

### PREPARATIVE EXAMPLE 26

### A. α,α-DIMETHYL-4-PYRIDYL ACETIC ACID

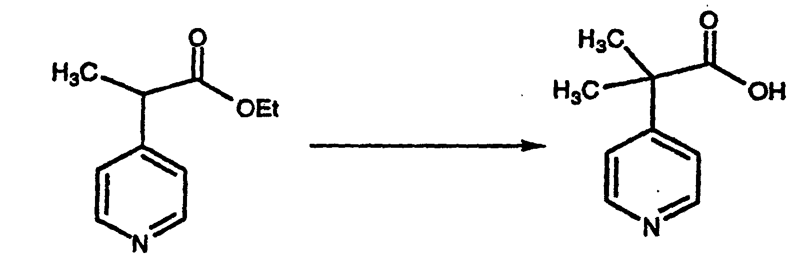

By essentialy the same procedure as set forth in Preparative Example 10A-B, but using ethyl α-methyl-4-pyridylacetic acid (from Preparative Example 25) instead of ethyl pyridyl acetic acid the title compound was obtained as an oil (MH⁺ 166).

### PREPARATIVE EXAMPLE 27

### ETHYL4-[4,8-DICHLORO-5,6-DIHYDRO-11H-BENZO[5,6]CYCLO-HEPTA[1,2-b]PYRIDIN-11-YLIDENE]-1-PIPERIDINECARBOXYLATE and ETHYL 4-[2,8-D]CHLORO-5,6-DIHYDRO-11H-BENZO[5,6]CYCLOHEPTA[1,2-b]PYRIDIN-11-YLIDENE]-1-PIPERIDINE-CARBOXYLATE

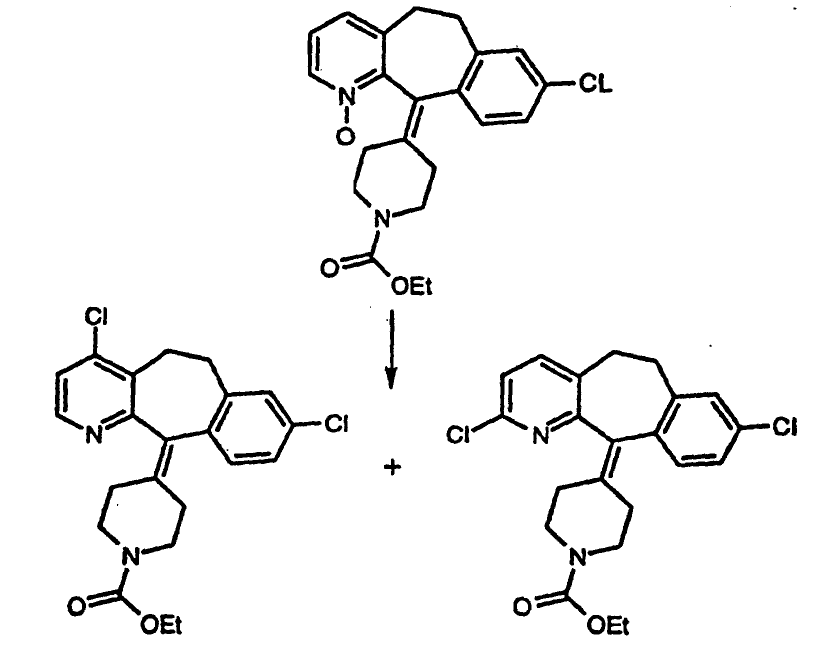

To phosphorous oxychloride (256 mL) stirring at reflux was added dropwise a solution of the title compound (109 grams) from Example 231A dissolved in chloroform (850 mL). After stirring the resulting solution for an additional 20 minutes at reflux, the reaction mixture was cooled to room temperature and the chloroform removed *in vacuo*. The resulting solution was cooled in an ice-water bath and to it was slowly added 1*N* aqueous sodium hydroxide (850 mL) followed by 50% aqueous sodium hydroxide until the resulting mixture was slightly basic. Extraction with ethyl acetate, drying of the organic phase over anhydrous magnesium sulfate, concentration *in vacuo*, and purification by flash column chromatography provided the 4,8-dichloro product (27 grams, 23% yield, mp 141.6-145.6 °C) and the 2,8-dichloro product (9 grams, 8% yield, 176.5-177.9 °C).

### PREPARATIVE EXAMPLE 28

### 4,8-DICHLORO-11-(4-PIPERIDYLIDENE)-6,11-5H-BENZO[5,6]-CYCLOHEPTA[1,2-b]PYRIDINE

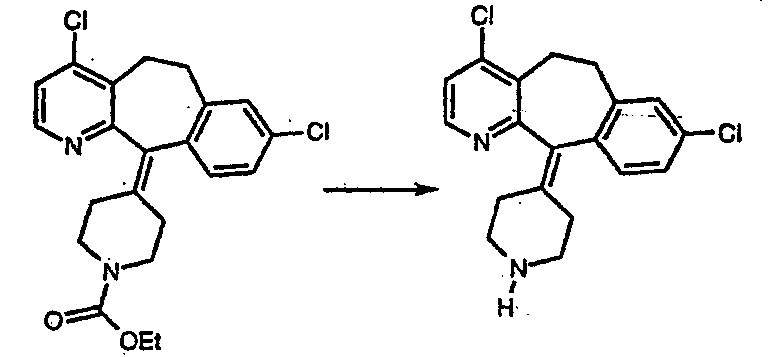

A solution of the 4,8-dichloro compound from Preparative Example 27 (2.6 grams) dissolved in absolute ethanol (50 mL) and concentrated hydrochloric acid (100 mL) was stirred at reflux for 48 hours. The reaction mixture was cooled in an ice-water bath and was made basic by addition of solid potassium hydroxide. Concentration *in vacuo* afforded a solid which was diluted with dichloromethane and water. The organic phase was dried over anhydrous magnesium sulfate and concentrated *in vacuo* to provide the title compound (2.0 grams, 93% yield, mp = 181.1-183.2°C)

### PREPARATIVE EXAMPLE 29

### ETHYL 4-[4,8-DICHLORO-5,6-DIHYDRO-11H-BENZO[5,6]CYCLO-HEPTA[1,2-b]PYRIDIN-11-YLIDENE]-1-PIPERIDINE CARBOXYLATE, N-OXIDE

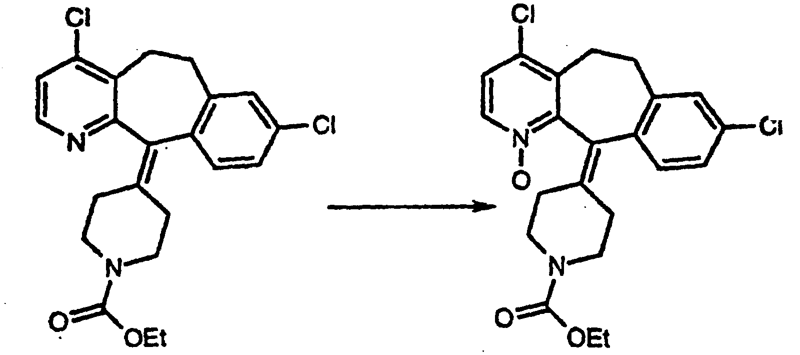

To a cooled (0°C) solution of the 4,8-dichloro compound from Preparative Example 27 (9.5 grams) dissolved in dichloromethane (300) mL) under N₂ was added dropwise a solution of *meta*-chloroperoxybenzoic acid (6.8 grams) dissolved in ethyl acetate (200 mL). The resulting mixture was washed with 1*N* aqueous sodium hydroxide, dried over anhydrous magnesium sulfate and concentrated *in vacuo*. The residue was purified by flash column chromatography (silica gel) using 100% ethyl acetate then 10% methanol-dichloromethane to afford the title compound (4.9 grams, 50%, MH⁺ 433).

### PREPARATIVE EXAMPLE 30

### ETHYL 4-[4-(2-AMINOETHYLTHIO)-8-CHLORO-5,6-DIHYDRO-11H-BENZO[5,6]CYCLOHEPTA[1,2-b]PYRIDIN-11-YLIDENE]-1-PIPERIDINE CARBOXYLATE

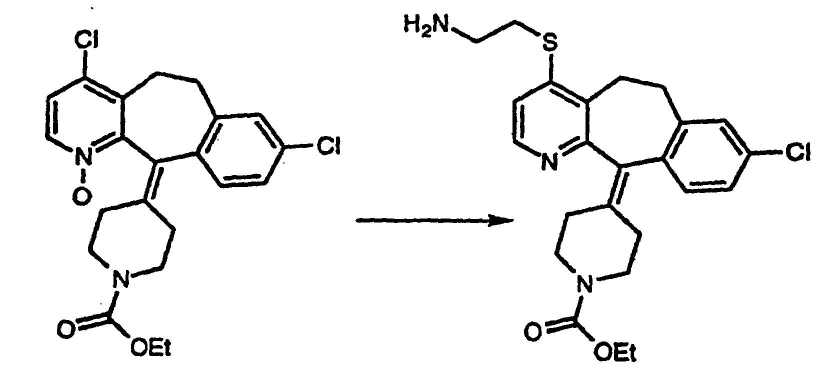

A mixture of the title compound from Preparative Example 29 (0.53 grams), 2-aminoethanethiol hydrochloride (0.74 grams) and absolute ethanol(15 mL) was stirred at reflux for 48 hours. The mixture was cooled to 25°C, diluted with dichloromethane and washed with 1*N* aqueous sodium hydroxide. The organic phase was dried over anhydrous magnesium sulfate and concentrated *in vacuo* to provide the title compound (0.5 grams, 88%, MH⁺ 458).

### PREPARATIVE EXAMPLE 31

### 1,1-DIMETHYLETHYL [2-[[8-CHLORO-6,11-DIHYDRO-11-(1-ETHOXYCARBONYL)-4-PIPERIDINYLIDENE]-5H-BENZO[5,6]CYCLO-HEPTA[1,2-b]PYRIDIN-4-YL]THIO]ETHYL]CARBAMATE

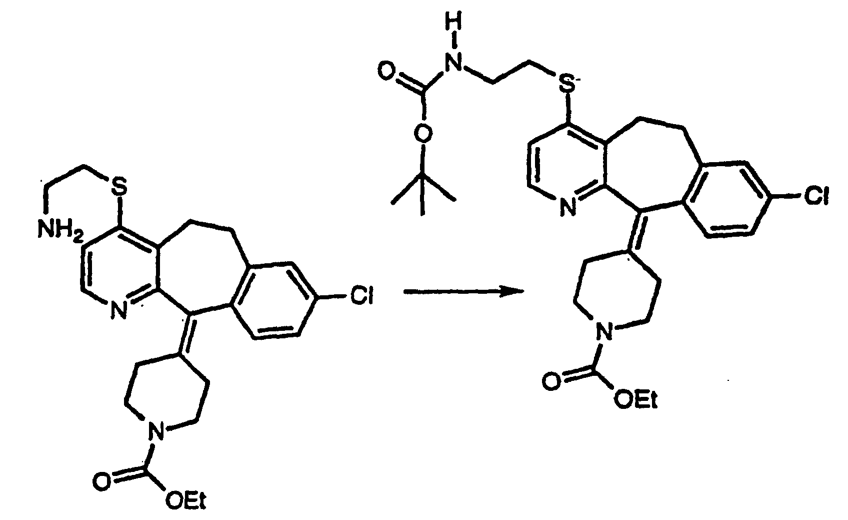

To the title compound from Preparative Example 30 (0.33 grams) dissolved in dichloromethane (60 mL) was added di-*tert*-butyldicarbonate (0.17 grams). The solution was stirred at 25°C under N₂ overnight. An additional 0.1 grams of di-*tert*-butyldicarbonate was added and after 4 hours the reaction mixture was diluted with dichloromethane, washed with 1*N* aqueous sodium hydroxide and concentrated *in vacuo* to afford the title compound (0.5 grams, 100%, MH⁺ 558).

### PREPARATIVE EXAMPLE 32

### 1,1-DIMETHYLETHYL [2-[[8-CHLORO-6,11-DIHYDRO-11-[4-PIPERIDINYLIDENE]-5H-BENZO[5,6]CYCLOHEPTA[1,2-b]PYRIDIN-4-YLITHIO]ETHYL]CARBAMATE

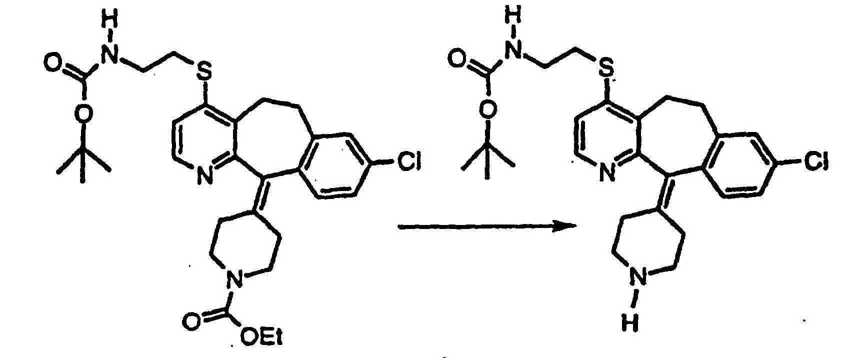

To the title compound from Preparative Example 31 (0.22 grams) dissolved in absolute ethanol (5 mL) was added water (5 mL) and solid potassium hydroxide (0.33 grams). The solution was stirred at reflux for 4 days, then cooled to 25°C, diluted with dichloromethane and washed with water. The organic phase was concentrated *in vacuo* and the residue purified by flash column chromatography (silica gel) using 5% methanol-dichloromethane saturated with ammonium hydroxide to afford the title compound (0.04 grams, 19%, MH⁺ 486).

### PREPARATIVE EXAMPLE 33

### 3-PYRIDYLISOCYANATE, HYDROCHLORIDE

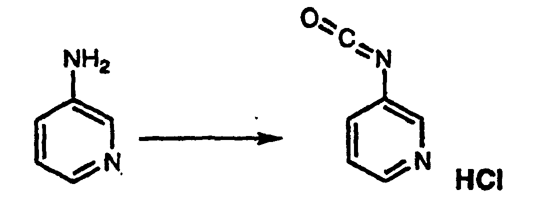

A 1.93 solution of phosgene in toluene (20%) (584 mL) was diluted with dry dichloromethane (1 L) and the mixture was stirred at 0°C under nitrogen atmosphere. A solution of 3-aminopyridine (21.1 grams) and dry pyridine (19 mL) dissolved in dry dichloromethane (600 mL) was added dropwise to the stirred solution at 0°C over a period of 5.5 hours. The mixture was stirred at 0-25°C for an additional 48 hours. A stream of nitrogen was passed through the solution to remove most of the phosgene and the solution was then evaporated until almost all of the solvent was removed to give the title compound which was then taken up in dry pyridine (850 mL) to give a stock solution of the title compound.

### PREPARATIVE EXAMPLE 34

### A. 8-CHLORO-11-(1-ETHOXYCARBONYL-4-PIPERIDINYL)-11H-BENZO[5,6]CYCLOHEPTA(1,2-b)PYRIDINE

### B. 8-CHLORO-11-(1-ETHOXYCARBONYL-4-PIPERIDINYL)-9-ETHYL-11H-BENZO[5,6]CYCLOHEPTA(1,2-b)PYRIDINE

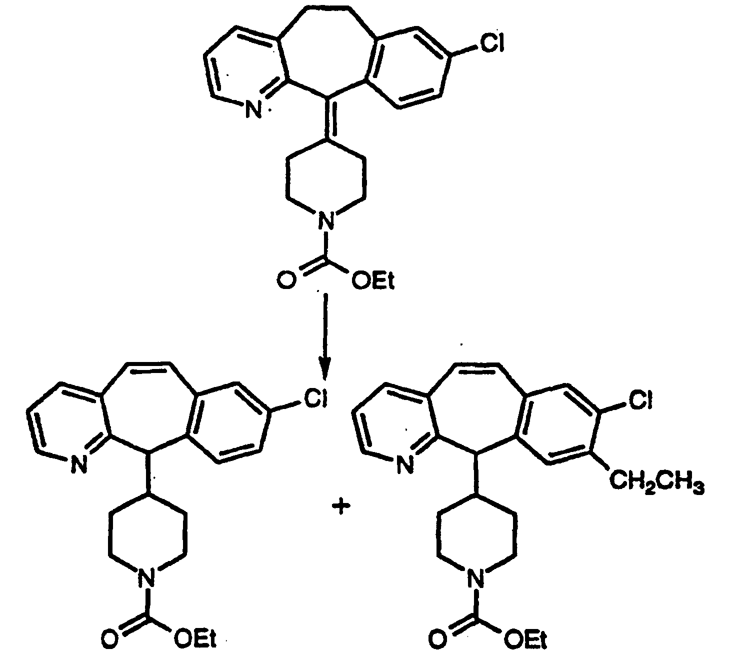

The title compound of Preparative Example 1F above (51.15 grams, 0.1336 mole) was dissolved in trifluoromethanesulfonic acid (170 mL). The dark mixture was heated to reflux for 70h. The solution was cooled to room temperature and was then poured into 800 mL of an ice/water slurry and the resulting mixture stirred. Concentrated ammonium hydroxide solution (175 mL) was added to the mixture in small portions so that the temperature of the mixture was below 20°C. The resulting basic mixture was extracted with dichloromethane. The dichloromethane extract was washed with brine and was then evaporated to give a brown residue. This residue was dissolved in dichloromethane (750 mL) and the solution cooled to 0°C. Ethyl chloroformate (14.8 grams, 0.136 mole) was added over 5 minutes and the resulting mixture stirred at 0° C for 15 minutes. Saturated sodium bicarbonate solution (150 mL) was added and the cooling bath was removed. The resulting biphasic mixture was stirred rapidly for 3h. The layers were separated and the dichloromethane layer was filtered through silica gel. The filtrate was evaporated to dryness and the residue chromatographed on silica gel using a gradient of hexane-dichloromethane-acetone 16:2.5:1.5 to hexane-dichloromethane-acetone 28:7.5:4.5 as eluent to give title compound A (25.02g 49% MH⁺ 383) and title compound B (4.85g, 9%, MH⁺ 411).

### C. 8-CHLORO-11-(4-PIPERIDINYL)-11H-BENZO[5,6]CYCLO-HEPTA(1,2-b)PYRIDINE

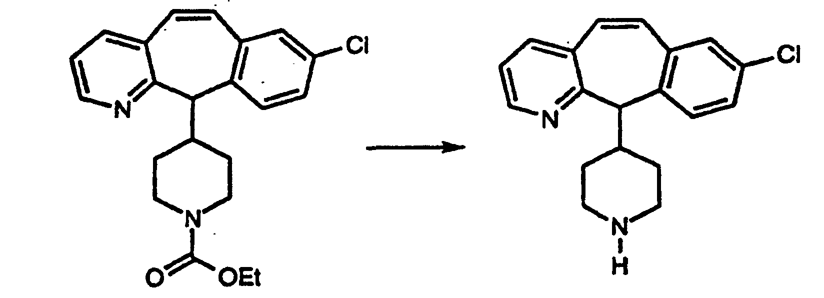

Hydrolyze the title compound of Preparative Example 34A by dissolving in 50% aqueous sulfuric acid (v/v) and heating to 90° to 100°C for 16 h. The cooled acidic mixture was neutralized with 25% sodium hydroxide solution (w/v). The resulting mixture was extracted with ethyl acetate and the ethyl acetate extract was dried with sodium sulfate. Filtration and evaporation of the ethyl acetate afforded the title compound(MH⁺ 311).

### PREPARATIVE EXAMPLE 35

### 8-CHLORO-9-ETHYL-11-(4-PIPERIDINYL)-11H-BENZO[5,6]CYCLOHEPTA[1,2-b]PYRIDINE

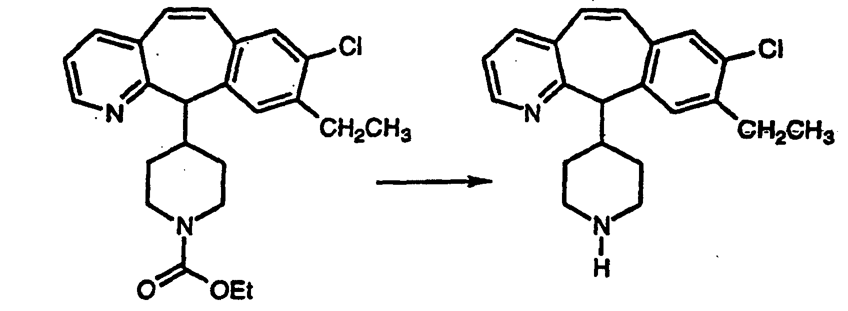

Hydrolyze the title compound of Preparative Example 34B following the procedure described in Preparative Example 34C to provide the title compound. 9Decomposes between 205.7-215.4°C. heating 2-3°C per minute.

### PREPARATIVE EXAMPLE 36

### A. 8-CHLORO-11-(1-ETHOXYCARBONYL-4-PIPERIDINYL)-11H-BENZO[5,6]CYCLOHEPTA[1,2-b]PYRIDINE-1-OXIDE

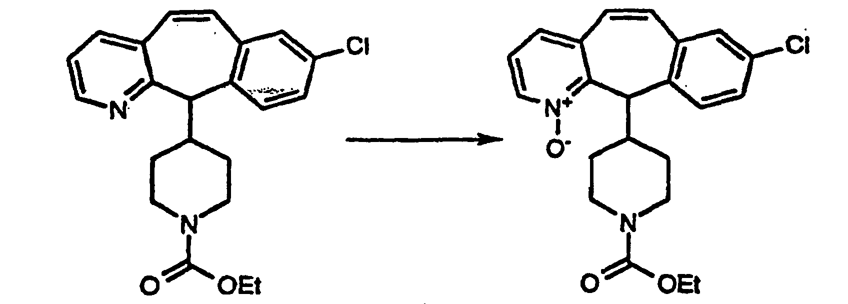

The title compound from Preparative Example 34A above (20.23 grams, 52.84 mmoles) was dissolved in dichloromethane (250 mL). 3-Chloroperoxybenzoic acid (1.25 equivalents) was added in one portion and this solution was stirred for 45 minutes. Sodium bisulfite solution (20% w/v) was added and the biphasic mixture rapidly stirred for 30 minutes. The layers were separated and the organic layer was washed with saturated sodium carbonate solution and dried with sodium sulfate. Filtration and evaporation afforded the title compound (21g, 99%, mp 78.6-89.4°C, MH+ 399).

### B. 4,8-DICHLORO-11-(1-ETHOXYCARBONYL-4-PIPERIDINYL)-11H-BENZO[5,6]CYCLOHEPTA[1,2-b]PYRIDINE (A) and 2,8-DICHLORO-11-(1-ETHOXYCARBONYL-4-PIPERIDIN-YL)-11H-BENZO[5,6]CYCLOHEPTA[1,2-B]PYRIDINE (B)

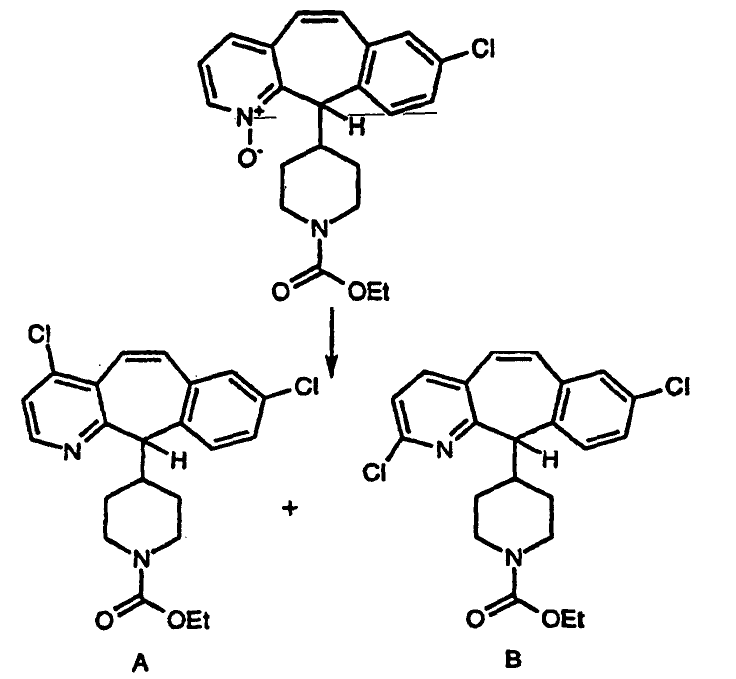

The title compound from Preparative Example 36A (21 grams, 53 mmoles) above was dissolved in anhydrous dichloroethane (250 mL) and the solution cooled to 0°C. POCl₃ (49.4 grams, 0.322 mole) was added dropwise to the dichloroethane solution over 15 minutes. After the POCI₃ was added the reaction mixture was warmed to 45 - 50°C and stirred for 18h. Additional POCl₃ (8.2 grams) was added and the mixture heated to reflux for 9h. The mixture was cooled and added to an ice cooled, stirred solution of sodium hydroxide (15% w/v). The resulting biphasic mixture was stirred rapidly for 18h. The layers were separated and the aqueous layer was extracted with dichloromethane. The combined organic layers were washed with water followed by brine and dried (sodium sulfate). The mixture was filtered and evaporated, and the residue chromatographed on silica gel using a gradient of 25% ethyl acetate in hexane to 45% ethyl acetate in hexane as eluent. The title compound A was obtained as a yellow solid (5.98 g M⁺ 417), and title compound B was obtained as a yellow solid (1.0 g, mp 84.4-90.6°C).

### C. 4,8-DICHLORO-11-(4-PIPERIDINYL)-11H-BENZO[5,6]-CYCLOHEPTA[1,2-b]PYRIDINE

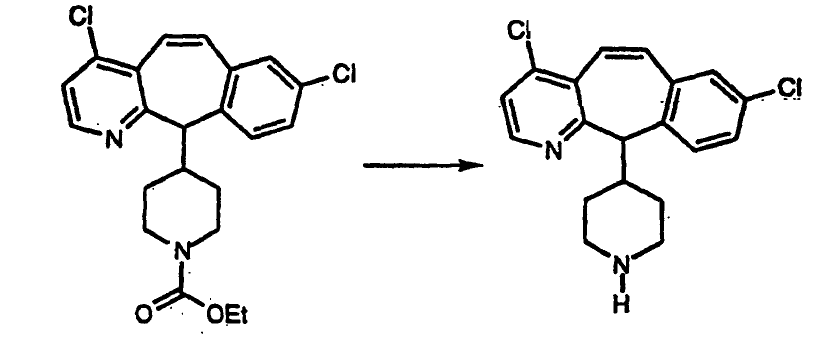

The title compound A from Preparative Example 36B was hydrolyzed under the conditions described in Preparative Example 34C to give the title compound (M⁺ 345).

### PREPARATIVE EXAMPLE 37

### A. 4-(8-CHLORO-11H-BENZO[5,6]CYCLOHEPTA[1,2-b]PYRIDIN-11-YLIDENE)-1-(ETHOXYCARBONYL)-PIPERIDINE

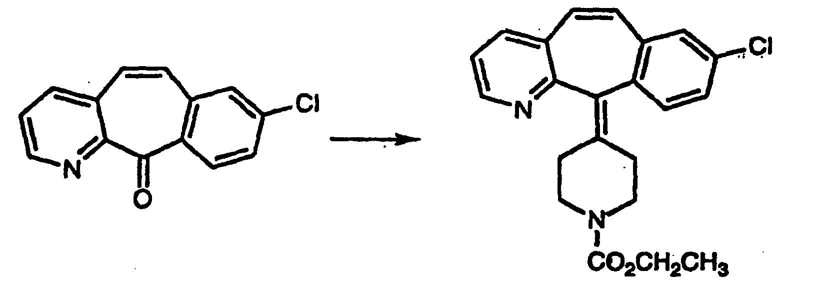

The preparation of the starting material for this reaction was described in *The Journal of Organic Chemistry,* **1990,** *55*, pp. 3341-3350 by Piwinski, J.J.; Wong, J.K.; Chan, T.-M.; Green, M.J.; and Ganguly, A.K. By substituting in Preparative Example 2, 8-chloro-11H-benzo[5,6]-cyclohepta[1,2-b]pyridin-11-one for 9-fluoro-5,6-dihydro-11H-benzo[5,6]-cyclohepta[1,2-b]pyridin-11-one and employing basically the same methods as steps D through F of Preparative Example 2, one obtains the title compound (mp 154.7 - 155.5°C).

### B. 8-CHLORO-11-(4-PIPERIDINYL)-BENZO[5,6]CYCLO-HEPTA[1,2-b]PYRIDINE

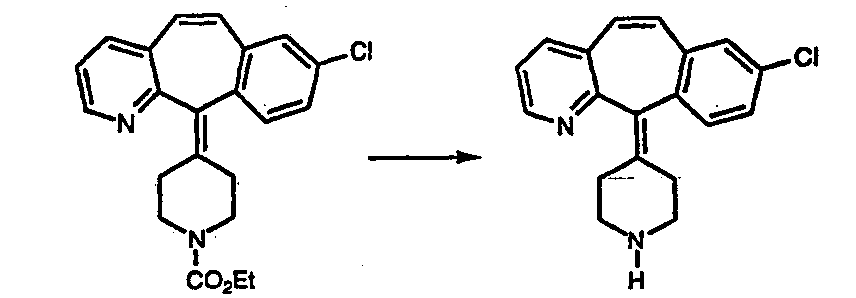

Hydrolyze the title compound of Preparative Example 37A following the procedure described in Preparative Example 334C (mp 168.5 - 171.2°C, decomposition).

### PREPARATIVE EXAMPLE 38

### 8-CHLORO-11-(1-PIPERAZINYL)-11H-BENZO[5,6]CYCLO-HEPTA[1,2b]PYRIDINE

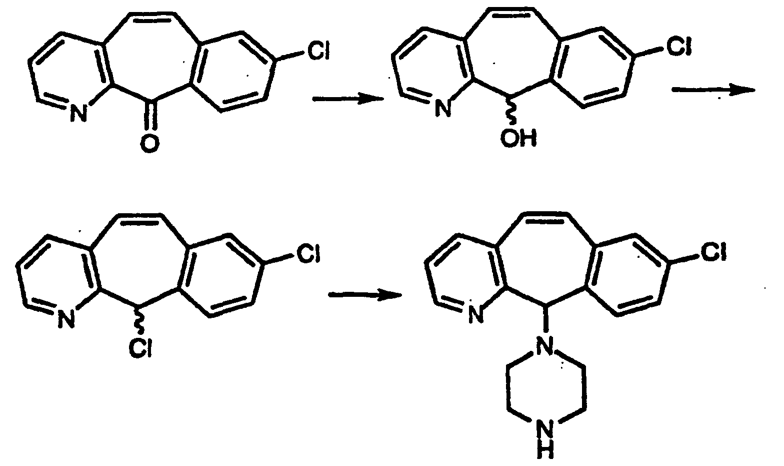

The preparation of the starting material for this reaction was described in *The Journal of Organic Chemistry,* **1990**, *55*, pp. 3341-3350 by Piwinski, J.J.; Wong, J.K.; Chan, T.-M.; Green, M.J.; and Ganguly, A.K. By substituting in Preparative Example 7A, 8-chloro-11H-benzo[5,6]cyclohepta[1,2-b]pyridin-11-one (11.53g) (47.71mmoles) for 8-chloro-5,6-dihydro-11H-benzo[5,6]cyclohepta[1,2-b]pyridin-11-one and employing basically the same methods as steps A through C of Preparative Example 7, one obtains 11.53g (36%) of the title compound (MH⁺ 312).

### PREPARATIVE EXAMPLE 39

### A. ETHYL α,α-DIMETHYL-3-PYRIDYLACETIC ACID N-OXIDE

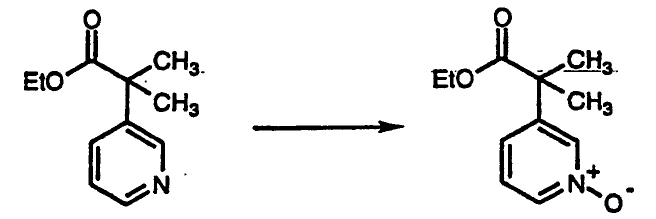

By substituting in Preparative Example 8A, ethyl α,α-dimethyl-3-pyridylacetic acid (4.0g, 20.7mmoles) for ethyl 3-pyridylacetic acid and using the same method as described in Preparative Example 8A, one obtains the title compound (3.2g, 74%, MH⁺ 210).

### B. α,α-DIMETHYL-3-PYRIDYLACETIC ACID N-OXIDE

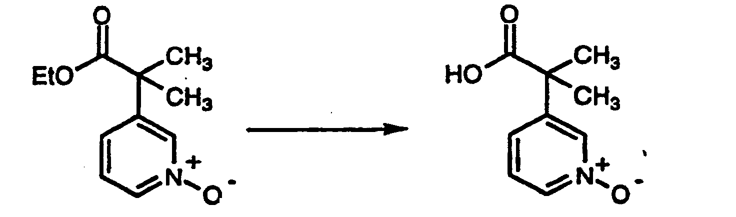

By substituting in Preparative Example 8B, ethyl α,α-dimethyl-3-pyridylacetic acid N-oxide (0.142g, 0.68mmoles) (Preparative Example 39A) for ethyl 3-pyridylacetic acid N-oxide and using the same method as described in Preparative Example 8B, one obtains the title compound.

### PREPARATIVE EXAMPLE 40

### 4-BROMO-8-CHLORO-11-(1-PIPERAZINYL)-6,11-DIHYDRO-5H-BENZO[5,6]CYCLOHEPTA[1,2-b]PYRIDINE

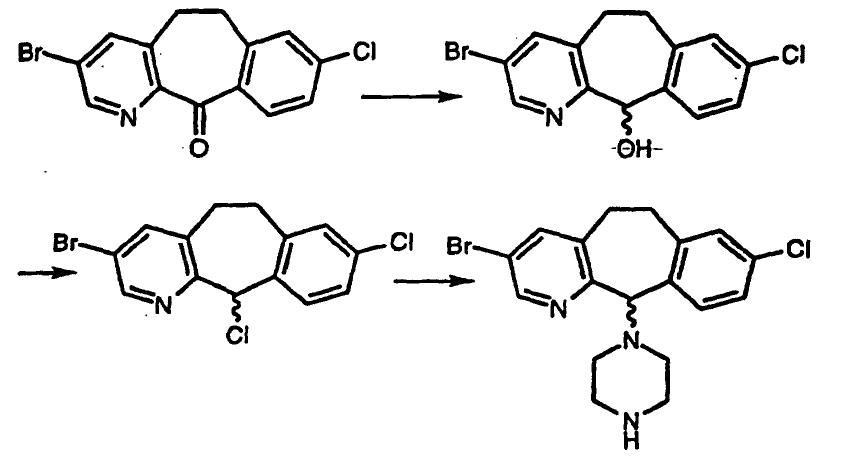

By substituting in Preparative Example 7A, 4-bromo-8-chloro-11-(1-piperazinyl)-5,6-dihydro-11H-benzo[5,6]cyclohepta[1,2-b]pyridin-11-one (1.5g, 4.65mmoles) (Preparative Example 20A) for 8-chloro-5,6-dihydro-11H-benzo[5,6]cyclohepta[1,2-b]pyridin-11-one and using the same methods as described in steps A through C of Preparative Example 7, one obtains the title compound (1.31g, 72%, MH⁺ 392).

### PREPARATIVE EXAMPLE 41

### 4,8-DICHLORO-11-(1-PIPERAZINYL)-6,11-DIHYDRO-5H-BENZO[5,6]CYCLOHEPTA[1,2-b]PYRIDINE

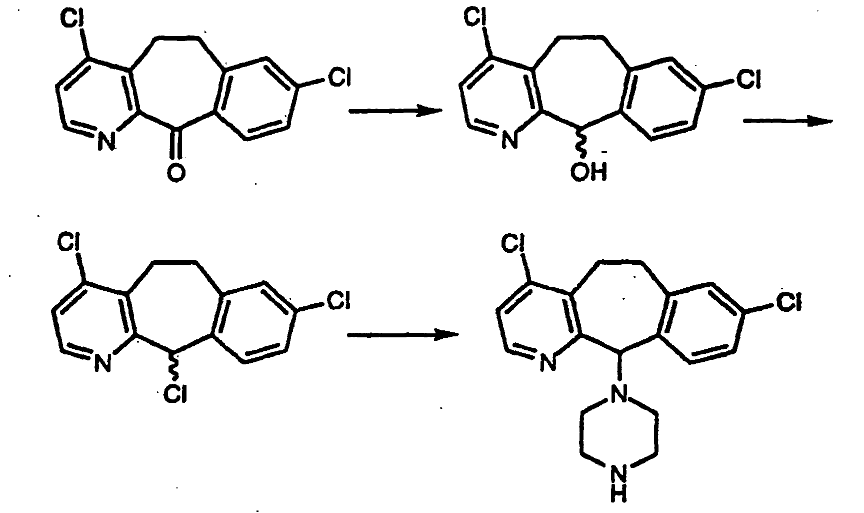

By substituting in Preparative Example 7A 4,8-Dichloro-5,6-dihydro-11H-benzo[5,6]cyclohepta[1,2-b]pyridin-11-one (6.64g, 28.37mmoles) (Preparative Example 5B) for 8-chloro-5,6-dihydro-11H-benzo[5,6] cyclohepta [1,2-b]pyridin-11-one and using the same methods as described in steps A through C of Preparative Example 7, one obtains the title compound (2.59g, 26%, MH⁺ 348).

### PREPARATIVE EXAMPLE 42

### ETHYL 4-[4-[(1H-BENZOTRIAZOL-1-YL)OXY]-8-CHLORO-5,6-DIHYDRO-11H-BENZO[5,6]CYCLOHEPTA[1,2-b]PYRIDIN-11-YLIDENE]-1-PIPERIDINE CARBOXYLATE

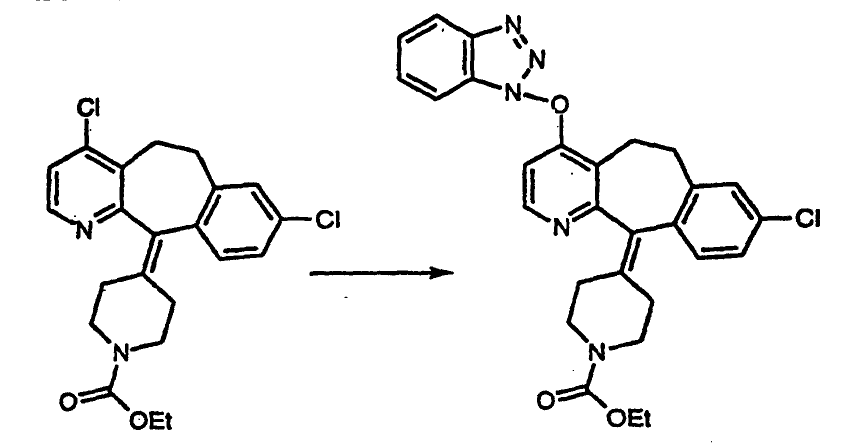

To a solution of the 4,8-dichloro compound from Preparative Example 27 (1.5 grams) in dry dimethylformamide (20 mL) was added 1-hydroxybenzotriazole (1.5 grams). After stirring for 14 days at 25°C, sodium hydride (0.84 grams, 60% in mineral oil) was added and after an additional 24 hours, the mixture was poured into water. Filtration provided the title compound (Yield: 1.7 grams, 89%, mp = 181.5 - 183.9 °C, MH⁺ 516).

### PREPARATIVE EXAMPLE 43

### ETHYL 4-[4-HYDROXY-8-CHLORO-5,6-DIHYDRO-11H-BENZO[5,6]CYCLOHEPTA[1,2-b]PYRIDIN-11-YLIDENE]-1-PIPERIDINE CARBOXYLATE

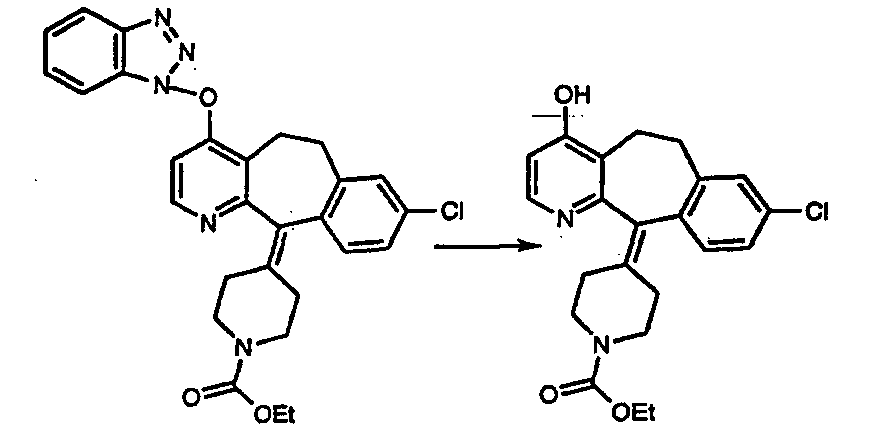

To a solution of the title compound from Preparative Example 42 (0.15 grams) and glacial acetic acid (5 mL) was added zinc dust (0.2 grams). After stirring at 25°C for 1 hour, the mixture was filtered through celite and the filtrate concentrated *in vacuo*. The residue was diluted with ethyl acetate, washed with saturated aqueous sodium bicarbonate and brine. The organic layer was separated, dried over magnesium sulfate and concentrated *in vacuo* to give the title compound (Yield: 0.11 grams, 95%, MH+ 399).

### PREPARATIVE EXAMPLE 44

### ETHYL 4-[3-BROMO-4-HYDROXY-8-CHLORO-5,6-DIHYDRO-11H-BENZO[5,6]CYCLOHEPTA[1,2-b]PYRIDIN-11-YLIDENE]-1-PIPERIDINE CARBOXYLATE

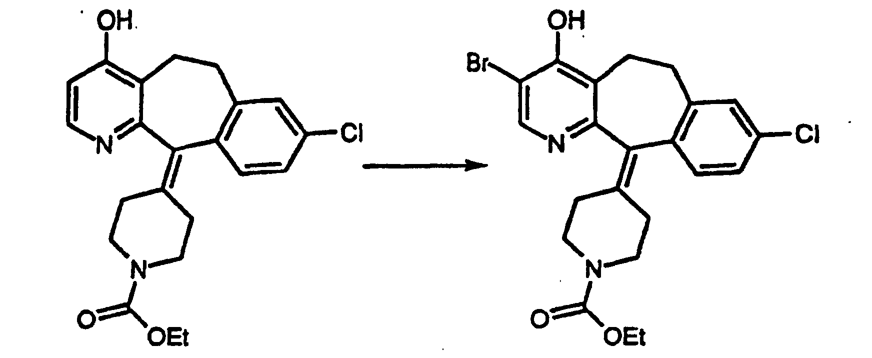

To a solution of the title compound from Preparative Example 43 (1.3 grams) and glacial acetic acid (5 mL) was added a 0.7 *M* bromine-acetic acid solution (4 mL) at 25°C under N₂. The solution was poured into 200 mL of water and the resulting solid was filtered, then washed with water. The solid was dried under vacuum overnight to provide the title compound (Yield: 1.2 grams, 81%, MH⁺ 477).

### PREPARATIVE EXAMPLE 45

### ETHYL 4-[3-BROMO-4,8-DICHLORO-5,6-DIHYDRO-11H-BENZO[5,6]CYCLOHEPTA[1,2-b]PYRIDIN-11-YLIDENE]-1-PIPERIDINE CARBOXYLATE

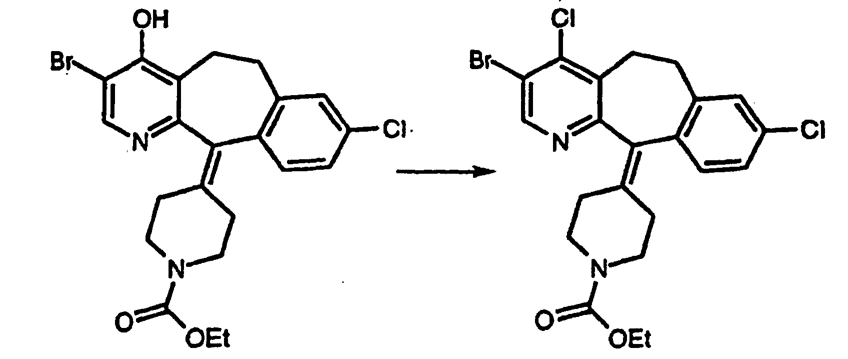

A mixture of the title compound from Preparative Example 44 (5.1 grams), phosphorous oxychloride (20 mL) and chloroform (40 mL) was stirred at reflux over night. The reaction mixture was made basic by the slow addition of 1*N* aqueous sodium hydroxide, and the resultant mixture was diluted with dichloromethane. The mixture was shaken well and after separation of the phases, the organic phase was washed with 1*N* aqueous sodium hydroxide. The organic phase was dried over anhydrous magnesium sulfate, filtered and concentrated *in vacuo* to provide a solid which was mixed with methanol and filtered. Concentration of the filtrate provided the title compound as a solid (Yield: 5.7 grams,MH⁺ 497).

### PREPARATIVE EXAMPLE 46

### 3-BROMO-4,8-DICHLORO-11-(4-PIPERIDYLIDENE)-6,11-5H-BENZO[5,6]CYCLOHEPTA[1,2-b]PYRIDINE

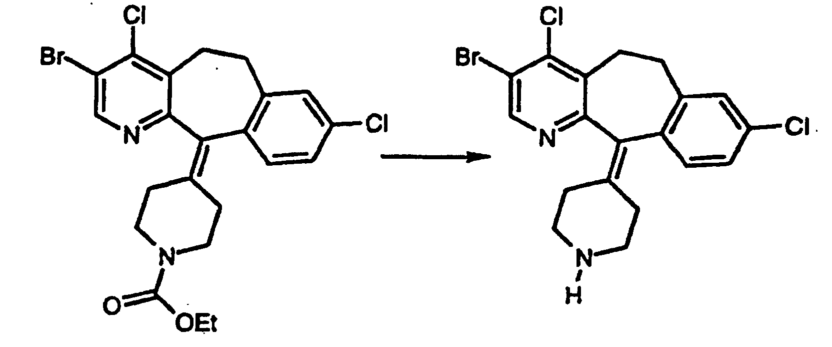

A solution of the title compound from Preparative Example 45 (5.7 grams) dissolved in absolute ethanol (100 mL) and concentrated hydrochloric acid (200 mL) was stirred at reflux for 24 hours. The reaction mixture was cooled in an ice-water bath and was made basic by the addition of solid potassium hydroxide. Extraction with dichloromethane and concentration of the organic phase *in vacuo* afforded the title compound as a solid (1.7 grams, 35% yield, MH⁺ 425).

### PREPARATIVE EXAMPLE 47

### A 4-(8-CHLORO-3-NITRO-5,6-DIHYDRO-11H-BENZO[5,6]-CYCLOHEPTA[1,2-b]PYRIDIN-11-YLIDENE)-1-PIPERIDINE-1-CARBOXYLIC ACID ETHYL ESTER

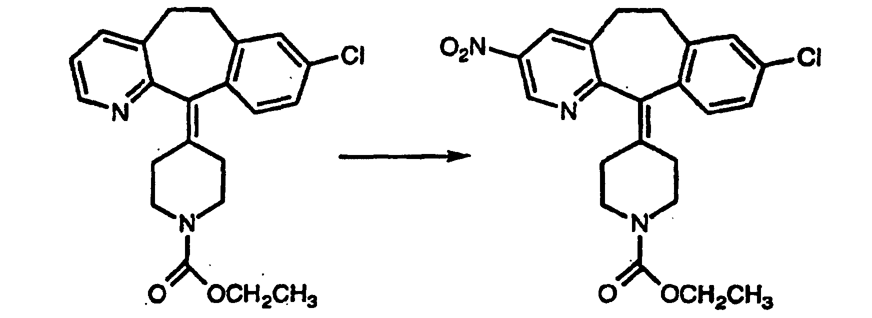

Tetrabutyl ammonium nitrate(4.98g, 16.3 mmol) was dissolved in dichloromethane(20 mL) and trifluoroacetic anhydride(3.12g,14.9 mmol, 2.1 mL) was then added. The solution was cooled to 0°C and then added (by cannulation) to a solution of 4-(8-chloro-5,6-dihydro-11H-benzo[5,6]-cyclohepta[1,2-b]pyridin-11-ylidene)-1-piperidine-1-carboxylic aid ethyl ester (5.69g, 14.9 mmol) in methylene chloride (35 mL) also cooled to 0°C. The reaction mixture was stirred at 0°C for 3h and then allowed to go to room temperature (25°C) overnight. The reaction mixture was then extracted with saturated sodium bicarbonate (60 mL) dried over magnesium sulfate and concentrated to give a semi-solid material that was chromatographed on silica gel eluting first with 10% and then 20% ethyl acetate -hexane. Removal of the organic solvents gave the title compound in 44% yield as a light yellow solid. MP = 90.4-91.0°C, MH⁺ 428.

### B. 4-(8-CHLORO-3-AMINO-5,6-DIHYDRO-11H-BENZO[5,6]-CYCLOHEPTA[1,2-b]PYRIDIN-11-YLIDENE)-1-PIPERIDINE-1-CARBOXYLIC ACID ETHYL ESTER

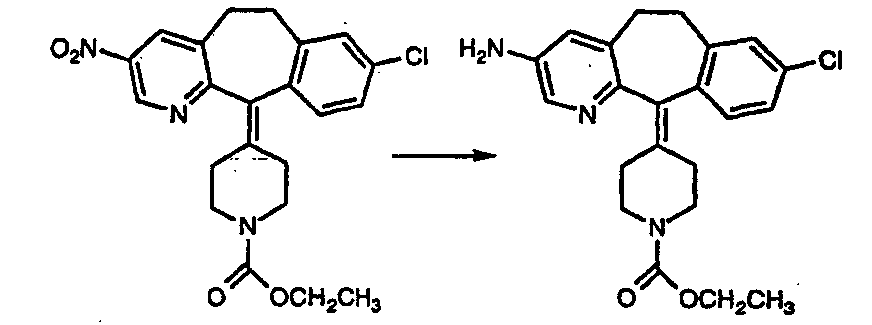

The title compound from Preparative Example 47A (5.99g, 14 mmol) was dissolved in 85% aqueous ethanol. To this solution was added iron filings (7.01g, 125.57 mmol) and calcium chloride (0.69g, 6.29 mmol) and the reaction mixture was refluxed for 16h. The reaction mixture was filtered through a bed of celite while hot and the celite was washed with hot ethanol (700 mL). The ethanol solution was then decolorized with activated charcoal (2.4g) and then filtered through celite. Ethanol was then rotary eavaporated to give the title compound in 100% yield as an off-white solid. MP= 102.4-103.1 °C, MH + 398.

### C. 4-(8-CHLORO-3-BROMO-5,6-DIHYDRO-11H-BENZO[5,6]-CYCLOHEPTA[1,2-b]PYRIDIN-11-YLIDENE)-1-PIPERIDINE-1-CARBOXYLIC ACID ETHYL ESTER

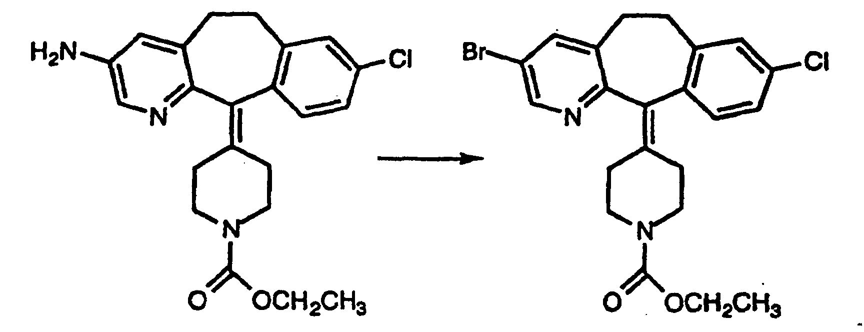

The title compound from Preparative Example 47B (3.00g, 7.60 mmol) was dissolved in hydrobromic acid (48%, 30 mL). The reaction mixture was cooled to -5°C (ice-ethylene glycol bath) and bromlne(2 mL) was added dropwise. The reaction mixture was stirred at -5°C for 15 minutes. Sodium nitrite (1.57g, 22.8 mmol) dissolved in water (15 mL) was slowly added to the reaction mixture. The reaction mixture was then stirred for 45 minutes and then quenched with 40% NaOH to pH ∼10. The aqueous phase was then extracted with ethyl acetate(3x100mL). Combined ethyl acetate fractions were dried over sodium sulfate and then concentrated to give the title compound in 83% yield as a light brown solid. Mp = 146- 148°C, MH+ 463.

The following examples illustrate methods by which compounds of the invention as well as intermediates suitable for preparing the compounds of the invention may be made. Examples marked with an asterisk (*) are not within the scope of the invention and are provided by way of illustrating analogous procedures by which compounds of the invention may be made.

### EXAMPLE 1*

### 1-(4-PYRIDYLACETYL)-4-(8-CHLORO-5,6-DIHYDRO-11H-BENZO[5,6]CYCLOHEPTA[1,2-b]PYRIDIN-11-YLIDENE)PIPERIDINE

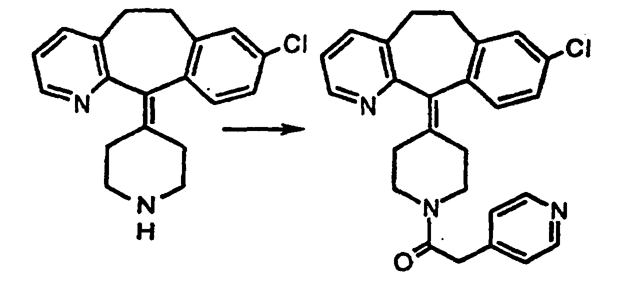

To a mixture of 528 mg (1.7 mmol) of 4-(8-chioro-5,6-dihydro-11H-benzo[5,6]cyclohepta[1,2-b]pyridin-11-ylidene)piperidine (product from Preparative Example 1, Step G), 274 mg (1.7 mmol) of 4-pyridyloicetic acid hydrochloride, and 242 mg (1.8 mmol) of 1-hydroxybenzotriazole hydrate in 5 mL of dry methylene chloride at -15 °C and under a nitrogen atmosphere was added dropwise 0.17 mL (1.5 mmol) of triethylamine followed by a solution of 363 mg (1.9 mmol) of 1-(3-dimethylaminopropyl)-3-ethylcarbodiimide hydrochloride (DEC) in 5 mL of dry methylene chloride. The reaction mixture was slowly allowed to warm to room temperature. After 4 hours the mixture was poured into water and extracted several times with methylene chloride. The combined organic portions were dried over MgSO₄, filtered, and concentrated *in vacuo* to give a product which was purified via flash chromatography (3% methanol saturated with ammonia in methylene chloride) 155 mg of 1-(4-pyridylacetyl)-4-(8-chloro-5,6-dihydro-11H-benzo[5,6]cyclohepta[1,2-b]pyridin-11-ylidene)piperidine as a solid: mp 152 - 155 °C.

By essentially the same procedure as set forth in Example 1, but using the carboxylic acids set forth in column 1, of Table 2 below, in place of 4-pyridylacetic acid, one can obtain the compounds listed in column 2 of Table 2. The compounds listed in Table 2 refer to compounds of Formula 500.00: 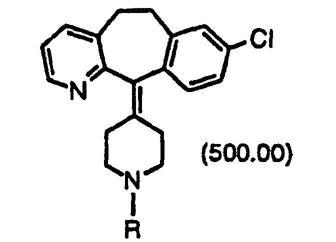 wherein R is the substituent in Column 2.

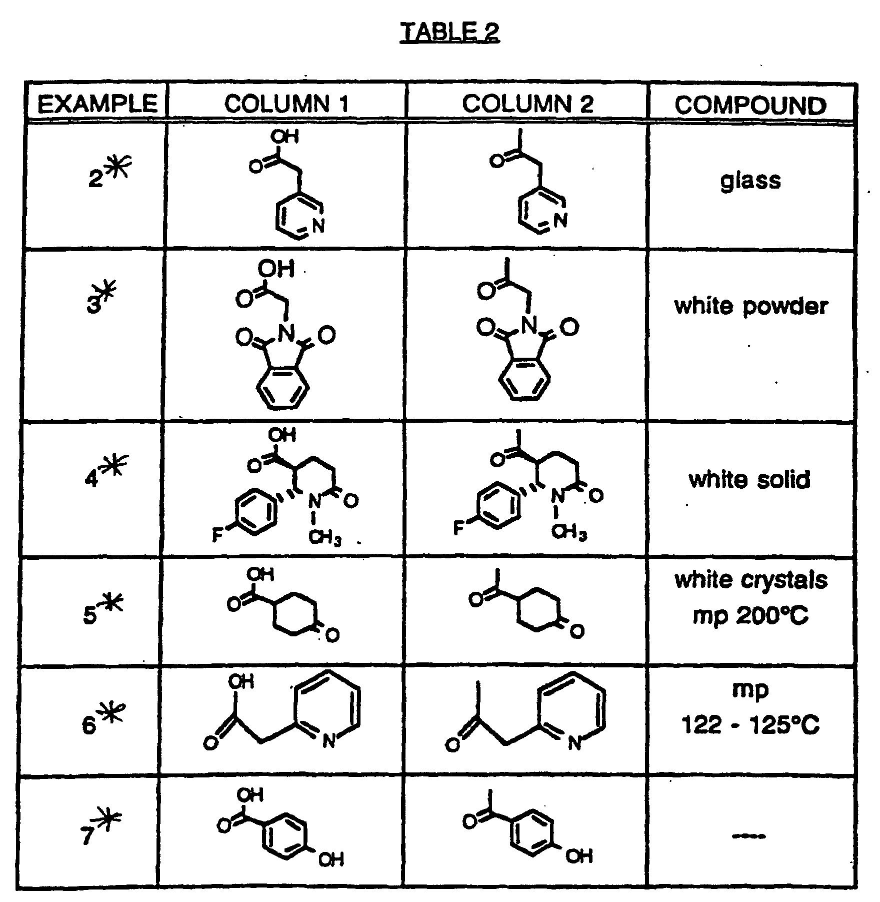

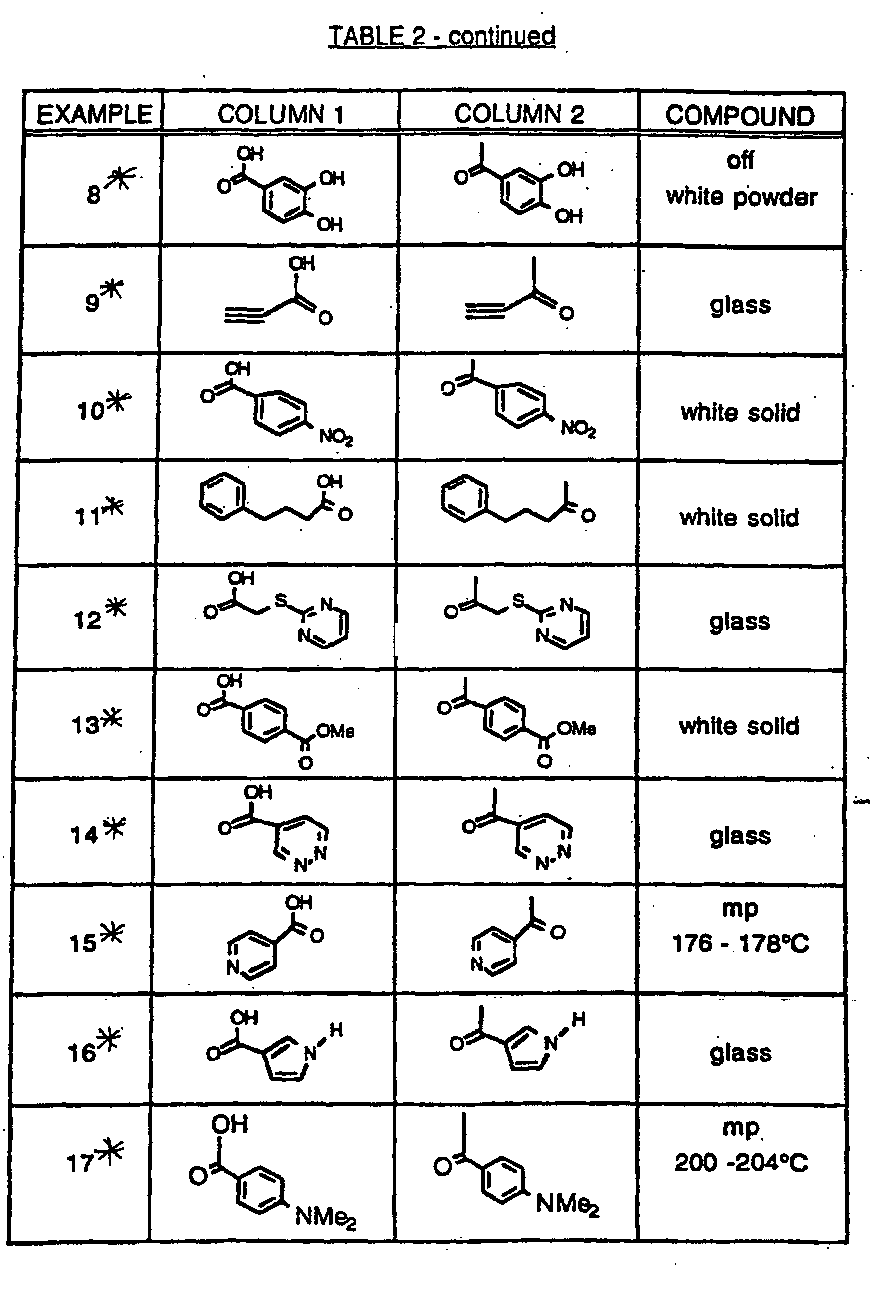

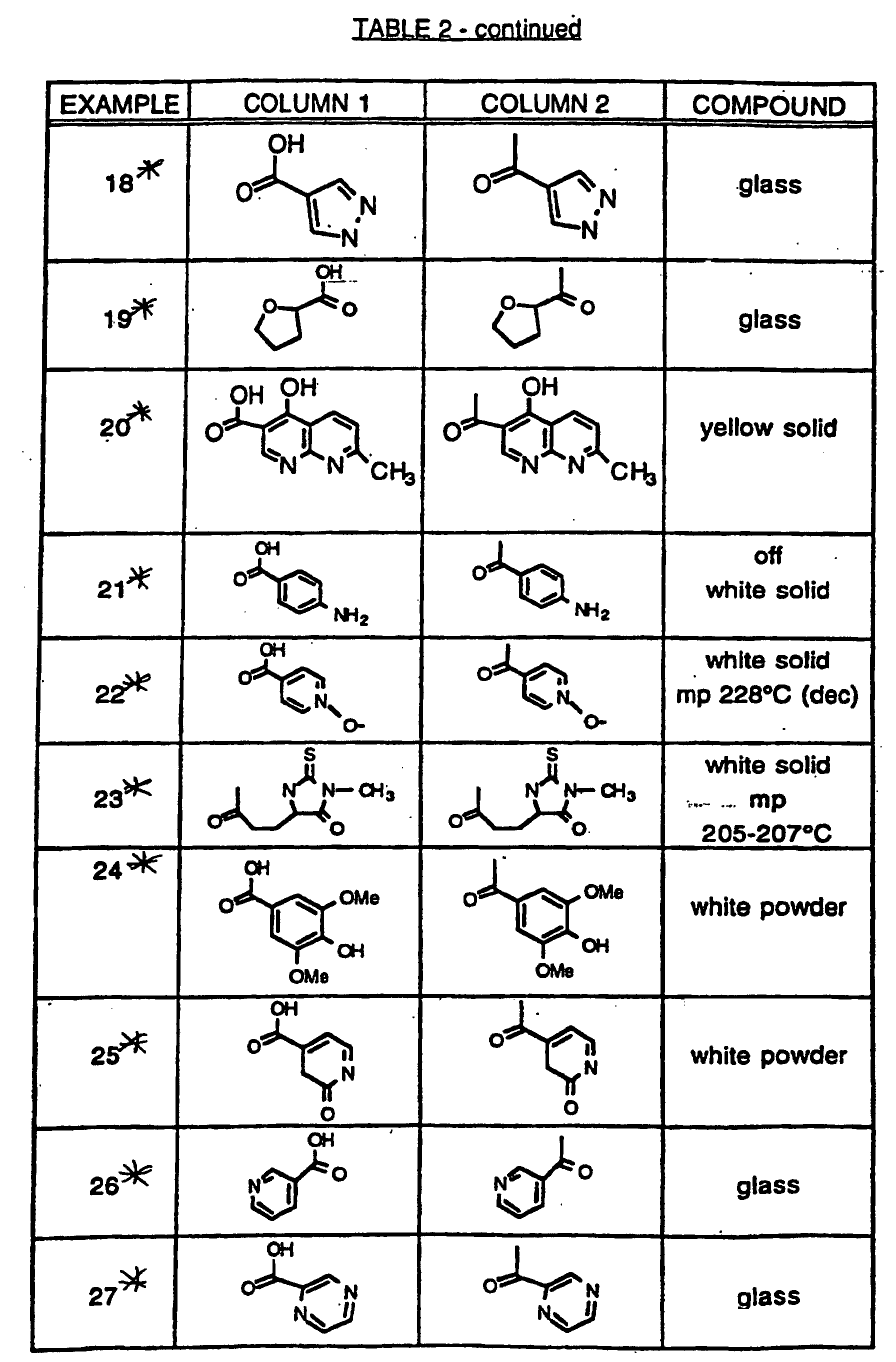

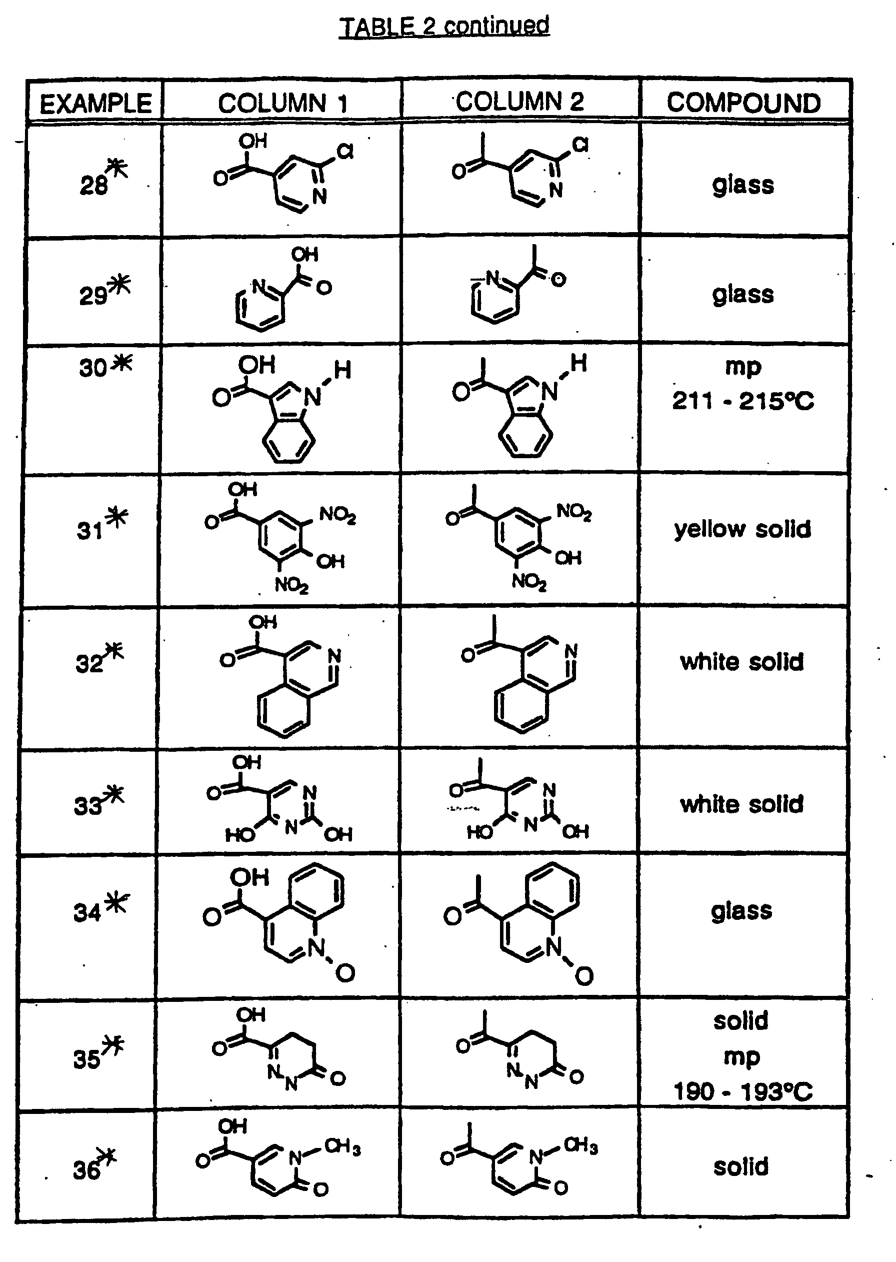

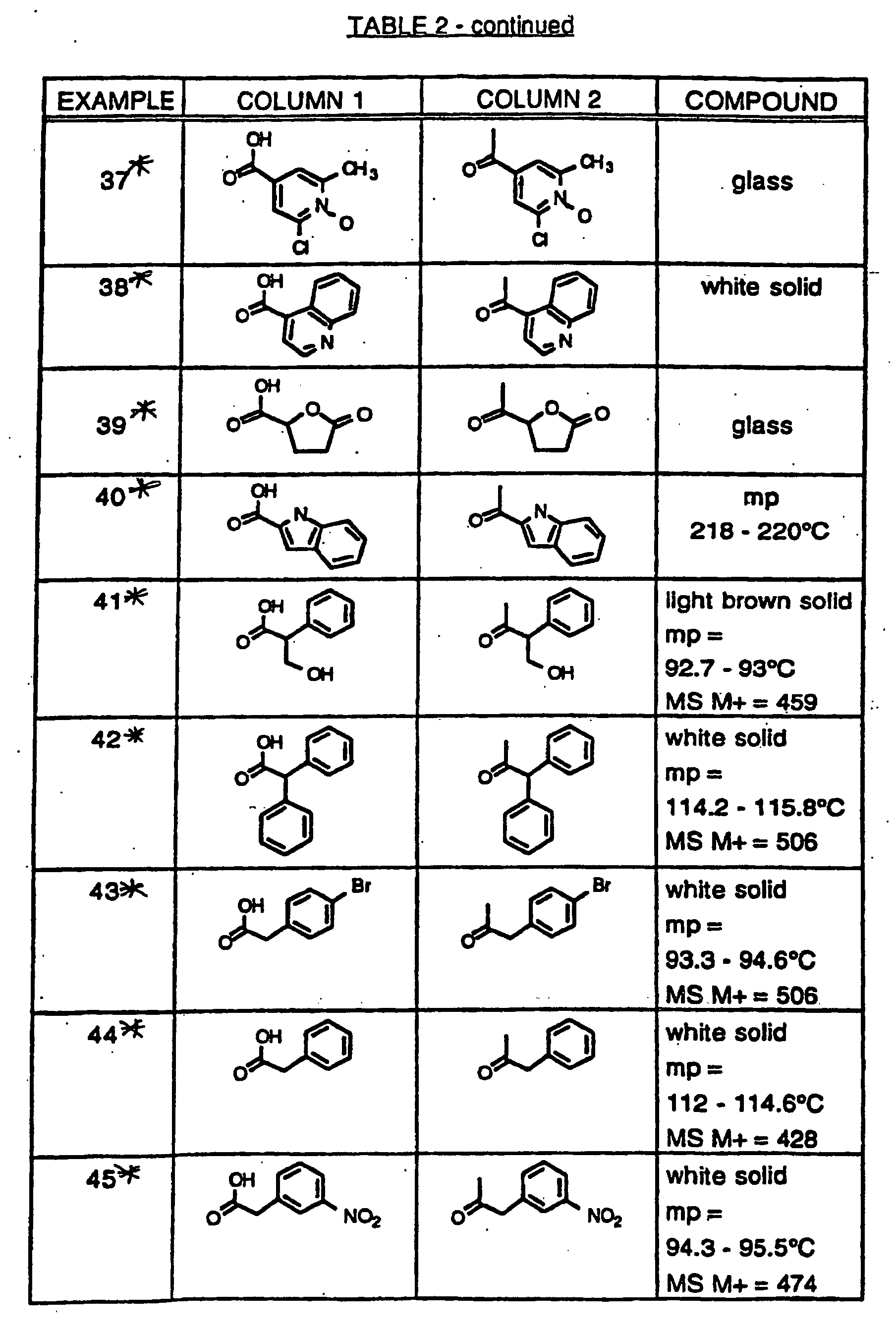

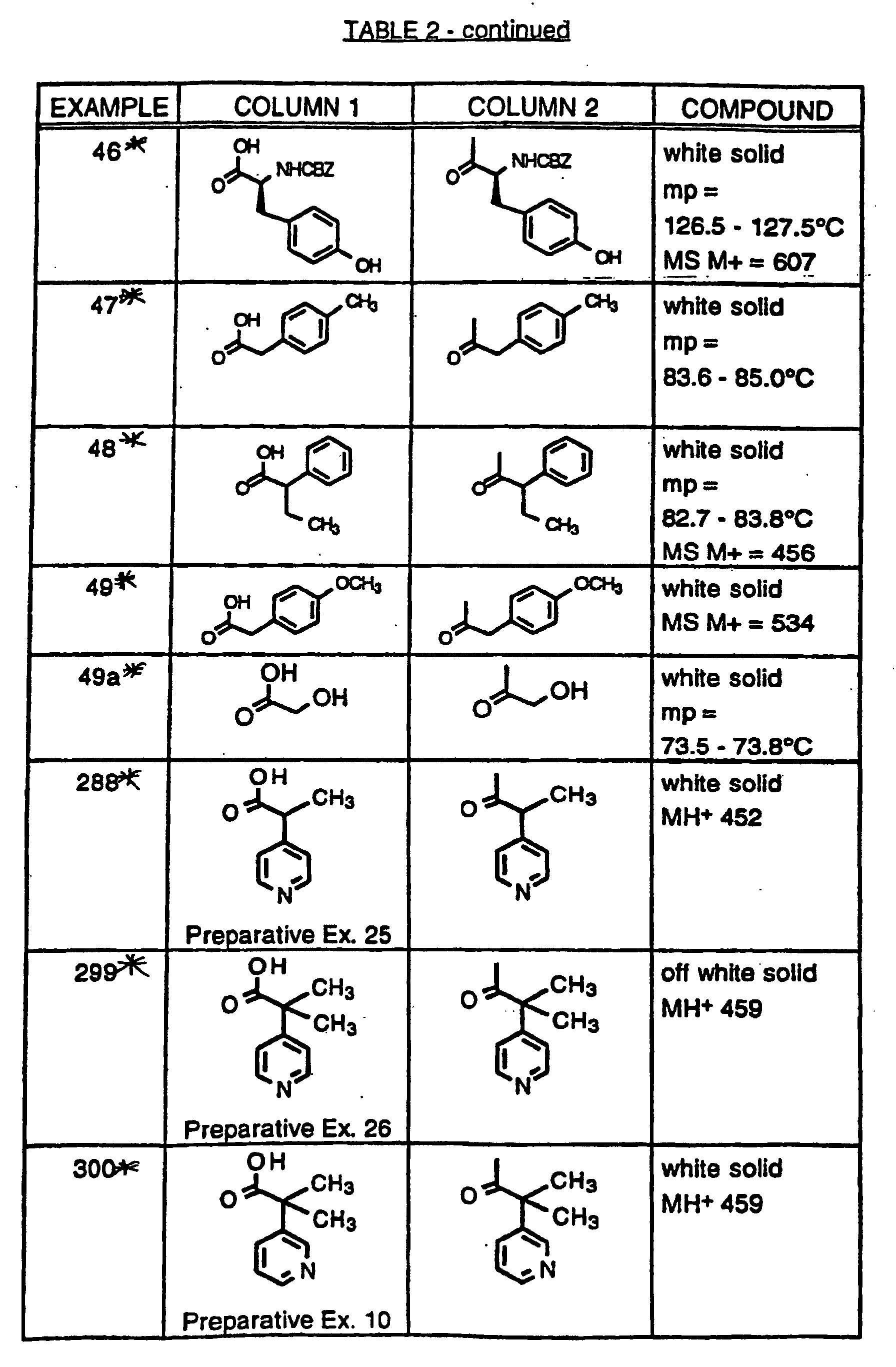

### EXAMPLE 50*

### 1-(2-THIOPHENEACETYL)-4-(8-CHLORO-5,6-DIHYDRO-11H-BENZO[5,6]CYCLOHEPTA[1,2-b]PYRIDIN-11-YLIDENE)PIPERIDINE

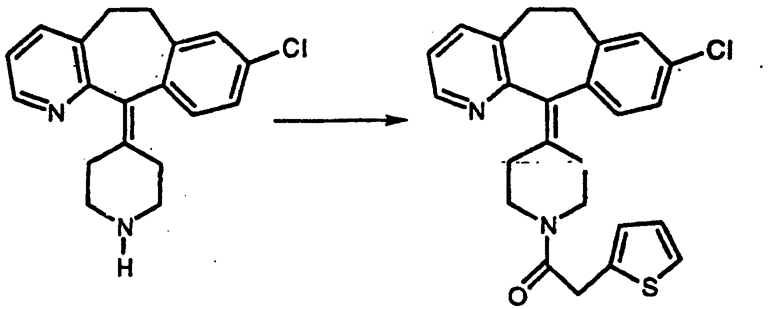

To a solution of 1.0 gm (3.22 mmole) of 4-(8-chloro-5,6-dihydro-11H-benzo[5,6]cyclohepta [1,2-b] pyridin-11-ylidene) piperidine and 0.29 mL of pyridine in 20 mL of dry methylene chloride at 0°C and under an argon atmosphere was added dropwise 0.438 mL (3.55 mmol) of 2-thiopheneacetyl chloride. After 30 minutes the mixture was washed with 1.0 N aqueous sodium hydroxide and then brine. The organic portion was dried over sodium sulfate, filtered and converted in vacuo to provide a residue which was purified via flash chromatography (3% methanol in methylene chloride) and treated with activated carbon to provide the title compound as a glass.

### EXAMPLE 51*

By essentially the same procedure as set forth in Example 50, but using the acid chlorides set forth in Column 1, in Table 3 below, in place of 2-thiopheneacetyl chloride, one can obtain the compounds listed in Column 2 of Table 3. The compounds listed in Table 3 refer to compounds of Formula 500.00: 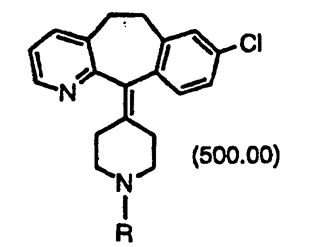 wherein R is the substituent in Column 2

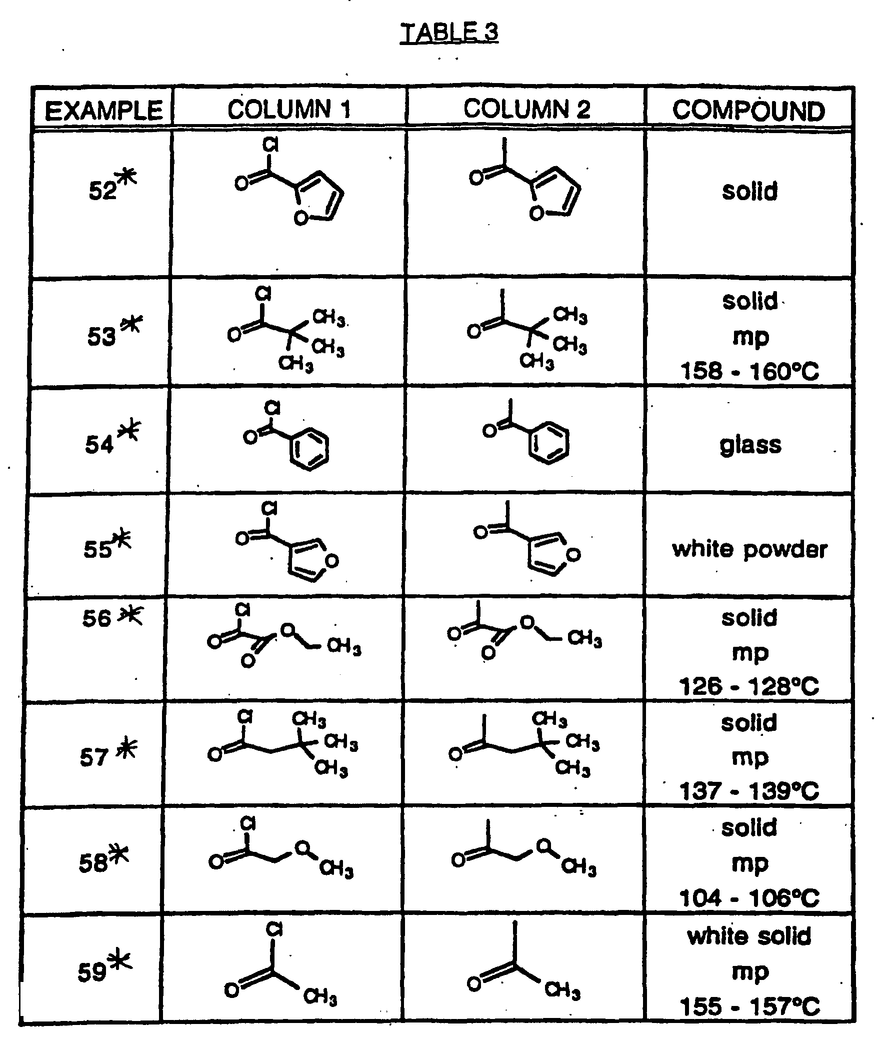

### EXAMPLE 65

By essentially the same procedures as set forth in Example 50 above, or Example 4 of US 5,089,496, but using 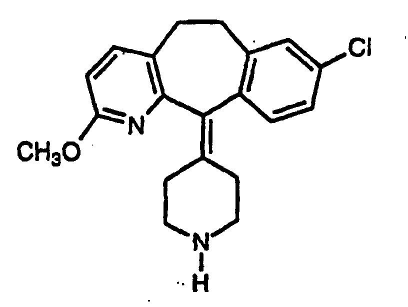 in place of 4-(8-chloro-5,6-dihydro-11H-benzo[5,6]cyclohepta[1,2-b]-pyridin-11ylidene)piperidine, one can obtain the compound 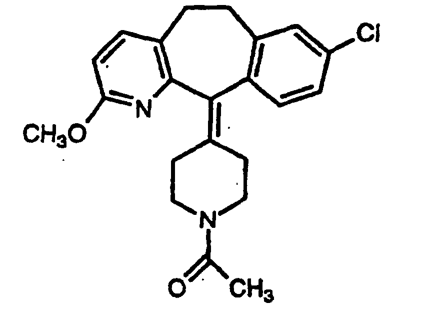 as a white solid.

### EXAMPLE 75*

### 1-(8-CHLORO-5,6-DIHYDRO-11H-BENZO[5,6]CYCLO-HEPTA[1,2-b]PYRIDIN-11-YL-4-(4-PYRIDYLACETYL)-PIPERAZINE

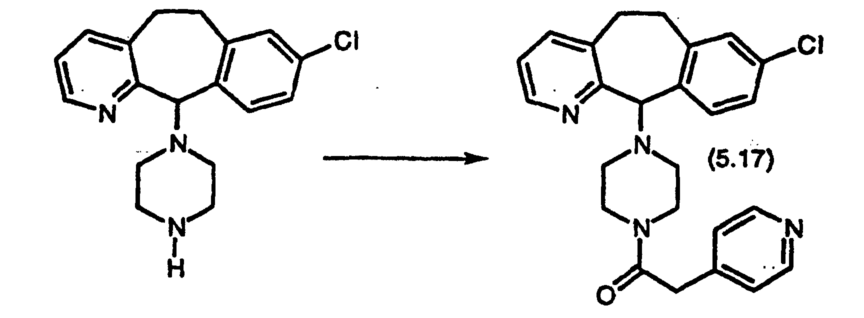

To a mixture of 8.5 g (27.2 m mole) of 8-chloro-11-(1-piperazinyl)-6,11-dihydro-5H-benzo[5,6]cyclohepta[1,2-b]pyridine (Preparative Example 7) in 256 mL of anhydrous dimethylformamide at room temperature and under an argon atmosphere was added 2.98 g (27.2 m mole of 4-methylmorpholine, 7.81 g (27.2 m mole) of 1-(3-dimethylaminopropyl)-3-ethylcarbodiimide hydrochloride, 3.68 g (27.2 m mole) of 1-hydroxybenzotriazole, and 3.72 g (27.2 m mole) pf 4-pyridylacetic acid. The mixture was stirred at room temperature for 22 hours. The mixture was poured into 3300 mL of methylene chloride and washed with 500 mL of water. The aqueous layer was extracted with 500 mL of methylene chloride. The combined organic portions were dried over magnesium Sulfate, filtered, and concentrated in vacuo. The residue was purified by silica gel column chromatography using a solution of 1.5% (10% ammonium hydroxide in methanol) in methylene chloride. The product was obtained as a white amorphous solid, M.S. (Mass Spec) M+ = 433.

By essentially the same procedures as set forth in Example 75 above but using the compounds set forth in Column 1, Table 4 below, In place of 4-pyridylacetic acid, one can obtain compounds of the formula 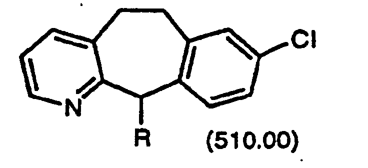 wherein R is as listed in Column 2 of Table 4.

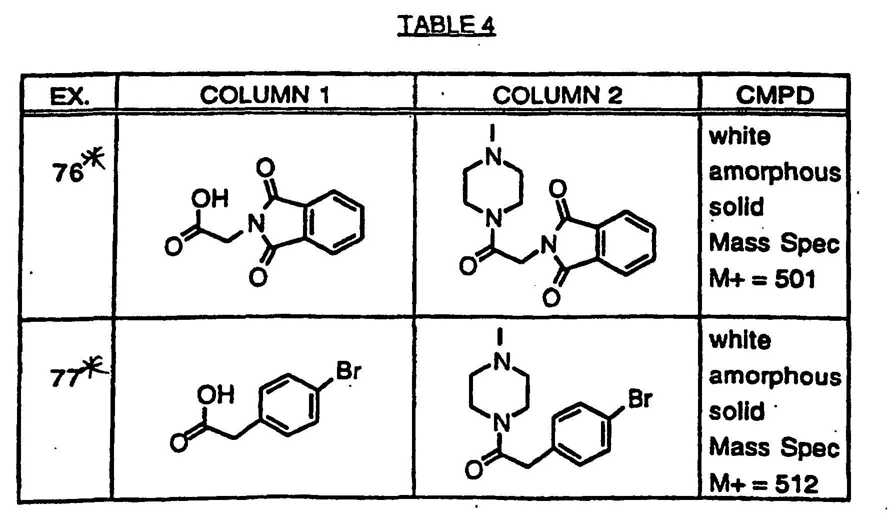

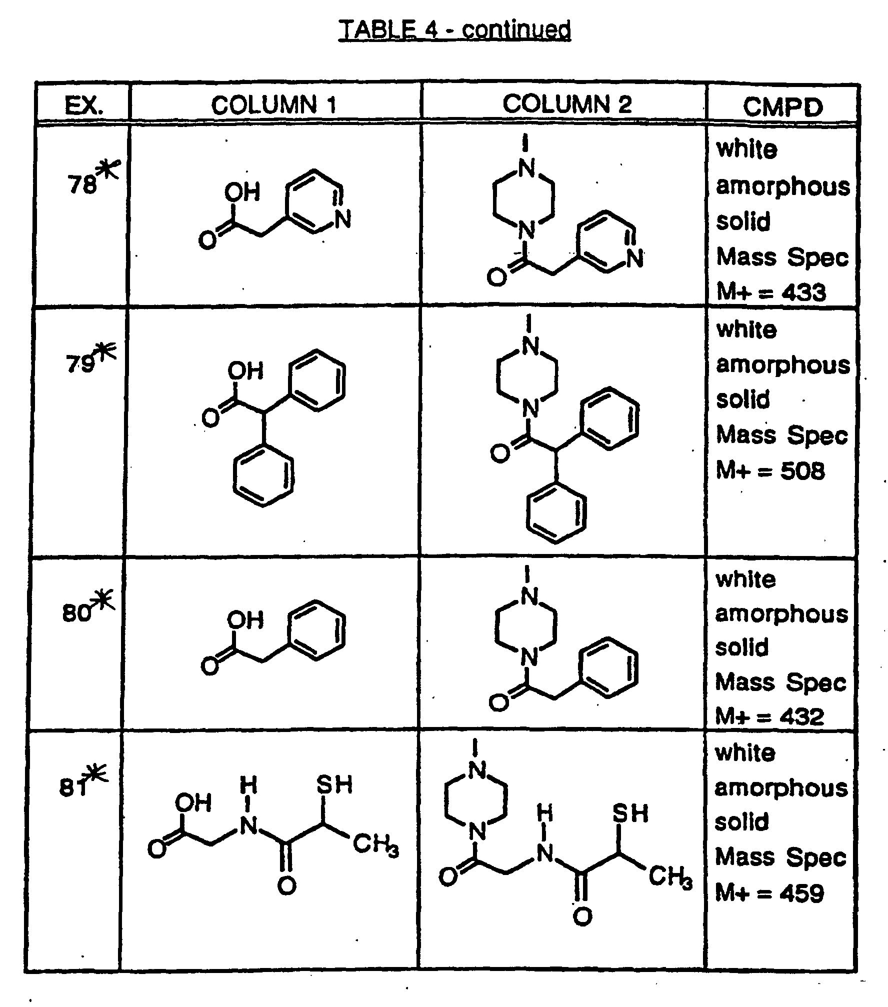

### EXAMPLE 82*

### 8-CHLORO-11-[1-(2-(4-PYRIDYL)ACETYL)-4-PIPERIDYL]-6,11-DIHYDRO-5H-BENZO[5,6]CYCLOHEPTA[1,2-b]-PYRIDINE

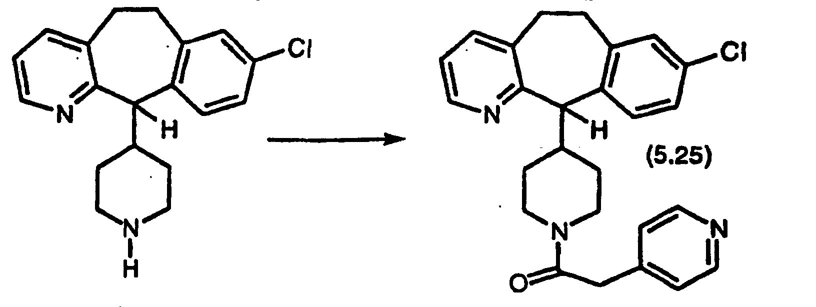

Dissolve 0.1 g (0.32 m mole) of 8-chloro-11-4-piperidyl]-6,11-dihydro-5H-benzo[5,6]cyclohepta[1,2-b]-pyridine (from Example 233), 0.06 g (0.32 m mole) of 4-pyridylacetic acid, 0.092 g (0.48 m mole) of 1-(3-dimethylaminopropyl)-3-ethylcarbodiimide hydrochloride, 0.065 g (0.48 m mole) of N-hydroxy benzotriazole and 0.048 g (0.50 m mole) of N-methyl morpholine in 5 mL of dimethylformamide and stir at room temperature for 18 hours under nitrogen. Concentrate under vacuo and partition between 100 mL each of ethyl acetate and water. Dry the organic layer over sodium sulfate and concentrate under vacuo. The resulting residue is chromatographed on silica gel using 98% dichloro methane and 2% methanol, saturated with ammonia as the solvent, giving the product as a white waxy solid, mass spec M+ = 431.

### EXAMPLE 82A*

### 8-CHLORO-11-[1-(2-(PYRIDYL)ACETYL)-4-PIPERIDYL]-6,11-DIHYDRO-5H-BENZO[5,6]CYCLOHEPTA[1,2-b]PYRIDINE

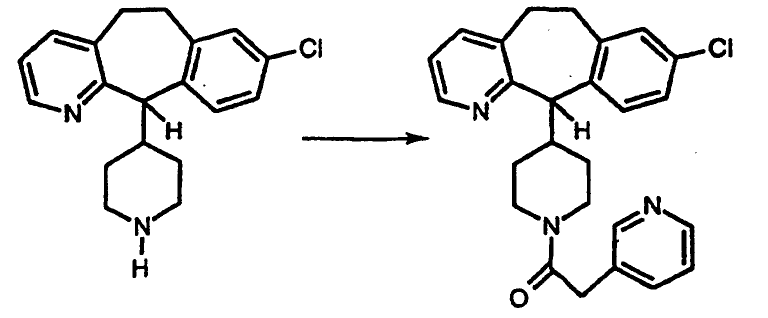

By essentially the same procedure as set forth in Example 82, but using 3-pyridylacetic acid instead of 4-pyridylacetic acid, the title compound is obtained as a white solid (M+ = 431, mp = 81.7-82°C).

### EXAMPLE 83*

### 8-CHLORO-11-[1-(2-METHYLSULFONYLOXY-1-PHENYLETHYL-CARBONYL)-4-PIPERIDYLIDENE]-6,11-DIHYDRO-5H-BENZO[5,6]-CYCLOHEPTA[1,2-b]-PYRIDINE

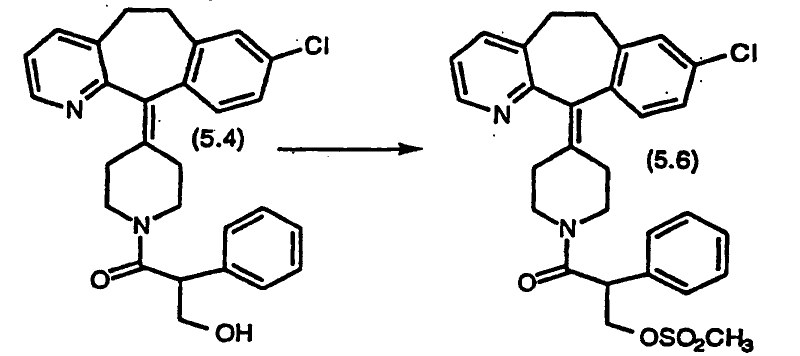

Dissolve 0.40 g (0.9 m mole) of 8-chloro-11-[1-(2-hydroxy-1-phenylethylcarbonyl)-4-piperidylidene]-6,11-dihydro-5H-benzo[5,6]-cyclohepta-[1,2-b]pyridine (Example 41 of Table 2) in 10 mL of pyridine and stir under nitrogen. Add 0.15 g (1.3 m mole) of methanesulfonyl chloride and stir for 20 hours. Concentrate under vacuo and triturate the residue with ether. Purify the resulting solid by silica gel chromatography using 2% methanol saturated with amonia, and 98% dichloromethane as the solvent. The product is obtained as a white solid, mp = 110.7-111.6° C.

### EXAMPLE 84*

### 8-CHLORO-11-[1-(2-ACETYLMERCAPTO-1-PHENYLETHYL-CARBONYL)-4-PIPERIDYLIDENE]-6,11-DIHYDRO-5H-BENZO[5,6]-CYCLOHEPTA[1,2-b]-PYRIDINE

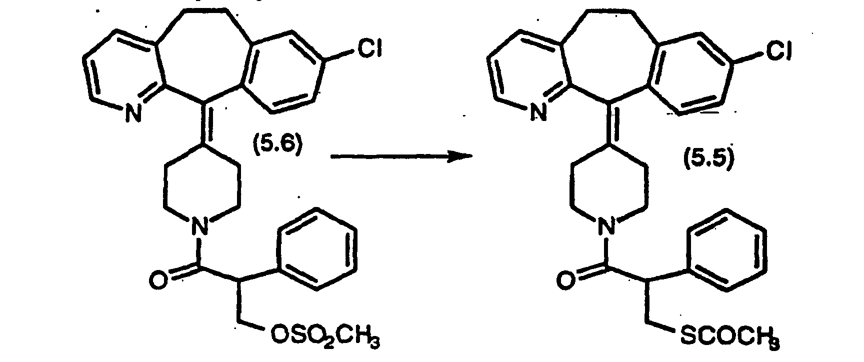

Dissolve 0.3 g (0.56 m mole) of 8-chloro-11-[1-(2-methanesulfonyloxy-1-phenylethylcarbonyl)-4-piperidylidene]-6,11-dihydro-5H-benzo[5,6]cyclohepta-[1,2-b]pyridine (Formula 5.6 of Example 83) in 5 mL of dimethylformamide and add 0.2 g (0.6 m mole) of cesium thioacetate (preparation described in Synthetic Communications, 13, 553, 1983). Stir the reaction at 80°C for twenty hours then concentrate under vacuo. Purify the residue by silica gel chromatography using 70% ethyl acetate and 30% hexane as the solvent. The product is obtained as a light brown solid, mp = 92.7-93°C.

### EXAMPLE 85*

### 8-CHLORO-11-[1-(1-(2,3-DIHYDRO-3-OXO-1,2-BENZOISO-THIAZOL-S,S-DIOXIDE-2-YL)METHYLCARBONYL)-4-PIPERIDYLIDENE]-6,11-DIHYDRO-5H-BENZO[5,6]CYCLOHEPTA[1,2-b]-PYRIDINE

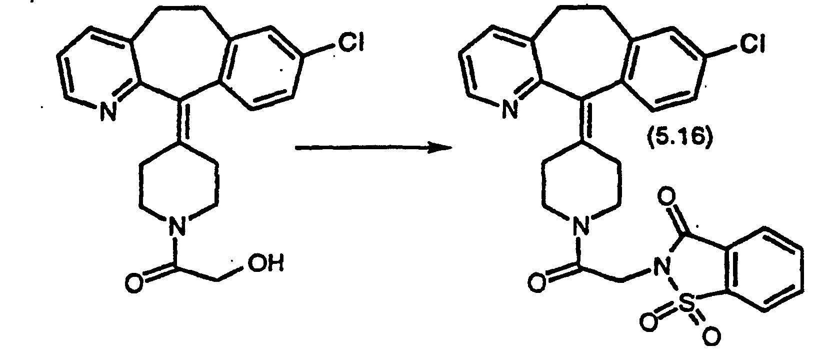

Dissolve 0.46g (1.7 m mole) of 8-chloro-11-[1-(2-hydroxyethylcarbonyl)-4-piperidylidene]-6,11-dihydro-5H-benzo[5,6]cyclohepta[1,2,b]pyridine (Example 49a of Table 2) in 30 mL of dimethylformamide and stir at 0°C under nitrogen. Add 0.55 g (2.1 m mole) of triphenyl phosphine and 0.36 g (2.1 m mole) of diethyl azodicarboxylate. Stir reaction mixture at 70°C for 3 days, then concentrate under vacuo. The residue was dissolved in 50 mL of 1 N hydrochloric acid and washed with 100 mL of ethyl acetate. The water layer was neutralized with 1 N sodium hydroxide and the mixture was extracted with ethyl acetate. The organic layer was dried over magnesium sulfate and concentrated under vacuo. The residue was purified by silica gel chromatography using 90% ethyl acetate and 10% hexane as the solvent, giving the product as a white solid, mass spec. M+ = 534.

### EXAMPLE 86*

### 8-CHLORO-11-[1-(1-(3-PYRIDYL)METHYLTHIOCARBONYL)-4-PIPERIDYLIDENE]-6,11-DIHYDRO-5H-BENZO[5,6]CYCLOHEPTA[1,2-b]-PYRIDINE

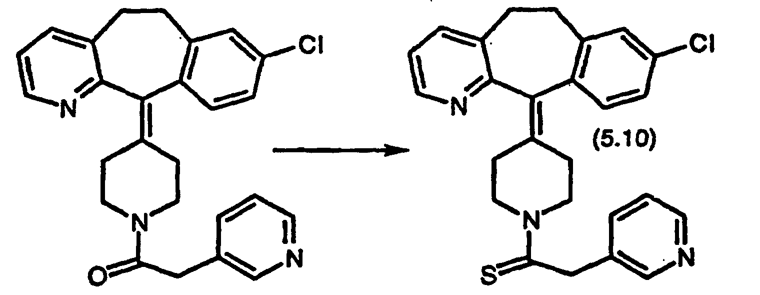

Dissolve 0.50 g (0.12 m mole) of 8-chloro-11-[1-(1-(3-pyridyl)-methylcarbonyl)-4-piperidylidene]-6,11-dihydro-5H-benzo[5,6]cyclohepta[1,2-b]-pyridine (Example 2 of Table 2) and 0.5 g (0.12 m mole) of 2,4-bis(4-methoxyphenyl)-1,3-dithia-2,4-diphosphetane-2,4-disulfide (Lawesson's Reagent) in 15 mL of toluene and stirr for 18 hours at room temperature and 18 hours at 80°C, under nitrogen. Filter the reaction mixture and concentrate under vacuo. Disolve the residue in 50 mL of 1N hydrochloric acid and extract with 200 mL of dichloromethane. The water layer was neutralized with sodium carbonate and extracted with dichloromethane. The organic layer was dried over magnesium sulfate and concentrated under vacuo giving the product as a white solid, mp = 92-93°C.

### EXAMPLE 87*

### 10,11-DIHYDRO-5-[1-(1-(4-PYRIDYL)METHYLCARBONYL)-4-PIPERIDYLIDENE]-5H-DIBENZO[a,d]CYCLOHEPTENE

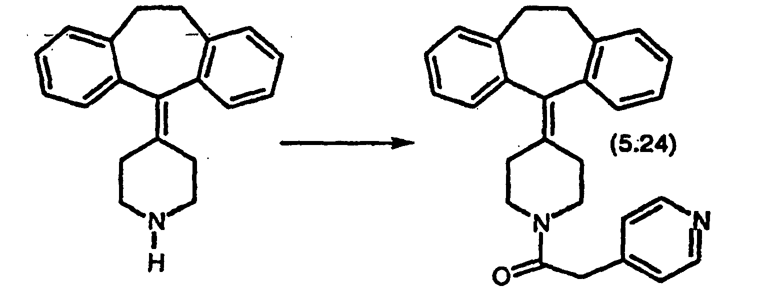

Dissolve 0.15 g (0.6m mole) of 10,11-dihydro-5-(4-piperidylidene)-5H-dibenzo[a,d]cycloheptene, 0.096 g (0.55 m mole) of 4-pyridylacetic acid hydrochloride, 0.16 g (0.83 m mole) of 1-(3-dimethylaminopropyl)-3-ethylcarbodiimide hydrochloride and 0.075 g (0.55 m mole) of N-hydroxy benzotriazole in 5 mL of dimethylformamide and stir at room temperature for 18 hours under nitrogen. Concentrate under vacuo and partition between 100 mL each of ethyl acetate and 10% aqueous sodium hydrogenphosphate. Dry the organic layer over magnesium sulfate and concentrate under vacuo. The resulting residue is chromatographed on silica gel using 98% dichloro methane and 2% methanol, saturated with ammonia as the solvent, giving the product as a white waxy solid, mp = 162.8-163.4 °C.

### EXAMPLE 180*

### 1-1-(4-PYRIDINYLACETYL)-4-[3,8-DICHLORO-5,6-DIHYDRO-11H-BENZO[5,6]CYCLOHEPTA[1,2-b]PYRIDIN-11-YLIDENE]-PIPERIDINE

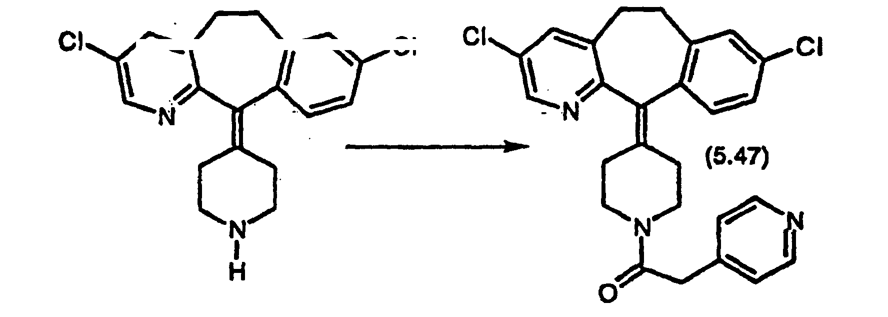

Dissolve 0.18 g (0.51 mmole) of 3,8-dichloro 11-(1-acetyl-4-piperidylidene)-6,11-dihydro-5H-benzo[5,6]cycohepta[1,2-b]pyridine, 0.088g (0.51 mmole ) 4-pyridylacetic acid, 0.117g (0.61 mmole) of 1-(3-dimethylaminopropyl)-3-ethylcarbodiimide hydrochloride, 0.082g (0.61 mmole) N-hydoxybenzotriazole and 0.071g (0.71 mmole) N-methyl morpholine in 5 mL of dimethylformamide and stir for 18 hours under nitrogen. Concentrate under vacuo and partion between ethyl acetate and water. Dry organic layer over sodium sulfate and concentrate in vacuo. The resulting residue is chromatogaphed on silica gel using 95% dichloromethane and 5% methanol, saturated with ammonia as the solvent. The product is obtained as white solid, mp = 113-114 °C.

### EXAMPLE 181*

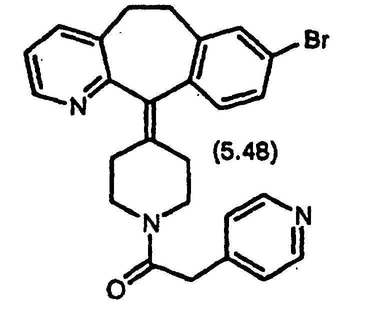

By essentially the same procedure as set forth in Exampe 180, but using 8-bromo-11-(1-acetyl-4-piperidylidene)-6,11-dihydro-5H-benzo[5,6]cyclohepta[1,2-b]pyridine instead of 3,8-dichloro-11-(1-acetyl-4-piperidylidene)-6,11-dihydro-5H-benzo[5,6]-cyclohepta[1,2-b]pyridine, compound 5.48 was obtained as an off-white solid, mp= 94.3-94,7 °C.

### EXAMPLE 182

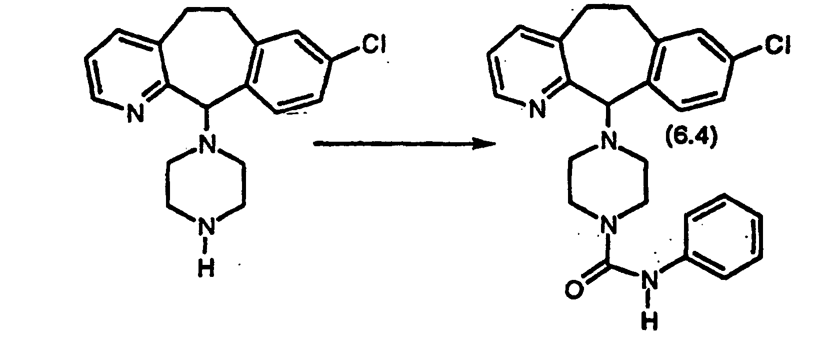

To a stirred solution of phenyl isocyanate (1.27 mmole) in 15 ml of anhydrous methylene chloride at room temperature and under an argon atmosphere was added dropwise over 20 minutes, a solution of 8-chloro-11-(1-piperazinyl)-6,11-dihydro-5H-benzo[5,6]cyclohepta[1,2-b]pyridine (1.27 mmole) in 5 ml of anhydrous methylene chloride. The mixture was stirred at room temperature for 20 hours. The mixture was poured into 700 ml of methylene chloride and washed with 100 ml of saturated sodium bicarbonate. The organic portion was dried over magnesium sulfate, filtered, and concentrated in vacuo. The residue was purified by silica gel chromatography using a solution of 1.0% (10% ammonium hydroxide in methanol) in methylene chloride. The product was obtained as a white amorphous solid, M.S. (Mass Spec) M+ = 433.

### EXAMPLE 183

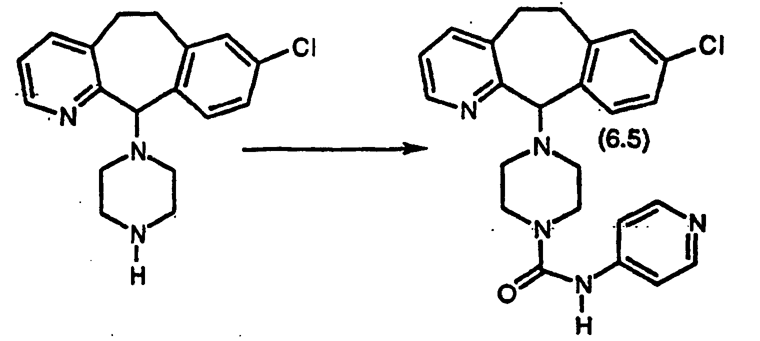

To a 5.0 ml reaction vial was added 8-chloro-11-(1-piperazinyl)-6,11-diydro-5H-benzo[5,6]cyclohepta[1,2-b]pyridine (1.0 mmole) and N-ethoxycarbonyl-4-aminopyridine (0.99 mmole). The vial was capped and placed in an oil bath at 170 °C and stirred for 5 hours. The residue was purified by silica gel chromatography using a solution of 3.0% (10% ammonium hydroxide in methanol) in methylene chloride. The product was obtained as a white amorphous solid, M.S. (Mass Spec) M+ = 434.

### EXAMPLE 184*

### 1-(8-CHLORO-6,11-DIHYDRO-5H-BENZO[5,6]CYCLOHEPTA [1,2-b]PYRIDIN-11-YL)-4-(3-PYRIDINYLACETYL)piperazine 1-N-OXIDE

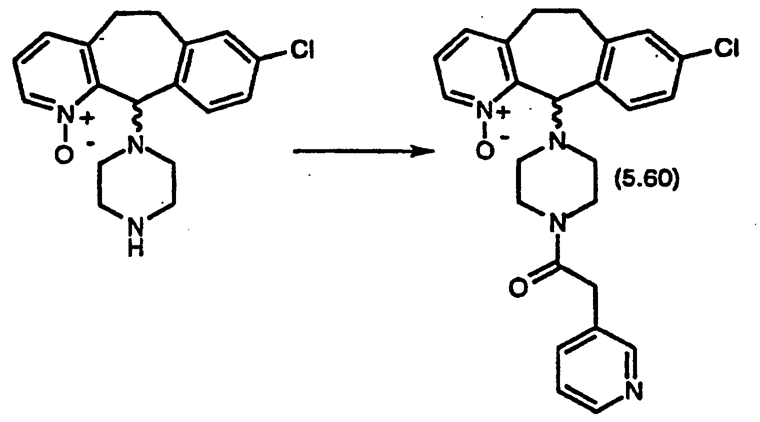

The title compound from Preparative Example 11D (0.5 grams) (1.5 mmoles) was reacted with 3-pyridylacetic acid (0.208 grams) (1.5 mmoles) under the conditions described in Example 75 to give the title compound (Yield: 0.439 grams, 95%, MH⁺ 449).

### EXAMPLE 185*

### 1-(8-CHLORO-6,11-DIHYDRO-5H-BENZO[5,6]CYCLOHEPTA [1,2-b]PYRIDIN-11-YL)-4-(3-PYRIDINYLACETYL 1-N-OXIDE)PIPERAZINE 1-N-OXIDE

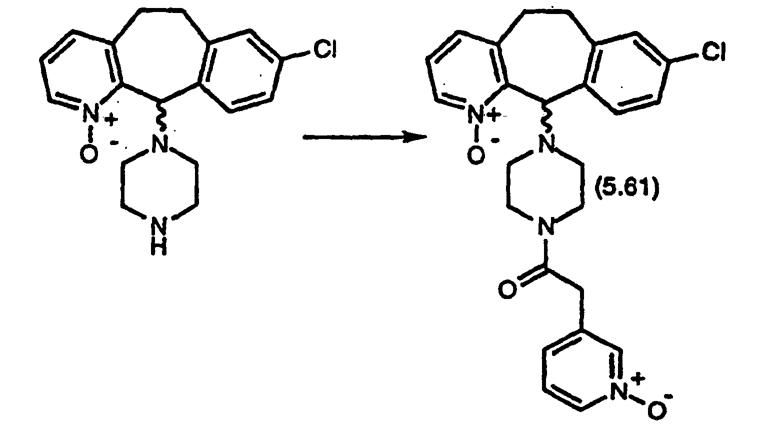

The title compound from Preparative Example 11D (0.5 grams) (1.5 mmoles) was reacted with the title compound from Preparative Example 8 (0.232 grams) (1.5 mmoles) under the conditions described in Example 75 to give the title compound (Yield: 0.6454 grams, 92%, MH⁺ 465.2).

### EXAMPLE 186*

### N-BENZYL 4-(8-CHLORO-6,11-DIHYDRO-5H-BENZO[5,6]CYCLO-HEPTA[1,2-b]PYRIDIN-11-YL)-1-PIPERAZINECARBOXAMIDE

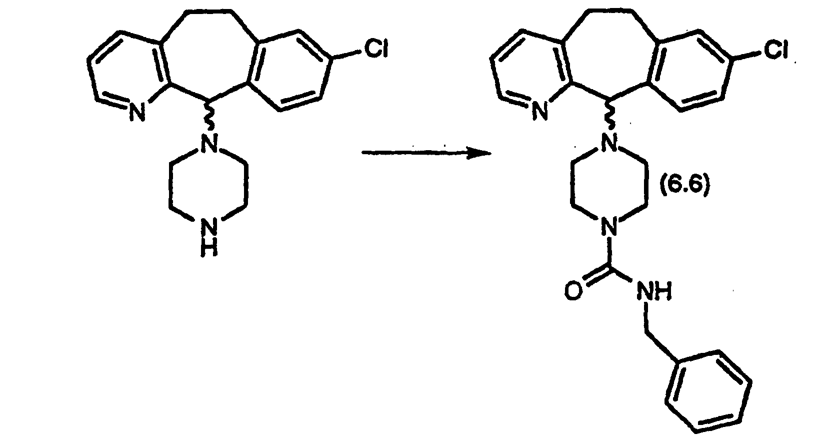

The title compound from Example 75 was reacted with benzyl isocyanate under the conditions described in Example 182 above to give the title compound (Yield: 79%, MH⁺ 447).

### EXAMPLE 187

By essentially the same procedure as Example 183, with the exception that 3-ethoxycarbonylaminopyridine or 2-ethoxycarbonylaminopyridine (Preparative Example 12) is used instead of using 4-ethoxycarbonylaminopyridine, the compound 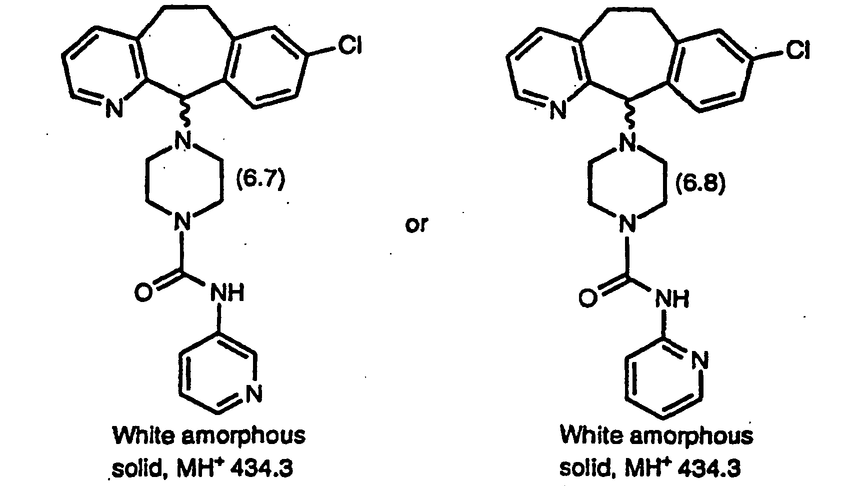 was obtained, respectively.

### EXAMPLE 188

### 4-(8-CHLORO-6,11-DIHYDRO-5H-BENZO[5,6]CYCLOHEPTA[1,2-b]PYRIDIN-11-YL)-N-METHYL-N-(3-PYRIDINYL)-1-PIPERAZINE-CARBOXAMIDE

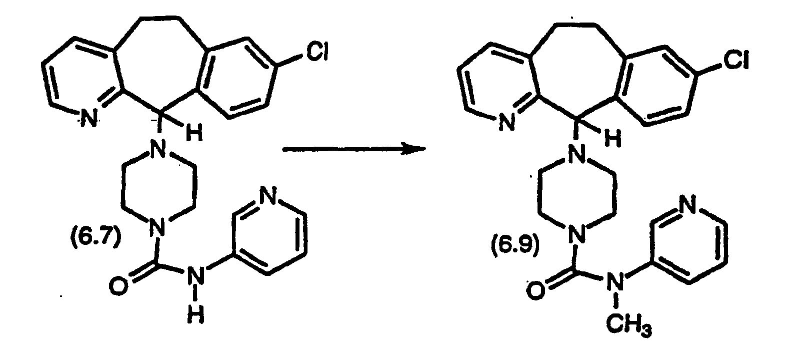

Compound 6.7 from Example 187 (10 grams) (23.1 mmoles) in DMSO (37.6 ml.) was added to a solution of powdered potassium hydroxide (2.62 grams) (23.1 mmoles) in DMSO (25 ml.) and the mixture was stirred at 25°C for 3 minutes. lodomethane (1.4518 ml.) (23.1 mmoles) was added and the mixture was stirred at 25°C for 15 minutes. The mixture was poured into water and extracted with dichloromethane. The latter was dried (magnesium sulphate), filtered and evaporated to dryness. The product was purified by chromatography on silica gel using 3-5%(10% concentrated ammonium hydroxide in methanol)-dichloromethane as the eluant to give the title compound (Yield: 6.28 grams, 61%, MH⁺ 448).

### EXAMPLES 189 - 218

By essentially the same procedures as set forth in Example 75 above but using the compounds set forth in Column 1, Table 5 below, in place of 4-pyridylacetic acid, one can obtain compounds of the formula 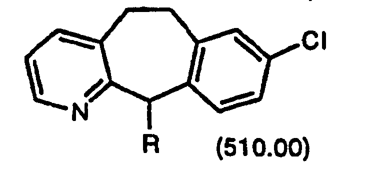 wherein R is as listed in Column 2 of Table 5.

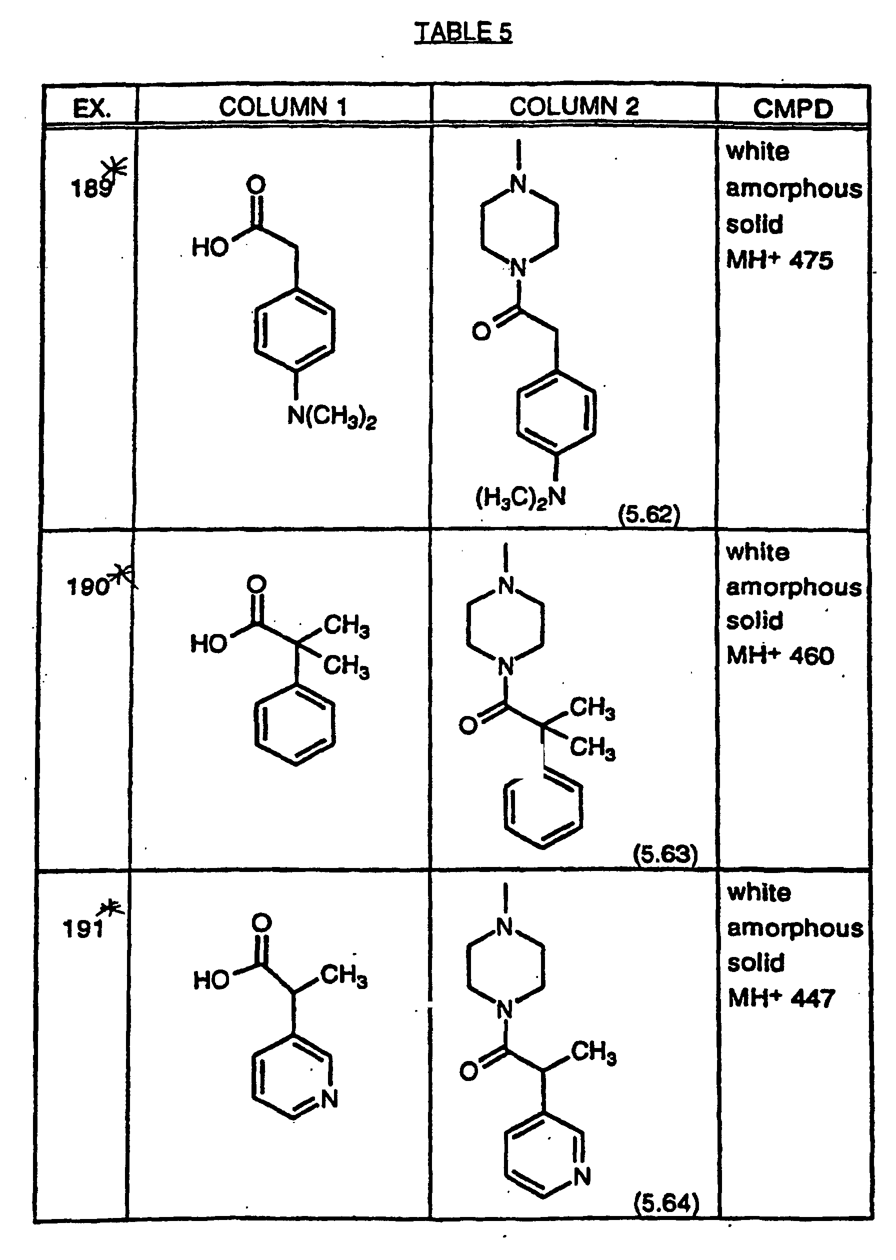

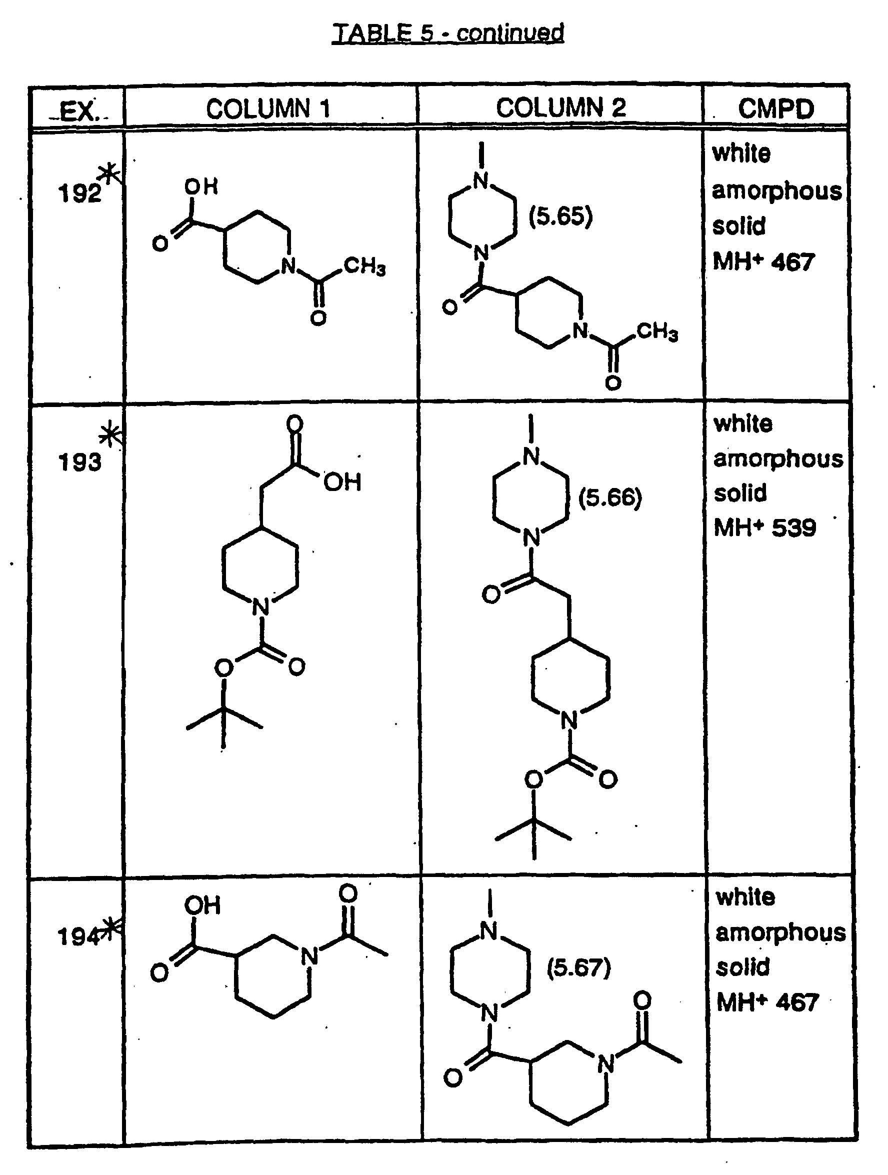

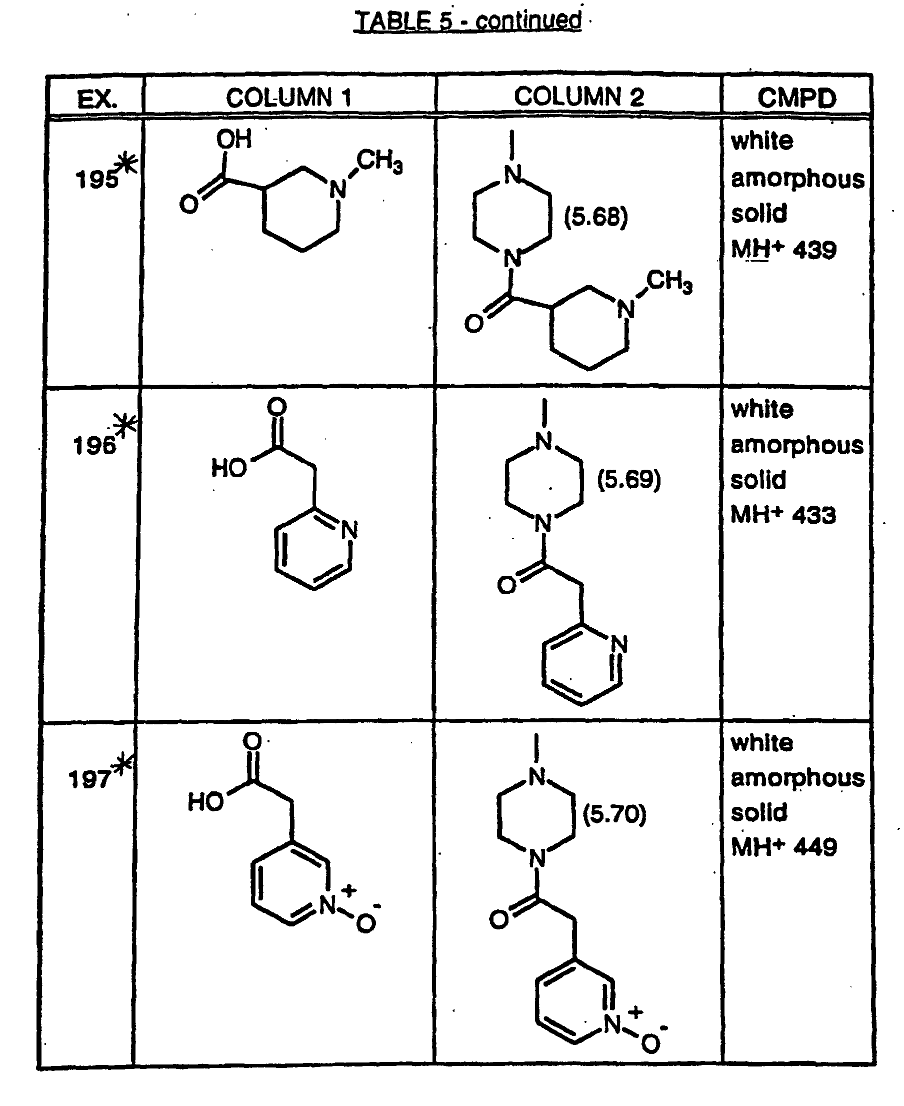

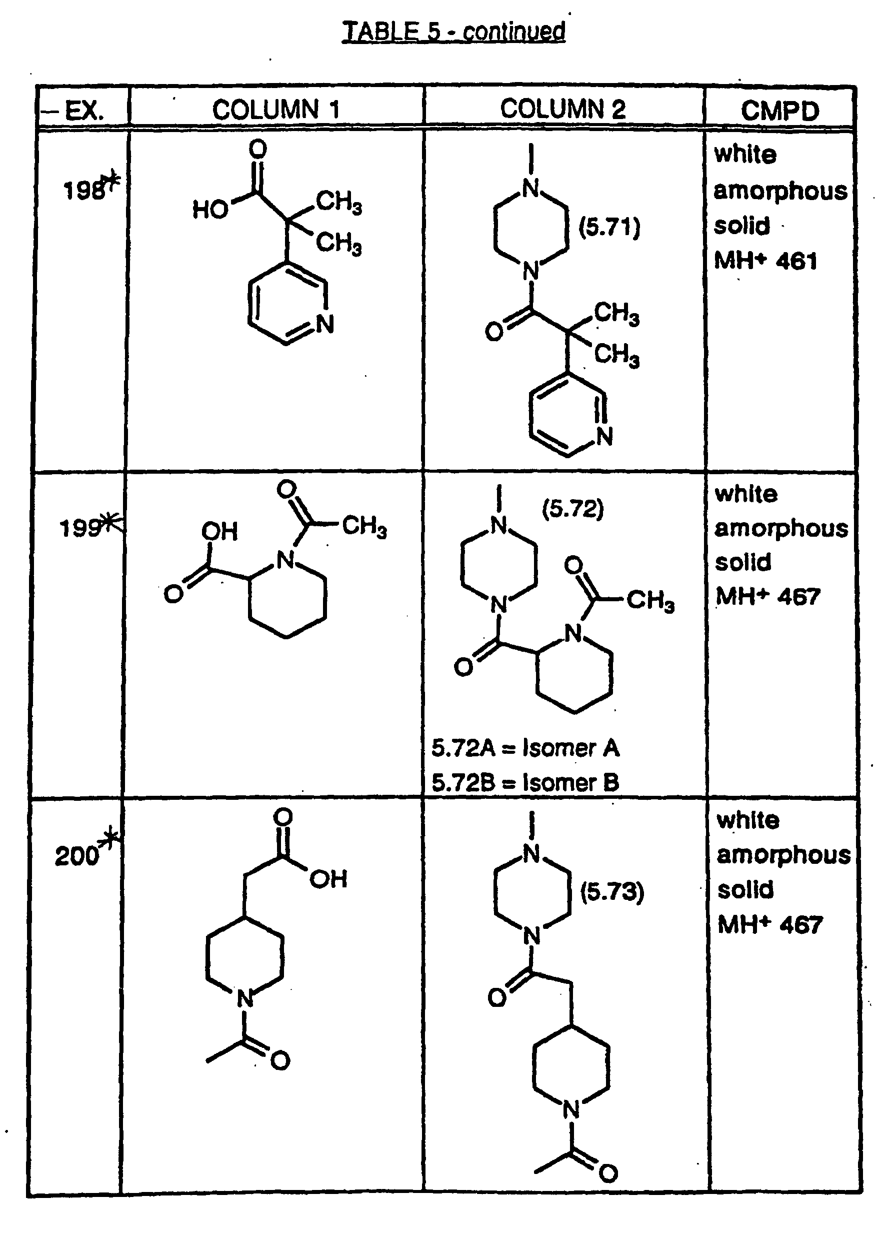

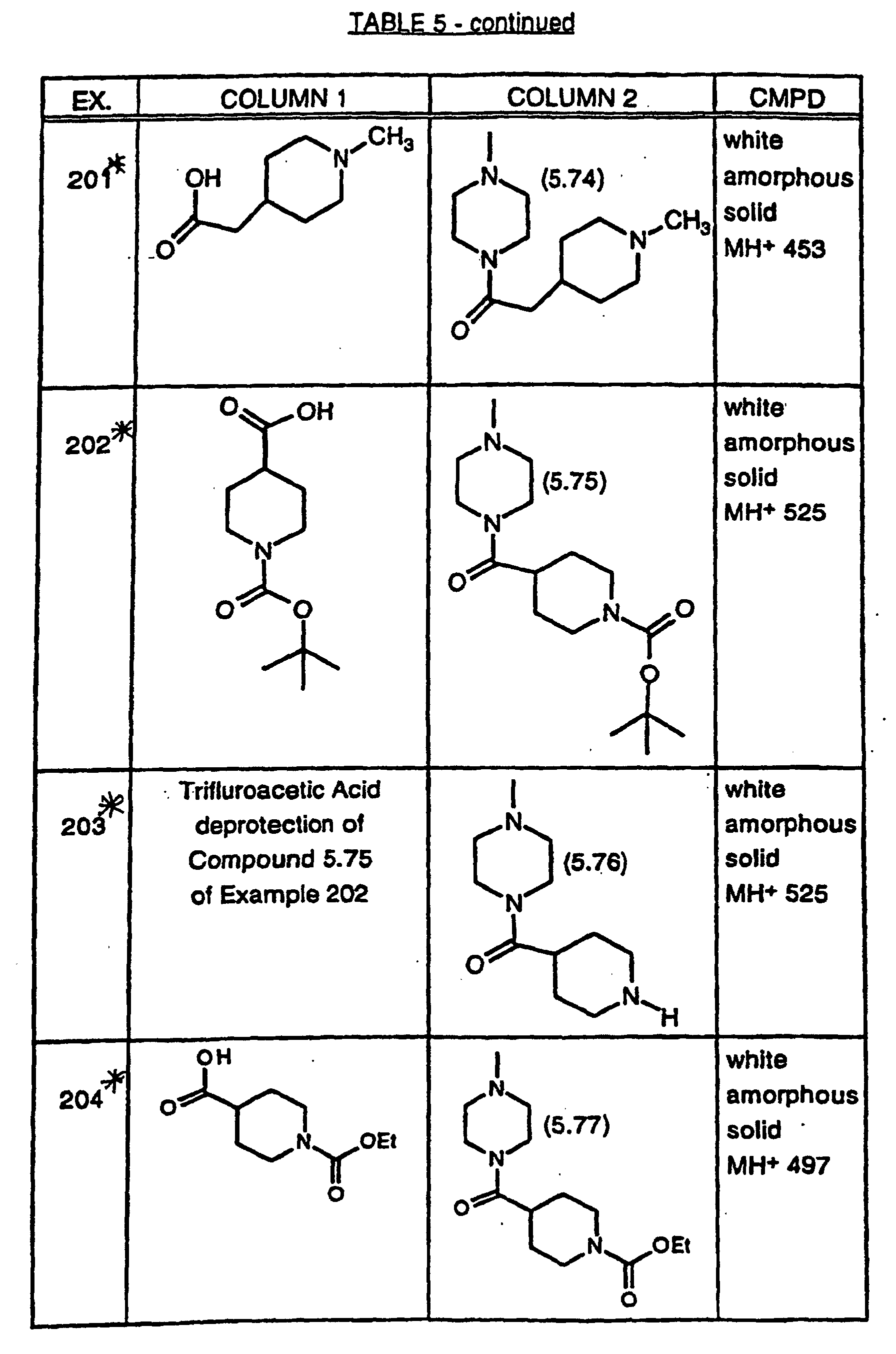

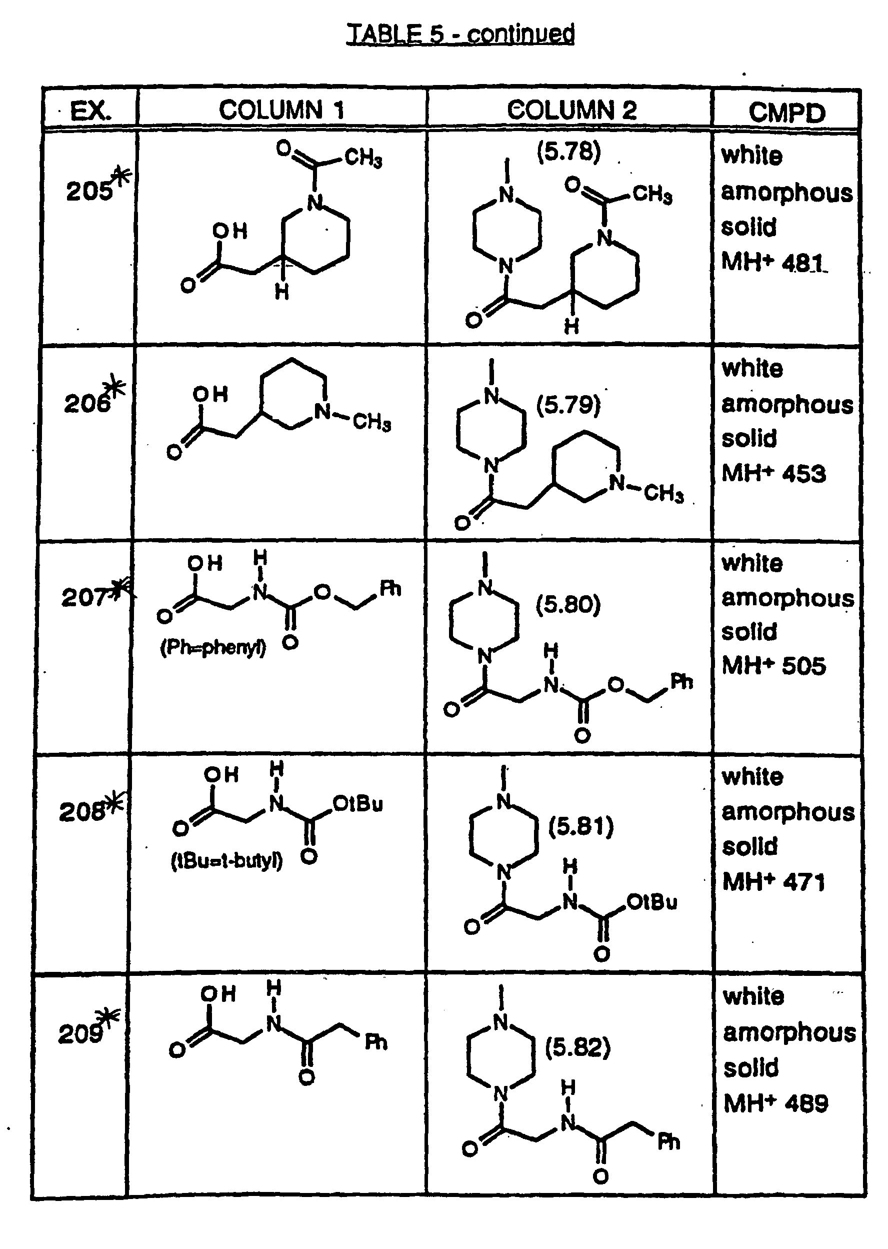

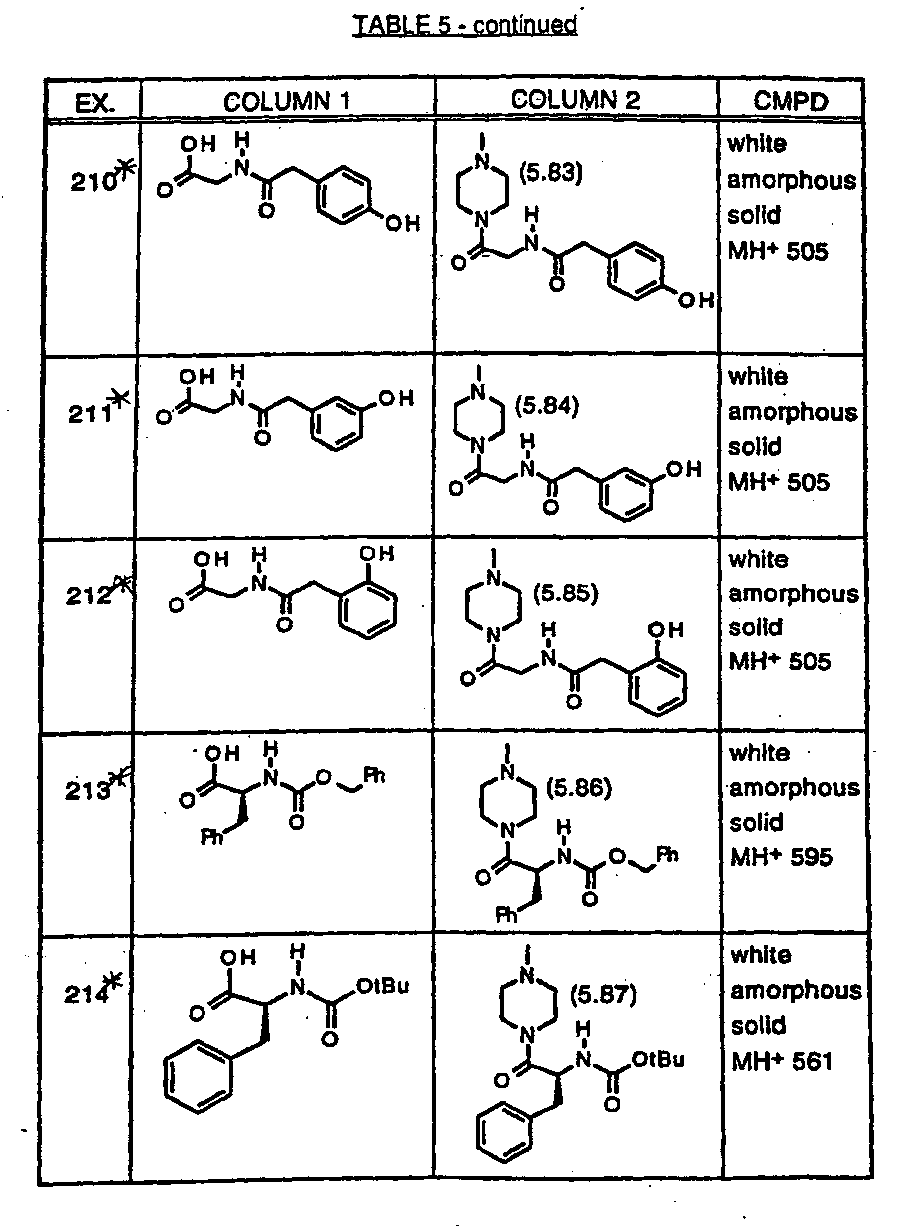

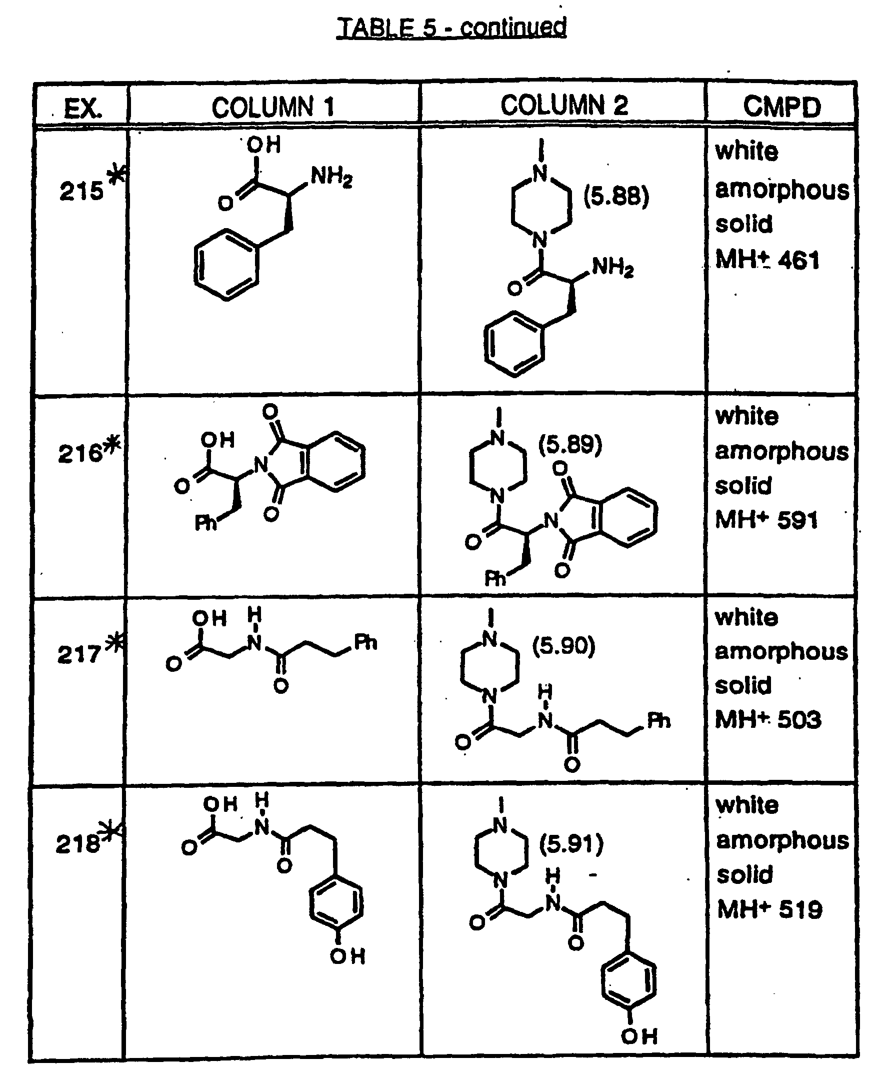

### EXAMPLE 219 - 222

By essentially the same procedure as set forth in Example 1, but using the acids set forth in Column 1 of Table 6 below in place of 4-pyridylacetic acid, the compounds listed in Column 2 of Table 6 are obtained. The compounds listed in Table 6 refer to compounds of Formula 500.00: 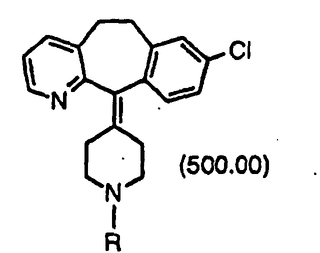 wherein R is the substituent in Column 2.

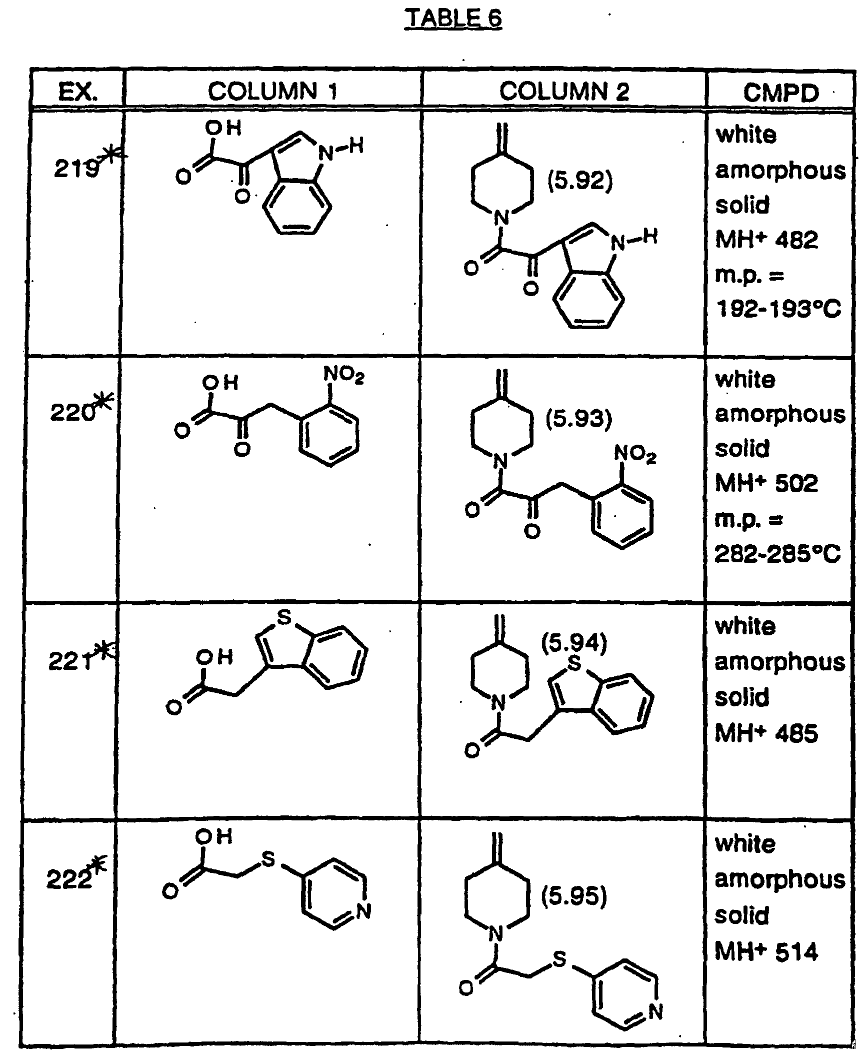

### EXAMPLE 223*

### A. (+)-1-(8-CHLORO-5,6-DIHYDRO-11H-BENZO[5,6]CYCLO-HEPTA[1,2-b]PYRIDIN-11(R)-YL)-4-(3-PYRIDINYLACETYL)PIPERAZINE

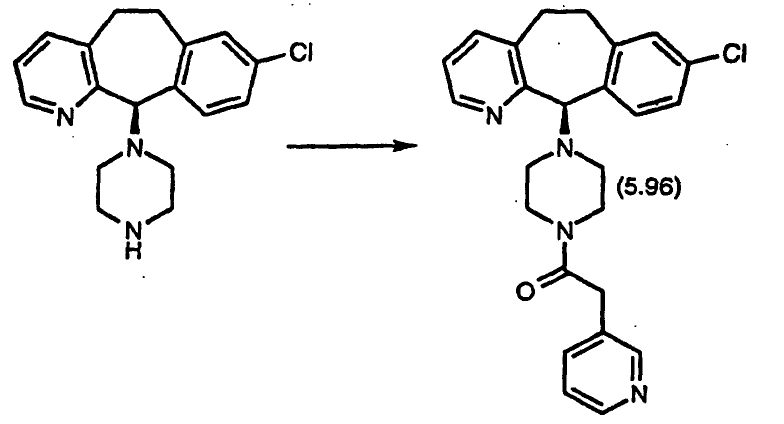

The title R(+) diastereoisomer from Preparative Example 19 was reacted with 3-pyridylacetic acid under the same conditions as described in Example 75 to give the title compound (Yield: 88%, MH⁺ 433).

### B. (-)-1-(8-CHLORO-5,6-DIHYDRO-11H-BENZO[5,6]CYCLO-HEPTA[1,2-b]PYRIDIN-11(S)-YL)-4-(3-PYRIDINYLACETYL)PIPERAZINE

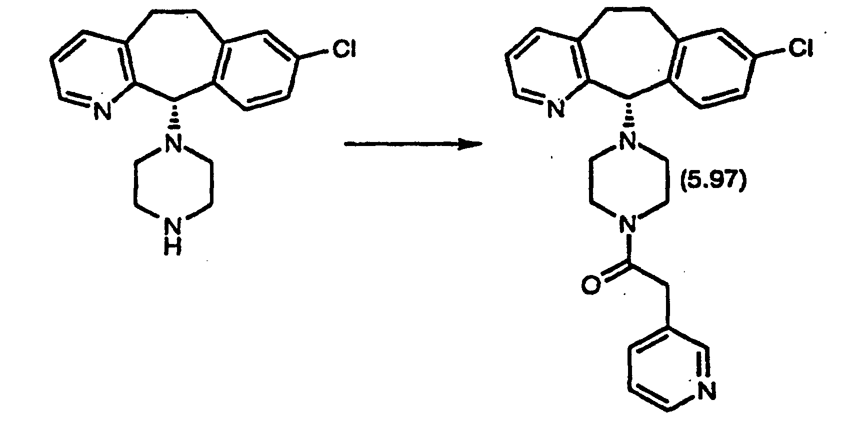

The title S(-) diastereoisomer from Preparative Example 19 above was reacted with 3-pyridylacetic acid under the same conditions as described in Example 75 to give the title compound (Yield: 96%, MH⁺ 433).

### EXAMPLE 224

### A. (+)-4-(8-CHLORO-6,11-DIHYDRO-5H-BENZO[5,6]CYCLO-HEPTA[1,2-b]PYRIDIN-11(R)-YL)-N-(3-PYRIDINYL)-1-PIPERAZINE-CARBOXYLATE

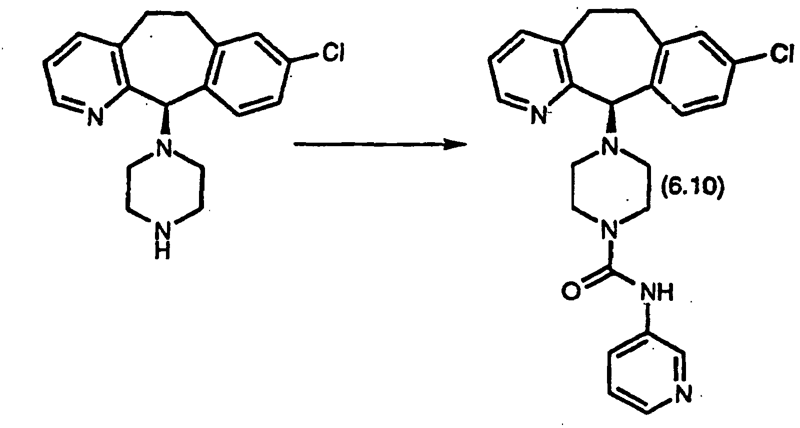

The title R(+) diastereoisomer from Preparative Example 19 was reacted with 3-ethoxycarbonylaminopyridine under the same conditions as described in Example 75 to give the title compound (Yield: 81%, MH⁺ 434).

### B. (-)-4-(8-CHLORO-6,11-DIHYDRO-5H-BENZO[5,6]CYCLO-HEPTA[1,2-b]PYRIDIN-11(S)-YL)-N-(3-PYRIDINYL)-1-PIPERAZINE-CARBOXAMIDE

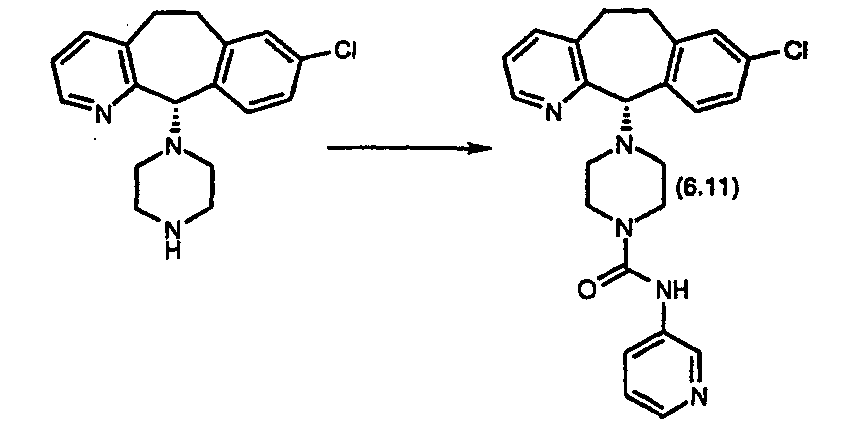

The title S(-) diastereoisomer from Preparative Example 19 was reacted with 3-ethoxycarbonylaminopyridine under the same conditions as described in Example 75 to give the title compound (Yield: 80%, MH⁺ 434).

### EXAMPLE 225*

### A. (+)-1-(8-CHLORO-5,6-DIHYDRO-11H-BENZO[5,6]CYCLO-HEPTA[1,2-b]PYRIDIN-11(R)-YL)-4-[(1-ACETYL-4-PIPERIDINYL)-ACETYL]PIPERAZINE

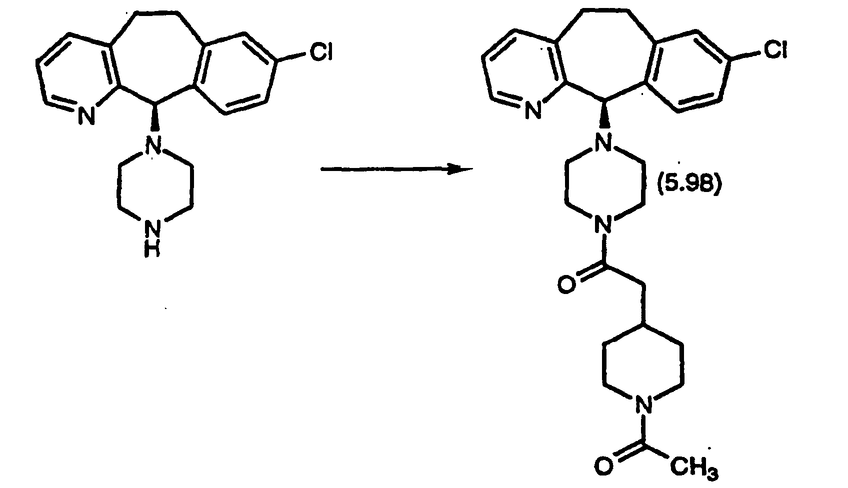

The title R(+) diastereoisomer from Preparative Example 19 above was reacted with 1-N-acetylpiperidinyl-3-acetic acid under the same conditions as described in Example 75 to give the title compound (Yield: 52%, MH⁺ 481).

### B. (-)-1-(8-CHLORO-5,6-DIHYDRO-11H-BENZO[5,6]CYCLO-HEPTA[1,2-b]PYRIDIN-11(S)-YL)-4-[(1-ACETYL-4-PIPERIDINYL)-ACETYL]PIPERAZINE

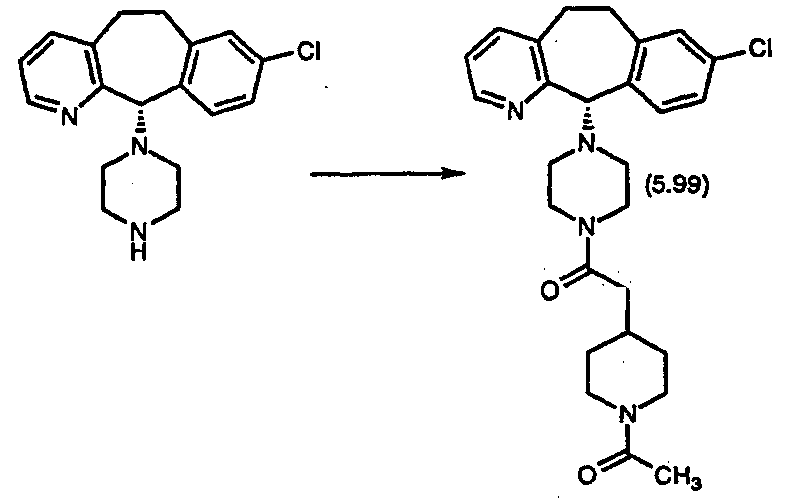

The title S(-) diastereoisomer from Preparative Example 19 above was reacted with 1-N-acetylpiperidinyl-3-acetic acid under the same conditions as described in Example 75 to give the title compound (Yield: 53%, MH⁺ 481).

### EXAMPLE 226*

### A. (+)-1-(8-CHLORO-6,11-DIHYDRO-5H-BENZO[5,6]CYCLO-HEPTA[1,2-b]PYRIDIN-11(R)-YL)-4-[(1-ACETYL-4-PIPERIDINYL)-CARBONYL]PIPERAZINE

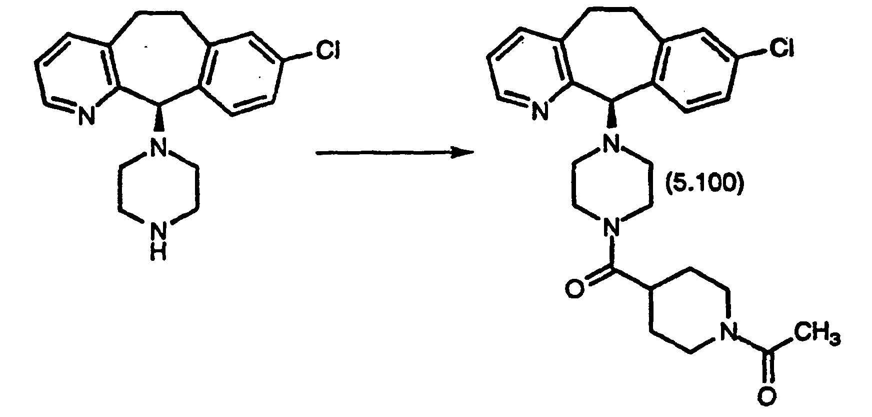

The title R(+) diastereoisomer from Preparative Example 19 was reacted with 1-N-acetylisonipecotic acid under the same conditions as described in Example 75 to give the title compound (Yield: 90%, MH⁺ 467).

### B. (-)-1-(8-CHLORO-6,11-DIHYDRO-5H-BENZO[5,6]CYCLO-HEPTA[1,2-b]PYRIDIN-11(S)-YL)-4-[(1-ACETYL-4-PIPERIDINYL)-CARBONYL]PIPERAZINE

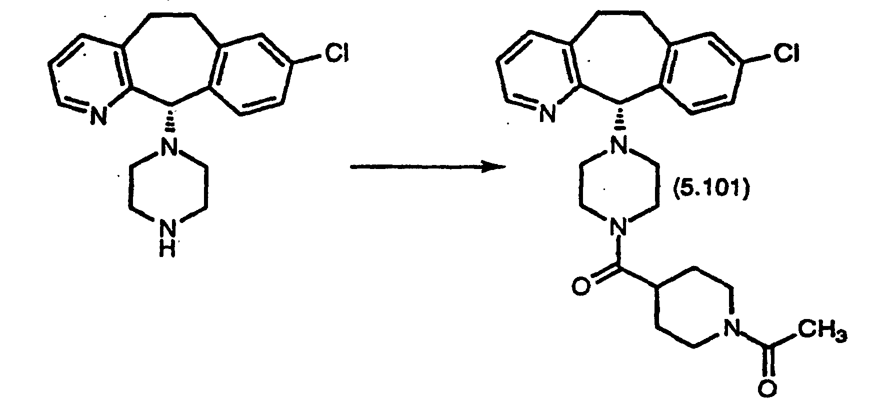

The title S(-) diastereoisomer from Preparative Example 19 was reacted with 1-N-acetylisonipecotic acid under the same conditions as described in Example 75 to give the title compound (Yield: 93%, MH⁺ 467).

### EXAMPLE 227*

### 4-(8-CHLORO-5,6-DIHYDRO-11H-BENZO-[5,6]CYCLOHEPTA[1,2-b]PYRIDIN-11-YLIDENE)-1-[(4-PYRIDINYL)ACETYL]- PIPERIDINE N1 OXIDE

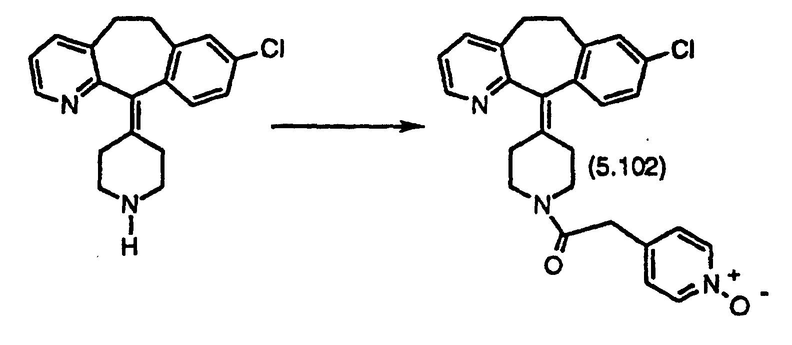

To a mixture of 0.933g(3 mmol) of 4-(8-chloro-5,6-dihydro-11H-benzo-[5,6]cyclohepta(1,2-b]pyridin-11-ylidene)-piperidine (product from Preparative Example 1, step G), 0.46g(3 mmol) of 4-pyridyl acetic acid N-oxide (prepared as described in Preparative Example 8) 1-hydroxybenzotriazole (0.40g, 3 mmol) in 20 mL of DMF at ∼ 4°C and under nitrogen atmosphere was added N- methyl morpholine(1.65 mL, 15 mmol) followed by 1-(3-dimethylaminopropyl)-3-ethylcarbodiimide hydrochloride salt (DEC) an reaction stirred overnight at room temperature. The volatiles were stripped off and the resulting semi-solid was partitioned between water and ethyl acetate. The aqueous phase was washed twice with ethyl acetate. Combined ethyl acetate fractions were dried over Na₂SO₄ and concentrated. The crude product was purified via flash chromatography on silica gel (first eluting with 3% and then 5% methanol saturated with ammonia in methylene chloride) to give the title compound as a light brown solid(0.2g mp=128-130 °C MH⁺446).

### EXAMPLE 228*

### 4-(8-CHLORO-5,6-DIHYDRO-11H-BENZO-[5,6]CYCLOHEPTA[1,2-b]PYRIDIN-11-YLIDENE)-1-[(3-PYRIDINYL)ACETYL]-PIPERIDINE N1 OXIDE

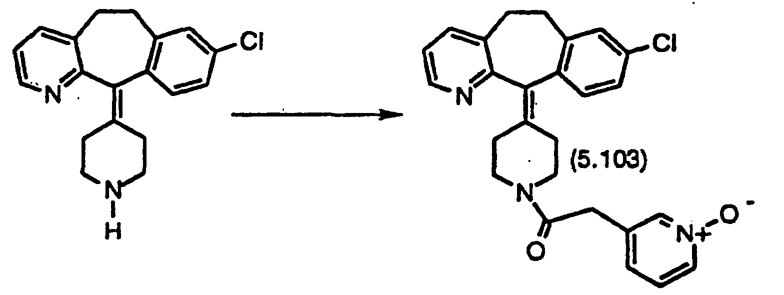

By essentially the same procedure as set forth in Example 227, but using 3-pyridyl acetic acid N-oxide (Preparative Example 9) instead of 4-pyridyl acetic acid N-oxidethe title compound was obtained as a white solid (mp = 120-121°C, MH⁺=466).

### EXAMPLE 229

### 4-(8-CHLORO-5,6-DIHYDRO-11H-BENZO-[5,6]CYCLOHEPTA[1,2-b]PYRIDIN-11-YLIDENE)-1-[(3-PYRIDINYL)ACETYL]- PIPERIDINE N4 OXIDE

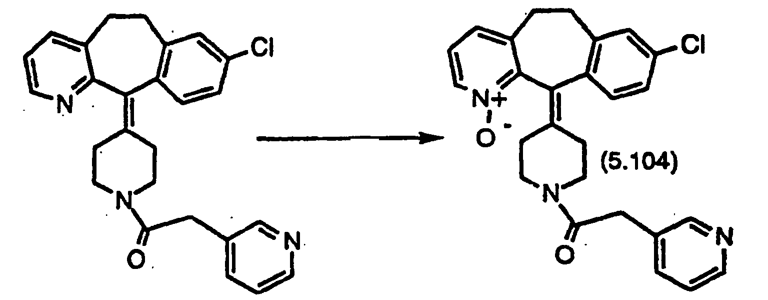

4-(8-chloro-5,6-dihydro-11H-benzo-[5,6]cyclohepta[1,2-b]pyridin-11-ylidene)-1-[(3-pyridinyl)acetyl]-piperidine (1.0g, 2.33mmol) was dissolved in dry methylene chloride(50mL) at -10°C. 3-Chloroperbenzoic acid (80-85% purity 1.1g, 5.13 mmol) was added and the reaction stirred at that temperature for 95 minutes. The reaction mixture was washed with sodium bisulfite and then with 10% NaOH. The organic phase was dried over magnesium sulfate and then concentrated. Purification on silica gel eluting, first with 4%, 6% and then 10% methanol in methylene chloride gave rise to the title compound as a white solid (0.2g, 0.77 mmol MH⁺=446).

### EXAMPLE 230*

### 4-(8-CHLORO-5,6-DIHYDRO-11H-BENZO-[5,6]CYCLOHEPTA[1,2-b]PYRIDIN-11-YLIDENE)-1-[(4-PYRIDINYL)ACETYL]- PIPERIDINE N4 OXIDE

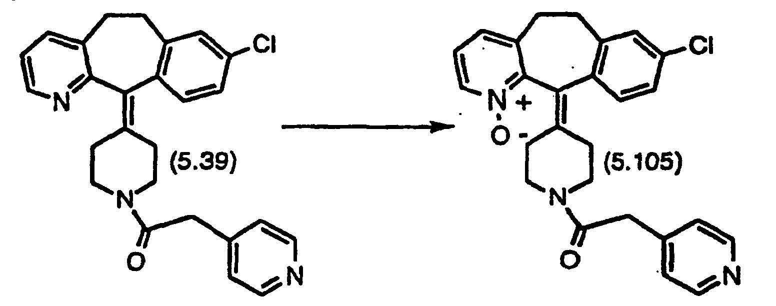

By essentially the same procedures as set forth in Example 229 above, but using 4-(8-chloro-5,6-dihydro-11H-benzo-[5,6]cyclohepta[1,2-b]pyridin-11-ylidene)-1-[(4-pyridinyl)acetyl]-piperidine instead of 4-(8-chloro-5,6-dihydro-11H-benzo-[5,6]cyclohepta[1,2-b]pyridin-11-ylidene)-1-[(3-pyridinyl)acetyl]-piperidine the title compound was obtained as an off-white solid (MH⁺=446).

### EXAMPLE 231*

### A. 8-CHLORO-11-(1-ETHOXYCARBONYL-4-PIPERIDYLIDENE)-6,11-DIHYDRO-5H-BENZO[5,6]CYCLOHEPTA[1,2-b]PYRIDINE N-OXIDE

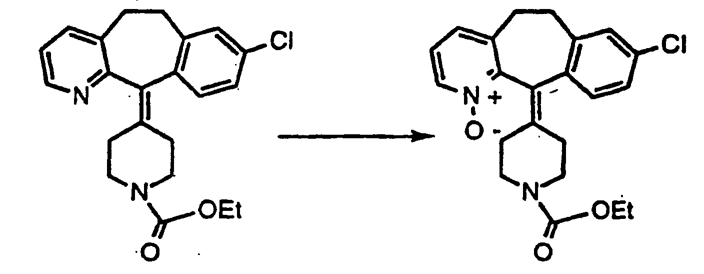

8-chloro-11-(1-ethoxycarbonyl-4-piperidylidene)-6,11-dihydro-5H-benzo[5,6]cyclohepta[1,2-b]pyridine (5g, 13.06 mmol) was dissolved in methylene chloride at -10°C. 3-Chlorobenzoic acid(4.9g, 15.67 mmmol) was then added and the reaction mixture stirred for 95 minutes. The reaction mixture was taken up in methylene chloride and extracted with sodium bisulfite, 10% sodium hydroxide. The crude reaction product was purified on silica gel eluting first with 1% and then with 2% methanol in methylene chloride to give the title compound (2.7g, MH⁺ 399).

### B. 8-CHLORO-11-(4-PIPERIDYLIDENE)-6,11-DIHYDRO-5H-BENZO[5,6]CYCLOHEPTA[1,2-b]PYRIDINE N-OXIDE

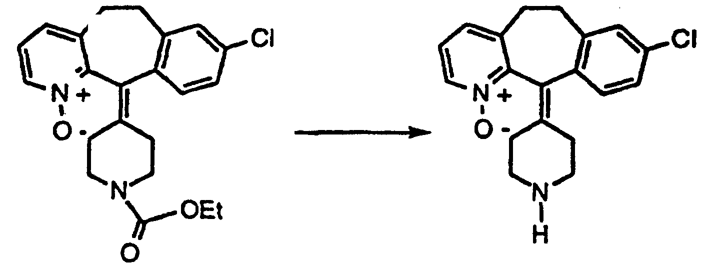

By essentially the same procedures as set forth in Preparative Example 1 step G, but using 8-chloro-11-(1-ethoxycarbonyl-4-piperidylidene)-6,11-dihydro-5H-benzo[5,6]cyclohepta[1,2-b]pyridine N-oxide instead of 8-chloro-11-(1-ethoxycarbonyl-4-piperidylidene)-6,11-dihydro-5H-benzo[5,6]cyclohepta[1,2-b]pyridine, the title compound was obtained and used for the next reaction without further purification (MH+ 327).

### C. 4-(8-CHLORO-5,6-DIHYDRO-11H-BENZO[5,6]CYCLO-HEPTA(1,2-b]PYRIDIN-11-YLIDENE)-1-[(3-PYRIDINYL)ACETYL]-PIPERIDINE N1,N4 DIOXIDE

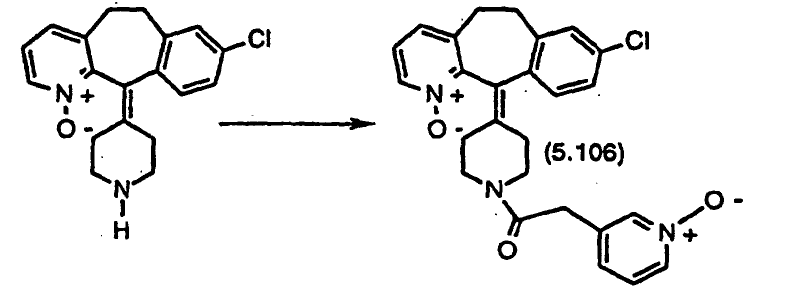

By essentially the same procedure as set forth in Example 227, but using 3-pyridyl acetic acid N-oxide (Preparative Example 9) instead of 4-pyridyl acetic acid N-oxide and 8-chloro-11-(4-piperidylidene)-6,11-dihydro-5H-benzo[5,6]cyclohepta[1,2-b]pyridine N-oxide instead of 8-chloro-11-(4-piperidylidene)-6,11-dihydro-5H-benzo[5,6]cyclohepta[1,2-b]pyridine, the title compound was obtained as a white solid (mp=105-107°C, MH⁺=462).

### EXAMPLE 232*

### 4-(8-CHLORO-5,6-DIHYDRO-11H-BENZO-[5,6]CYCLOHEPTA[1,2-b]PYRIDIN-11-YLIDENE)-1-[(4-PYRIDINYL)ACETYL]-PIPERIDINE N1,N4 DIOXIDE

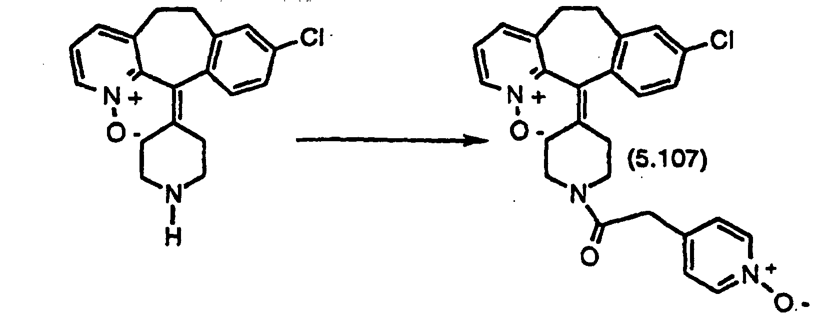

By essentially the same procedure as set forth in Example 227, but using 8-chloro-11-(4-piperidylidene)-6,11-dihydro-5H-benzo[5,6]cyclohepta[1,2-b]pyridine N-oxide instead of 8-chloro-11-(4-piperidylidene)-6,11-dihydro-5H-benzo[5,6]cyclohepta[1,2-b]pyridine, the title compound was obtained as a light brown solid( MH⁺=462).

### EXAMPLE 233*

### A. 8-CHLORO-6,11-DIHYDRO-11-(4-PIPERIDINYL)-5H-BENZO[5,6]CYCLOHEPTA[1,2-b]PYRIDINE (Product A) and 6,11-DIHYDRO-11-(4-PIPERIDINYL)-5H-BENZO[5,6]-CYCLOHEPTA[1,2-b]PYRIDINE (Product B)

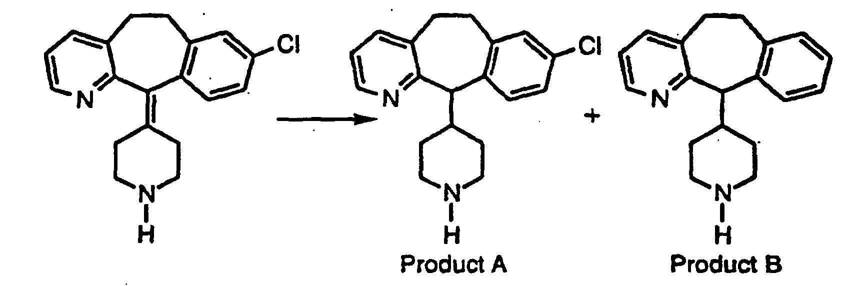

To a solution 66.27g (0.21 mole) of 4-(8-chloro-5,6-dihydro-11H-benzo[5,6]cyclohepta(1,2-b]pyridin-11-ylidene)-piperidine (product from Preparative Example 1 Example, step G), in THF (1L) was added lithium aluminum hydride (24.32g, 0.64 mole) and the reaction mixture was heated to reflux overnight. The reaction mixture was then cooled to room temperature and ∼ 3L of diethyl ether is added followed by dropwise addition of saturated sodium sulfate until a white gray precipitate forms. Magnesium sulfate was then added to the separated organic layer and stirred for 30 minutes. All the volatiles were then removed and the resulting crude mixture was chromatographed on a silica gel column eluting with 10% methanol saturated with ammonia in methylene chloride. The material obtained contained both the desired compound and the des-chloro compound. Separation on HPLC using reverse phase column and eluting with 40% methanol-water afforded the desired compounds as white solids (Product A's mp = 95.2-96.1°C, Product B's mp = 145.1-145.7°C).

### B. 4-(8-CHLORO-6,11-DIHYDRO-5H-BENZO[5,6]CYCLO-HEPTA[1,2-b]PYRIDIN-11-YL)-1-[(3-PYRIDINYL)ACETYL]-PIPERIDINE N1 OXIDE

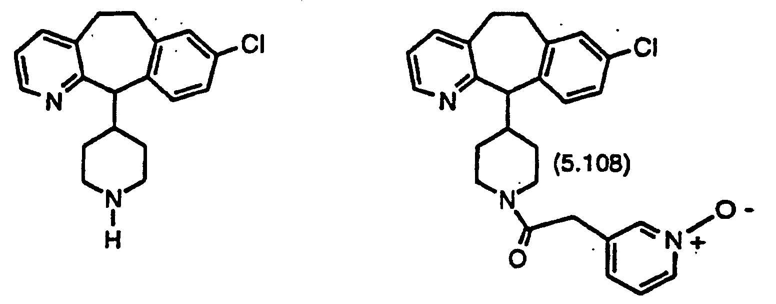

By essentially the same procedure as set forth in Example 227, but using 3-pyridyl acetic acid N-oxide(Preparative Example 9) instead of 4-pyridyl acetic acid N-oxide and 8-chloro-6,11-dihydro-11-(4-piperidinyl)-5H-benzo[5,6]cyclohepta[1,2-b]pyridine (product from Example 233A ) instead of 4-(8-chloro-5,6-dihydro-11H-benzo-[5,6]cyclohepta(1,2-b]pyridin-11-ylidene)-piperidine, the title compound was obtained as a white solid (mp=117-118°C, MH⁺=414).

### EXAMPLE 234*

### 4-(8-CHLORO-6,11-DIHYDRO-5H-BENZO[5,6]CYCLOHEPTA(1,2-b]PYRIDIN-11-YL)-1-[(4-PYRIDINYL)ACETYL]-PIPERIDINE N1 OXIDE

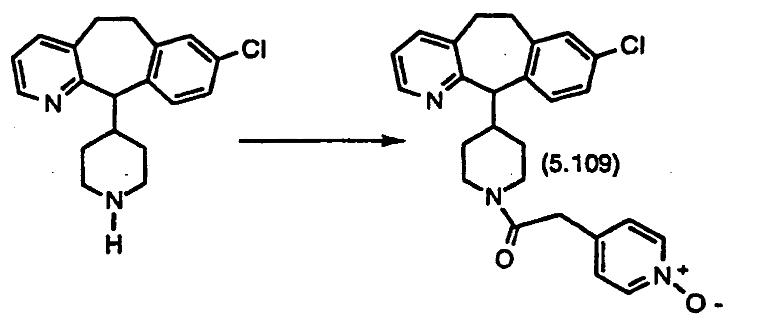

By essentially the same procedure as set forth in Example 227, but using 8-chloro-6,11-dihydro-11-(4-piperidinyl)-5H-benzo[5,6]cyclohepta[1,2-b]pyridine (product from Example 233A) instead of 4-(8-chloro-5,6-dihydro-11H-benzo-[5,6]cyclohepta(1,2-b]pyridin-11-ylidene)-piperidine (product from Preparative Example 1, step G), the title compound was obtained as a white solid (mp=125-126°C, MH⁺=414).

### EXAMPLE 235*

### A. ETHYL 4-(8-CHLORO-6,11-DIHYDRO-5H-BENZO[5,6]CYCLOHEPTA(1,2-b]PYRIDIN-11-YL)-1- PIPERIDINE-CARBOXYLATE

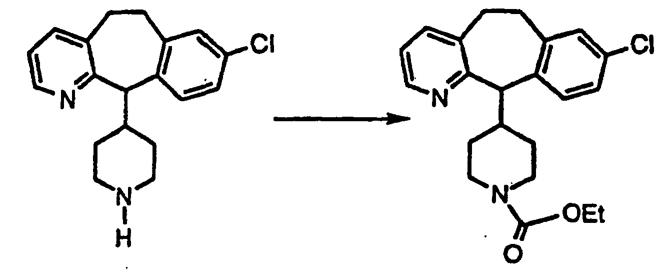

8-Chloro-6,11-dihydro-11-(4-piperidinyl)-5H-benzo[5,6]cyclohepta[1,2-b]pyridine (product from Example 233A) (4.18g, 13mmol) was dissolved in toluene (175mL). Ethyl chloroformate(11.6g,110 mmol, 10.2 mL) was then added and the reaction mixture was heated to ∼1.20°C overnight. All volatiles were stripped off and the crude product was purified on silica gel column eluting with 50% ethyl acetate- hexanes to give the title compound as a white solid(MH⁺ 385).

### B. ETHYL 4-(8-CHLORO-6,11-DIHYDRO-5H-BENZO[5,6]CYCLOHEPTA(1,2-b]PYRIDIN-11-YL)-1-PIPERIDINECARBOXYLATE N OXIDE

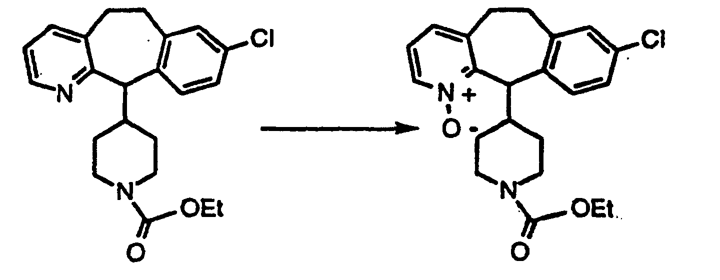

By essentially the same procedure as set forth in Example 231, but using ethyl 4-(8-chloro-6,11-dihydro-5H-benzo-[5,6]cyclohepta(1,2-b]pyridin-11-yl)-1-piperidinecarboxylate (product from Example 235A) instead of 8-chloro-11-(1-ethoxycarbonyl-4-piperidylidene)-6,11-dihydro-5H-benzo[5,6]cyclohepta[1,2-b]pyridine, the title compound was obtained as a white solid (mp= 81.7-82.5°C, MH⁺=400).

### C. 4-(8-CHLORO-6,11-DIHYDRO-5H-BENZO-[5,6]CYCLOHEPTA(1,2-b]PYRIDIN-11-YL)-1- PIPERIDINE N OXIDE

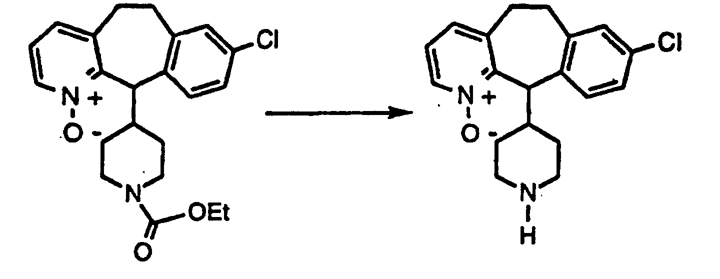

By essentially the same procedure as set forth in Preparative Example 1 step G, but using ethyl 4-(8-chloro-6,11-dihydro-5H-benzo[5,6]cyclohepta[1,2-b]pyridin-11-yl)-1- piperidinecarboxylate N1 oxide (product from Example 235B) instead of 8-chloro-11-(1-ethoxycarbonyl-4-piperidylidene)-6,11-dihydro-5H-benzo[5,6]cyclohepta[1,2-b]pyridine, the title compound was obtained as a solid (MH⁺=329).

### D. 4-(8-CHLORO-6,11-DIHYDRO-5H-BENZO[5,6]CYCLOHEPTA(1,2-b]PYRIDIN-11-YL)-1-[(3-PYRIDINYL)ACETYL]-PIPERIDINE N4 OXIDE

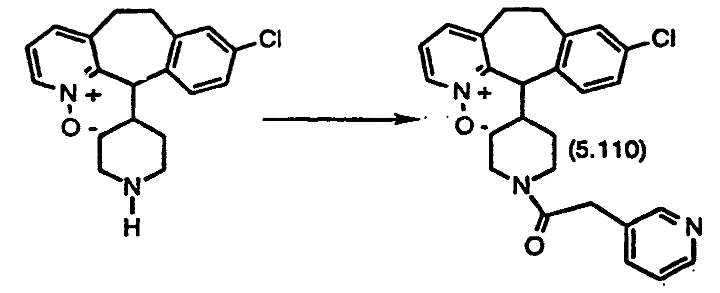

By essentially the same procedure as set forth in Example 227, but using 3-pyridyl acetic acid instead of 4-pyridyl acetic acid N-oxide and 4-(8-chloro-6,11-dihydro-5H-benzo-[5,6]cyclohepta[1,2-b]pyridin-11-yl)-1-piperidine N oxide (product from Example 235C) instead of 4-(8-chloro-5,6-dihydro-11H-benzo-[5,6]cyclohepta[1,2-b]pyridin-11-ylidene)-piperidine, the title compound was obtained as a white solid (mp=61.8-62.3°C, MH⁺=448).

### EXAMPLE 236*

### 4-(8-CHLORO-6,11-DIHYDRO-5H-BENZO-[5,6]CYCLOHEPTA(1,2b]PYRIDIN-11-YL)-1-[(4-PYRIDINYL)ACETYL]-PIPERIDINE N4 OXIDE

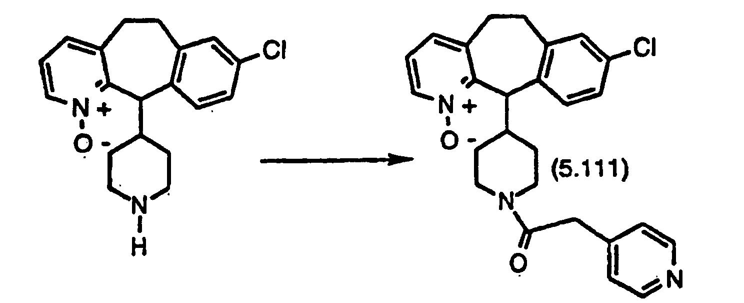

By essentially the same procedure as set forth in Example 227, but using 4-pyridyl acetic acid instead of 4-pyridyl acetic acid N-oxide and 4-(8-chloro-6,11-dihydro-5H-benzo-[5,6]cyclohepta(1,2-b]pyridin-11-yl)-1-piperidine N oxide (product from Example 235C) instead of 4-(8-chloro-5,6-dihydro-11H-benzo-[5,6]cyclohepta[1,2-b]pyridin-11-ylidene)-piperidine, the title compound was obtained as a white solid (mp=116.7-117.6°C, MH⁺=448).

### EXAMPLE 237*

### 4-(8-CHLORO-6,11-DIHYDRO-5H-BENZO-[5,6]CYCLOHEPTA(1,2-b]PYRIDIN-11-YL)-1-[(3-PYRIDINYL)ACETYL]- PIPERIDINE N1, N4 OXIDE

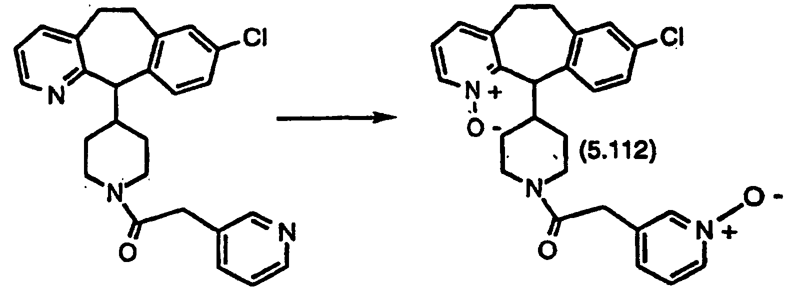

4-(8-Chloro-6,11-dihydro-5H-benzo-[5,6]cyclohepta(1,2-b]pyridin-11-yl)-1-[(3-pyridinyl)acetyl]-piperidine, from Example 82A, (0.5g, 1.2mmol) was disolved in methylene chloride at about -18°C. 3-Chloroperbenzoic acid (0.62g, 3.6 mmol) was added and the reaction stirred for 1.5 hours. The reaction mixture was extracted with 10% sodium bisulfite, 10% sodium hydroxide and then dried with magnesium sulfate, filtered and concentrated. The crude product was purified on silica gel eluting with 7% methanol saturated with ammonia in methylene chloride to give the title compound as a white solid(0.51g, 91% yield MH⁺ 464)

### EXAMPLE 238*

### 4-(8-CHLORO-6,11-DIHYDRO-5H-BENZO-[5,6]CYCLOHEPTA(1,2-b]PYRIDIN-11-YL)-1-[(4-PYRIDINYL)ACETYL]-PIPERIDINE N1, N4 OXIDE

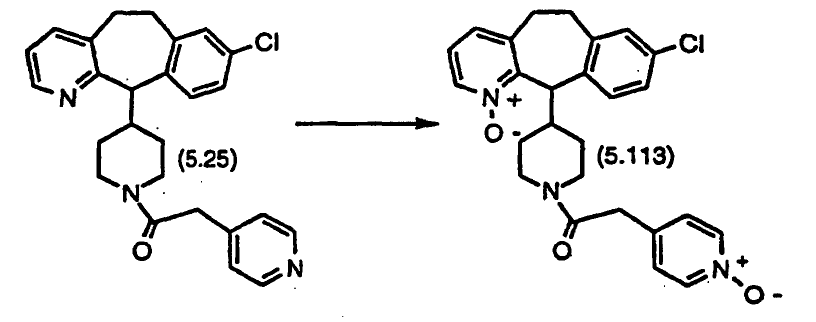

By essentially the same procedure as set forth in Example 237, but using 4-(8-chloro-6,11-dihydro-5H-benzo-[5,6]cyclohepta[1,2-b]pyridin-11-yl)-1-[(4-pyridinyl)acetyl]-piperidine (product from Example 82 ) instead of 4-(8-chloro-6,11-dihydro-5H-benzo-[5,6]cyclohepta[1,2-b]pyridin-11-yl)-1-[(3-pyridinyl)acetyl]-piperidine, the title compound was obtained as a white solid (mp=85-85.6 °C, MH⁺=464).

### EXAMPLE 239*

### 4-(8-CHLORO-3-METHOXY-5,6-DIHYDRO-11H-BENZO[5,6]-CYCLOHEPTA[1,2-b]PYRIDIN-11-YLIDENE)-1-[(3-PYRIDINYL)ACETYL]-PIPERIDINE

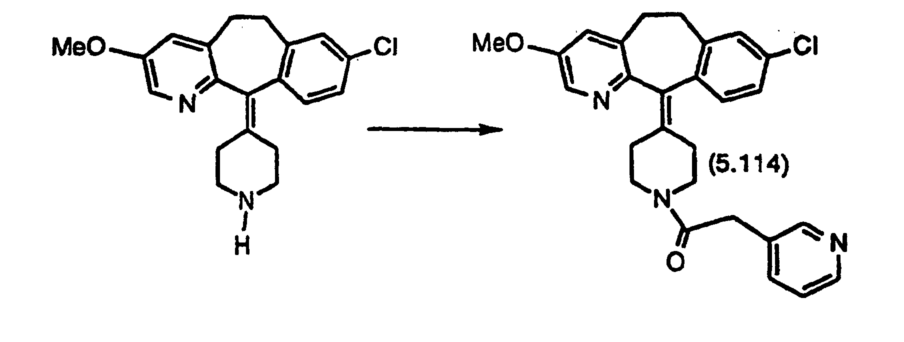

By essentially the same procedure as set forth in Example 180, but using 8-chloro-3-methoxy-11-(4-piperidylidene)-6,11-dihydro-5H-benzo[5,6]-cyclohepta[1,2-b]pyridine (Preparative Example 20) instead of 3,8-dichloro-11-(1-acetyl-4-piperidylidene)-6,11-dihydro-5H-benzo[5,6]-cyclohepta[1,2-b]pyridine the title compound was obtained as a white solid (MH⁺ 460).

### EXAMPLE 240*

### 4-(8-CHLORO-3-HYDROXY-5,6-DIHYDRO-11H-BENZO[5,6]-CYCLOHEPTA[1,2-b]PYRIDIN-11-YLIDENE)-1-[(3-PYRIDINYL]-ACETYL]-PIPERIDINE

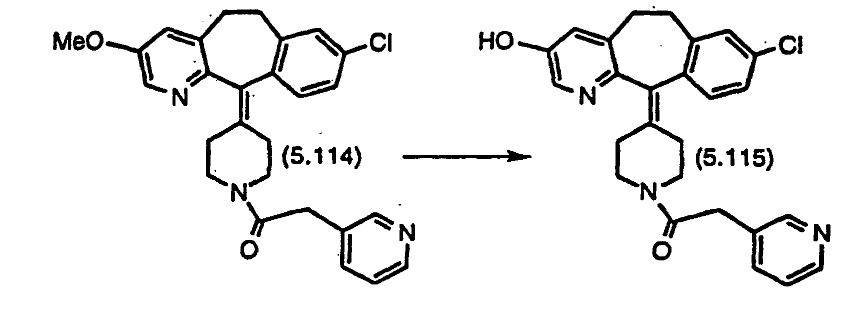

4-(8-Chloro-3-methoxy-5,6-dihydro-11H-benzo[5,6]cyclohepta[1,2-b]pyridin-11-ylidene)-1-[(3-pyridinyl]acetyl]-piperidine (0.24g, 0.54 mmol) (Example 239) was dissolved in methylene chloride at 0°C under nitrogen atmosphere. Bromine tribromide(0.9g, 3.6 mmol, 3.6 mL) was added and the reaction was run at room temperature for two days. The reaction mixture was concentrated and chromatographed on a silica gel column eluting with 3% methanol saturated with ammonia in methylene chloride to give an off white borate salt solid (0.14g, 61% yield, MH⁺ 446).

### EXAMPLE 246*

### 1-1(4-PYRIDINYLACETYL)-4-[3-BROMO8-CHLORO5-6-DIHYDRO-11H-BENZO[5,6]CYCLOHEPTA[1,2-b]PYRIDIN-11YLIDENE]-PIPERIDINE

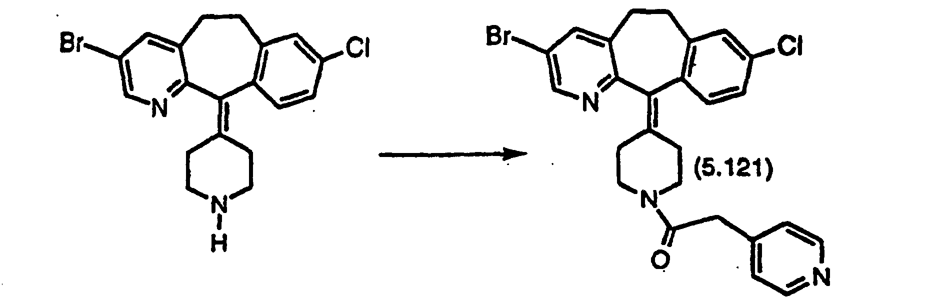

By essentialy the same procedure as set forth in Example 180 but using 4-(3-bromo-8-chloro-5,6-dihydro-11H-benzo[5,6]cyclohepta(1,2-b]pyridin-11-ylidene)-piperidine instead of 4-(3,8-dichloro-5,6-dihydro-11H-benzo-[5,6]cyclohepta(1,2-b]pyridin-11-ylidene)-piperidine, the title compound was obtained as a glassy solid ( MH⁺ 508).

### EXAMPLE 247*

### 1-1(3-PYRIDINYLACETYL)-4-[3-BROMO8-CHLORO5-6-DIHYDRO-11H-BENZO[5,6]CYCLOHEPTA[1,2-b]PYRIDIN-11YLIDENE]-PIPERIDINE

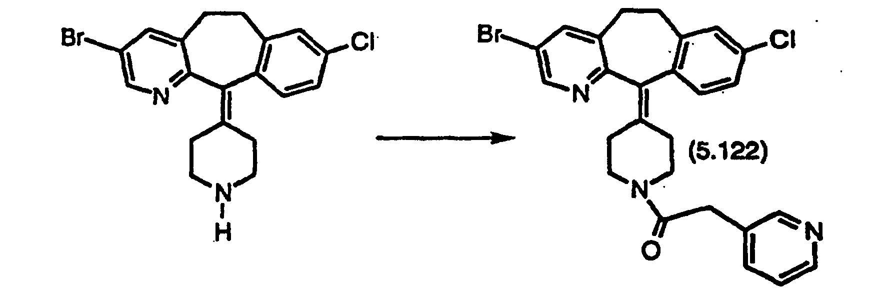

By essentialy the same procedure as set forth in Example 180, but using 4-(3-bromo-8-chloro-5,6-dihydro-11H-benzo[5,6]cyclohepta(1,2-b]pyridin-11-ylidene)-piperidine instead of 4-(3,8-dichloro-5,6-dihydro-11H-benzo[5,6]cyclohepta[1,2-b]pyridin-11-ylidene)-piperidine and 3-pyridyl acetic acid instead of 4-pyridyl acetic acid, the title compound was obtained as a white solid (mp=92-93°C MH⁺ 508).

### EXAMPLE 248*

### 4-[4,8-DICHLORO-5,6-DIHYDRO-11H-BENZO[5,6]CYCLOHEPTA[1,2-b]PYRIDIN-11-YLIDENE]-1-(4-PYRIDINYLACETYL)-PIPERIDINE and 4-[2,8-DICHLORO-5,6-DIHYDRO-11H-BENZO[5,6]CYCLOHEPTA[1,2-b]PYRIDIN-11-YLIDENE]-1-(4-PYRIDINYLACETYL)-PIPERIDINE

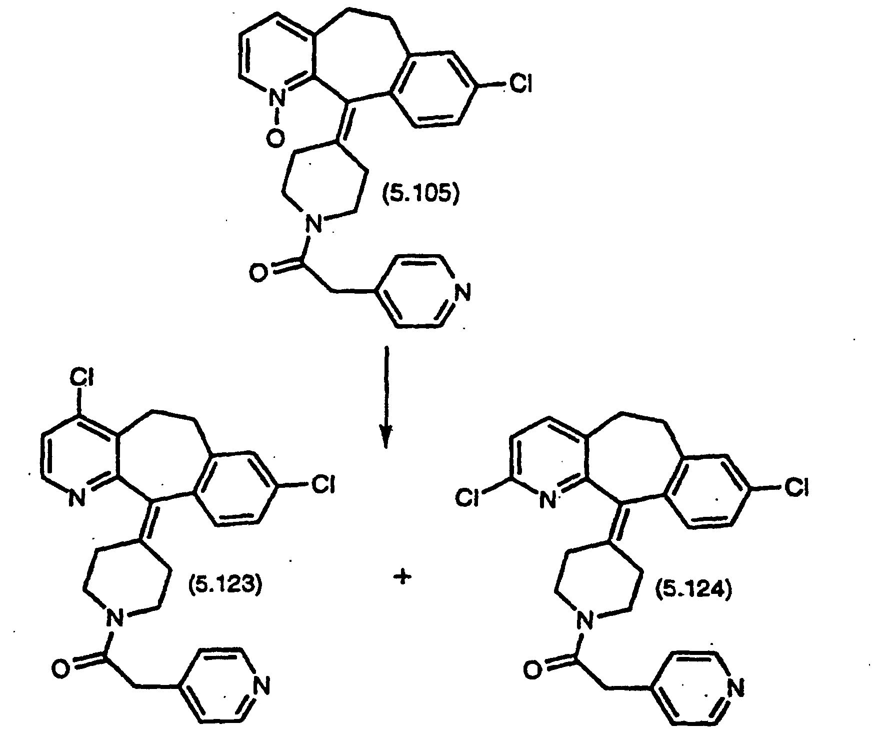

A solution of the title compound from Example 230 (1.7 grams) and phosphorous oxychloride (21 mL) dissolved in chloroform (12 mL) was stirred at reflux for 1 hour. Concentration *in vacuo* provided a residue which was diluted with dichloromethane and washed with saturated aqueous sodium bicarbonate and brine. The organic phase was dried over anhydrous magnesium sulfate, concentrated *in vacuo*, and purified by flash column chromatography (silica gel) using 2% methanol-dichloromethane to afford the title 4,8-dichloro compound (0.34 grams, 20% yield, mp 84-91 °C, MH⁺ 464) and the title 2,8-dichloro compound (0.18 grams, 11% yield, mp 163.8-164.6 °C, MH⁺ 464).

### EXAMPLE 249*

### 4-[4-[(1H-BENZOTRIAZOL-1-YL)OXY]-8-CHLORO-5,6-DIHYDRO-11H-BENZO[5,6]CYCLOHEPTA[1,2-b]PYRIDIN-11-YLIDENE]-1-(4-PYRIDINYLACETYL)-PIPERIDINE

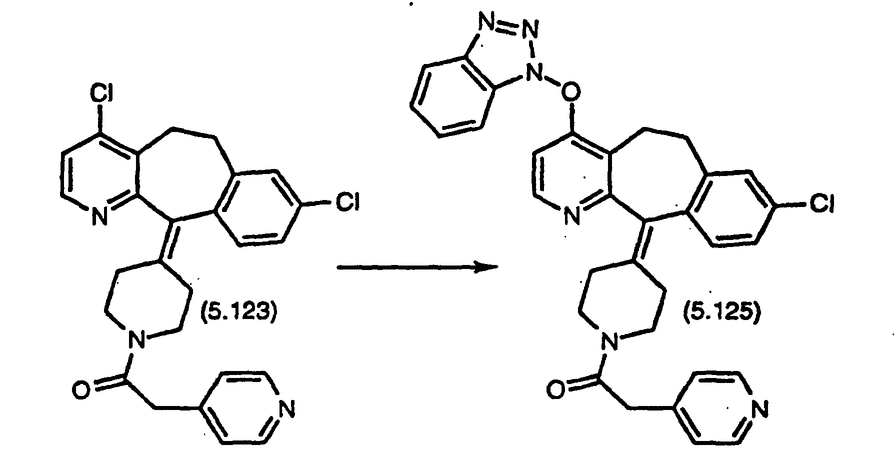

A mixture of the 4,8-dichloro compound from Example 248 (0.5 grams), 1-hydroxybenzotriazole hydrate (0.4 grams) and anhydrous dimethylformamide (20 mL) was stirred at 25°C under N₂ for 5 days. The mixture was concentrated *in vacuo*, diluted with dichloromethane, and washed with 1*N* aqueous sodium hydroxide. The organic phase was dried over anhydrous magnesium sulfate, concentrated *in vacuo* and purified by flash column chromatography (silica gel) using 3-5% methanol-dichloromethane to give the title compound (0.58 grams, 96%, mp 98.6-101.6 °C, MH⁺ 563).

### EXAMPLE 250*

### 4-[4,8-DICHLORO-5,6-DIHYDRO-11H-BENZO[5,6]CYCLO-HEPTA[1,2-b]PYRIDIN-11-YLIDENE]-1-(3-PYRIDINYLACETYL)-PIPERIDINE

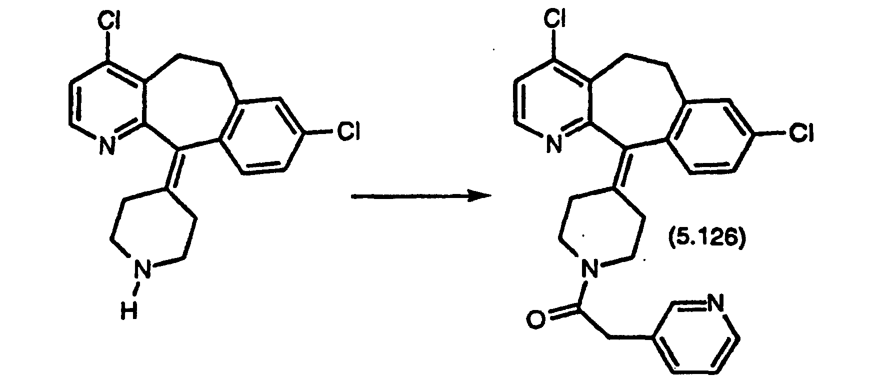

A mixture of the 4,8-dichloro product from Preparative Example 28 (1.91 grams), 3-pyridylacetic acid hydrochloride (2.1 grams), 1-(3-dimethylaminopropyl)-3-ethyl carbodiimide hydrochloride (1.6 grams), 4-methylmorpholine (1.4 mL) and anhydrous dimethylformamide (100 mL) was stirred at 25 °C overnight. Concentration *in vacuo* provided a residue which was diluted with dichloromethane and water. The organic phase was dried over anhydrous magnesium sulfate and concentrated *in vacuo* to provide the title compound (2.2 grams, 87%, mp 59.8-63.5 °C, MH⁺ 464).

### EXAMPLE 251*

### 4-[4-[(1H-BENZOTRIAZOL-1-YL)OXY]-8-CHLORO-5,6-DIHYDRO-11H-BENZO[5,6]CYCLOHEPTA[1,2-b]PYRIDIN-11-YLIDENE]-1-(3-PYRIDINYLACETYL)-PIPERIDINE

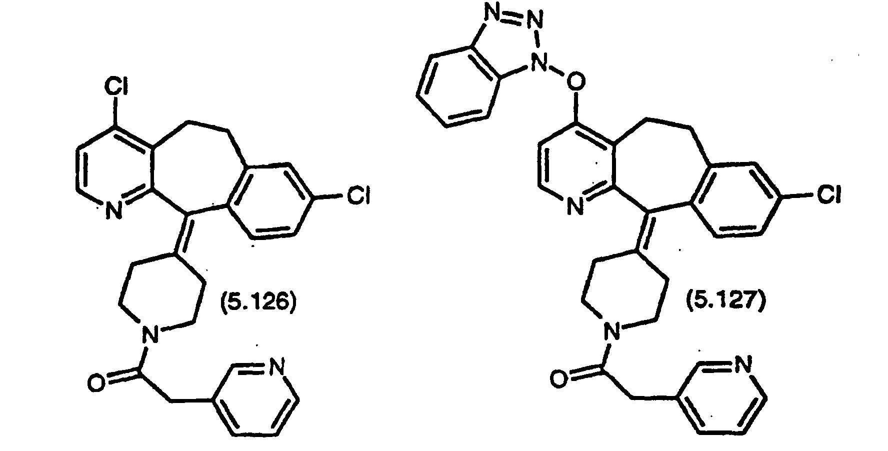

The 4,8-dichloro compound from Example 250 (0.8 grams) was added to a solution of 1-hydroxybenzotriazole hydrate (1.2 grams) and sodium hydride (0.14 grams, 60% in mineral oil) in anhydrous dimethylformamide (60 mL). The resulting solution was irradiated with a 200 W lamp while stirring at 25°C for 60 hours. The solution was poured into 1N aqueous sodium hydroxide while stirring and an additional 400 mL of water was added to the resulting mixture. Filtration provided a solid which was washed with water several times. The solid was dissolved in dichloromethane-acetone, dried over anhydrous magnesium sulfate, and concentrated in vacuo to proved the title compound (0.87 grams, 90%, mp = 120-122°C. MH⁺ 563).

### EXAMPLE 252*

### 4-[4-HYDROXY-8-CHLORO-5,6-DIHYDRO-11H-BENZO[5,6]-CYCLOHEPTA[1,2-b]PYRIDIN-11-YLIDENE]-1-(3-PYRIDINYLACETYL)-PIPERIDINE

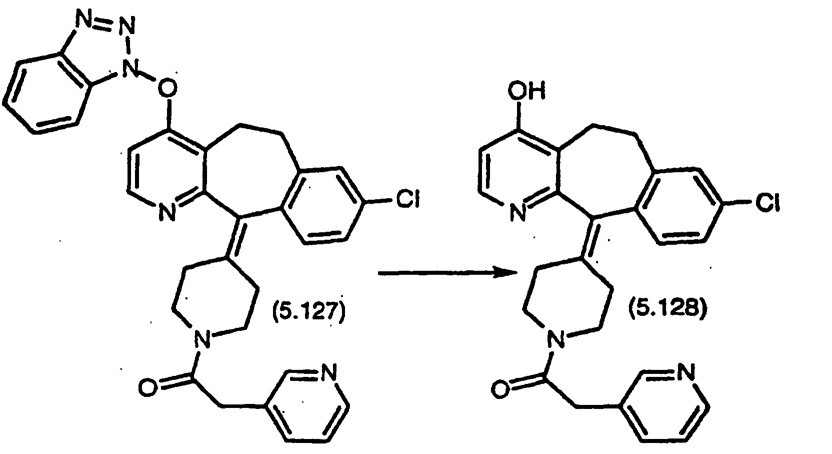

To a solution of the title compound form Example 251 (0.8 grams) and glacial acetic acid (30 mL) was added zinc dust (0.4 grams). After stirring at 25°C for 18 hour, the mixture was filtered through celite and the filtrate concentrated *in vacuo*. The residue was diluted with ethyl acetate, washed with saturated aqueous sodium bicarbonate and brine. The organic layer was separated, dried over magnesium sulfate and concentrated *in vacuo* to give the title compound (Yield 0.346 grams, 58%, MH+ 446).

### EXAMPLE 253*

### 4-[3-BROMO-4-HYDROXY-8-CHLORO-5,6-DIHYDRO-11H-BENZO[5,6]CYCLOHEPTA[1,2-b]PYRIDIN-11-YLIDENE]-1-(3-PYRIDINYLACETYL)-PIPERIDINE

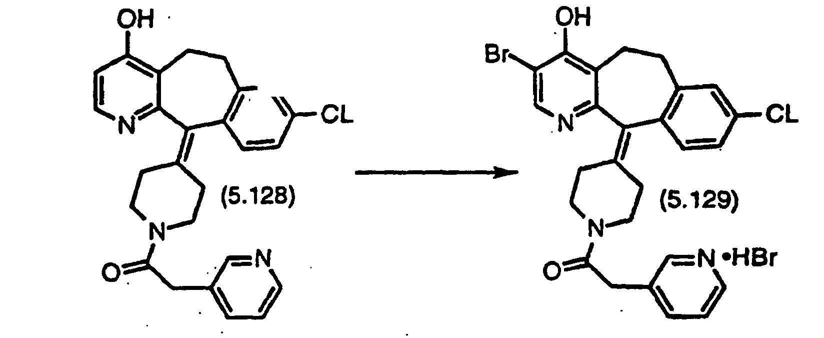

To a solution of the title compound from Example 252 (0.19 grams) and glacial acetic acid (4 mL) was added a 0.7 M bromine-acetic acid solution (0.7 mL) at 25°C under N₂. After 10 minutes, water was added and the resulting solid was filtered and washed with water several times and dried to give the title compound (0.18 grams, 71%, MH⁺ 526).

### EXAMPLE 255*

### 4-[8-CHLORO-4-(METHYLTHIO)-5,6-DIHYDRO-11H-BENZO[5,6]CYCLOHEPTA[1,2-b]PYRIDIN-11-YLIDENE]-1-(3-PYRIDINYLACETYL)-PIPERIDINE

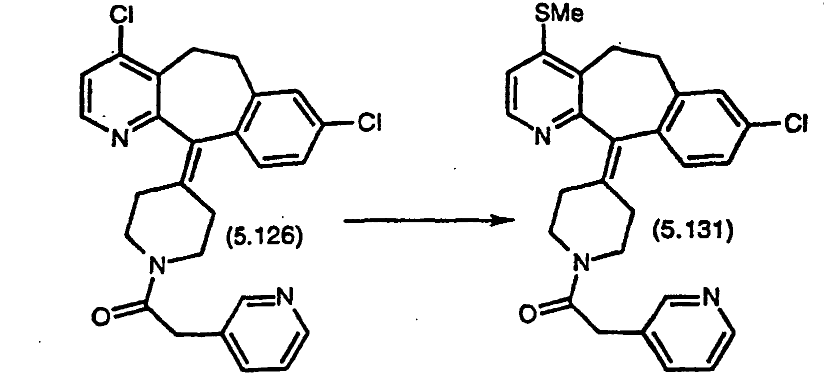

A mixture of the title compound from Example 250 (0.26 grams), sodium methylthiolate (0.06 grams) and anhydrous dimethylformamide (10 mL) was stirred while being irradiated with a 200 W lamp for 1 hour. After stirring an additional 12 hours at room temperature without irradiation, the mixture was concentrated *in vacuo*, diluted with dichloromethane, and washed with 1*N* aqueous sodium hydroxide and brine. The organic phase was dried over anhydrous magnesium sulfate and concentrated *in vacuo* to afford the title compound as a white foam (0.3 grams, 100%, MH⁺ 476).

### EXAMPLE 256*

### 4-[8-CHLORO-4-(METHYLSULFINYL)-5,6-DIHYDRO-11H-BENZO[5,6]CYCLOHEPTA[1,2-b]PYRIDIN-11-YLIDENE]-1-(3-PYRIDINYLACETYL)-PIPERIDINE

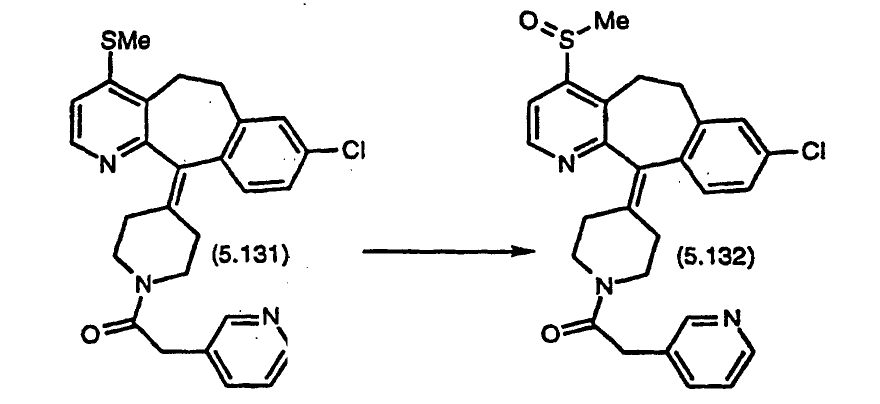

To the title compound from Example 255 (0.18 grams) dissolved in anhydrous tetrahydrofuran (10 mL) was added 30% aqueous hydrogen peroxide (3 mL) and the resulting solution was stirred for 12 hours at 73°C. The solution was concentrated *in vacuo*, diluted with dichloromethane, and washed with water. The organic phase was dried over anhydrous magnesium sulfate and concentrated *in vacuo* to afford the title compound after preparative plate chromatography (silica gel) using 3% methanol-dichloromethane (0.04 grams, 26%, MH⁺ 492).

### EXAMPLE 257*

### METHYL [[8-CHLORO-6,11-DIHYDRO-11-[1-[1-OXO-2-(3-PYRIDINYL)ETHYL]-4-PIPERIDINYLIDENE]-5H-BENZO[5,6]CYCLOHEPTA[1,2-b]PYRIDIN-4-YL]THIO]ACETATE

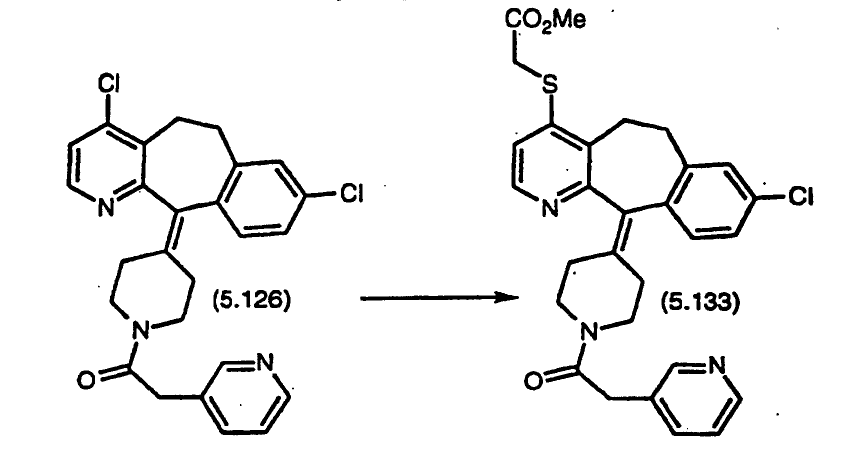

A mixture of the title compound from Example 250 (0.26 grams), sodium hydride (0.08 grams, 60% in mineral oil), methyl thioglycolate (0.19 mL) and anhydrous dimethylformamide (15 mL) was stirred while being irradiated with a 200 W lamp for 16 hours. The mixture was diluted with methanol, concentrated *in vacuo*, diluted with dichloromethane and water, and washed with 1*N* aqueous sodium hydroxide and brine. The organic phase was dried over anhydrous magnesium sulfate and concentrated *in vacuo* and the residue purified by preparative plate chromatography (silica gel) using 3% methanol-dichloromethane to afford the title compound (0.05 grams, 15%, MH⁺ 534).

### EXAMPLE 258*

### 4-[8-CHLORO-5,6-DIHYDRO-4-(PHENYLMETHYLTHIO)-11H-BENZO[5,6]CYCLOHEPTA[1,2-b]PYRIDIN-11-YLIDENE]-1-(3-PYRIDINYLACETYL)-PIPERIDINE

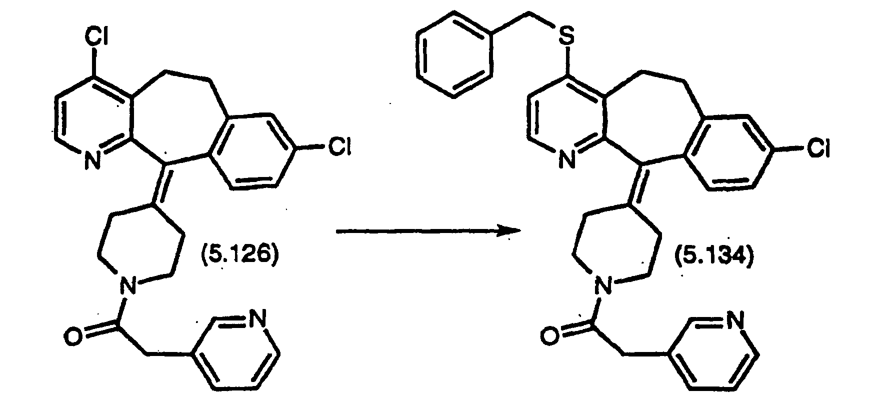

A mixture of the title compound from Example 250 (0.25 grams), sodium hydride (0.11 grams, 60% in mineral oil), benzyl mercaptan (0.13 mL) and anhydrous dimethylformamide (15 mL) was stirred while being irradiated with a 200 W lamp for 10 days. Isolation and purification as in Example 257 provided the title compound (0.02 grams, 8%, MH⁺ 552).

### EXAMPLE 259*

### 4-[8-CHLORO-5,6-DIHYDRO-4-[(2-METHYL-2H-TETRAZOL-5-YL)THIO]-11H-BENZO[5,6]CYCLOHEPTA[1,2-b]PYRIDIN-11-YLIDENE]-1-(3-PYRIDINYLACETYL)-PIPERIDINE

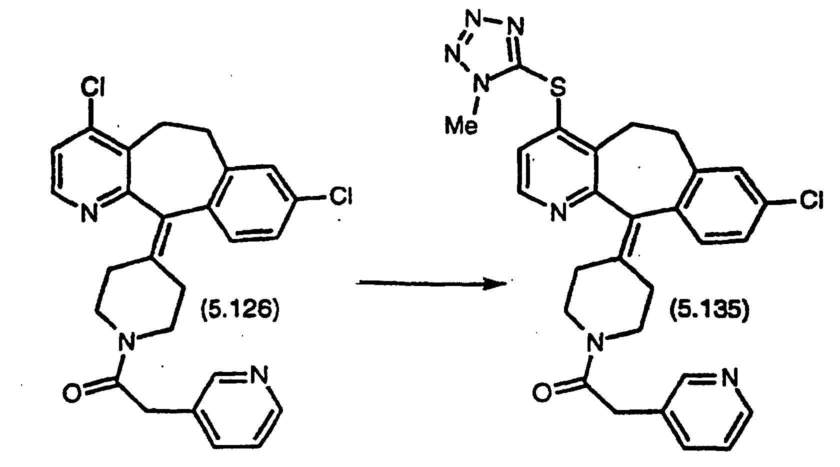

A mixture of the title compound from Example 250 (0.24 grams), 5-mercapto-1-methyltetrazole sodium salt (0.6 grams) and anhydrous dimethylformamide (10 mL) was stirred while being irradiated with a 200 W lamp for 10 days. Isolation and purification as in Example 257 provided the title compound (0.2 grams, 68%, MH⁺ 544).

### EXAMPLE 260*

### 1,1-DIMETHYLETHYL [2-[[8-CHLORO-6,11-DIHYDRO-11-[1-[1-OXO-2-(3-PYRIDINYL)ETHYL]-4-PIPERIDINYLIDENE]-5H-BENZO[5,6]-CYCLOHEPTA[1,2-b]PYRIDIN-4-YL]THIO]ETHYL]CARBAMATE

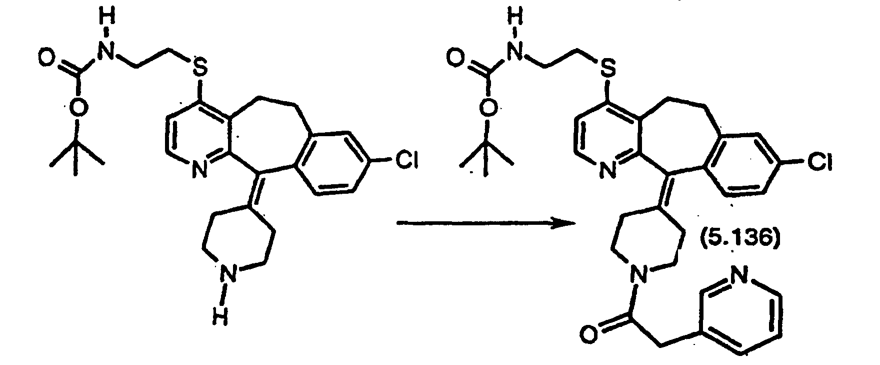

A mixture of the title compound from Preparative Example 32 (0.032 grams), 3-pyridylacetic acid hydrochloride (0.05 grams), 1-(3-dimethylaminopropyl)-3-ethyl carbodiimide hydrochloride (0.03 grams), triethylamine (0.08 mL) and anhydrous dimethylformamide (4 mL) was stirred at 25 °C for 48 hours. Concentration *in vacuo* provided a residue which was diluted with dichloromethane and washed with 1*N* aqueous sodium hydroxide. The organic phase was dried over anhydrous magnesium sulfate and concentrated *in vacuo* to provide the title compound (0.02 grams, 50%, mp 59.8-63.5 °C, MH⁺ 605).

### EXAMPLE 261

### 4-(4,8-DICHLORO-5,6-DIHYDRO-11H-BENZO[5,6]CYCLO-HEPTA[1,2-b]PYRIDIN-11-YLIDENE)-N-(3-PYRIDYL)-1-PIPERIDINE-CARBOXAMIDE

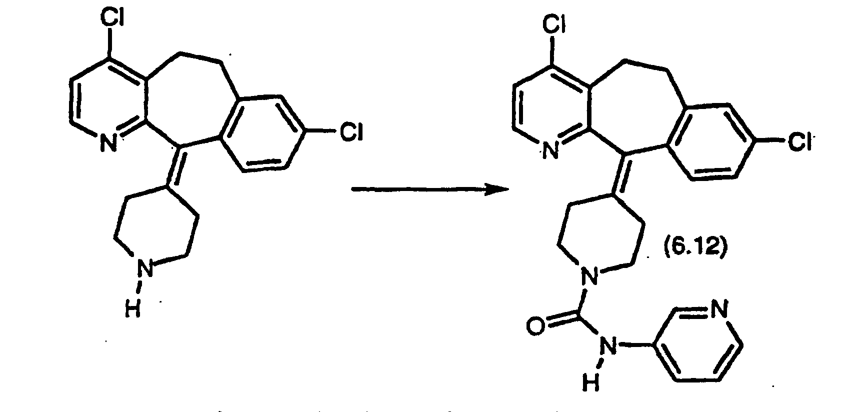

A portion of the stock solution of 3-pyridylisocyanate (32 mL) prepared as described in Preparative Example 33 was added to the 4,8-dichloro product from Preparative Example 28 (1.37 grams) and the mixture was stirred at 25°C for 4 days. The mixture was evaporated to dryness and the residue was taken up in dichloromethane and washed with saturated aqueous sodium bicarbonate and then water. The organic solution was dried over magnesium sulfate, filtered and evaporated to dryness. The residue was purified by flash column chromatography silica gel) using 2% methanol-dichloromethane as eluent to give the title compound (Yield 1.25 grams, 70%, MH+ 465).

### EXAMPLE 262

### 4-[4-[(1H-BENZOTRIAZOL-1-YL)OXY]-8-CHLORO-5,6-DIHYDRO-11H-BENZO[5,6]CYCLOHEPTA[1,2-b]PYRIDIN-11-YLIDENE]-N-(3-PYRIDYL)-1-PIPERIDINECARBOXAMIDE

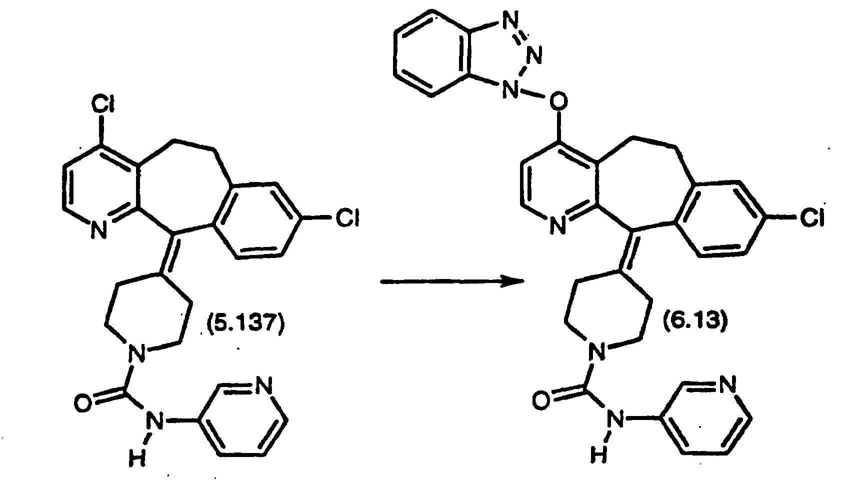

To a solution of the title compound from Example 261 (1.0 grams) in dry dimethylformamide (60 mL) was added 1-hydroxybenzotriazole (1.4 grams), sodium hydride (0.2 grams, 60% in mineral oil) and distilled water (0.5 mL). The solution was stirred at 25°C under nitrogen while being irradiated with a 200 Watt lamp for 20 hours. The reaction mixture was concentrated in vacuo, diluted with dichloromethane and saturated aqueous sodium bicarbonate and after two hours, the organic phase was separated, dried over magnesium sulfate and concentrated. Purification by flash column chromatography (silica gel) using 3-5% methanol-dichloromethane afforded the title compound (Yield 1.1 grams, 87%, MH+ 564).

### EXAMPLE 263

### 4-[4-HYDROXY-8-CHLORO-5,6-DIHYDRO-11H-BENZO[5,6]-CYCLOHEPTA[1,2-b]PYRIDIN-11-YLIDENE]-N-(3-PYRIDYL)-1-PIPERIDINECARBOXAMIDE

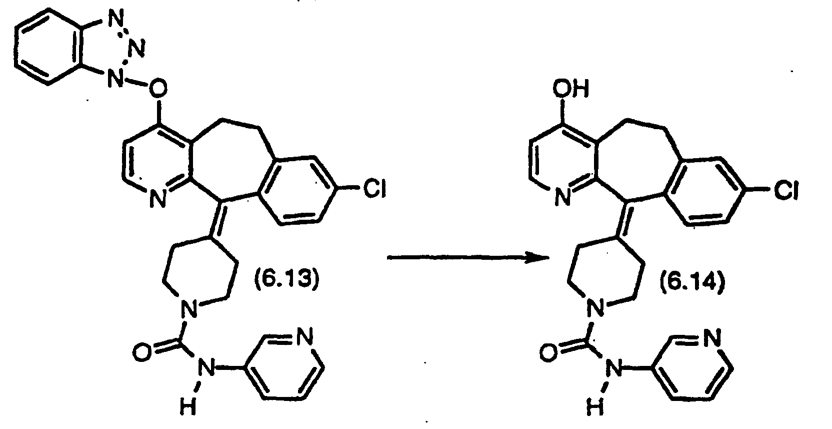

To a solution of the title compound form Example 262 (0.86 grams) and glacial acetic acid (20 mL) was added zinc dust (0.5 grams). After stirring at 25°C for 1.5 hours, the mixture was filtered through celite and the filtrate concentrated *in vacuo.* The residue was purified by flash column chromatography (silica gel) using 5-10% methanol-dichloromethane saturated with ammonium hydroxide to give the title compound (Yield 0.47 grams, 69%, MH+448).

### EXAMPLE 264

### 4-[3-BROMO-4-HYDROXY-8-CHLORO-5,6-DIHYDRO-11H-BENZO[5,6]CYCLOHEPTA[1,2-b]PYRIDIN-11-YLIDENE]-N-(3-PYRIDYL)-1-PIPERIDINECARBOXAMIDE

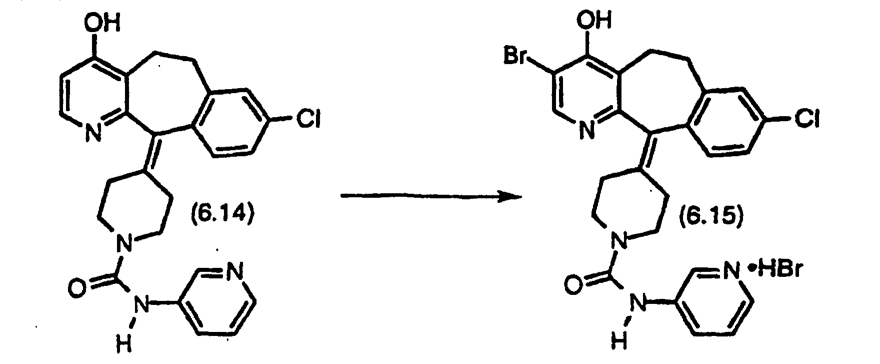

To a solution of the title compound from Example 263 (0.34 grams) and glacial acetic acid (10 mL) was added a 0.7 *M* bromine-acetic acid solution (4 mL) at 25°C under N₂. After 10 minutes, water was added and the resulting solid was filtered and washed with water several times and dried to give the title compound (Yield 0.31 grams, 67%, MH⁺ 527).

### EXAMPLE 266*

### 4-(8-CHLORO-5H-BENZO[5,6]CYCLOHEPTA[1,2-b]PYRIDIN-11-YL)-1-(3-PYRIDINYLACETYL)-PIPERIDINE

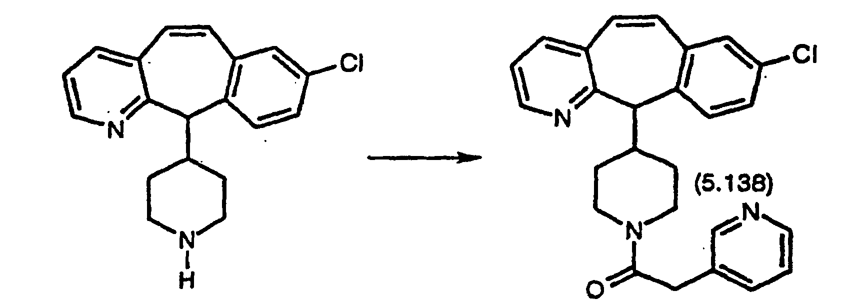

The title compound from Preparative Example 34C (2.0g, 6.4 mmole) was dissolved in anhydrous dimethylformarnide (70 mL) and the solution was cooled with an ice bath for 30 minutes. 4-Methylmorpholine (3.3 g, 32 mmole), 1-(3-dimethylaminopropyl)-3-ethylcarbodiimide hydrochloride (1.8 g, 9.7 mmole) and 1-hydroxybenzotriazole (0.87g 6.4 mmole) were added to the cold solution. 3-Pyridylacetic acid (0.88 g, 6.4 mmole) was added and the cooling bath removed. Stir the mixture at room temperature for 18 hours. The reaction mixture was evaporated to dryness and the residue was diluted with water (50 mL). The aqueous mixture was extracted with ethyl acetate and the combined extracts dried (MgSO₄), filtered and evaporated. The resulting residue was purifed by silica gel chromatography using a gradient of 97% dichloromethane/ 3% methanol saturated with ammonia to 93% dichlormethane/7% methanol saturated with ammonia as eluent to give the title compound (0.87g MH⁺ 430).

### EXAMPLE 267

### E. 4-(8-CHLORO-11H-BENZO[5,6]CYCLOHEPTA[1,2-b]PYRIDIN-11-YL)-N-(3-PYRIDINYL)-1-PIPERIDINECARBOXAMIDE

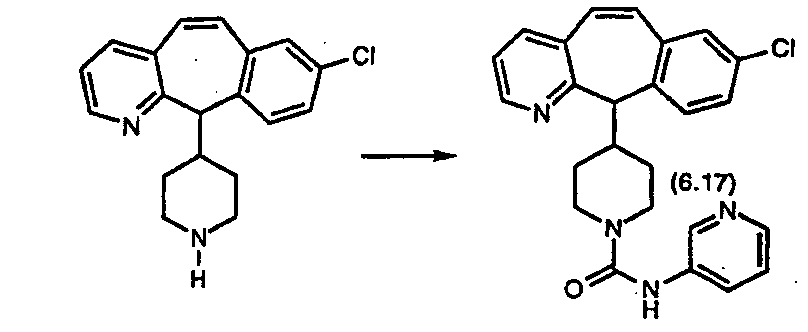

The title compound from Preparative Example 34C was treated with 3-pyridylisocyanate, similar to the procedure in Example 261, to afford the title compound (MH⁺ 431).

### EXAMPLE 268*

### 4-(8-CHLORO-5H-BENZO[5,6]CYCLOHEPTA[1,2-b]PYRIDIN-11-YL)-1-[2-METHYL-2-(3-PYRIDINYL)-1-OXOPROPYL]-PIPERIDINE

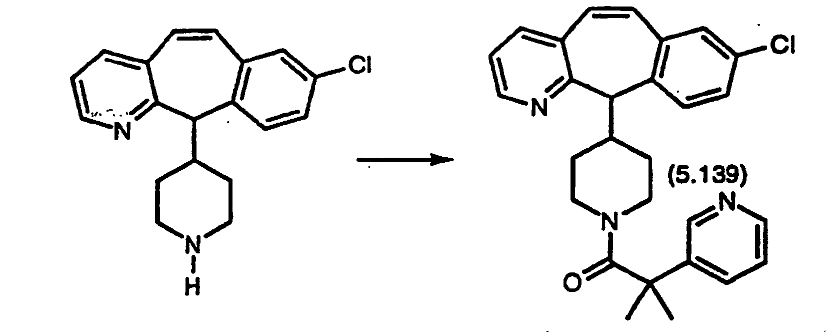

The title compound from Preparative Example 34C was treated as described in Example 266, using α,α-dimethyl-3-pyridylacetic acid (described in Preparative Example 10B) in place of 3-pyridylacetic acid, to afford the title compound (M+ 458).

### EXAMPLE 269*

### 4-(8-CHLORO-5H-BENZO[5,6]CYCLOHEPTA[1,2-b]PYRIDIN-11-YL)-1-(4-PYRIDINYLACETYL)-PIPERIDINE

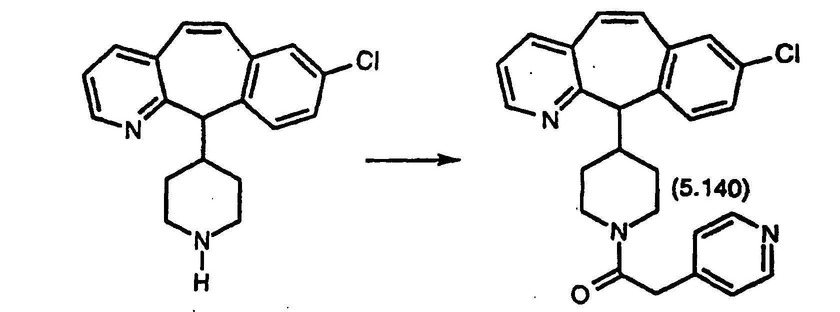

The title compound from Preparative Example 34C above was treated as descibed in Example 266, using 4-pyridylacetic acid in place of 3-pyridylacetic acid, to give the title compound (M⁺ 430).

### EXAMPLE 270*

### 4-(8-CHLORO-9-ETHYL-5H-BENZO[5,6]CYCLOHEPTA-[1,2-b]PYRIDIN-11-YL)-1-(3-PYRIDINYLACETYL)-PIPERIDINE

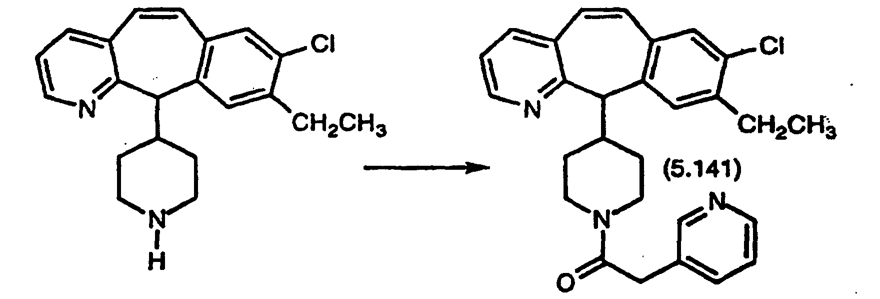

The title compound from Preparative Example 2A was treated as descibed in Example 266 to give the title compound (M+ = 458, mp = 67.2-69.8°C).

### EXAMPLE 273*

### 4-(4,8-DICHLORO-5H-BENZO[5,6]CYCLOHEPTA[1,2-B]PYRIDIN-11-YL)-1-(3-PYRIDINYLACETYL)-PIPERIDINE

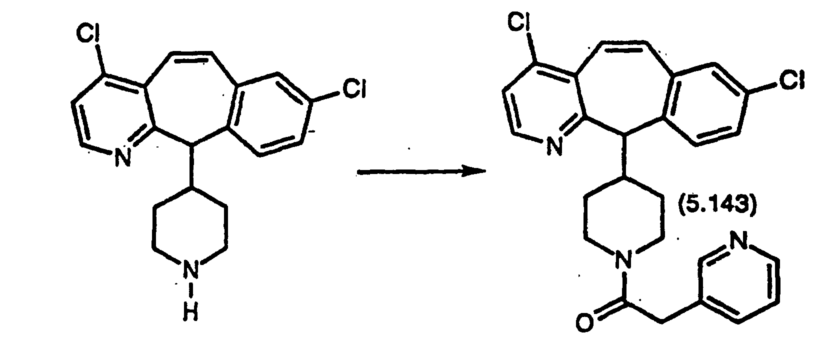

The title compound from Preparative Example 36C was treated as descibed in Example 266 to give the title compound (mp 100.1 - 103.4°C).

### EXAMPLE 274

### 4-(4,8-DICHLORO-11H-BENZO[5,6]CYCLOHEPTA[1,2-b]PYRIDIN-11-YL)-N-(3-PYRIDINYL)-1-PIPERIDINECARBOXAMIDE

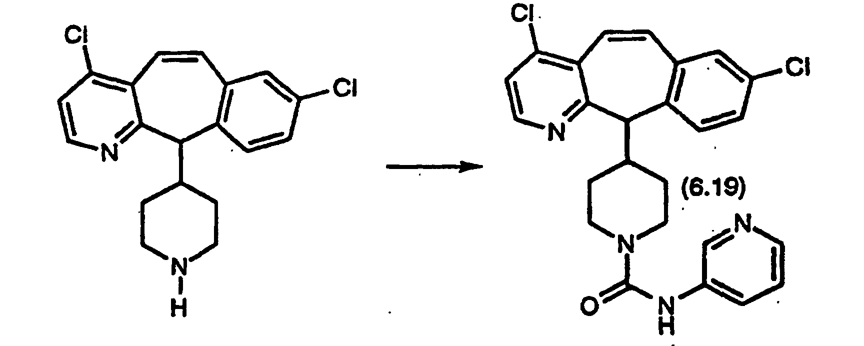

The title compound from Preparative Example 36C (0.75g, 2.17mmol) was treated with a pyridine solution of 3-pyridylisocyanate (from Preparative Example 33). The reaction mixture was evaporated to dryness and the residue dissolved in dichloromethane. This solution was washed with saturated sodium bicarbonate solution and brine, dried (MgSO₄), filtered and evaporated to give a dark syrup. The syrup was purified by silica gel chromatography using a gradient of 97% dichloromethane/3% methanol saturated with ammonia to 93% dichloromethane/7% methanol saturated with ammonia. The title compound was obtained as a yellow solid (0.13g, 13%, M+ 465)

### EXAMPLE 276*

### 4-(8-CHLORO-11H-BENZO[5,6]CYCLOHEPTA[1,2-b]PYRIDIN-11YLIDENE)-1-(3-PYRIDINYLACETYL)-PIPERIDINE

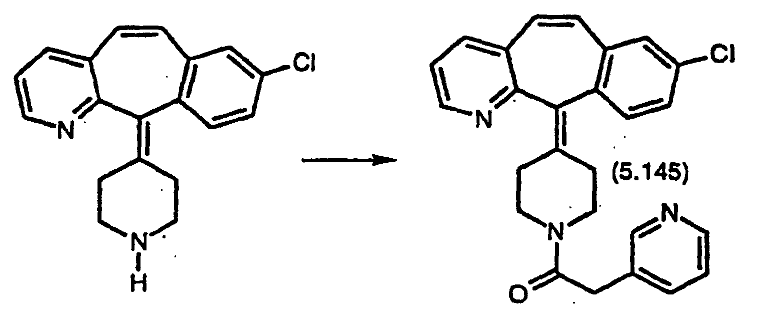

The title compound from Preparative Example 37B was treated as descibed in Example 266 to give the title compound (MH⁺ 428).

### EXAMPLE 277

### 4-(8CHLORO-11H-BENZO[5,6]CYCLOHEPTA[1,2-b]PYRIDIN-11YLIDENE)-N-(3-PYRIDINYL)-1-PIPERIDINECARBOXAMIDE

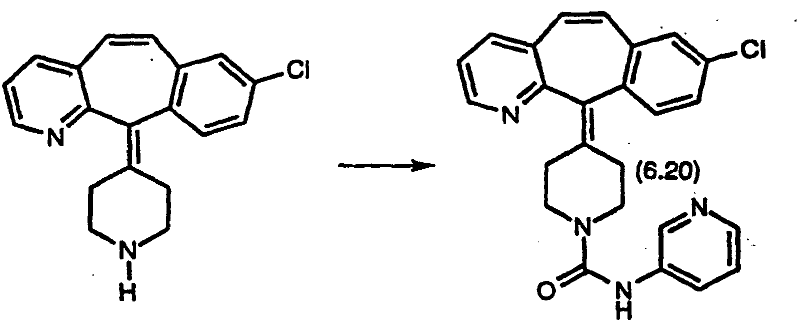

The title compound from Preparative Example 37B above was treated as descibed in Example 261 above to give the title compound (mp 95.9 - 97.6°C).

### EXAMPLE 278*

### 4-(8-CHLORO-11H-BENZO[5,6]CYCLOHEPTA[1,2-b]PYRIDIN-11YLIDENE)-1-[2-METHYL-2-(3-PYRIDINYL)-1-OXO-PROPYL]-PIPERIDINE

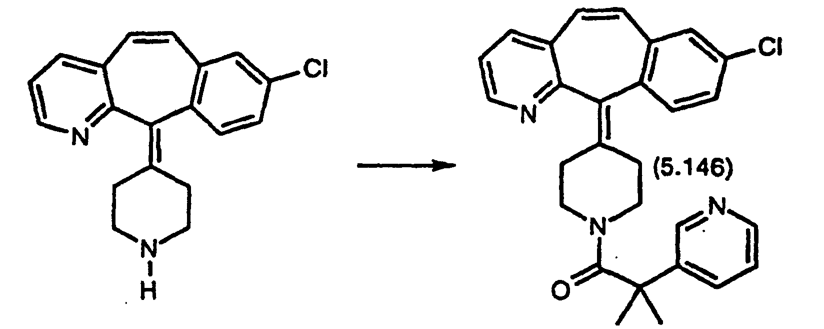

The title compound from Preparative Example 37B was treated as descibed in Example 266 using α,α-dimethyl-3-pyridylacetic acid (described in Preparative Example 10B) in place of 3-pyridylacetic acid, to give the title compound (M+ 456).

### EXAMPLE 279*

### 4-(8-CHLORO-5,6-DIHYDRO-5-OXO-11H-BENZO[5,6]-CYCLO-HEPTA[1,2-b]PYRIDIN-11YLIDENE)-1-(3-PYRIDINYLACETYL)-PIPERIDINE

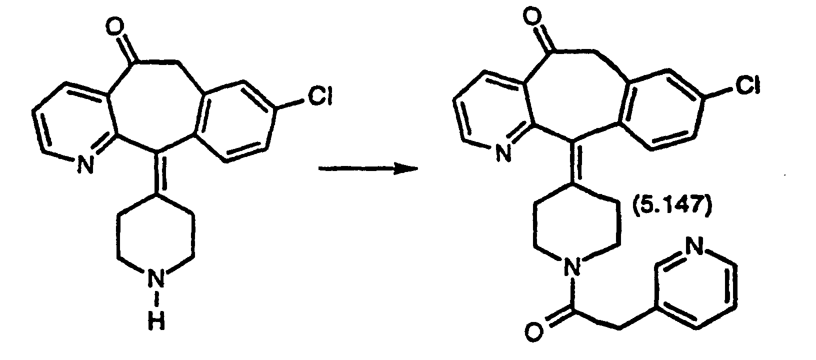

The preparation of the starting material for this reaction was described in *The Journal of Organic Chemistry,* **1990,** *55*, pp. 3341-3350 by Piwinski, J.J.; Wong, J.K.; Chan, T.-M.; Green, M.J.; and Ganguly, A.K. The procedure described in Example 266 was followed using 8-chloro-6,11-dihydro-11-(4-piperidinylidene)-5H-benzo[5,6]cyclohepta[1,2-b]-pyridin-5-one to give the title compound (M⁺ 443).

### EXAMPLE 280*

### 4-(8-CHLORO-5,6-DIHYDRO-5-HYDROXY-11H-BENZO[5,6]CYCLOHEPTA[1,2-b]PYRIDIN-11YLIDENE)-1-(3-PYRIDINYLACETYL)-PIPERIDINE

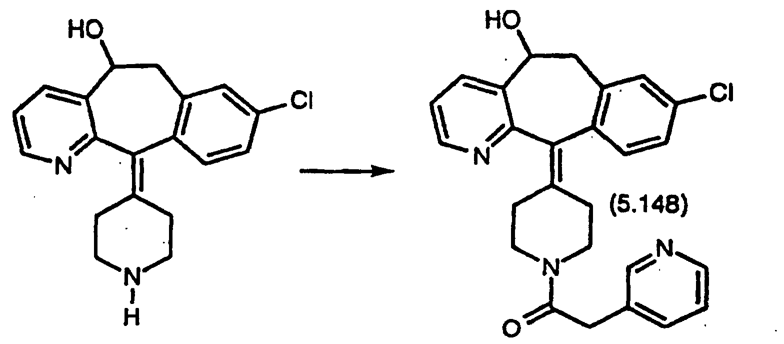

The preparation of the starting material for this reaction was described in *The Journal of Organic Chemistry,* **1990**, *55,* pp. 3341-3350 by Piwinski, J.J.; Wong, J.K.; Chan, T.-M.; Green, M.J.; and Ganguly, A.K. The procedure described in Example 266 was followed using 8-chloro-6,11-dihydro-5-hydroxy-11-(4-piperidinylidene)-5H-benzo[5,6]cyclohepta[1,2-b]pyridine to give the title compound (MH⁺ 446).

### EXAMPLE 281*

### 4-(8-CHLORO-5,6-DIHYDRO-5-OXO-11H-BENZO[5,6]-CYCLOHEPTA[1,2-b]PYRIDIN-11YLIDENE)-1-(4-PYRIDINYLACETYL)-PIPERIDINE

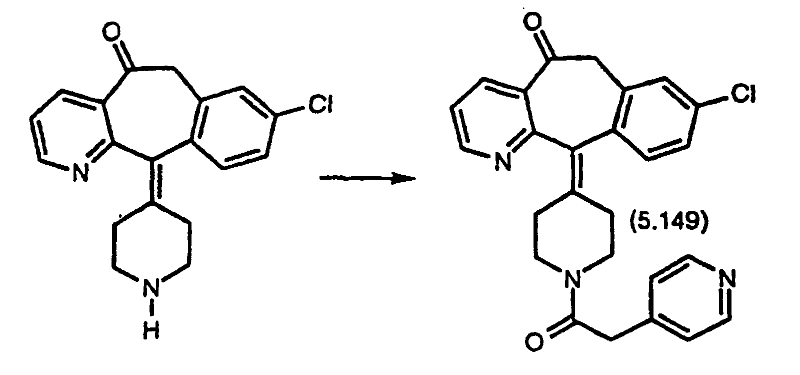

The procedure of Example 279 was followed with the exception that 4-pyridylacetic acid was used in place of 3-pyridylacetic acid to give the title compound (MH⁺ 444).

### EXAMPLE 282*

### 4-(8-CHLORO-5,6-DIHYDRO-5-HYDROXY-11H-BENZO[5,6]CYCLOHEPTA[1,2-b]PYRIDIN-11YLIDENE)-1-(4-PYRIDINYL-ACETYL)-PIPERIDINE

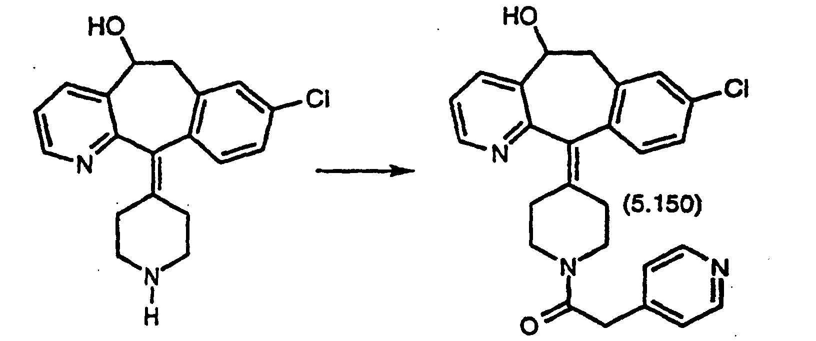

The procedure of Example 280 was followed with the exception that 4-pyridylacetic acid was used in place of 3-pyridylacetic acid to give the title compound (MH⁺ 446).

### EXAMPLE 283*

### 4-(8-CHLORO-5,6-DIHYDRO-6-OXO-11H-BENZO[5,6]-CYCLOHEPTA[1,2-b]PYRIDIN-11YLIDENE)-1-(3-PYRIDINYLACETYL)-PIPERIDINE

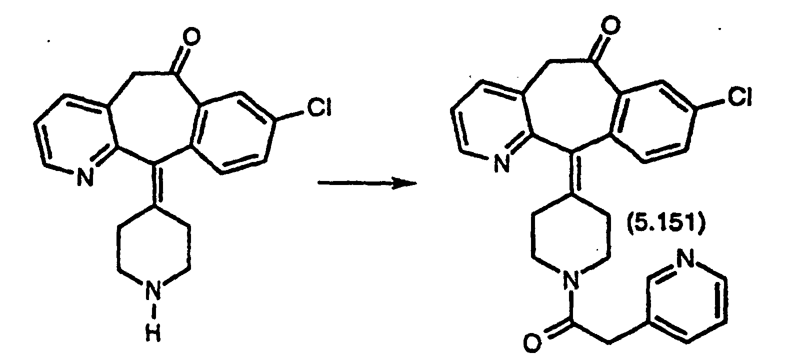

The preparation of the starting material for this reaction was described in *The Journal of Organic Chemistry*, **1990**, *55*, pp. 3341-3350 by Piwinski, J.J.; Wong, J.K.; Chan, T.-M.; Green, M.J.; and Ganguly, A.K. The procedure described in Example 266 was followed using 8-chloro-6,11-dihydro-11-(4-piperidinylidene)-5H-benzo[5,6]cyclohepta[1,2-b]pyridin-6-one to give the title compound (M⁺ 444).

### EXAMPLE 284*

### 4-(8-CHLORO-5,6-DIHYDRO-6-HYDROXY-11H-BENZO[5,6]CYCLOHEPTA[1,2-b]PYRIDIN-11YLIDENE)-1-(3-PYRIDINYL-ACETYL)-PIPERIDINE

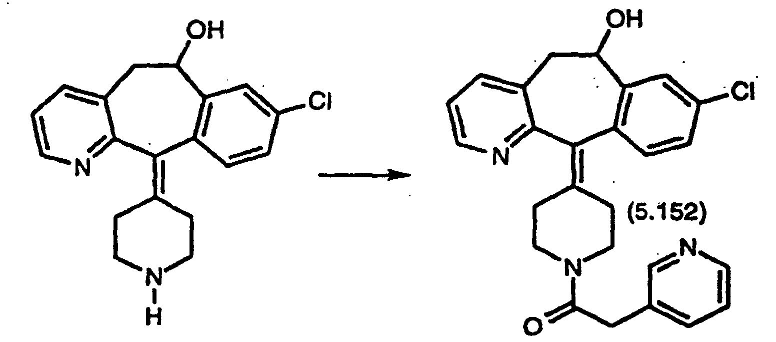

The preparation of the starting material for this reaction was described in *The Journal of Organic Chemistry,* **1990,** *55*, pp. 3341-3350 by Piwinski, J.J.; Wong, J.K.; Chan, T.-M.; Green, M.J.; and Ganguly, A.K. The procedure described in Example 266 above was followed using 8-chloro-6,11-dihydro-6-hydroxy-11-(4-piperidinylidene)-5H-benzo[5,6]-cyclohepta[1,2-b]pyridine to give the title compound (MH⁺ 446).

### EXAMPLE 285*

### 4-(8-CHLORO-5,6-DIHYDRO-6-OXO-11H-BENZO[5,6]-CYCLOHEPTA[1,2-b]PYRIDIN-11YLIDENE)-1-(4-PYRIDINYLACETYL)-PIPERIDINE

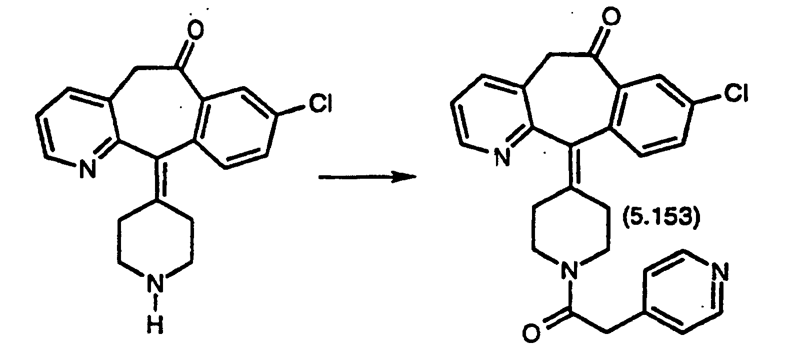

The procedure of Example 283 was followed with the exception that 4-pyridylacetic acid was used in place of 3-pyridylacetic acid to give the title compound (M⁺ 444).

### EXAMPLE 286*

### 4-(8-CHLORO-5,6-DIHYDRO-6-HYDROXY-11H-BENZO[5,6]CYCLOHEPTA[1,2-b]PYRIDIN-11YLIDENE)-1-(4-PYRIDINYLACETYL)-PIPERIDINE

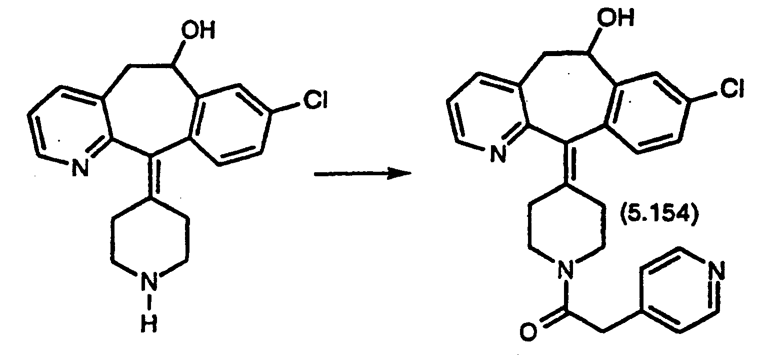

The procedure of Example 284 was followed with the exception that 4-pyridylacetic acid was used in place of 3-pyridylacetic acid to give the title compound (MH⁺ 446).

### EXAMPLES 287*, 289* AND 290*

By essentially the same procedure as in Example 1, but using either (R)-(+)-α-methoxy-α-(trifluromethyl)-phenylacetic acid (Example 290), (S)-(-)-α-methoxy-α-(trifluromethyl)-phenylacetic acid (Example 287), or α,α-dimethylphenylacetic acid (Example 289), the compounds of Example 290, 287 and 289 were obtained. The structures for these compounds are in Table 7. Data for these compounds are: compound of Example 290, white solid MH+ 527; compound of Example 287, white solid MH+ 527; and compound of Example 289, white solid M+ 457.

### EXAMPLES 291, 292, 294, 313 AND 314

By essentially the same procedure as in Example 183, and using either 4-, 3-, or 2-ethoxycarbonylaminopyridine and either 4-(8-chloro-5,6-dihydro-11H-benzo[5,6]cyclohepta[1,2-b]pyridin-11-ylidene)piperidine or 8-chloro-6,11-dihydro-11-(4-piperidinyl)-5H-benzo[5,6]cyclohepta[1,2-b]pyridine (product of Example 233A), the compounds of Examples 291, 292, 294, 313 and 314 were obtained. The structures for the compounds of Examples 291, 292, and 294 are given in Table 7. The structures for the compounds of Examples 313 and 314 are given in Table 10. Data are: the compound of Example 291 was a yellow solid (MH⁺ 431), the compound of Example 292 was an off white solid (MH⁺ 431), the compound of Example 294 was an off white solid (MH⁺ 431), the compound of Example 313 was a white solid (MH⁺ 433), and the compound of Example 314 was a white solid (MH⁺ 433).

### EXAMPLE 301*

### 1-1-(4-PYRIDINYLACETYL)-4-[3-METHYL-8-CHLORO-5,6-DIHYDRO-11H-BENZO[5,6]CYCLOHEPTA[1,2-b]PYRIDIN-11-YLIDENE]-PIPERIDINE

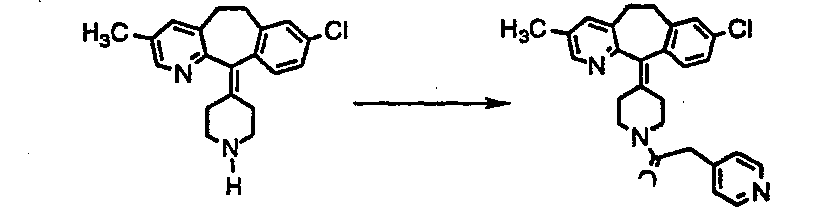

By essentially the same procedure as set forth in Example 180, but using 4-(8-chloro-3-methyl-5,6-dihydro-11-(4-piperidylidene)-11H-benzo[5,6]cyclohepta[1,2-b]pyridine (from Preparative Example 3E) instead of 4-(3,8-dichloro-5,6-dihydro-11-(4-piperidylidene)-11H-benzo[5,6]cyclohepta[1,2-b]pyridine, the title compound was obtained as an off-white solid MH+ 444

### EXAMPLE 303*

### 1-1-(3-PYRIDINYLACETYL)-4-[3-METHYL-8-CHLORO-5,6-DIHYDRO-11H-BENZO[5,6]CYCLOHEPTA[1,2-b]PYRIDIN-11-YLIDENE]-PIPERIDINE

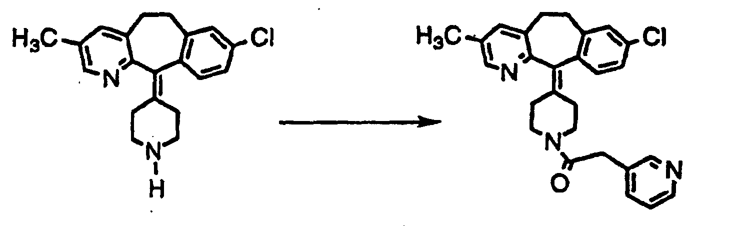

By essentially the same procedure as set forth in Example 180, but using 4-(8-chloro-3-methyl-5,6-dihydro-11-(4-piperidylidene)-11H-benzo[5,6]cyclohepta[1,2-b]pyridine (from Preparative Example 3E) instead of 4-(3,8-dichloro-5,6-dihydro-11-(4-piperidylidene)-11H-benzo[5,6]cyclohepta[1,2-b]pyridine, and 3-pyridylacetic acid instead of 4-pyridylacetic acid, the title compound was obtained as white solid MH+ 444.

### EXAMPLE 307*

By essentially the same procedure as in Example 1, using the title compound from Preparative Example 37B, and 4-pyridylacetic acid the compound of Example 307, identified in Table 8, was obtained, MH⁺ 428.

### EXAMPLE 309*

### 1-1-(4-PYRIDINYLACETYL)-4-[2-METHYL-8-CHLORO-5,6-DIHYDRO-11H-BENZO[5,6]CYCLOHEPTA][1,2-b]PYRIDIN-11-YLIDENE]-PIPERIDINE

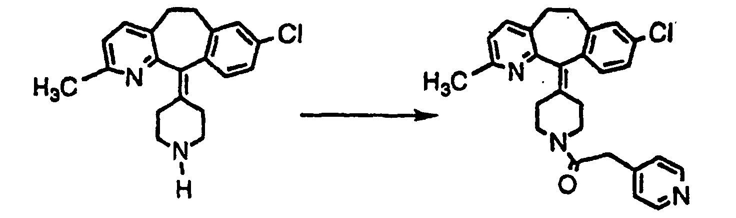

By essentially the same procedure as set forth in Example 180, but using 4-(8-chloro-2-methyl-5,6-dihydro-11-(4-piperidylidene)-11H-benzo[5,6]cyclohepta[1,2-b]pyridine (from Preparative Example 3E) instead of 4-(3,8-dichloro-5,6-dihydro-11-(4-piperidylidene)-11H-benzo[5,6]cyclohepta[1,2-b]pyridine, and 3-pyridylacetic acid instead of 4-pyridylacetic acid, the title compound was obtained as white solid MH+ 444.

### EXAMPLE 311*

### 1-1-(4-PYRIDINYLACETYL)-4-[8,9 DICHLORO-5,6-DIHYDRO-11H-BENZO[5,6]CYCLOHEPTA[1,2-b]PYRIDIN-11-YLIDENE] - PIPERIDINE

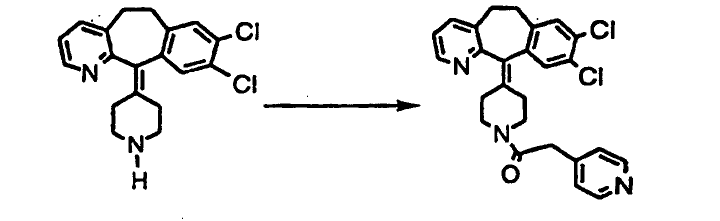

By essentially the same procedure as set forth in Example 180, but using 4-(8,9-dichloro-5,6-dihydro-11-(4-piperidylidene)-11H-benzo[5,6]-cyclohepta[1,2-b]pyridine (from Preparative Example 1H) instead of 4-(3,8-dichloro-5,6-dihydro-11-(4-piperidylidene)-11H-benzo[5,6]cyclohepta[1,2-b]pyridine, and 3-pyridylacetic acid instead of 4-pyridylacetic acid, the title compound was obtained as white solid MH+ 464.

### EXAMPLE 312

By essentially the same procedure as in Example 182, with the exception that 8-chloro-6,11-dihydro-11-(4-piperidinyl)-5H-benzo[5,6]cyclohepta[1,2-b]pyridine is used instead of 8-chloro-11-(1-piperazinyl)-6,11-dihydro-5H-benzo[5,6]cyclohepta[1,2-b]pyridine, the compound of Example 312 was obtained as a white solid (MH⁺ 432). The structure for this compound is given in Table 10.

### EXAMPLE 350*

### 8-CHLORO-11H-BENZO[5,5]CYCLOHEPTA[1,2-b]PYRIDIN-11-YLIDENE)-4-(3-PYRIDINYLACETYL)PIPERAZINE

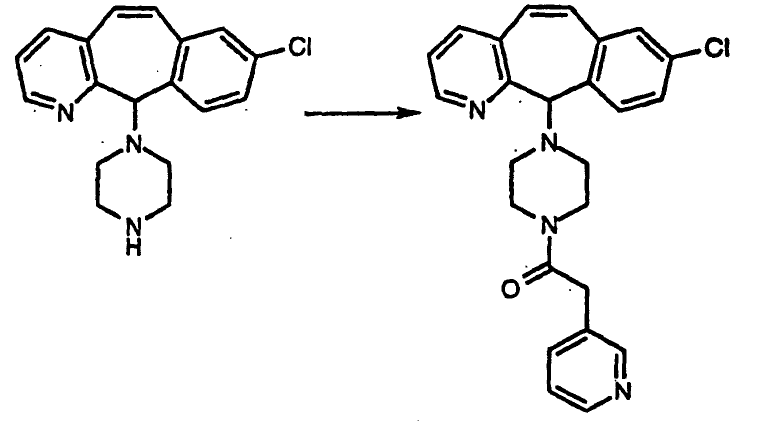

By substituting in Example 75, 0.4g (1.28mmoles) of 8-chloro-11-(1-piperazinyl)-11H-benzo[5,6]cyclohepta[1,2-b]pyridine (Preparative Example 38) for 8-chloro-11-(1-piperazinyl)-6,11-dihydro-5H-benzo[5,6]-cyclohepta[1,2-b]pyridine and 0.1765g (1.28mmoles) of 3-pyridylacetic acid for 4-pyridylacetic acid and using the same method as described in Example 75, one obtains the title compound (0.513g, 93%, MH⁺ 431).

### EXAMPLE 351*

### 1-(3-BROMO-8-CHLORO-6,11-DIHYDRO-5H-BENZO[5,6]CYCLO-HEPTA[1,2-b]PYRIDIN-11-YL)-4-(3-PYRIDINYLACETYL)PIPERAZINE

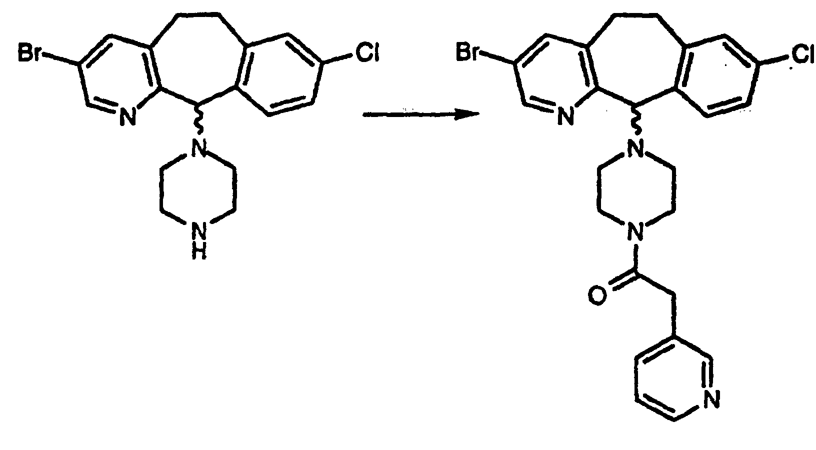

By substituting in Example 75, 3-bromo-8-chloro-11-(1-piperazinyl)-6,11-dihydro-5H-benzo[5,6]cyclohepta[1,2-b]pyridine (0.32g, 0.81mmoles) (Preparative Example 40) for 8-chloro-11-(1-piperazinyl)-6,11-dihydro-5H-benzo[5,6]cyclohepta[1,2-b]pyridine and 3-pyridylacetic acid (0.117g, 0.86mmoles) for 4-pyridylacetic acid and using the method described in Example 50, one obtains the title compound (0.3942g, 95%, MH⁺ 511).

### EXAMPLES 352*-353*

By essentially the same procedures as set forth in Example 351, but using 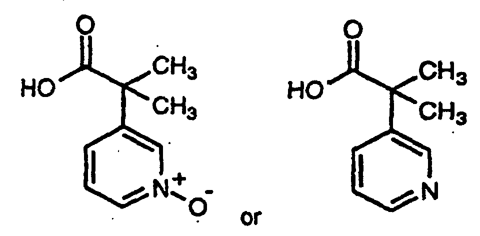 in place of 3-pyridylacetic acid, one obtains compounds of the formulas 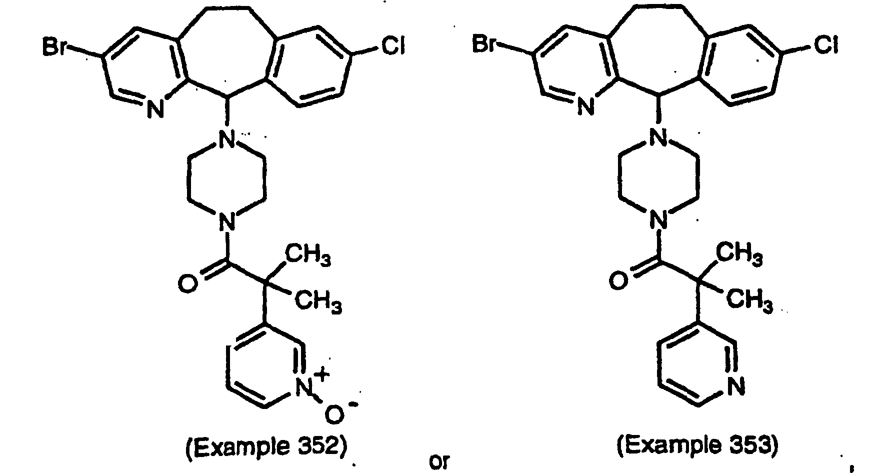 respectively. The compound of Example 352 is a white amorphous solid, yield 65%, Mass Spec MH⁺ 555. The compound of Example 353 is a white amorphous solid, yield 59%, Mass Spec MH⁺ 539.

### EXAMPLE 354

### 4-(3-BROMO 8-CHLORO-6,11-DIHYDRO-5H-BENZO[5,6]CYCLO-HEPTA[1,2-b]PYRIDIN-11-YL)-N-(3-PYRIDINYL)-1-PIPERAZINE-CARBOXAMIDE

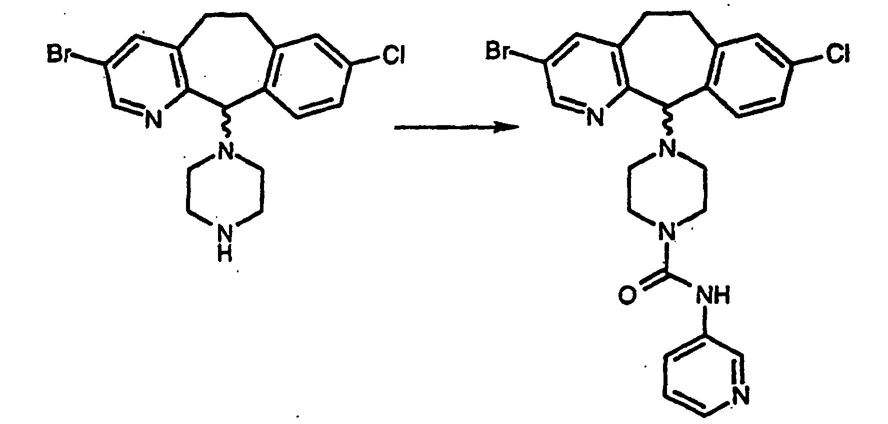

The title compound from Preparative Example 40 (0.37g, 0.94mmoles) was reacted with 3-ethoxycarbonylaminopyridine (Preparative Example 12) (0.123g, 0,94mmoles) under essentially the same conditions as described in Example 183, to give the title compound (0.3164g, 66%, MH⁺ 512).

### EXAMPLE 355*

### 1-(4,8-DICHLORO-6,11-DIHYDRO-5H-BENZO[5,6]CYCLOHEPTA[1,2-b]PYRIDIN-11-YL)-4-(3-PYRIDYLACETYL)PIPERAZINE

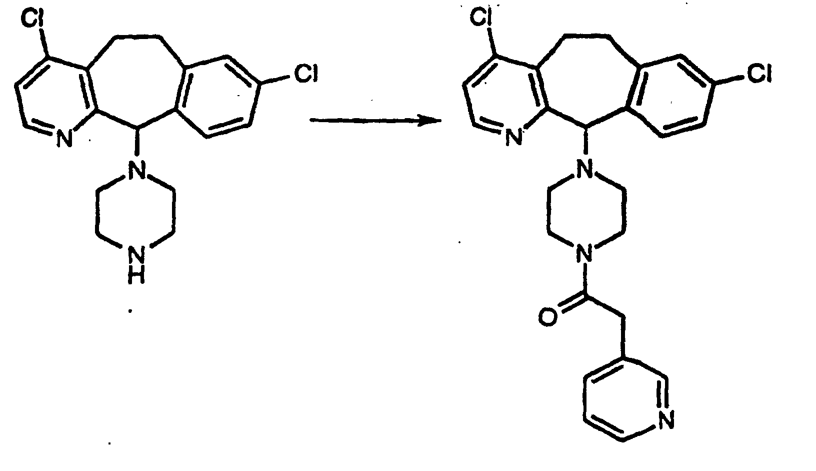

By substituting in Example 75, 4,8-chloro-11-(1-piperazinyl)-6,11-dihydro-5H-benzo[5,6]cyclohepta[1,2-b]pyridine (0.3g, 0.86mmoles) (Preparative Example 41) for 8-chloro-11-(1-piperazinyl)-6,11-dihydro-5H-benzo[5,6]cyclohepta[1,2-b]pyridine and 3-pyridylacetic acid (0.1181g, 0.86mmoles) for 4-pyridylacetic acid and using the method described in Example 50 ,one obtains the title compound (0.357g, 88%, MH⁺ 467).

### EXAMPLE 356*

### 4-[3-BROMO-4,8-DICHLORO-5,6-DIHYDRO-11H-BENZO[5,6]-CYCLOHEPTA[1,2-b]PYRIDIN-11-YLIDENE]-1-(4-PYRIDINYLACETYL)-PIPERIDINE

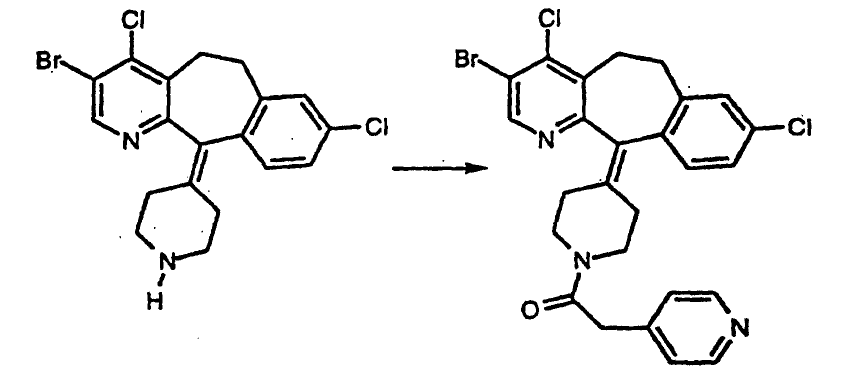

A mixture of the title compound from Preparative Example 46 (0.68 grams), 4-pyridylacetic acid hydrochloride (0.60 grams), 1-(3-dimethylaminopropyl)-3-ethyl carbodiimide hydrochloride (0.65 grams), 4-methylmorpholine (0.6 mL) and anhydrous dimethylformamide (20 mL) was stirred at 25°C for 48 hours. Concentration *in vacuo* provided a residue which was diluted with dichloromethane and washed with 1*N* aqueous sodium hydroxide and brine. The organic phase was dried over anhydrous magnesium sulfate and concentrated *in vacuo* to provide a residue which was purified by flash column chromatography (silica gel) using 2-5% methanol-dichloromethane saturated with ammonium hydroxide to afford the title compound (0.06 grams, 7%, MH⁺ 544).

### EXAMPLE 358*

### A. 4-(8-CHLORO-3-NITRO-5,6-DIHYDRO-11-(4-PIPERIDYLIDENE)-11H-BENZO[5,6]CYCLOHEPTA[1,2-b]PYRIDINE

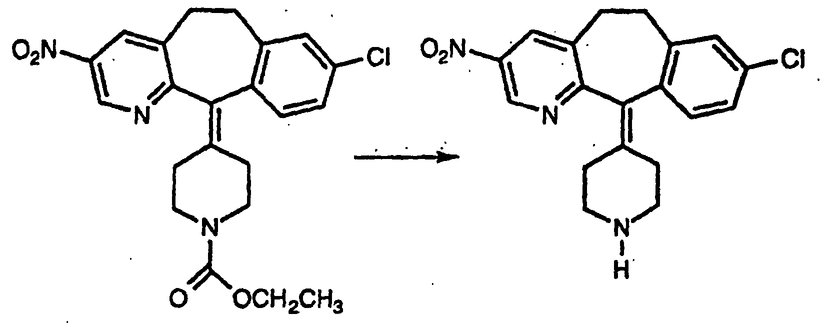

Hydrolyze the title compound of Preparative Example 47A (10.0g, mmol) by dissolving in conc. HCI (250mL) and heating to 100°C for 16h. The cooled acidic mixture was neutralized with 1M NaOH (950 mL). The mixture was extracted with methylene chloride. The latter was dried over magnesium sulfate. Filtration and concentration afforded the title compound in 99% yield as a solid. MH+ 358.

### B. 1-1-(4-PYRIDINYLACETYL)-4-[3-BROMO-8-CHLORO-5,6-DIHYDRO-11H-BENZO[5,6]CYCLOHEPTA[1,2-b]PYRIDIN-11-YLIDENE]-PIPERIDINE

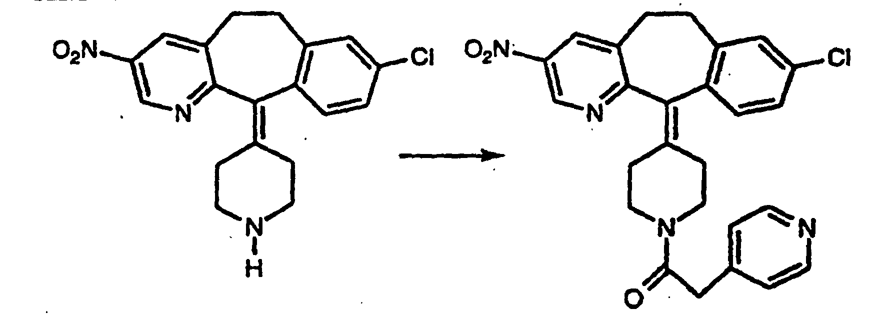

By essentially the same procedure as set forth in Example 180, but using 4-(8-chloro-3-nitro-5,6-dihydro-11-(4-piperidylidene)-11H-benzo[5,6]cyclohepta[1,2-b]pyridine instead of 4-(3,8-dichloro-5,6-dihydro-11-(4-piperidylidene)-11H-benzo[5,6]cyclohepta[1,2-b]pyridine, the title compound was obtained as an off-white solid. Mp = 111.3-112.2°C, MH+ 475.

### ASSAYS

### 1. In vitro enzyme assays: Inhibition of farnesyl protein transferase and geranylgeranyl protein transferase.

Both farnesyl protein transferase (FPT) and geranylgeranyl protein transferase (GGPT) I were partially purified from rat brain by ammonium sulfate fractionation followed by Q-Sepharose (Pharmacia, Inc.) anion exchange chromatography essentially as described by Yokoyama et al (Yokoyama, K., et al., (1991), A protein geranylgeranyltransferase from bovine brain: Implications for protein prenylation specificity, **Proc. Natl. Acad. Sci USA 88: 5302-5306**, the disclosure of which is incorporated herein by reference thereto). Human farnesyl protein transferase was also expressed in E. coli, using cDNA clones encoding both the α any β subunits. The methods used were similar to those published (Omer, C. et al., (1993), Characterization of recombinant human farnesyl protein transferase: Cloning, expression, farnesyl diphosphate binding, and functional homology with yeast prenyl-protein transferases, Biochemistry 32:5167-5176). Human farnesyl protein transferase was partially-purified from the soluble protein fraction of E. coli as described above. The tricyclic farnesyl protein transferase inhibitors disclosed herein inhibited both human and rat enzyme with similar potencies. Two forms of val¹²-Ha-Ras protein were prepared as substrates for these enzymes, differing in their carboxy terminal sequence. One form terminated in cysteine-valine-laucine-serine (Ras-CVLS) the other in cystein-valine-leucine-leucine (Ras-CVLL). Ras-CVLS is a substrate for the famesyl protein transferase while Ras-CVLL is a substrate for geranylgeranyl protein transferase I. The cDNAs encoding these proteins were constructed so that the proteins contain an amino-terminal extension of 6 histidine residues. Both proteins were expressed in Escherichia coli and purified using metal chelate affinity chromatography. The radiolabelled isoprenyl pyrophosphate substrates, [³H]famesyl pyrophosphate and [³H]geranylgeranyl pyrophosphate, were purchased from DuPont/New England Nuclear.

Several methods for measuring farnesyl protein transferase activity have been described (Reiss et al 1990, **Cell 62**: 81; Schaber et al 1990, **J. Biol. Chem. 265:** 14701; Manne et al 1990, **PNAS 87:** 7541; and Barbacid & Manne 1993, U.S. Patent No. 5,185,248). The activity was assayed by measuring the transfer of [³H]farnesyl from [³H]famesyl pyrophosphate to Ras-CVLS using conditions similar to those described by Reiss et al. 1990 (Cell **62**: 81) The reaction mixture contained 40 mM Hepes, pH 7.5; 20 mM magnesium chloride; 5 mM dithiothreitol; 0.25 µM [³H]farnesyl pyrophosphate; 10 µl Q-Sepharose-purified farnesyl protein transferase; the indicated concentration of tricyclic compound or dimethylsulfoxide (DMSO) vehicle control (5% DMSO final); and 5 µM Ras-CVLS in a total volume of 100 µl. The reaction was allowed to proceed for 30 minutes at room temperature and then stopped with 0.5 ml of 4% sodium dodecyl sulfate (SDS) followed by 0.5 ml of cold 30% trichloracetic acid (TCA). Samples were allowed to sit on ice for 45 minutes and precipitated Ras protein was then collected on GF/C filter paper mats using a Brandel cell harvester. Filter mats were washed once with 6% TCA, 2% SDS and radioactivity was measured in a Wallac 1204 Betaplate BS liquid scintillation counter. Percent inhibition was calculated relative to the DMSO vehicle control.

The geranylgeranyl protein transferase I assay was essentially identical to the farnesyl protein transferase assay described above, with two exceptions: [³H]geranylgeranylpyrophosphate replaced farnesyl pyrophosphate as the isoprenoid donor and Ras-CVLL was the protein acceptor. This is similar to the assay reported by Casey et al (Casey, P.J., et al., (1991), Enzymatic modification of proteins with a geranylgeranyl isoprenoid, **Proc. Natl. Acad. Sci, USA 88: 8631-8635,** the' disclosure of which is incorporated herein by reference thereto).

### 2. Cell-Based Assay: Transient expression of val¹²-Ha-Ras-CVLS and val¹²-Ha-Ras-CVLL in COS monkey kidney cells: Effect of farnesyl protein transferase inhibitors on Ras processing and on disordered cell growth induced by transforming Ras.

COS monkey kidney cells were transfected by electroporation with the plasmid pSV-SPORT (Gibco/BRL) containing a cDNA insert encoding either Ras-CVLS or Ras-CVLL, leading to transient overexpression of a Ras substrate for either farnesyl protein transferase or geranylgeranyl protein transferase I, respectively (see above).

Following electroporation, cells were plated into 6-well tissue culture dishes containing 1.5 ml of Dulbecco's-modified Eagle's media (GIBCO, Inc.) supplemented with 10% fetal calf serum and the appropriate farnesyl protein transferase inhibitors. After 24 hours, media was removed and fresh media containing the appropriate drugs was re-added.

48 hours after electroporation cells were examined under the microscope to monitor disordered cell growth induced by transforming Ras. Cells expressing transforming Ras become more rounded and refractile and overgrow the monolayer, reminiscent of the transformed phenotype. Cells were then photographed, washed twice with 1 ml of cold phosphate-buffered saline (PBS) and removed from the dish by scraping with a rubber policeman into 1 ml of a buffer containing 25 mM Tris, pH 8.0; 1 mM ethylenediamine tetraacetic acid; 1 mM phenylmethylsulfonyl fluoride; 50 µM leupeptin; and 0.1 µM pepstatin. Cells were lysed by homogenization and cell debris was removed by centrifugation at 2000 x g for 10 min

. Cellular protein was precipitated by addition of ice-cold trichloroacetic acid and redissolved in 100 µl of SDS-electrophoresis sample buffer. Samples (5-10 µl) were loaded onto 14% polyacrylamide minigels (Novex, Inc.) and electrophoresed until the tracking dye neared the bottom of the gel. Proteins resolved on the gels were electroblotted onto nitrocellulose membranes for immunodetection.

Membranes were blocked by incubation overnight at 4°C in PBS containing 2.5% dried milk and 0.5% Tween-20 and then incubated with a Ras-specific monoclonal antibody, Y13-259 (Furth, M.E., et al., (1982), Monoclonal antibodies to the p21 products of the transforming gene of Harvey murine sarcome virus and of the cellular ras gene family, J. **Virol. 43: 294-304),** in PBS containing 1% fetal calf serum for one hour at room temperature. After washing, membranes were incubated for one hour at room temperature with a 1:5000 dilution of secondary antibody, rabbit anti-rat IgG conjugated to horseradish peroxidase, in PBS containing 1% fetal calf serum. The presence of processed and unprocessed Ras-CVLS or Ras-CVLL was detected using a colorimetric peroxidase reagent (4-chloro-1-naphthol) as described by the manufacturer (Bio-Rad).

### 3. Cell Mat Assay:

Normal human HEPM fibroblasts were planted in 3.5 cm dishes at a density of 5 x 10⁴ cells/dish in 2 ml growth medium, and incubated for 3-5d to achieve confluence. Medium was aspirated from each dish and the indicator tumor cells, T24-BAG4 human bladder carcinoma cells expressing an activated H-ras gene, were planted on top of the fibroblast monolayer at a density of 2 x 10³cells/dish in 2 ml growth medium, and allowed to attach overnight. Compound-induced colony inhibition was assayed by addition of serial dilutions of compound directly to the growth medium 24 h after tumor cell planting, and incubating cells for an additional 14 d to allow colony formation. Assays were terminated by rinsing monolayers twice with phosphate-buffered saline (PBS), fixing the monolayers with a 1% glutaraldehyde solution in PBS, then visualizing tumor cells by staining with X-Gal (Price, J., et al., Lineage analysis in the vertebrate nervous system by retrovirus-mediated gene transfer, Proc. Natl. Acad. Sci.**84**, 156-160(1987)). In the colony inhibition assay, compounds were evaluated on the basis of two IC₅₀ values: the concentration of drug required to prevent the increase in tumor cell number by 50% (tIC₅₀) and the concentration of drug required to reduce the density of cells comprising the cell mat by 50% (mlC₅₀). Both IC₅₀ values were obtained by determining the density of tumor cells and mat cells by visual inspection and enumeration of cells per colony and the number of colonies under the microscope. The therapeutic index of the compound was quantitatively expressed as the ratio of mIC₅₀/tIC₅₀, with values greater than one indicative of tumor target specificity.

### RESULTS OF ASSAYS - TABLES 7 TO 19

The compounds listed in Table 7 refer to compounds of Formula 500.00:

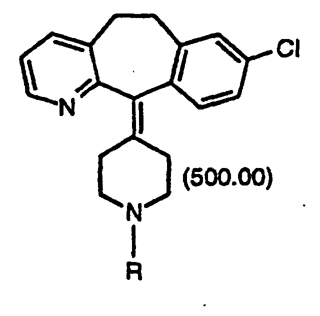

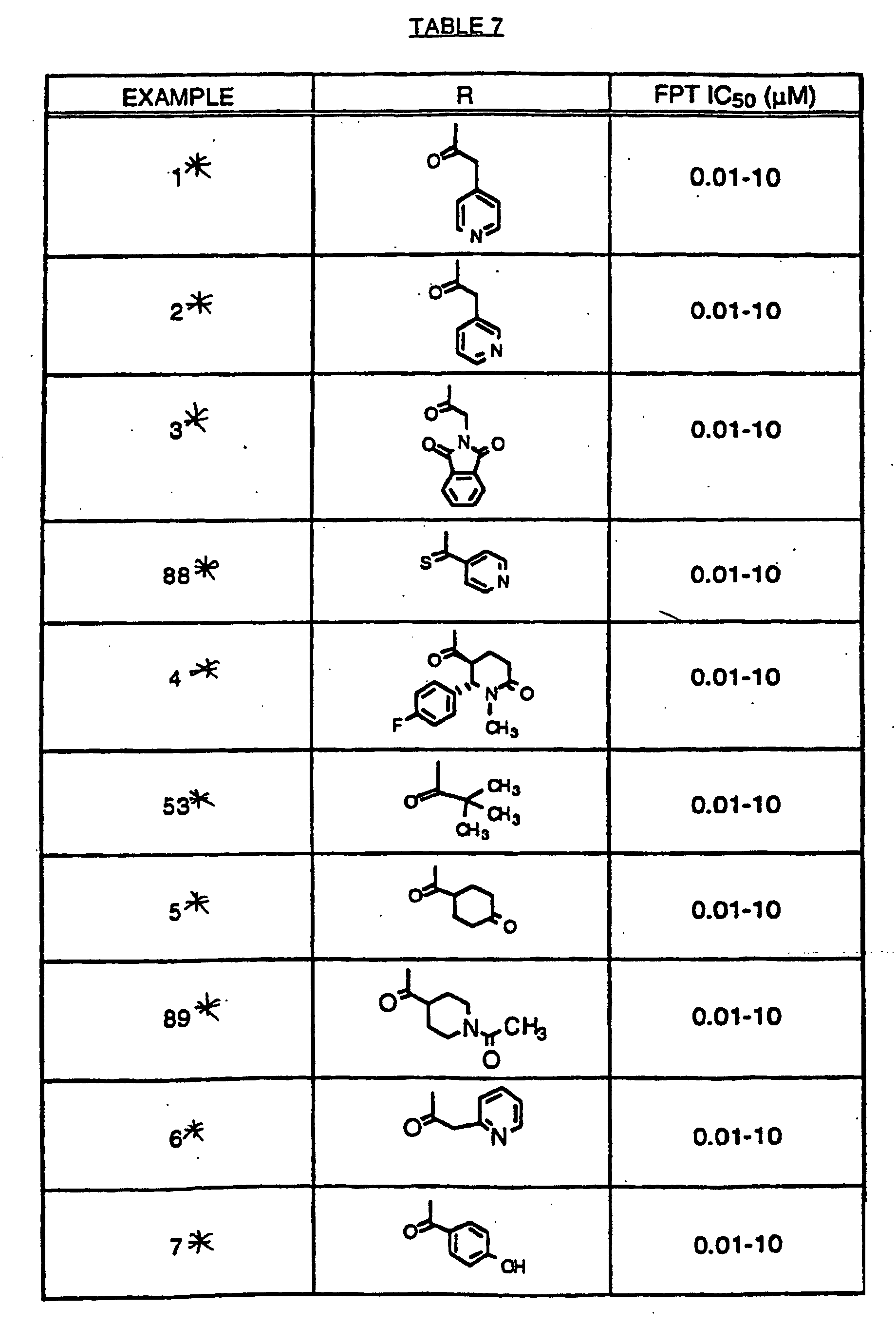

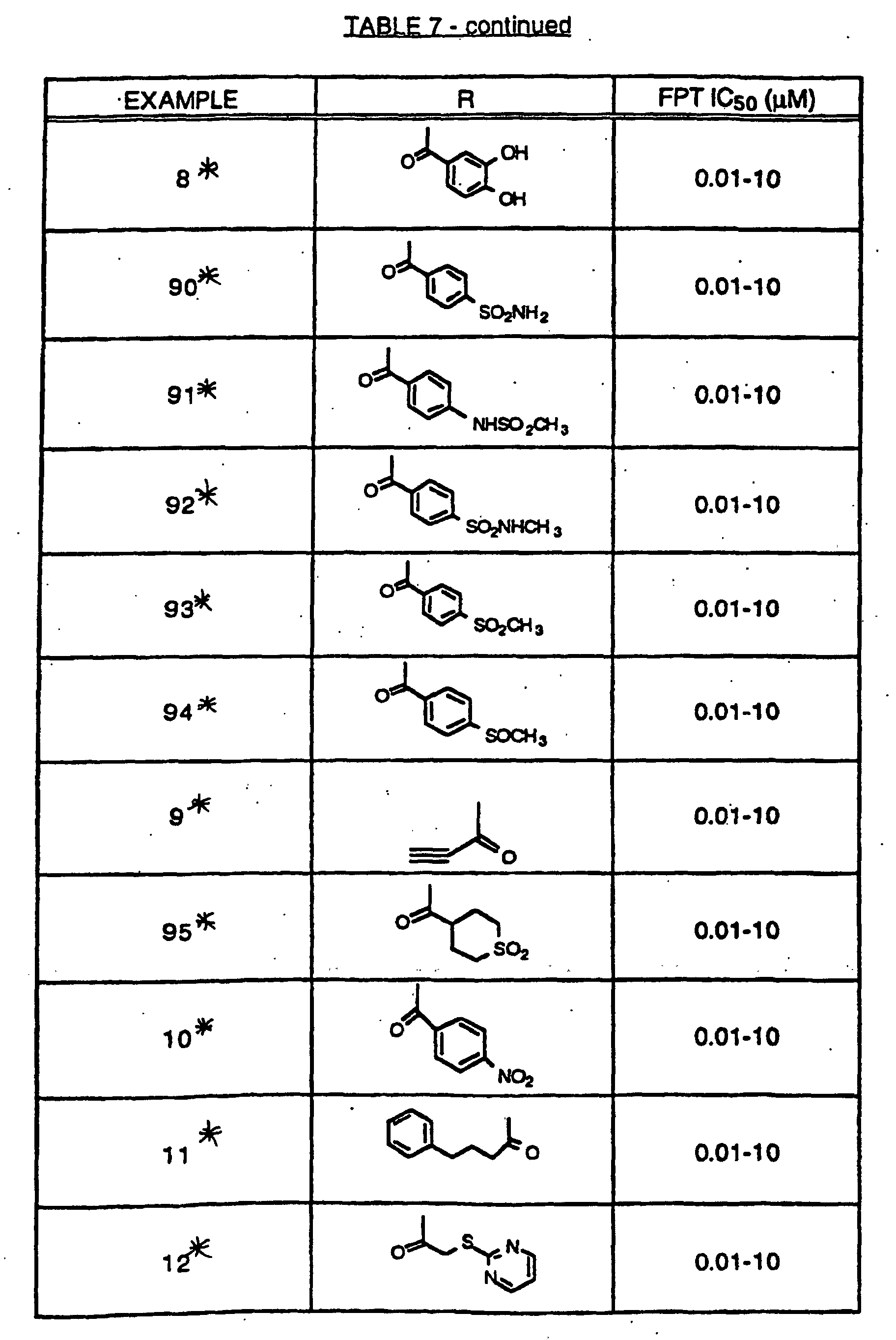

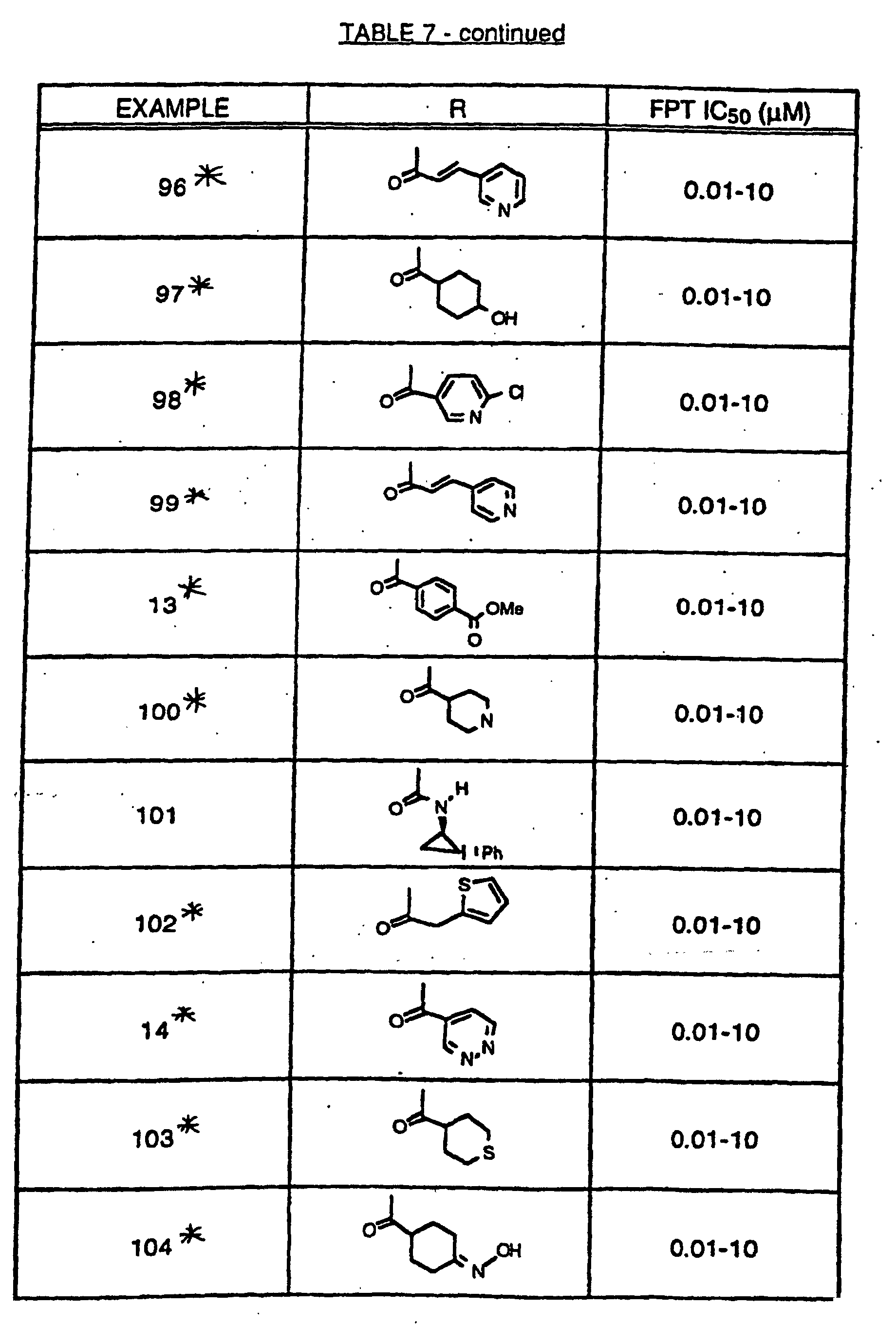

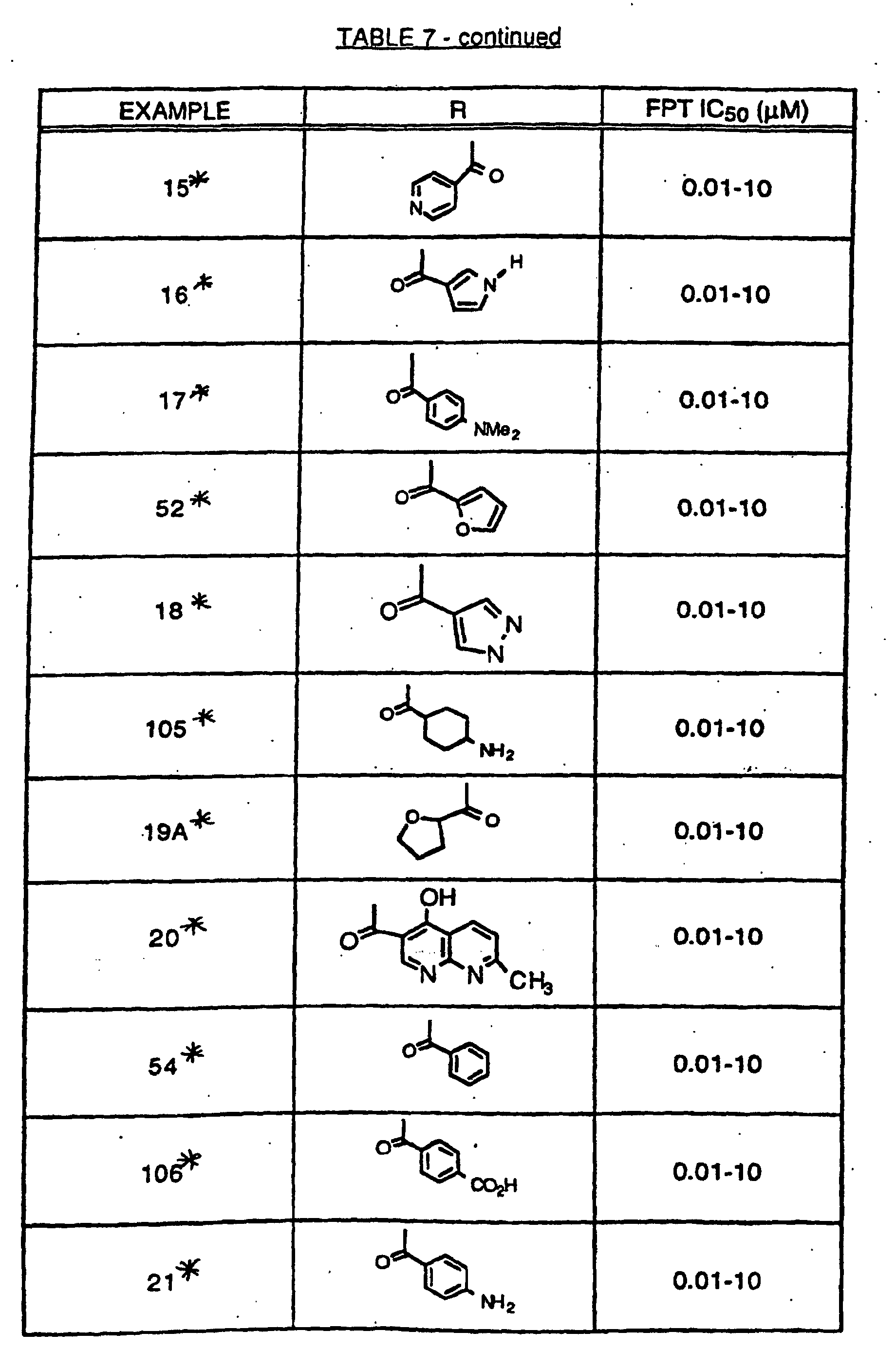

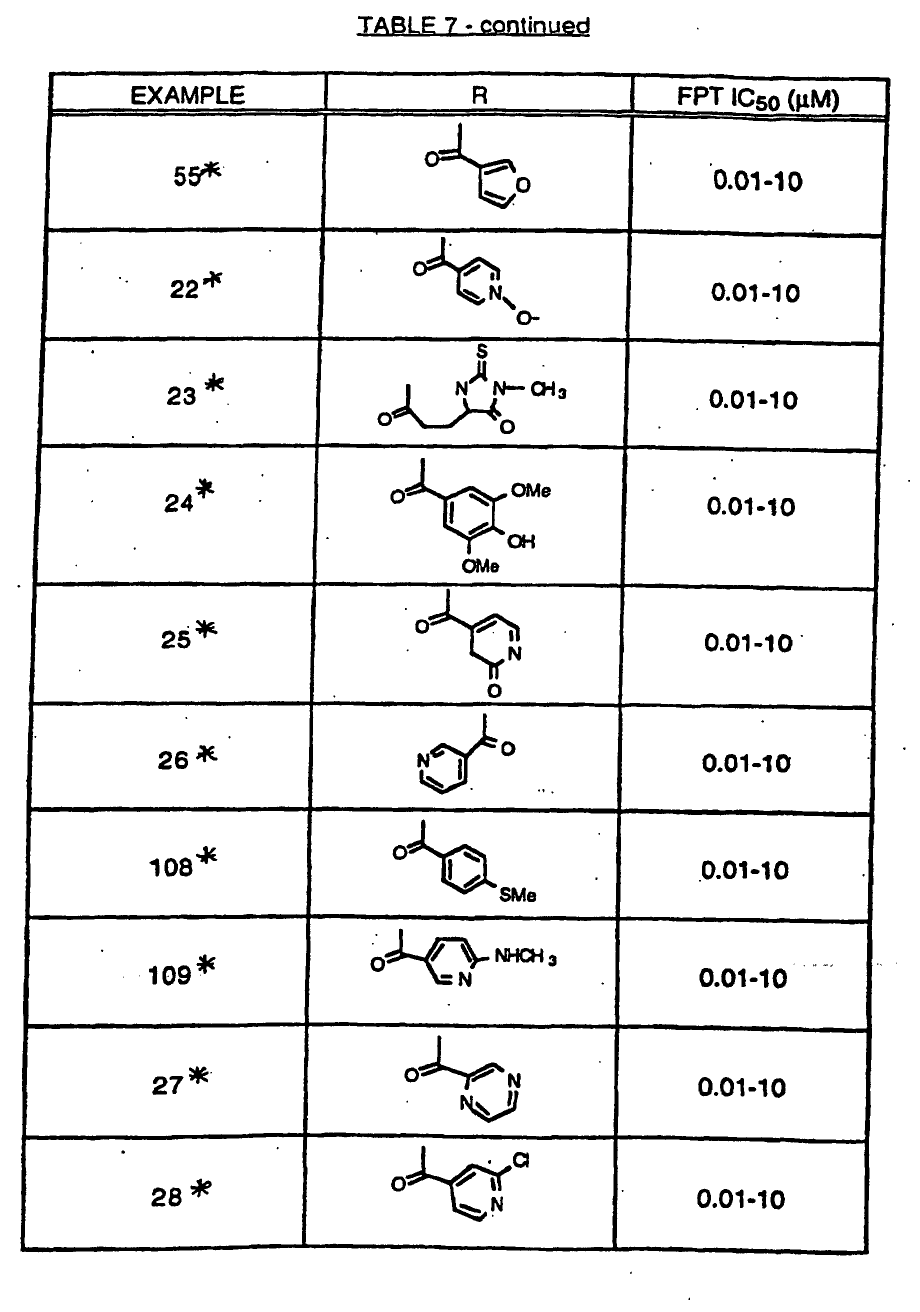

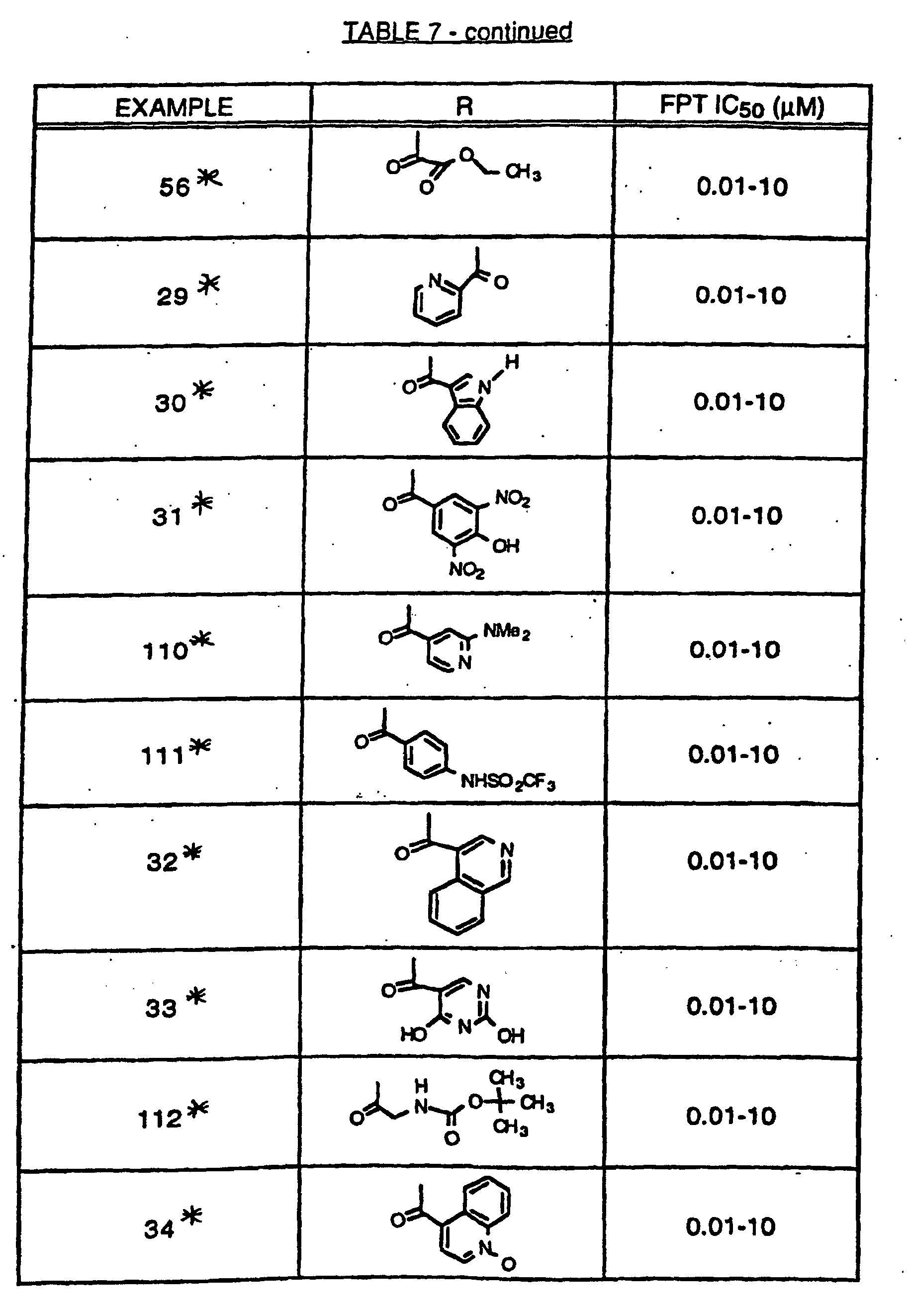

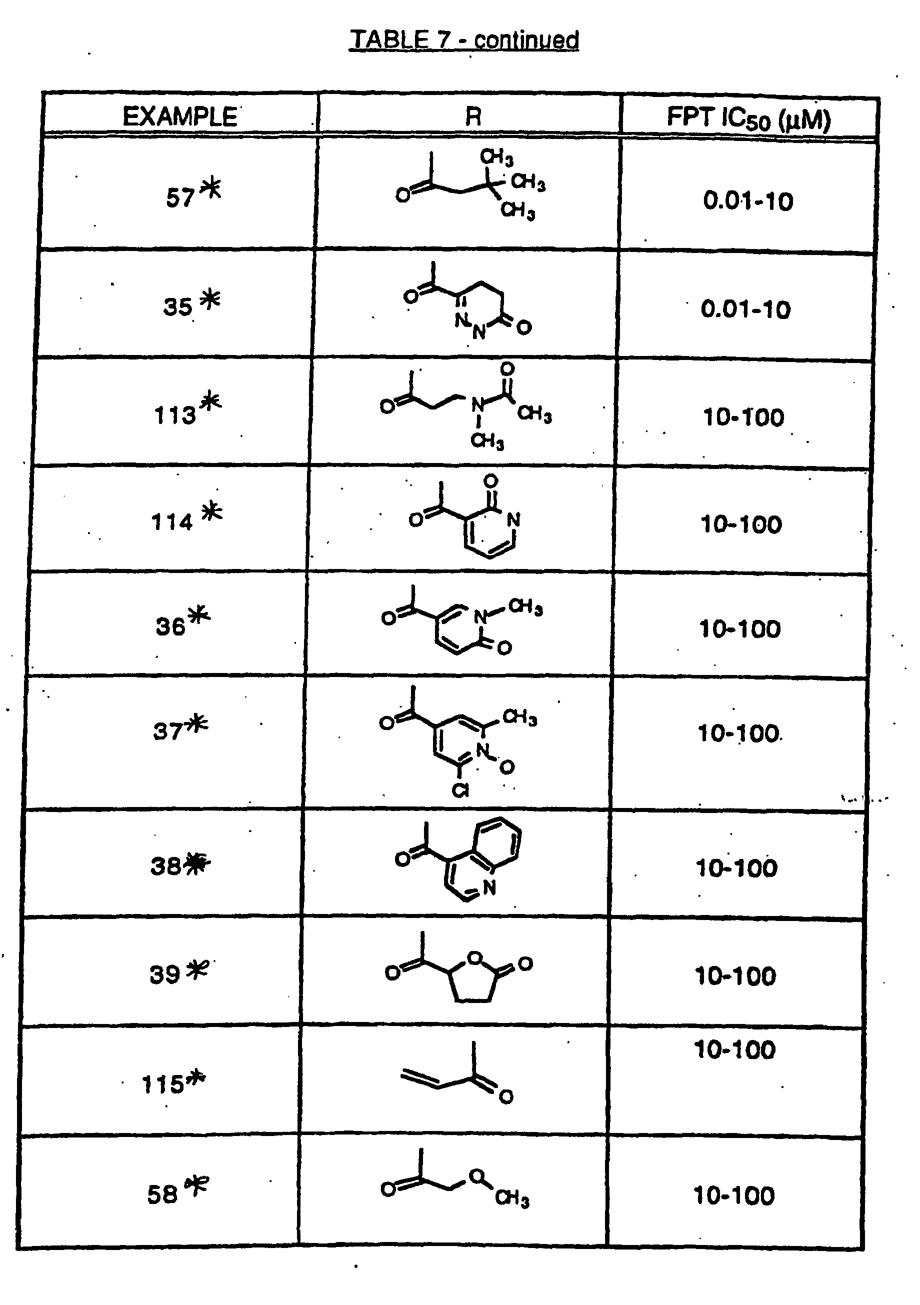

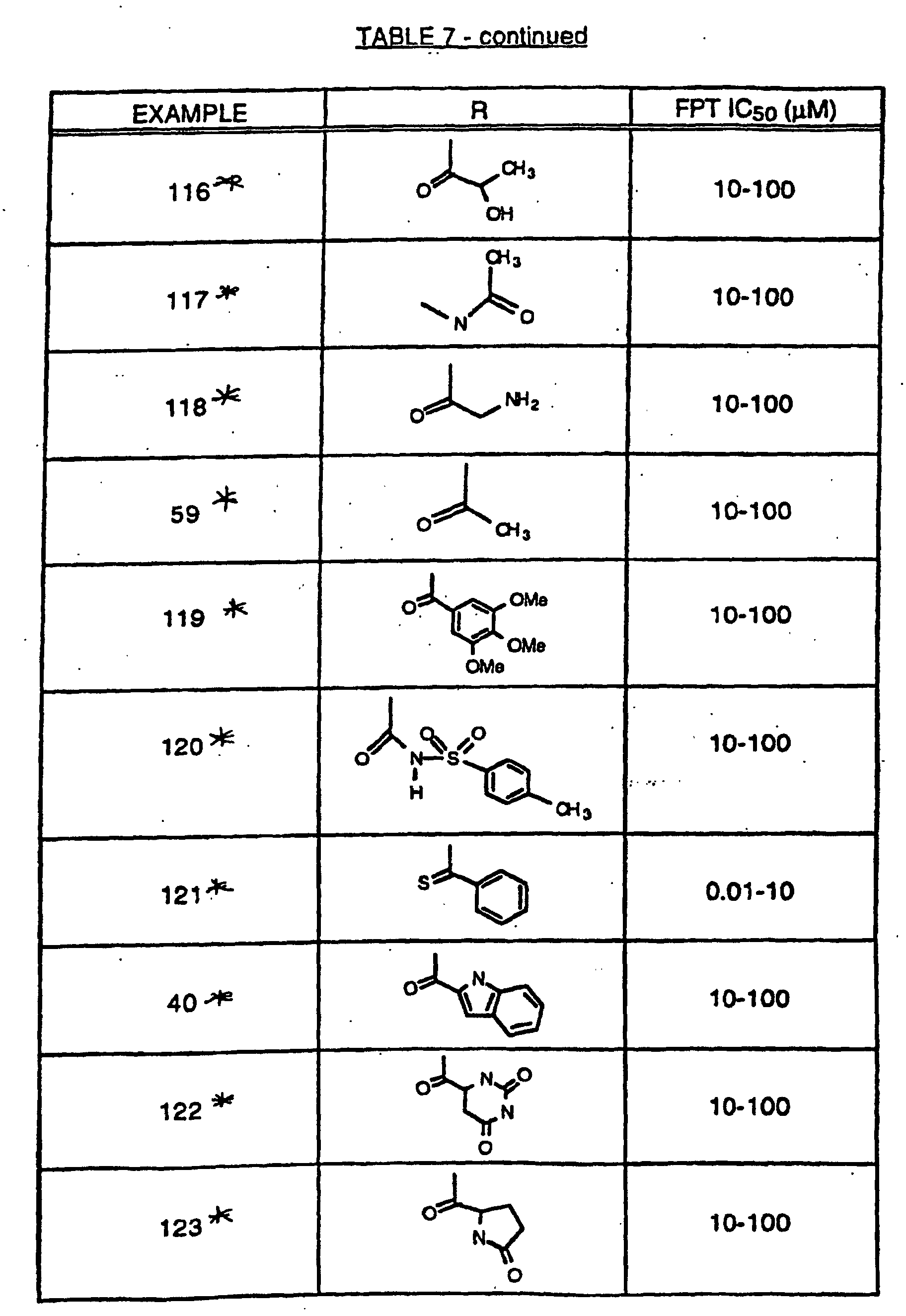

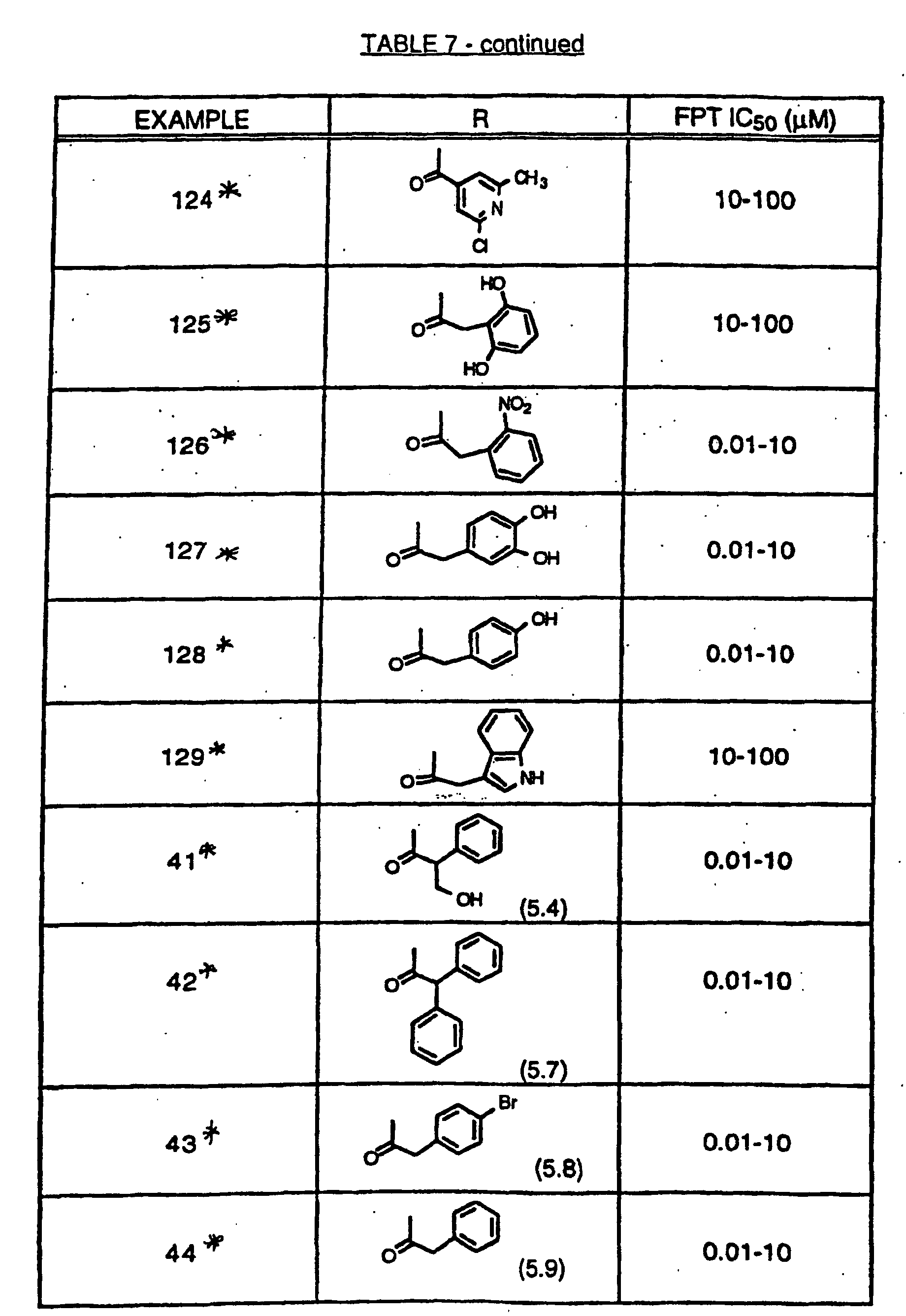

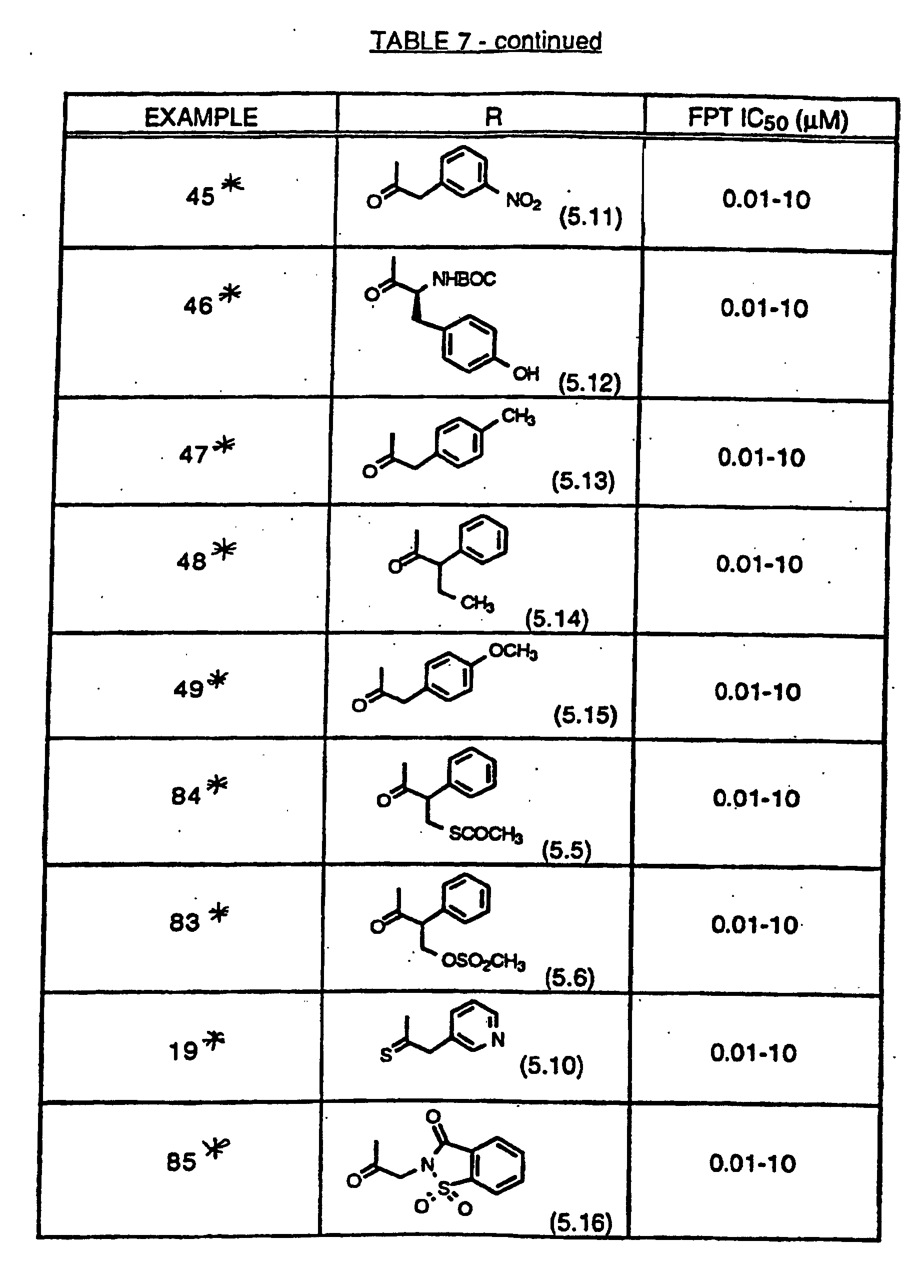

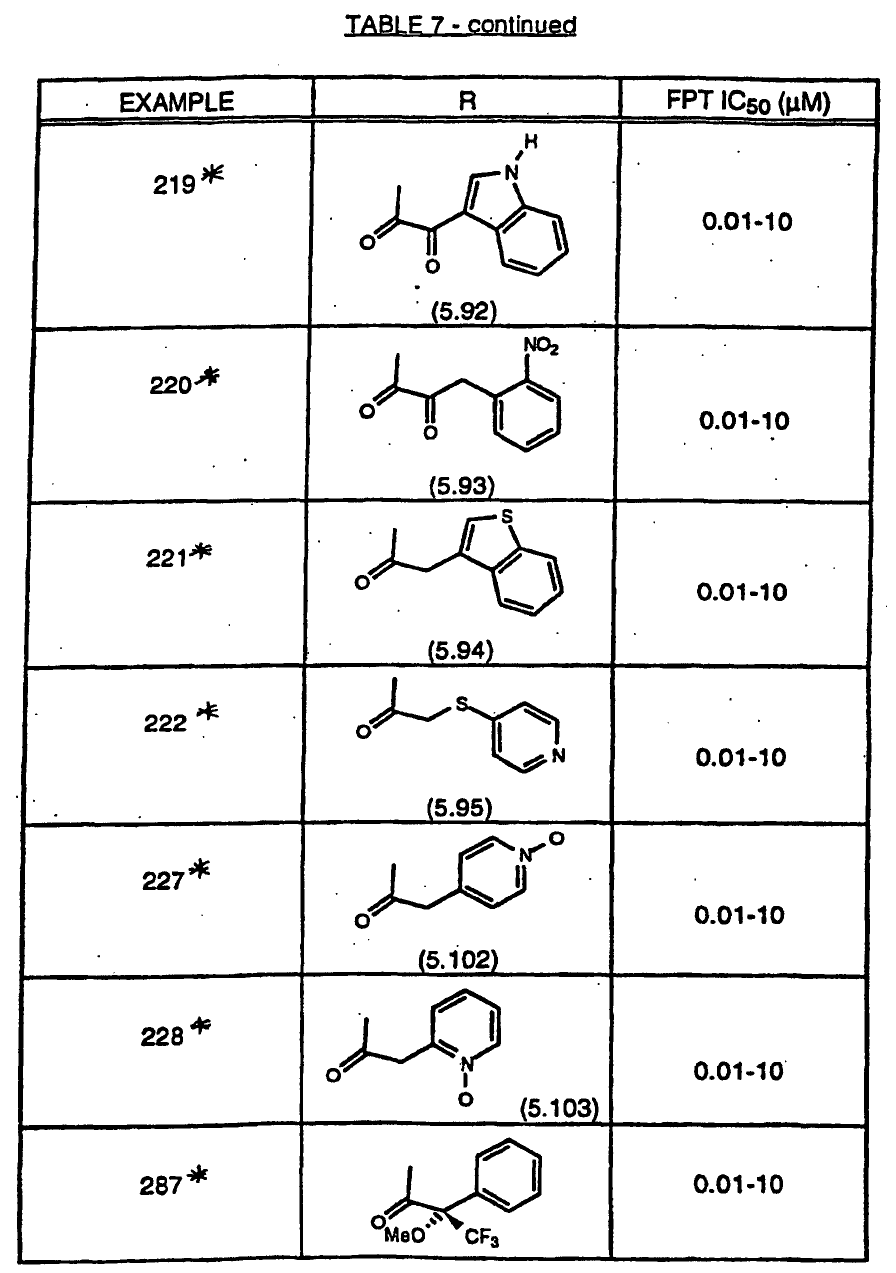

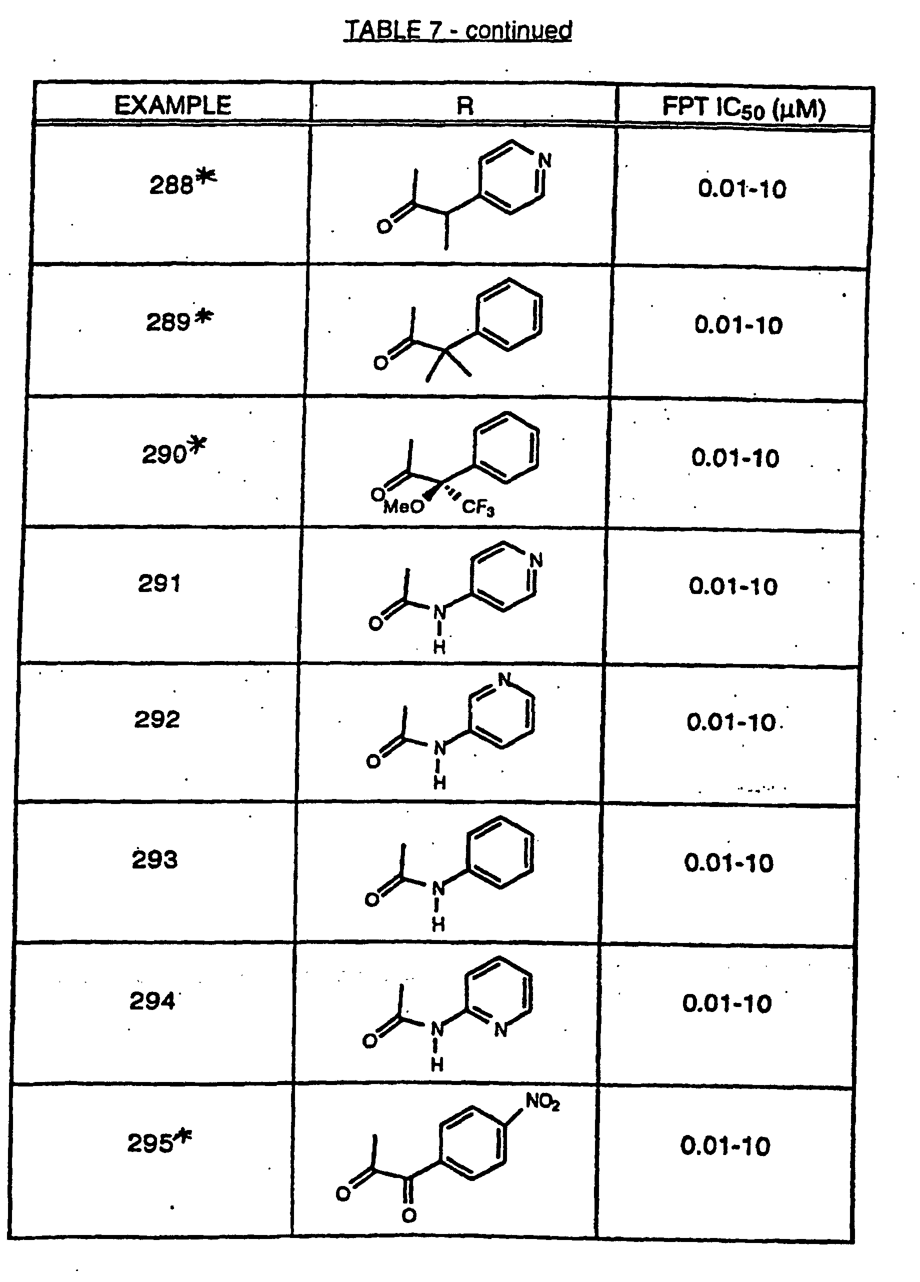

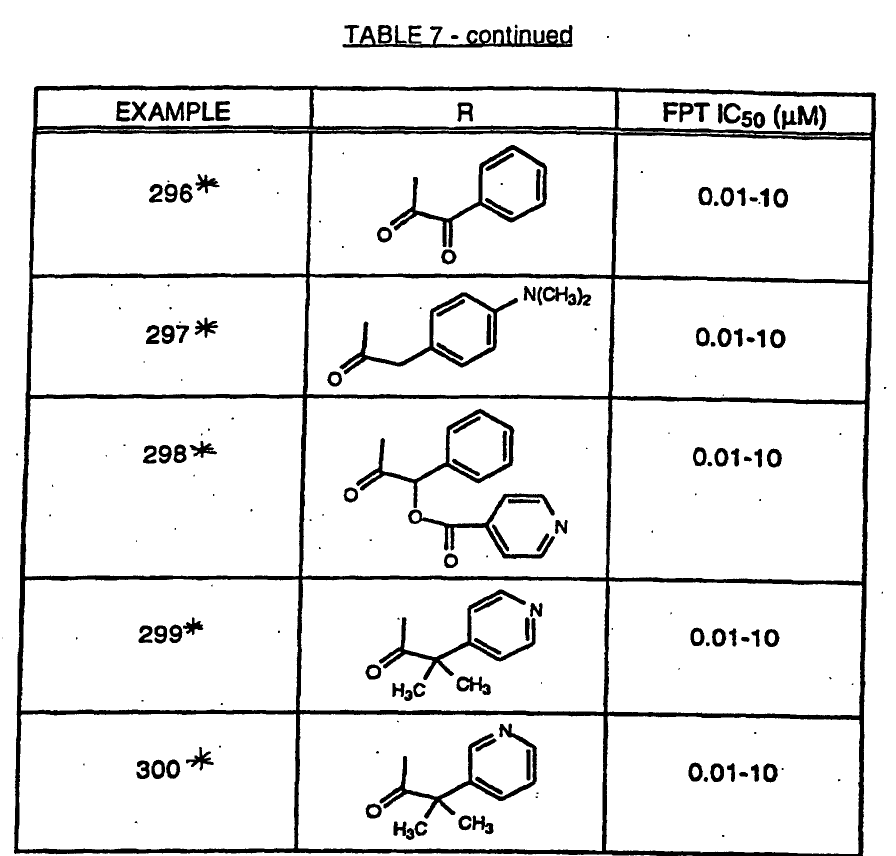

The compounds listed in Table 8 refer to compounds of Formula 505.00:

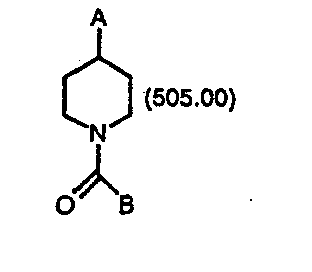

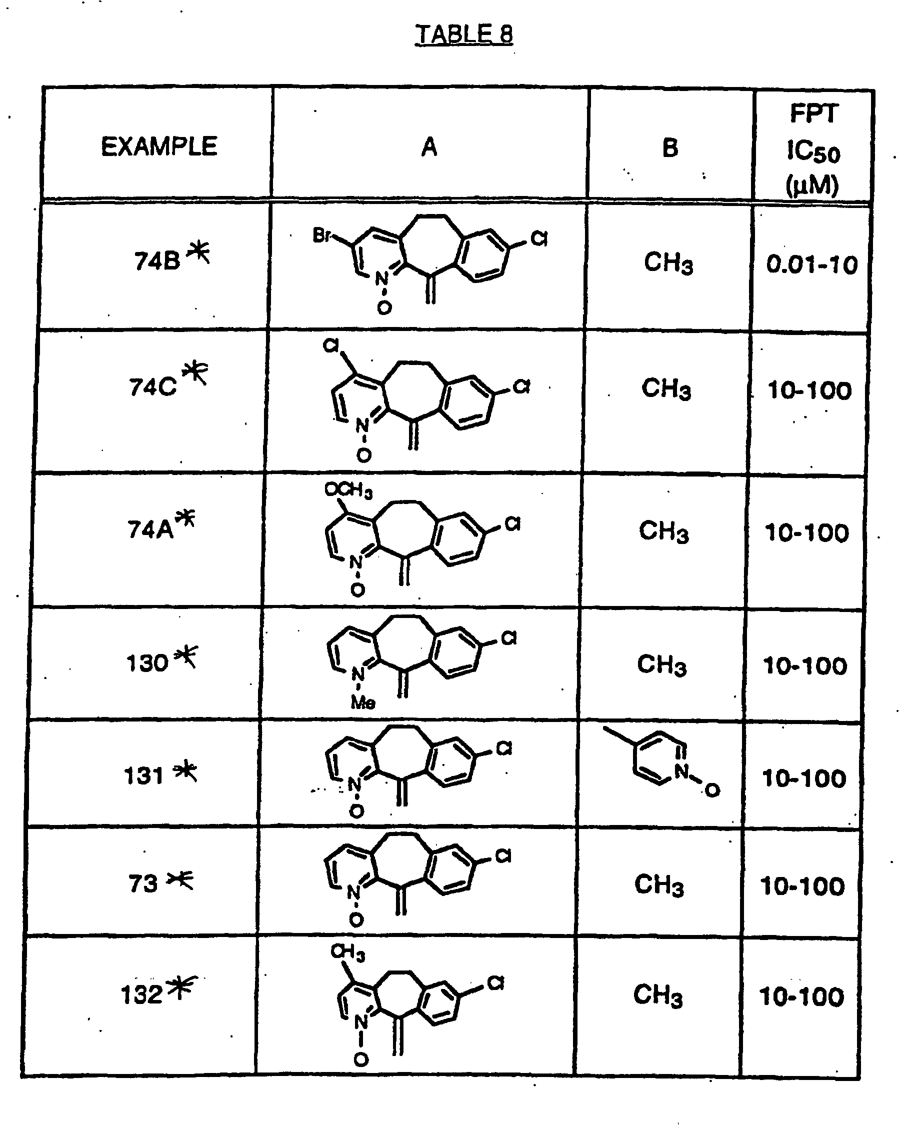

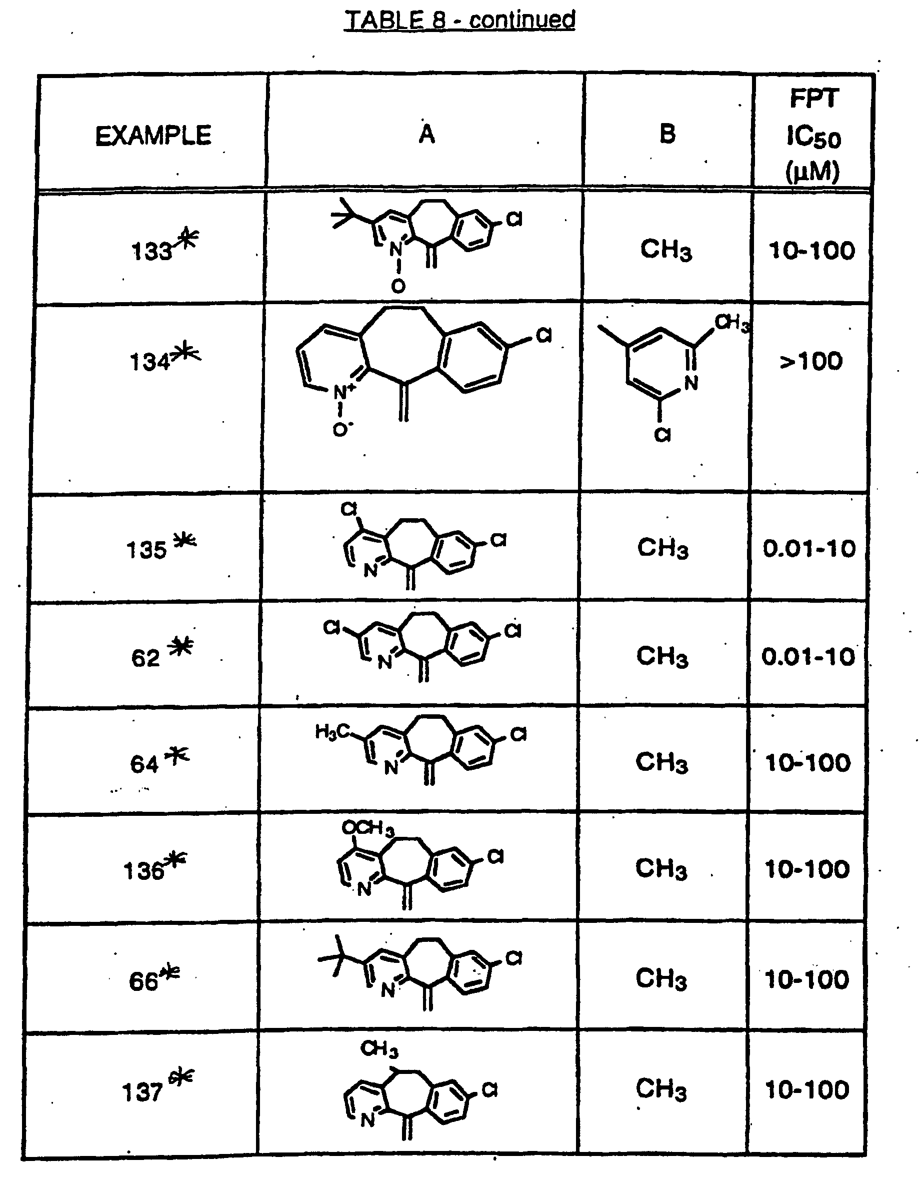

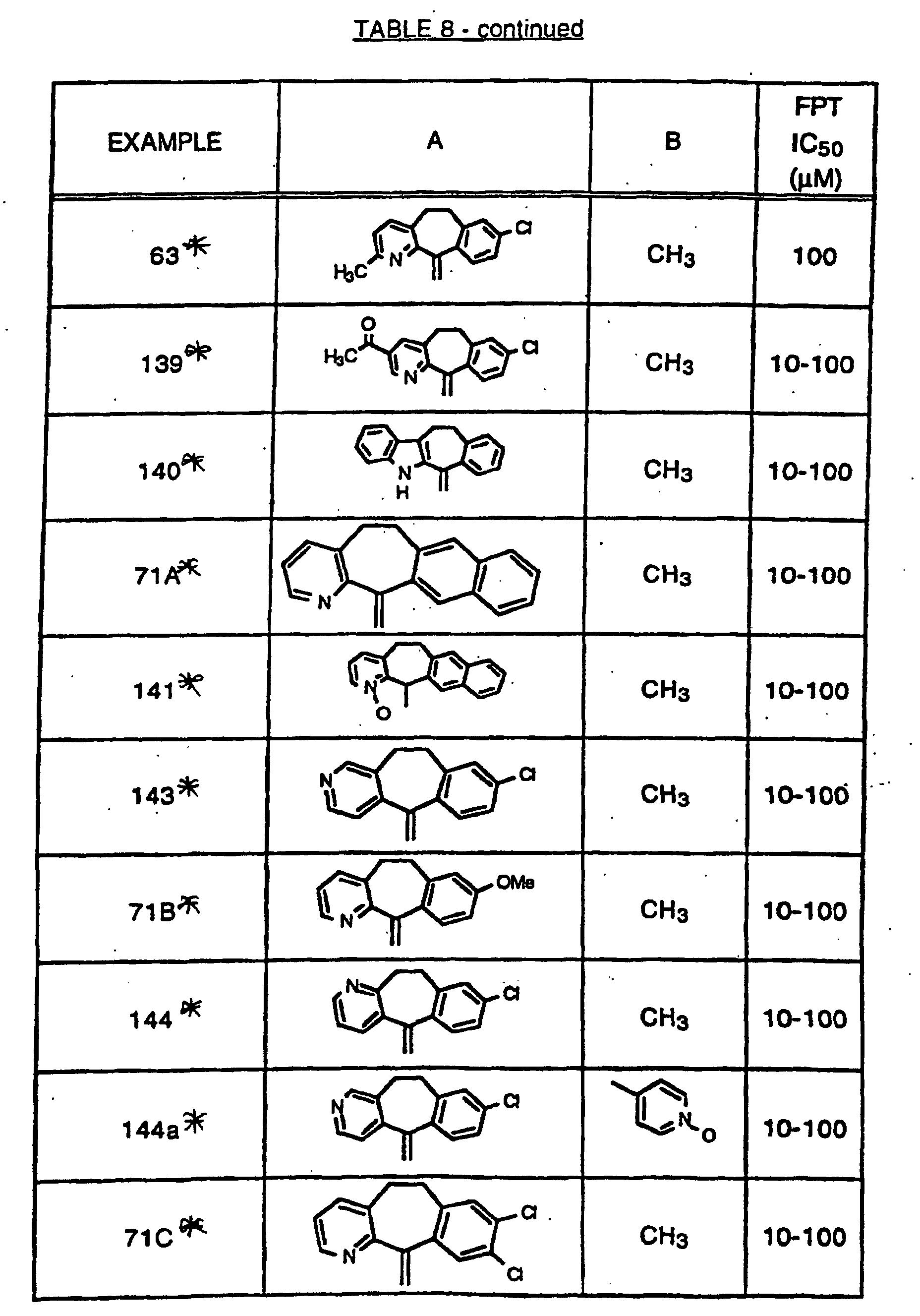

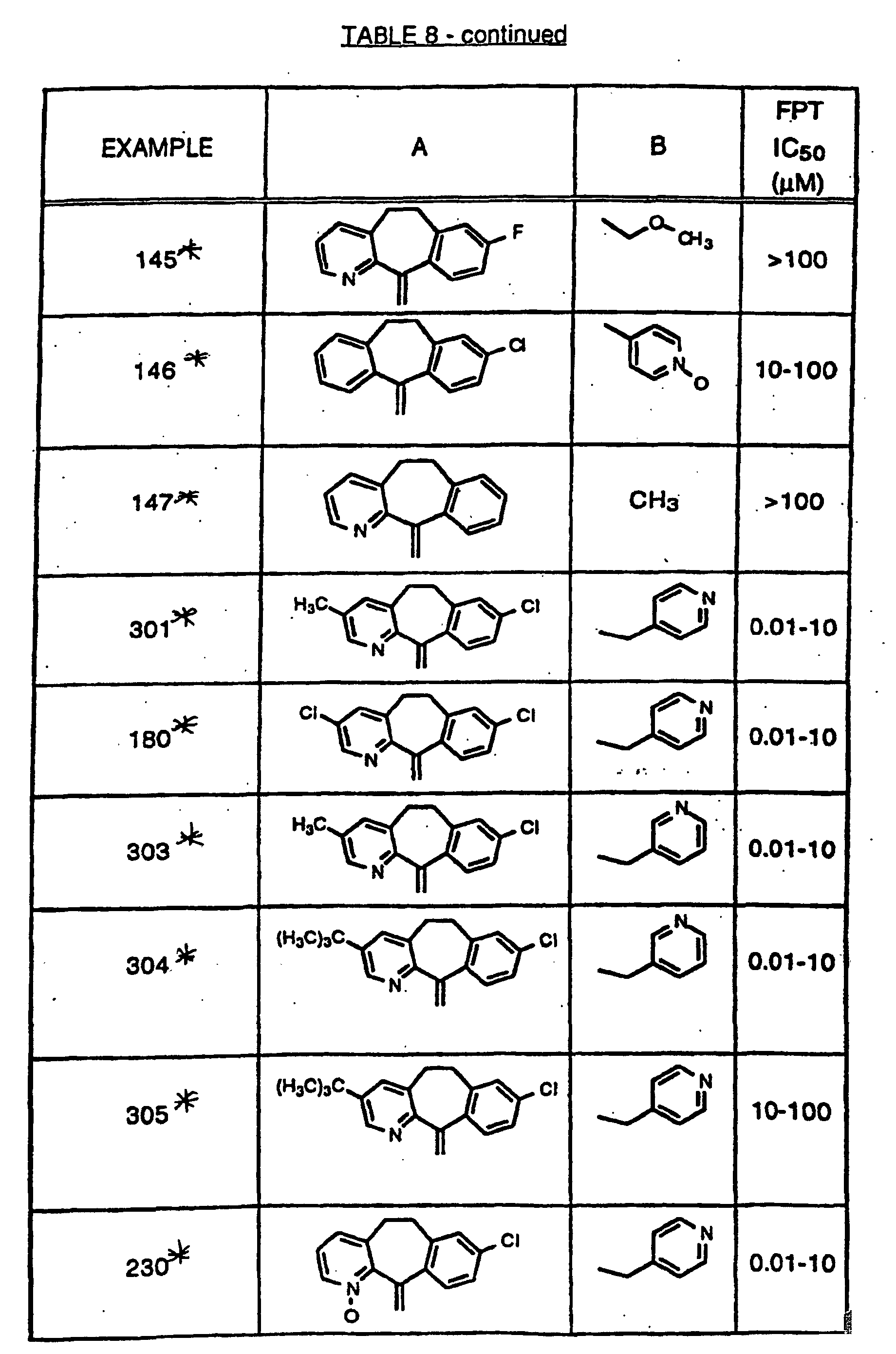

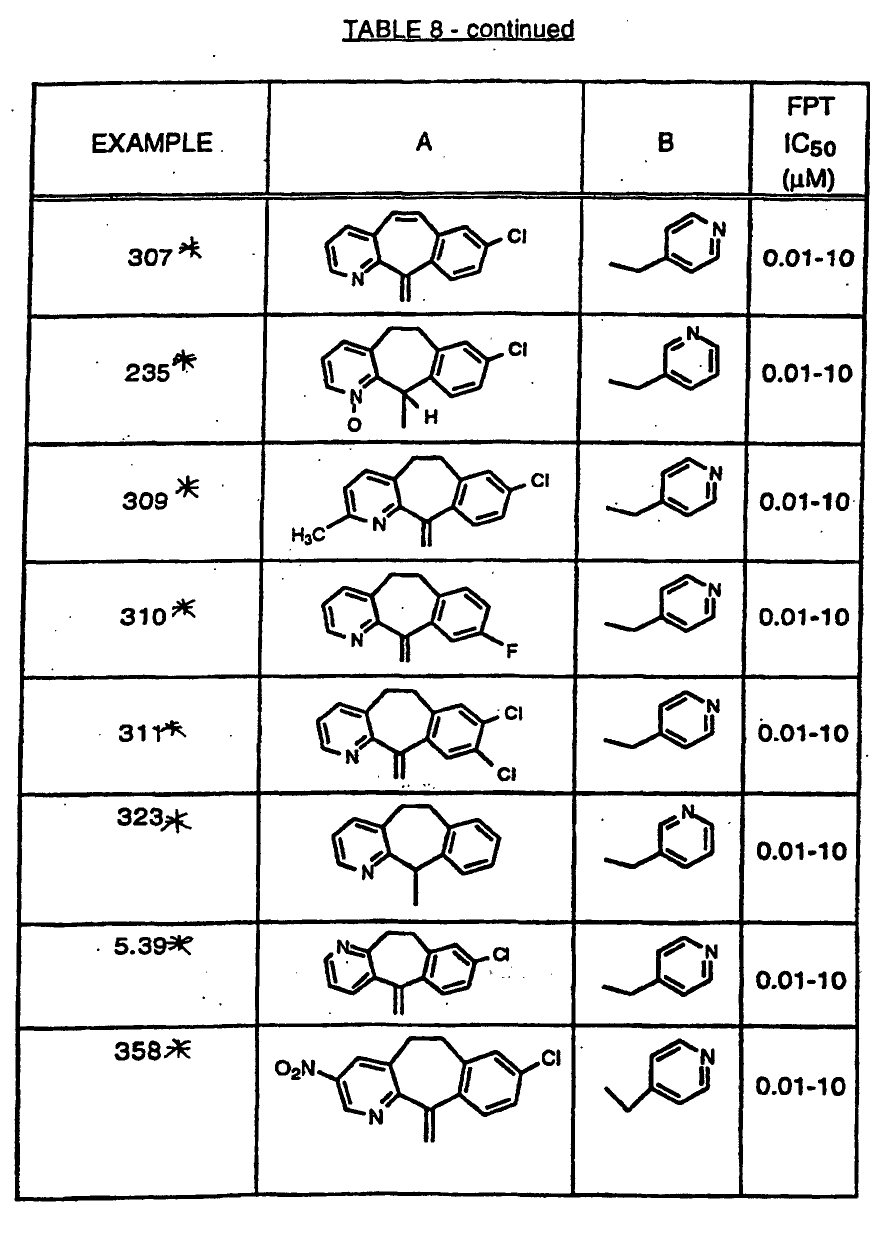

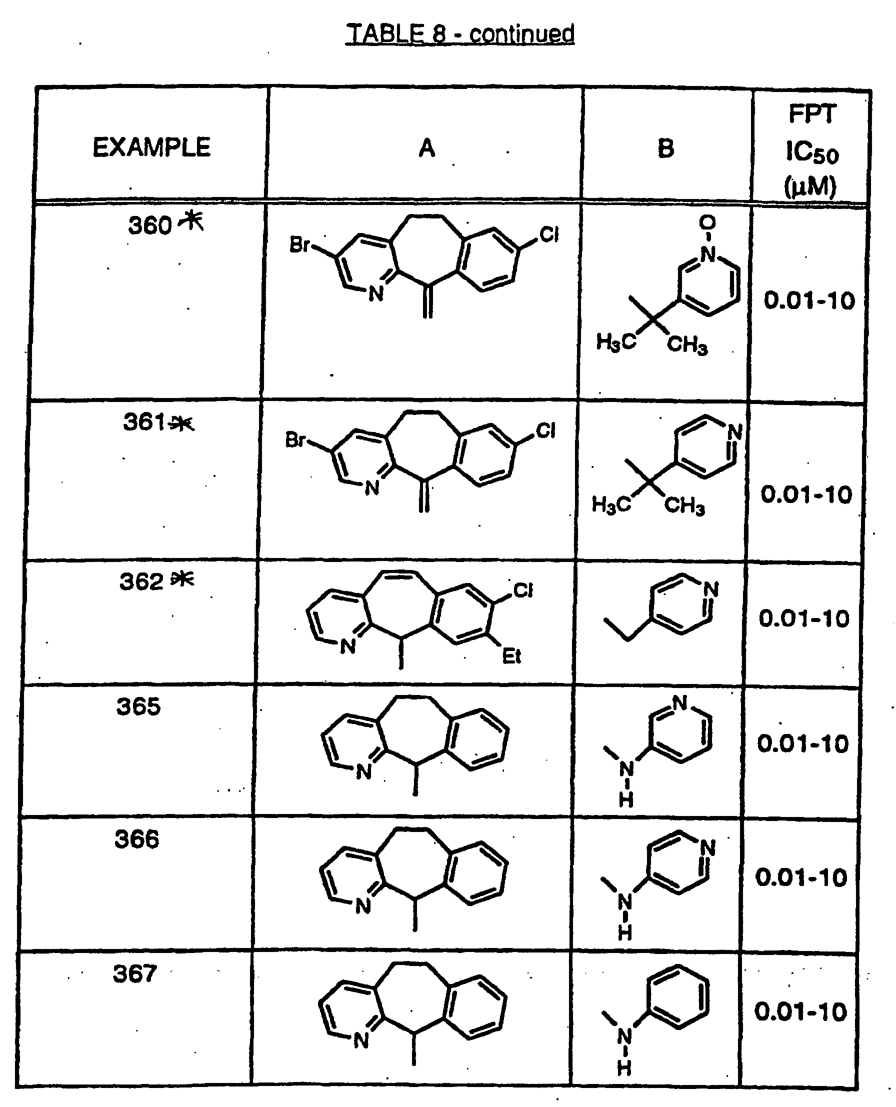

Table 9 lists FPT IC₅₀ results for additional compounds.

**TABLE 9**

| EXAMPLE | FPT IC₅₀ (µM) | EXAMPLE | FPT IC₅₀ (µM) | EXAMPLE | FPT IC₅₀ (µM) |
|---|---|---|---|---|---|
| 229* | 0.01-10 | 231* | 10-100 | 232* | 0.01-10 |
| (5.104) | | (5.106) | | (5.107) | |
| 236* | 0.01-10 | 237* | 10-100 | 238* | 10-100 |
| (5.111) | | (5.112) | | (5.113) | |
| 239* | 0.01-10 | 240* | 0.01-10 | 246* | 0.01-10 |
| (5.114) | | (5.115) | | (5.121) | |
| 247* | 0.01-10 | 248* | 0.01-10 | 248* | 0.01-10 |
| (5.122) | | (5.124) | | (5.123) | |
| 249* | 0.01-10 | 250* | 0.01-10 | 256* | 0.01-10 |
| (5.125) | | (5.126) | | (5.132) | |
| 257* | 0.01-10 | 258* | 10-100 | 259* | 0.01-10 |
| (5.133) | | (5.134) | | (5.135) | |
| 260* | 0.01-10 | 266* | 0.01-10 | 269* | 0.01-10 |
| (5.136) | | (5.138) | | (5.140) | |
| 276* | 0.01-10 | 279* | 0.01-10 | 280* | 10-100 |
| (5.145) | | (5.147) | | (5.148) | |
| 281* | 0.01-10 | 282* | 0.01-10 | 283* | 0.01-10 |
| (5.149) | | (5.150) | | (5.151) | |
| 284* | 0.01-10 | 285* | 0.01-10 | 286* | 0.01-10 |
| (5.152) | | (5.153) | | (5.154) | |

The compounds listed in Table 10 refer to compounds of Formula

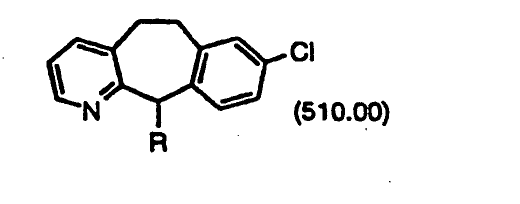

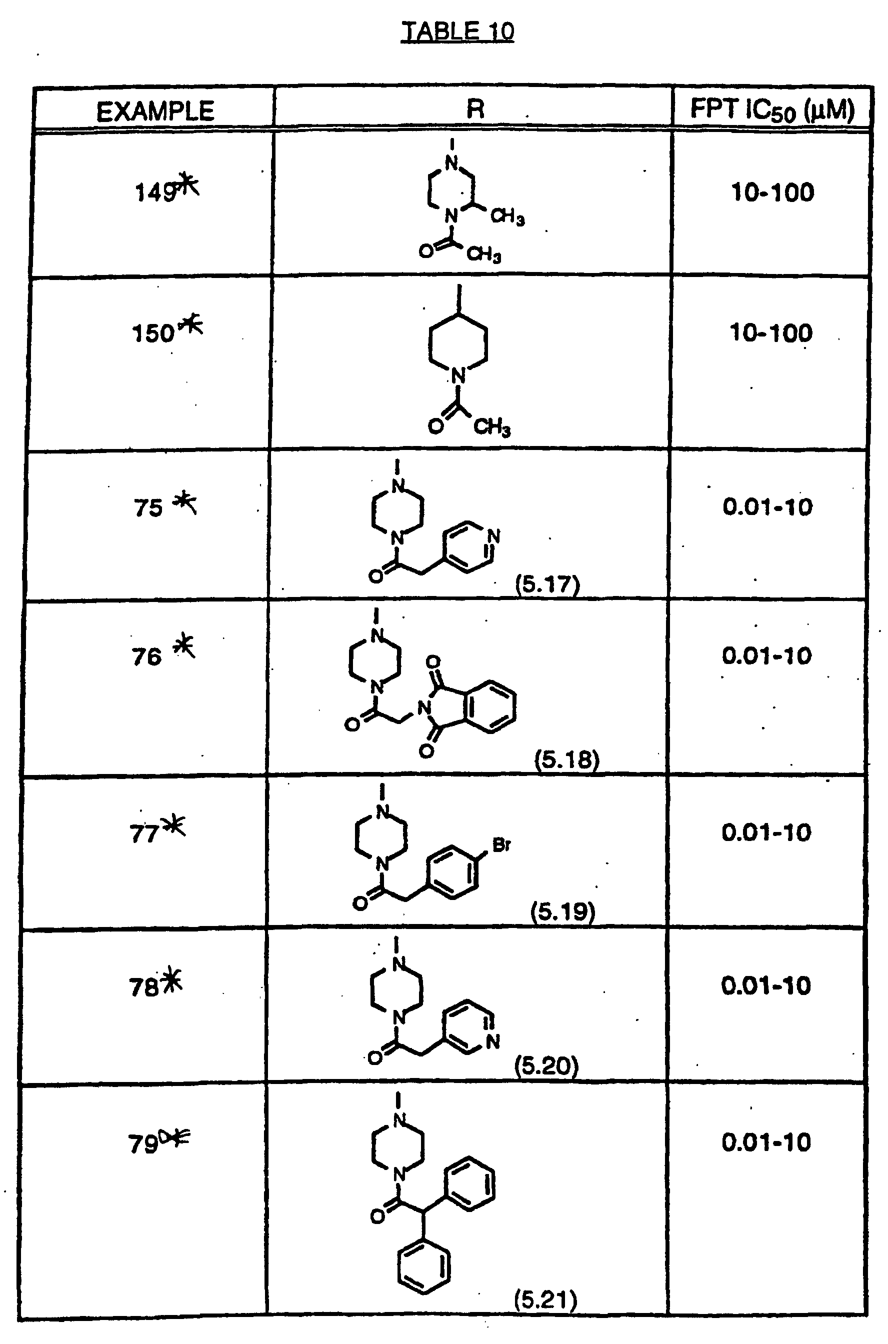

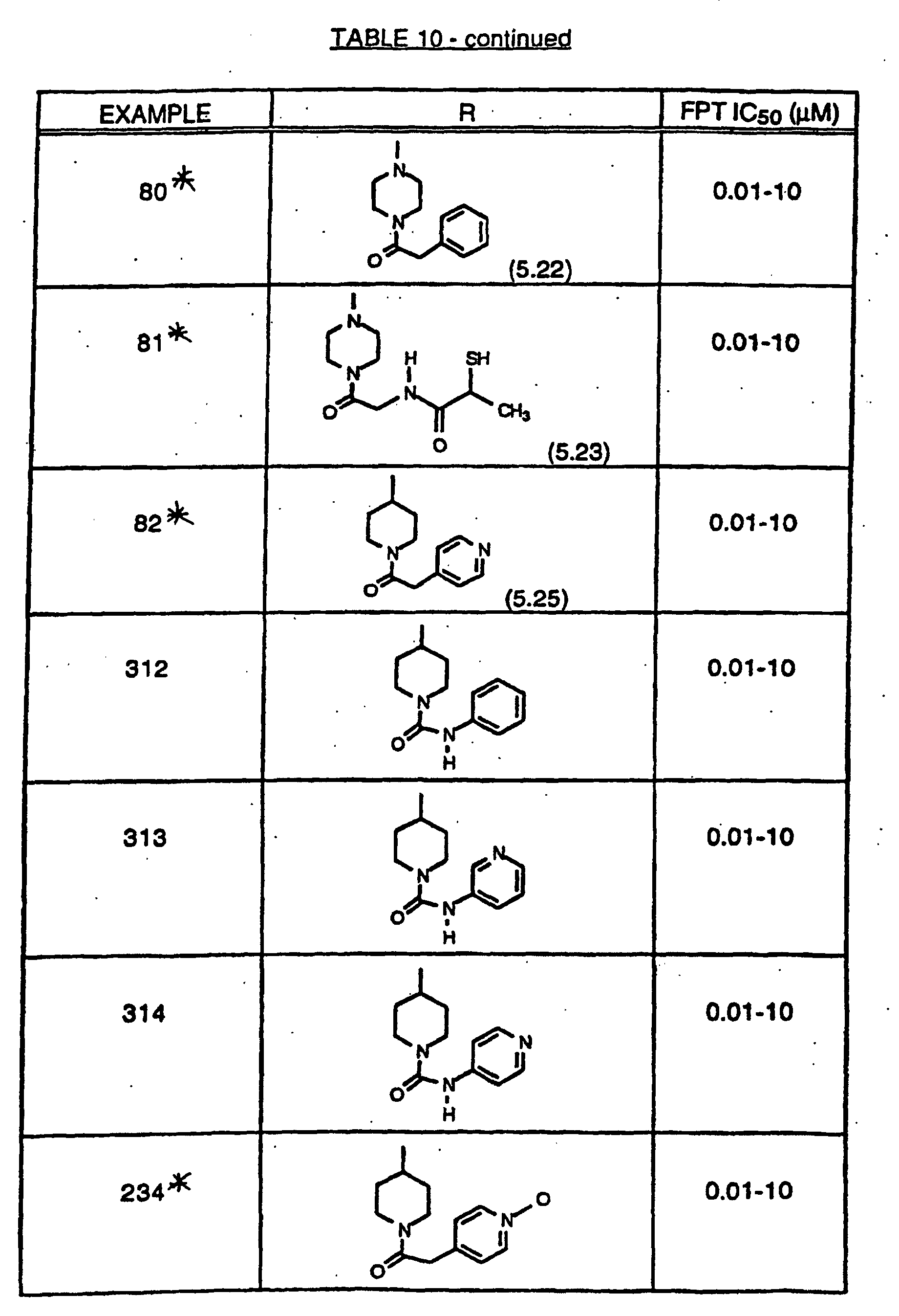

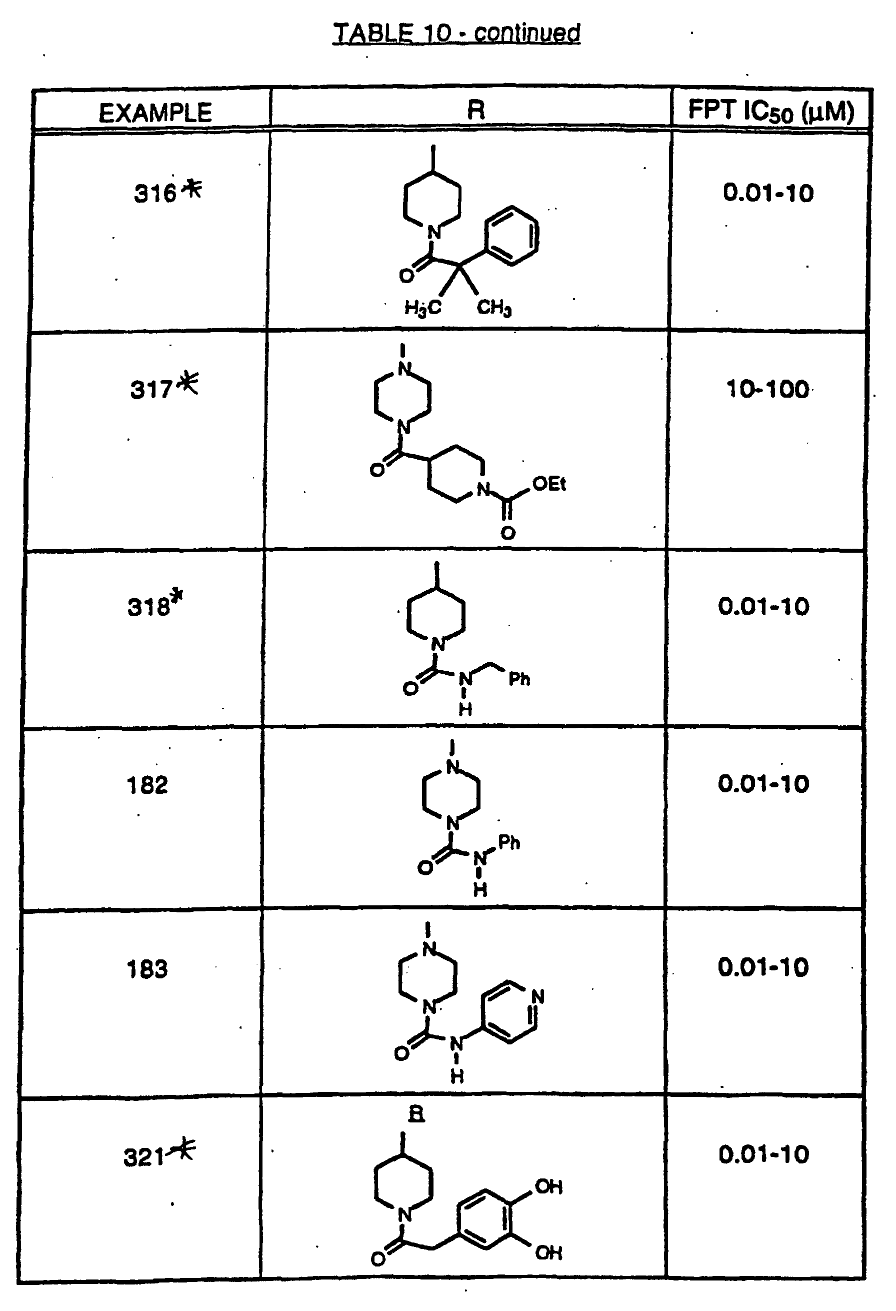

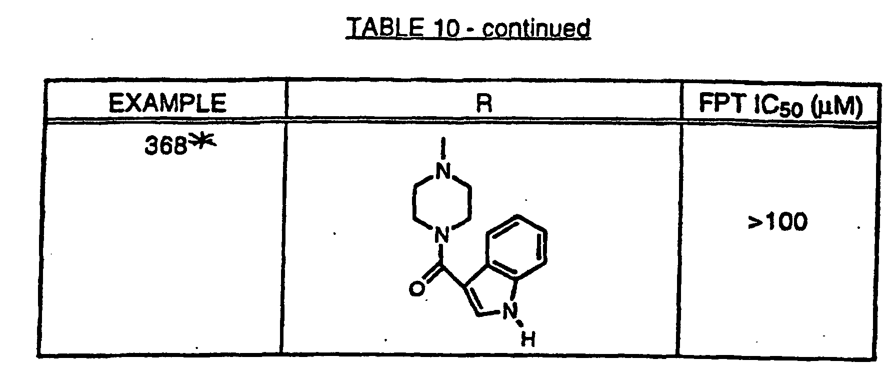

Table 11 lists FPT IC₅₀ results for additional compounds.

**TABLE 11**

| EXAMPLE | FPT IC₅₀ (µM) | EXAMPLE | FPT IC₅₀ (µM) | EXAMPLE | FPT IC₅₀ (µM) |
|---|---|---|---|---|---|
| 187 | 0.01-10 | 187 | 0.01-10 | 188 | 0.01-10 |
| (6.7) | | (6.8) | | (6.9) | |
| 189* | 0.01-10 | 190* | 0.01-10 | 191* | 0.01-10 |
| (5.62) | | (5.63) | | (5.64) | |
| 192* | 0.01-10 | 194* | 0.01-10 | 195* | 0.01-10 |
| (5.65) | | (5.67) | | (5.68) | |
| 196* | 0.01-10 | 197* | 0.01-10 | 198* | 0.01-10 |
| (5.69) | | (5.70) | | (5.71) | |
| 199* | 10-100 | 199* | 10-100 | 200* | 0.01-10 |
| (5.72A) | | (5.72B) | | (5.73) | |
| 201* | 0.01-10 | 202* | 10-100 | 203* | 10-100 |
| (5.74) | | (5.75) | | (5.76) | |
| 205* | 0.01-10 | 206* | 0.01-10 | 207* | 10-100 |
| (5.78) | | (5.79) | | (5.80) | |
| 208* | 0.01-10 | 209* | 0.01-10 | 210* | 0.01-10 |
| (5.81) | | (5.82) | | (5.83) | |
| 211* | 0.01-10 | 212* | 0.01-10 | 213* | 10-100 |
| (5.84) | | (5.85) | | (5.86) | |
| 214* | >100 | 215* | 10-100 | 216* | 10-100 |
| (5.87) | | (5.88) | | (5.89) | |
| 217* | 0.01-10 | 218* | 0.01-10 | 233* | 0.01-10 |
| (5.90) | | (5.91) | | (5.108) | |
| 251* | 0.01-10 | 261 | 0.01-10 | 351* | 0.01-10 |
| (5.127) | | (6.12) | | | |
| 352* | 0.01-10 | 353* | 0.01-10 | 354 | 0.01-10 |
| 355* | 0.01-10 | 273* | 0.01-10 | 267 | 0.01-10 |
| | | (5.143) | | (6.17) | |
| 356* | 0.01-10 | 264 (6.15) | >100 | 262 (6.13) | 0.01-10 |
| 263* | 0.01-10 | 253* | 0.01-10 | 350* | 0.01-10 |
| (6.14) | | (5.129) | | | |
| 252* | 0.01-10 | 182 | 0.01-10 | 268* | 0.01-10 |
| (5.128) | | (6.4) | | (5.139) | |
| 277 | 0.01-10 | ---- | ---- | ---- | ---- |
| (6.20) | | | | | |

The compounds listed in Table 12 refer to compounds of Formula 525.00:

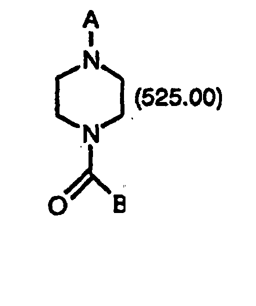

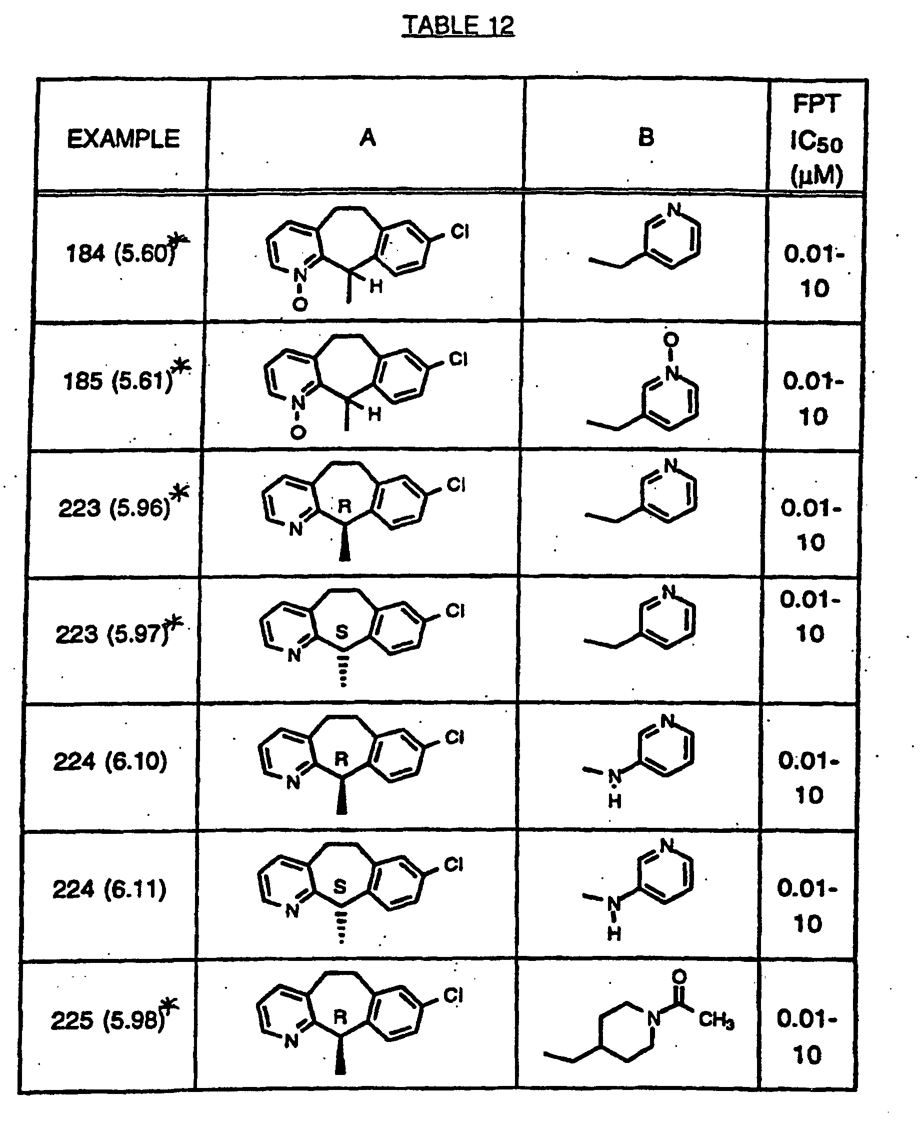

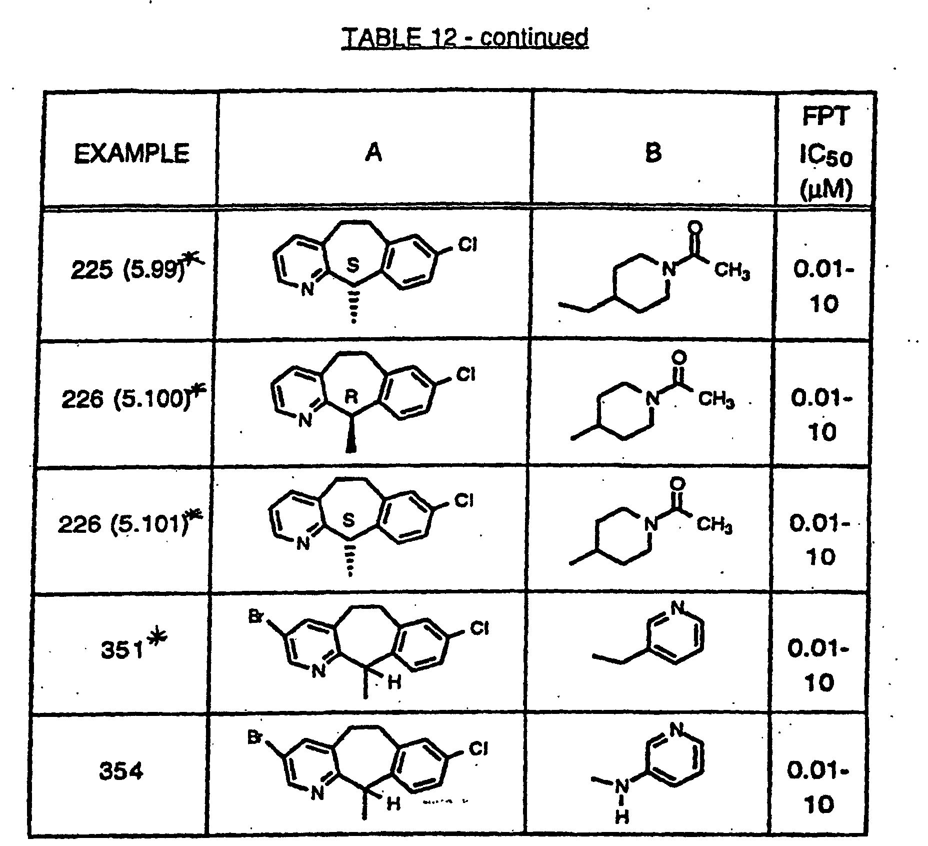

The compounds listed in Table 13 refer to compounds of Formula 515.00:

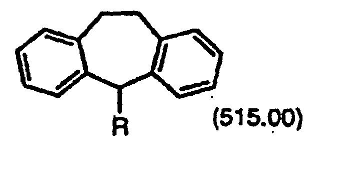

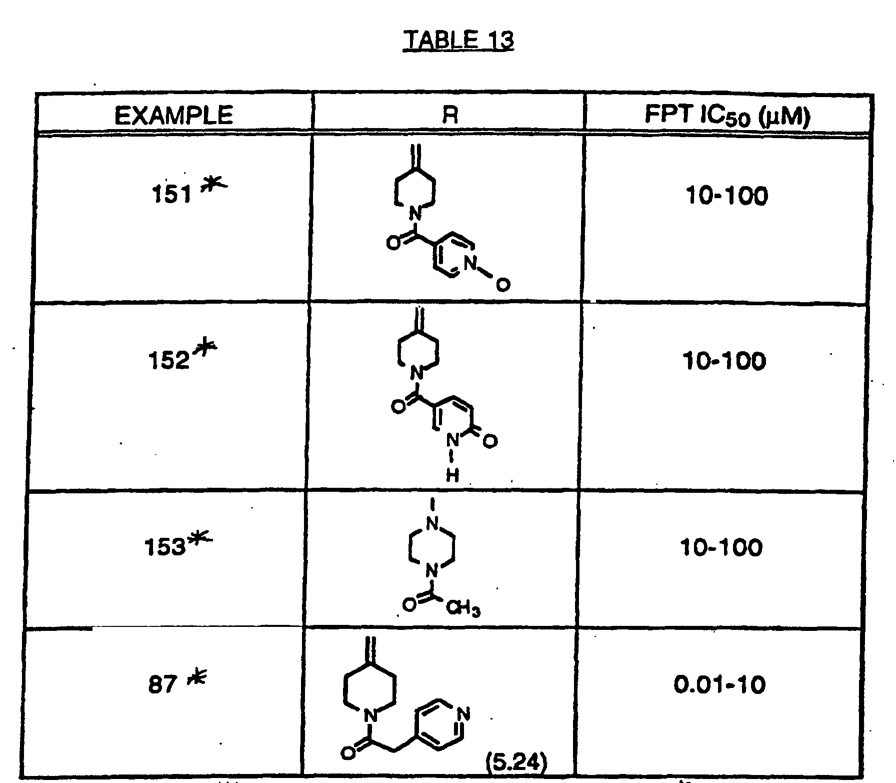

Additional FPT IC₅₀ results were: (1) Example 180*, compound 5.47, 0.01-10 µM; (2) Example 181*, compound 5.48, 0.01-10 µM; (3) Example 182, compound 6.4, 0.01-10 µM; and (4) Example 183, compound 6.5, 0.01-10 µM.

**TABLE 14**

| COMPARISON OF FPT INHIBITION AND GGPT INHIBITION | | |
|---|---|---|
| EXAMPLE | ENZYME INHIBITION FPT IC₅₀ µM | ENZYME INHIBITION GGPT IC₅₀ µM |
| 1* | 0.01-10 | >46 |
| 2* | 0.01-10 | >46 |
| 3* | 0.01-10 | >39 |
| 5* | 0.01-10 | >46 |
| 7* | 0.01-10 | >45 |
| 8* | 0.01-10 | 42 |
| 181* | 0.01-10 | >42 |

**TABLE 15**

| ACTIVITY IN COS CELLS | | | |
|---|---|---|---|
| Example | Inhibition of Ras Processing IC₅₀ (µM) | Example | Inhibition of Ras Processing IC₅₀ (µM) |
| 1* | 0.01-10 | ---- | ---- |
| 82* | 0.01-10 | 156 (5.46)* | 0.01-10 |
| 75* | 0.01-10 | 2* | 0.01-10 |
| 45* | 0.01-10 | 157* | 0.01-10 |
| 78* | 0.01-10 | 42* | 0.01-10 |
| 19* | 0.01-10 | 89* | 0.01-10 |
| 83* | 0.01-10 | 5* | 0.01-10 |
| 77* | 0.01-10 | 43* | 0.01-10 |
| 6* | 0.01-10 | 49* | 10-100 |
| 47* | 10-100 | 44* | 10-100 |
| 87* | 10-100 | 46* | 10-100 |
| 85* | 10-100 | 84* | 10-100 |
| 3* | 10-100 | 76* | 10-100 |
| 154 (5.28)* | 10-100 | 48* | 10-100 |
| 5* | 10-100 | 88* | 10-100 |
| 53* | 10-100 | 181 (5.48)* | 0.01-10 |

In Table 15, the numbers in parenthesis in the Example column refer to the formula number for the compound used in the indicated example. Also, the compound of Example 157* is:

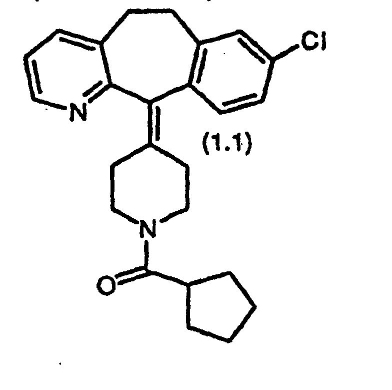

**TABLE 16**

| INHIBITION OF TUMOR CELL GROWTH MAT ASSAY | | | | | |
|---|---|---|---|---|---|
| Example | Tumor IC ₅₀ (µM) | Normal IC ₅₀ (µM) | Example | Tumor IC ₅₀ (µM) | Normal IC ₅₀ (µM) |
| 75* | 2.5 | >50.0 | ---- | ---- | ---- |
| 1* | 3.1 | 25.0 | 82* | 3.1 | 40.0 |
| 5* | 6.3 | >50.0 | 89* | 6.3 | >25.0 |
| 127* | 6.3 | >50.0 | 45* | 6.3 | >50.0 |
| 88* | 8.0 | >50.0 | 6* | 12.5 | 50.0 |
| 49* | 12.5 | >50.0 | 47* | 12.5 | >50.0 |
| 48* | 12.5 | 25.0 | 79* | 12.5 | >50.0 |
| 158 (5.36)* | 12.5 | 18.0 | 2* | 25.0 | >50.0 |
| 10* | 25.0 | >50.0 | 128* | 25.0 | >50.0 |
| 44* | 25.0 | 25.0 | 164 (5.30)* | 25.0 | >50.0 |
| 43* | 25.0 | 50.0 | 165 (5.34)* | 25.0 | 50.0 |
| 53* | 25.0 | >50.0 | 166 (5.26)* | 37.0 | >50.0 |
| 159 (5.31)* | 37.0 | >50.0 | 167 (5.32)* | 37.0 | 50.0 |
| 160 (5.39)* | 37.0 | 50.0 | 168 (5.44)* | 37.0 | >50.0 |
| 161 (5.45)* | 37.0 | >50.0 | 5* | 37.5 | 100.0 |
| 162 (5.29)* | 37.0 | >50.0 | 93* | 40.0 | >50.0 |
| 94* | 40.0 | 80.0 | 88* | >50.0 | >50.0 |
| 3* | >50.0 | >50.0 | 7* | 50.0 | 100.0 |
| 90* | 50.0 | >50.0 | 91* | 50.0 | 80.0 |
| 95* | >50.0 | >50.0 | 11* | >50.0 | >50.0 |
| 12* | 50.0 | >50.0 | 96* | 50.0 | >50.0 |
| 97* | >50.0 | >50.0 | 98* | 50.0 | >50.0 |
| 121* | 50.0 | >50.0 | 126* | 50.0 | >50.0 |
| 163 (5.27)* | 50.0 | >50.0 | 42* | 50.0 | >50.0 |
| 154 (5.28)* | >50.0 | >50.0 | 169 (5.33)* | >50.0 | >50.0 |
| 46* | 50.0 | >50.0 | 80* | >50.0 | >50.0 |
| 77* | >50.0 | >50.0 | 76* | >50.0 | >50.0 |
| 81* | >50.0 | >50.0 | 173 (5.35)* | >50.0 | >50.0 |
| 170 (5.37)* | 50.0 | >50.0 | 174 (5.38)* | 50.0 | 50.0 |
| 171 (5.40)* | 50.0 | >50.0 | 87* | 50.0 | >50.0 |
| 172 (5.42)* | >50.0 | >50.0 | 175 (5.43)* | >50.0 | >50.0 |
| 180 (5.47)* | 18 | >50.0 | 181 (5.48)* | <3.1 | >50.0 |

In Table 16, the numbers in parenthesis In the Example column refer to the formula number for the compound used in the indicated example.

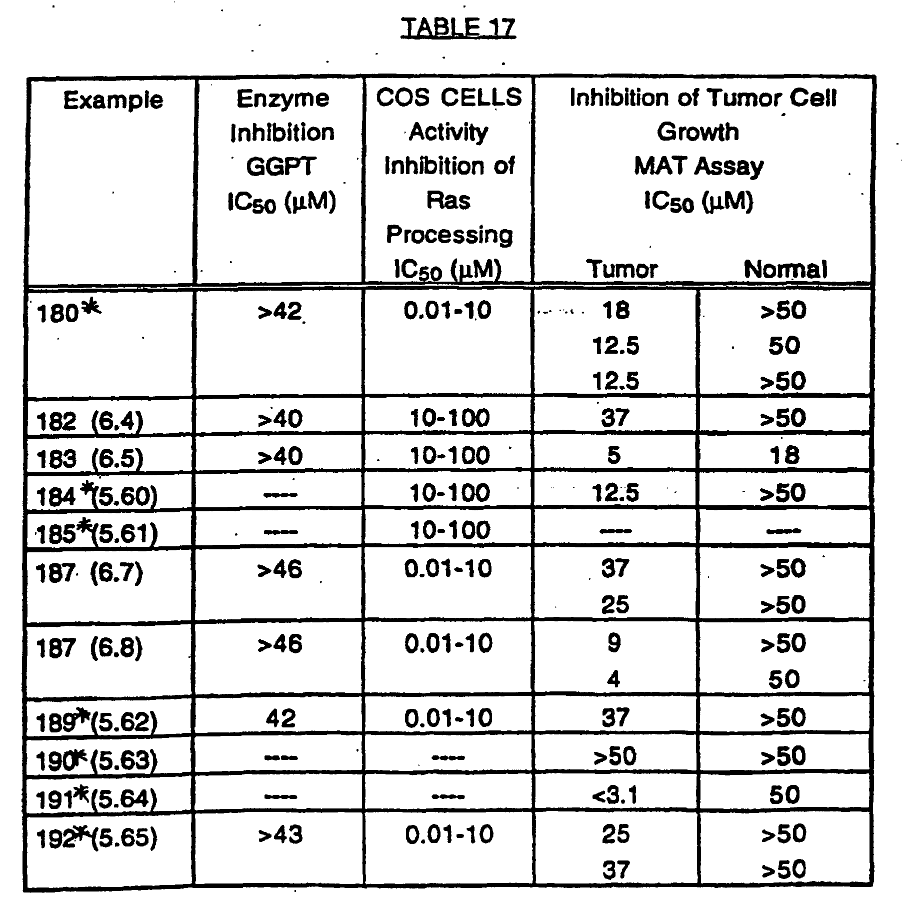

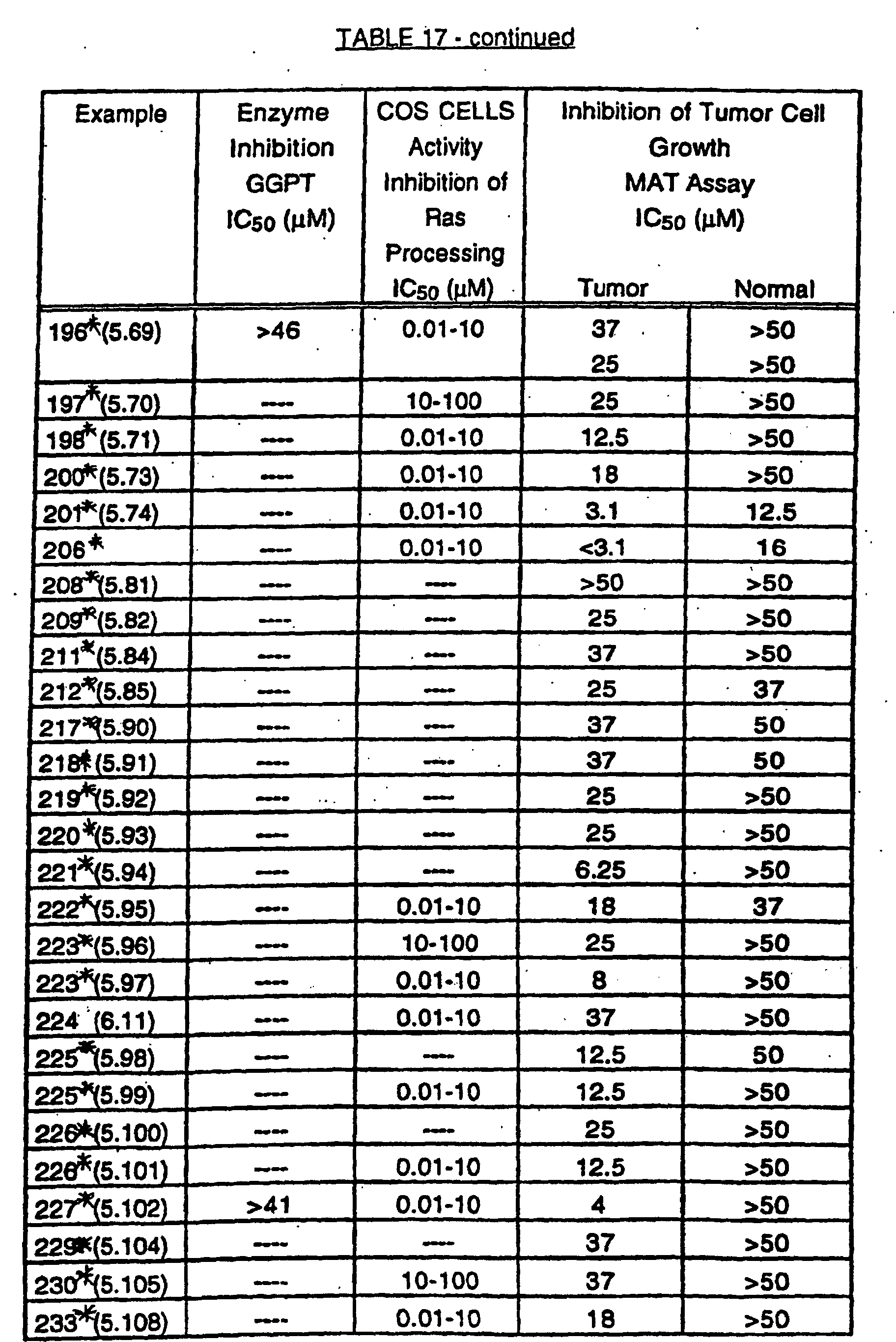

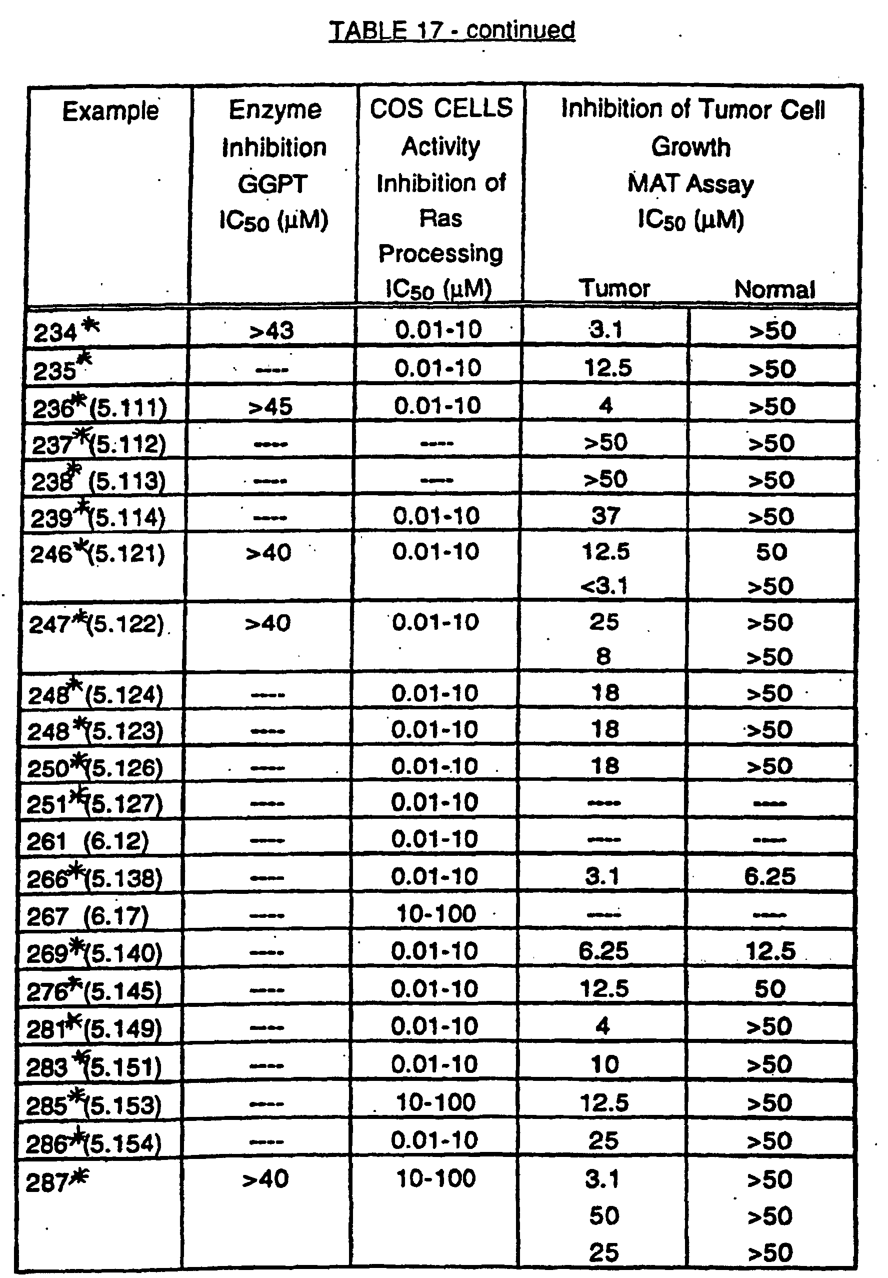

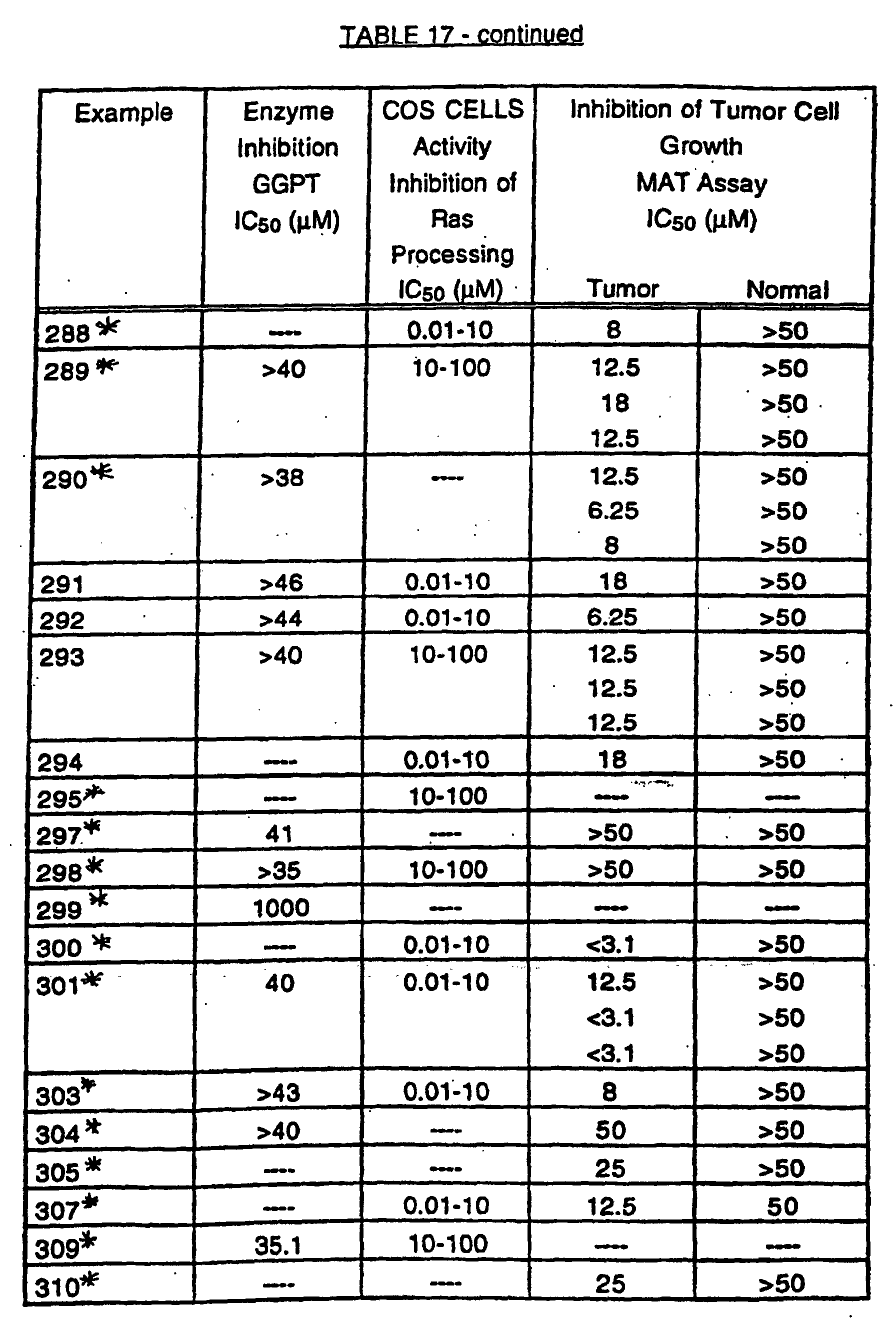

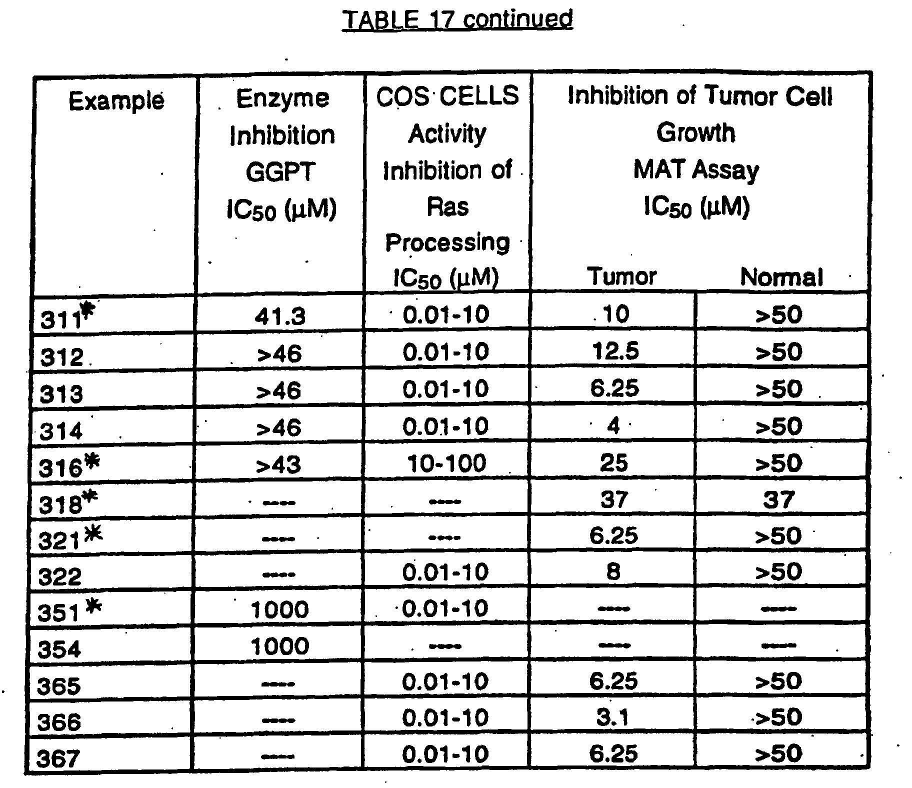

### RESULTS:

### 1. Enzymology:

The data demonstrate that the compounds of the invention are inhibitors of Ras-CVLS farnesylation by partially purified rat brain farnesyl protein transferase (FPT). The data also show that there are compounds of the invention which can be considered as potent (IC₅₀ <10 µM) inhibitors of Ras-CVLS farnesylation by partially purified rat brain FPT.

The data also demonstrate that compounds of the invention are poorer inhibitors of geranylgeranyl protein transferase (GGPT) assayed using Ras-CVLL as isoprenoid acceptor. Generally, the compounds of the invention are inactive or weakly active as geranylgeranyl transferase inhibitors at 20 µg/mL. For example, with reference to Table 14, the compound of Example 1 inhibits GGPT 24% at 46 µM and is at least 184-fold selective for FPT inhibition. The compound of Example 2, for example, inhibits GGPT 25% at 46 µM and is at least 98-fold selective for FPT inhibition. For another example, the compound of Example 3 inhibits GPPT 3% at 39 µM and is at least 59-fold selective for FPT. This selectivity is important for the therapeutic potential of the compounds used in the methods of this invention, and increases the potential that the compounds will have selective growth inhibitory properties against Ras-transformed cells.

### 2. Cell-Based: COS Cell Assay

Western blot analysis of the Ras protein expressed in Ras-transfected COS cells following treatment with the tricyclic farnesyl protein transferase inhibitors of this invention indicated that they inhibit Ras-CVLS processing, causing accumulation of unprocessed Ras (see Table 15). The compound of Example 1, for example, inhibited Ras-CVLS processing with an IC₅₀ value of 0.01-10 µM, but did not block the geranylgeranylation of Ras-CVLL at concentrations up to 20 µg/mL. Microscopic and photographic examination of the Ras-transfected COS cells following treatment with two of the tricyclic farnesyl transferase inhibitors of this invention indicated that they also blocked phenotypic changes induced by expression of oncogenic Ras. Cells expressing oncogenicRas-CVLS or Ras-CVLL overgrew the monolayer and formed dense foci of cells. The compound of Example 1 inhibited the morphological changes induced by Ras-CVLS in a dose-dependent manner over the concentration range of 2 to 20 µg/mL. The compound of Example 1 had little effect at 0.2 or 0.5 µg/mL. Importantly, 20 µg/mL of the compound of Example 1 did not prevent the morphological changes induced by Ras-CVLL.

These results provide evidence for specific inhibition of famesyl protein transferase, but not geranylgeranyl transferase I, by compounds of this invention in intact cells and indicate their potential to block cellular transformation by activated Ras oncogenes.

### 3. Cell-Based: Cell Mat Assay

Tricyclic farnesyl protein transferase inhibitors of this invention also inhibited the growth of Ras-transformed tumor cells in the Mat assay without displaying cytotoxic activity against the normal monolayer.

### In Vivo Anti-Tumor Studies:

Tumor cells (5 x 10⁵ to 8 x 10⁶ of M27 (mouse Lewis lung carcinoma), A431(human epidermal carcinoma) or SW620 (human colon adenocarcinoma [lymph node metastasis])) are innoculated subcutaneously into the flank of 5-6 week old athymic nu/nu female mice. For the C-f-1 (mouse fibroblast transformed with c-fos oncogene) tumor model, 2 mm³ tumor fragments are transplanted subcutaneously into the flank of 5-6 week old athymic nu/nu female mice. Tumor bearing animals are selected and randomized when the tumors are established. Animals are treated with vehicle (beta cyclodextran for i.p. or corn oil for p.o.) only or compounds in vehicle twice a day (BID) for 5 (1-5), 6 (1-6), or 7 (1-7) days per week for 2 (x2) or 4 (x4) weeks. The percent inhibition of tumor growth relative to vehicle controls are determined by tumor measurements. The results are reported in Table 18.

**TABLE 18**

| In-Vivo Anti-Tumor Results | | | | | | | | |
|---|---|---|---|---|---|---|---|---|
| s.c. Tumor | Route & Schedule | Ex 2* | Ex 1* | Ex 3* | Ex 7* | Ex 78* | Ex 79* | Ex 75* |
| M27 | po, BID, 1-7, x4 | 61.2 | ----- | 27.3 | 58.2 | ----- | ----- | ----- |
| A431 | ip, BID, 1-5, x4 | ----- | 20.5 | 0 | 0 | ----- | ----- | ----- |
| A431 | po, BID 1-5, x4 | 45.6 | ----- | 8 | 29.1 | ----- | ----- | ----- |
| A431 | po, BID, 1-5, x4 | 36.5 | ----- | 26 | ----- | ----- | ----- | ----- |
| A431 | po, BID, 1-6, x4 | ----- | ----- | ----- | ----- | 31 | 0 | 34.5 |
| C-f-1 | ip, BID, 1-5, x2 | 8 | 0 | 8 | 39.7 | ----- | ----- | ----- |
| C-f-1 | po, BID, 1-5, x4 | 9.6 | ----- | 0 | 39.3 | ----- | ----- | ------ |
| C-f-1 | po, BID, 1-5, x4 | ----- | ----- | ----- | ----- | 26.7 | 25 | 20 |
| SW-620 | ip, BID, 1-5, x4 | 0 | 0 | 27 | 19.6 | ----- | ----- | ----- |
| SW-620 | po, BID, 1-5, x2 | 46.1 | 0 | 15.8 | 48.6 | ----- | ----- | ----- |
| SW-620 | po, BID, 1-5, x4 | 33.5 | ----- | ----- | 0 | ----- | ----- | ----- |
| SW-620 | po, BID, 1-5, x4 | ----- | ----- | ----- | ----- | 59.6 | 26.7 | 43.4 |

Additional in-vivo anti-tumor results are reported in Table 19. In Table 23, LOX is a human memanoma cell line, and the schedule "10/wk, x4", for example, means 10 times per week (twice a day Monday to Friday) for 4 weeks.

**TABLE 19**

| In-Vivo Anti-Tumor Results | | | | |
|---|---|---|---|---|
| Example or Structure | Tumor | Dose (MPK) | Route & Schedule | Average % Tumor Inhibition |
| Ex.2* | SW620 | 100 | ip, 10/wk, x2 | 0 |
| | SW620 | 100 | po,10/wk,x2 | 0 |
| | SW620 | 100 | po, 10/wk, x4 | 1 |
| | M27 | 100 | po, 14/wk, x4 | 45 |
| Ex. 4* | SW620 | 100 | po, 10/wk, x4 | 2 |
| Ex. 7* | SW620 | 100 | po, 10wk, x2 | 13 |
| | SW620 | 100 | po, 10/wk, x4 | 0 |
| | M27 | 100 | po, 14/wk, x4 | 40 |
| Ex. 45* | SW620 | 100 | po, 10/wk, x4 | 0 |
| | SW620 | 100 | po, 10/wk, x4 | 19 |
| | M27 | 100 | po, 10/wk, x3 | 0 |
| Ex. 47* | SW620 | 100 | po, 10/wk, x4 | 0 |
| | SW620 | 100 | po, 10/wk, x4 | 30 |
| | M27 | 100 | po, 10/wk, x3 | 19 |
| Ex. 49* | SW620 | 100 | po, 10/wk, x4 | 0 |
| | SW620 | 100 | po, 10/wk, x4 | 27 |
| | M27 | 100 | p0, 10/wk, x3 | 30 |
| Ex. 75* | SW620 | 100 | po, 10/wk, x4 | 26 |
| | SW620 | 100 | po, 10/wk, x4 | 4 |
| | SW620 | 100 | po, 10/wk, x4 | 54 |
| | SW620 | 100 | po, 10/wk, x4 | 7 |
| | M27 | 100 | po, 10/wk, x4 | 0 |
| Ex. 82* | SW620 | 100 | po, 10/wk, x4 | 25 |
| | SW620 | 100 | po, 10/wk, x4 | 32 |
| Ex. 88* | SW620 | 100 | po, 10/wk, x4 | 43.25 * |
| | M27 | 100 | po, 10/wk, x4 | 19 |
| | SW620 | 100 | po, 10/wk, x4 | 38* |
| | LOX | 100 | po, 10/wk, x4 | 70 |
| | SW620 | 100 | po, 10/wk, x4 | 38 |
| | SW620 | 100 | po, 10/wk, x4 | 37 |
| | SW620 | 50 | po, 10/wk, x4 | 30 |
| | SW620 | 50 | po, 10/wk, x4 | 30 |
| | SW620 | 25 | po, 10/wk, x4 | 4 |
| | SW620 | 25 | po, 10/wk, x4 | 0 |
| | SW620 | 100 | po, 10/wk, x4 | 27.4* |
| | LOX | 100 | po, 10/wk, x4 | 33 |
| | SW620 | 100 | po, 10/wk, x4 | 28 |
| | SW620 | 100 | po, 10/wk, x4 | 38 |
| Ex. 127* | SW620 | 100 | po, 10/wk, x4 | 25 |
| | SW620 | 100 | po, 10/wk, x4 | 42 |
| | M27 | 100 | po, 10/wk, x3 | 22 |
| Ex. 187 (6.8) | SW620 | 100 | po, 10/wk, x4 | 11 |
| | SW620 | 100 | po, 10/wk, x4 | 21 |
| Ex. 192* | SW620 | 100 | po, 10/wk, x4 | 29 |
| | SW620 | 100 | po, 10/wk, x4 | 40 |
| Ex. 287* | SW620 | 100 | po, 10/wk, x4 | 14 |
| | SW620 | 100 | po, 10/wk, x4 | 0 |
| Ex. 290* | SW620 | 100 | po, 10/wk, x4 | 41 |
| | SW620 | 100 | po, 10/wk, x4 | 16 |
| Ex. 293 | SW620 | 100 | po, 10/wk, x4 | 5 |
| | SW620 | 100 | po, 10/wk, x4 | 47 |
| Ex. 301* | SW620 | 100 | po, 10/wk, x4 | 16 |
| | SW620 | 100 | po, 10/wk, x4 | 0 |
| Ex. 82A* | SW620 | 100 | po, 10/wk, x4 | 27 |
| | SW620 | 100 | po, 10/wk, x4 | 26 |
| Ex. 342 | SW620 | 100 | po, 10/wk, x4 | 39 |
| | SW620 | 100 | po, 10/wk, x4 | 31 |
| 5.21* | SW620 | 100 | po, 10/wk, x4 | 19 |
| | SW620 | 100 | po, 10/wk, x4 | 17 |
| | M27 | 100 | po, 10/wk, x4 | 0 |
| 5.25* | SW620 | 100 | po, 10/wk, x4 | 7 |
| | SW620 | 100 | po, 10/wk, x4 | 36 |

| | | | | |
|---|---|---|---|---|
| * Average of several results | | | | |

The compound of Example 342 (Table 19) is:

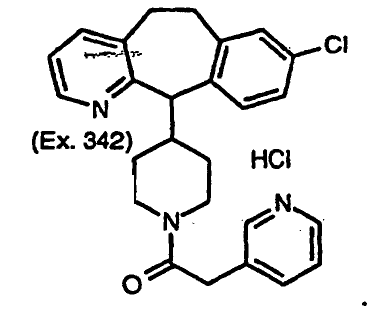

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose.

The quantity of active compound in a unit dose of preparation may be varied or adjusted from about 0.1 mg to 1000 mg, more preferably from about 1 mg. to 300 mg, according to the particular application.

The actual dosage employed may be varied depending upon the requirements, of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended dosage regimen is oral administration of from 10 mg to 2000 mg/day preferably 10 to 1000 mg/day, in two to four divided doses to block tumor growth. The compounds are non-toxic when administered within this dosage range.

The following are examples of pharmaceutical dosage forms which contain a compound of the invention. The scope of the invention in its pharmaceutical composition aspect is not to be limited by the examples provided.

### Pharmaceutical Dosage Form Examples

### EXAMPLE A

| Tablets | | | |
|---|---|---|---|
| No. | Ingredients | mg/tablet | mg/tablet |
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| Total | | 300 | 700 |

### Method of Manufacture

Mix Item Nos. 1 and 2 in a suitable mixer for 10-15 minutes. Granulate the mixture with Item No. 3. Mill the damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

### EXAMPLE B

| Capsules | | | |
|---|---|---|---|
| No. | Ingredient | mg/capsule | mg/capsule |
| 1. | Active compound | 100 | 500 |
| 2. | Lactose USP | 106 | 123 |
| 3. | Corn Starch, Food Grade | 40 | 70 |
| 4. | Magnesium Stearate NF | 7 | 7 |
| Total | | 253 | 700 |

### Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

## Claims

1. The use in the manufacture of a pharmaceutical composition for inhibiting the abnormal growth of cells of a compound of Formula 1.0: 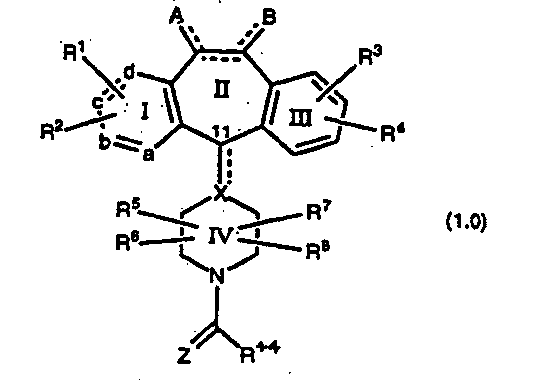 or a pharmaceutically acceptable salt or solvate thereof, wherein:
one of a, b, c and d represents N or NR⁹ wherein R⁹ is O⁻, -CH₃ or -(CH₂)ₙCO₂H wherein n is 1 to 3, and the remaining a, b, c and d groups represent CR¹ or CR²; or
each of a, b, c, and d are independently selected from CR¹ or CR²;
each R¹ and each R² is independently selected from H, halo, -CF₃, -OR¹⁰, -COR¹⁰, -SR¹⁰, -S(O)ₜR¹¹ (wherein t is O, 1 or 2), -N(R¹⁰)₂, -NO₂, -OC(O)R¹⁰, -CO₂R¹⁰, -OCO₂R¹¹, -CN, -NR¹⁰COOR¹¹, -SR¹¹C(O)OR¹¹, -SR¹¹N(R⁷⁵)₂ (wherein each R⁷⁵ is independently selected from H and -C(O)OR¹¹), benzotriazol-1-yloxy, tetrazol-5-ylthio, or substituted tetrazol-5-ylthio, alkynyl, alkenyl or alkyl, said alkyl or alkenyl group optionally being substituted with halo, -OR¹⁰ or -CO₂R¹⁰;
R³ and R⁴ are the same or different and each independently represents H, any of the substituents of R¹ and R², or R³ and R⁴ taken together represent a saturated or unsaturated C₅-C₇ fused ring to the benzene ring;
R⁵, R⁶, R⁷ and R⁸ each independently represents H, -CF₃, -COR¹⁰, alkyl or aryl, said alkyl or aryl optionally being substituted with -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ or one of R⁵, R⁶, R⁷ and R⁸ can be taken in combination with R⁴⁴ as defined below to represent -(CH₂)ᵣ- wherein r is 1 to 4 which can be substituted with lower alkyl, lower alkoxy, -CF₃ or aryl, or R⁵ is combined with R⁶ to represent =O or =S and/or R⁷ is combined with R⁸ to represent =O or =S;
R¹⁰ represents H, alkyl, aryt, or aralkyl;
R¹¹ represents alkyl or aryl;
X represents N, CH or C, which C may contain an optional double bond, represented by the dotted line, to carbon atom 11;
the dotted line between carbon atoms 5 and 6 represents an optional double bond, such that when a double bond is present, A and B independently represent -R¹⁰, halo, -OR¹¹, -OCO₂R¹¹ or -OC(O)R¹⁰, and when no double bond is present between carbon atoms 5 and 6, A and B each independently represent H₂, -(OR¹¹)₂; H and halo, dihalo, alkyl and H, (alkyl)₂, -H and -OC(O)R¹⁰, H and -OR¹⁰, =O, aryl and H, =NOR¹⁰ or -O-(CH₂)ₚ-O- wherein p is 2, 3 or 4;
R⁴⁴ represents 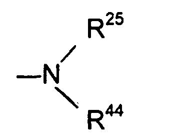 wherein R²⁵ represents heteroaryl or aryl; and R⁴⁸ represents H or alkyl; or R⁴⁴ can be taken in combination with R⁵, R⁶, R⁷ or R⁸ as defined above, and
Z represents O or S;
wherein unless indicated otherwise, the terms "alkyl", "alkenyl", "alkynyl", "aryl", "halo" and "heteroaryl" have the following meanings:
alkyl-(including the alkyl portions of alkoxy, alkylamino and dialkylamino)-represents straight and branched carbon chains and contains from one to twenty carbon atoms, preferably one to six carbon atoms;
alkenyl-represents straight and branched carbon chains having at least one carbon to carbon double bond and containing from 2 to 12 carbon atoms, preferably from 2 to 6 carbon atoms and most preferably from 3 to 6 carbon atoms;
alkynyl-reproserds straight and branched carbon chains having at least one carbon to carbon triple bond and containing from 2 to 12 carbon atoms, preferably from 2 to 6 carbon atoms;
aryl (including the aryl portion of aryloxy and aralkyl)-represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, CF₃, amino, alkylamino, dialkylamino, -COOR¹⁰ or -NO₂; and
halo-represents fluoro, chloro, bromo and iodo; and
heteroaryl-represents cyclic groups, optionaliy substituted with R³ and R⁴, having at least one heteroatom selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms, e.g., 2-, 3- or 4-pyridyl or pyridyl N-oxide (optionally substituted with R³ and R⁴),wherein pyridyl N-oxide can be represented as:

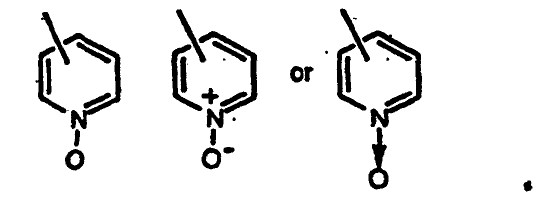

2. Use according to Claim 1 wherein one of a, b, c and d represents N or NO and the remaining a, b, c and d groups represent CR¹ or CR².

3. Use according to Claim 2 wherein a represents N and b, c and d represent CR¹ or CR².

4. Use according to any preceding claim wherein each R¹ and each R² is independently selected from H, halo, -CF₃, lower alkyl or benzotriazol-1-yloxy

5. Use according to Claim 4 wherein R¹ is Cl or H, and R² is H, Cl or Br.

6. Use according to any preceding claim wherein R¹ is at the C-4 position and R² is at the C-3 position.

7. Use according to any preceding claim wherein R³ and R⁴ are the same or different and each independently represents H, halo, -CF₃, -OR¹⁰ or alkyl.

8. Use according to Claim 7 wherein R³ and R⁴ independently represent H or halo.

9. Use according to any preceding claim wherein R³ is at the C-8 position and R⁴ is at the C-9 position.

10. Use according Claim 9 wherein R³ is Cl at the C-8 position and R⁴ is H at the C-9 position.

11. Use according to any preceding claim wherein R⁵, R⁶, R⁷ and R⁸ each independently represents H.

12. The use of Claim wherein a is N and b, c, and d are carbon; R¹ and R² are the same or different and each is independently selected from H, halo, -CF₃, lower alkyl, or benzotriazol-1-yloxy, and R¹ is at the C-4 position and R² is at the C-3 position; R³ and R⁴ are the same or different and each is independently selected from H or halo, and R³ is at the C-8 position and R⁴ is at the C-9 position; when the double bond between carbon atoms 5 and 6 is present, A and B independently represent H, lower alkyl or alkyloxy; and when the double bond between carbon atoms 5 and 6 is absent, A and B independently represent H₂, (-H and -OH) or =O; R⁵, R⁶, R⁷, and R⁸ are H; Z is O; and R represents R⁴⁴ and the R²⁵ represents pyridyl or phenyl.

13. The use of Claim 12 wherein R²⁵ represents 3-pyridyl or phenyl; R³ is Cl; R⁴ is H; R⁴⁸ represents are H or methyl; and R¹ and R² are individually selected from H, benzotriazol-1-yloxy, C₁ to C₄ alkyl or halo.

14. The use of Claim 13 wherein R¹ and R² are individually selected from H, Br, Cl, methyl or benzotriazol-1-yloxy.

15. The use according to any preceding claim wherein the the cells inhibited are tumor cells expressing an activated ras oncogene.

16. The use of Claim 15 wherein the cells inhibited are pancreatic tumor cells, lung cancer cells, myeloid leukemia tumor cells, thyroid follicular tumor cells, myelodysplastic tumor cells, epidermal carcinoma tumor cells, bladder carcinoma tumor cells or colon tumors cells.

17. The use of Claim 1 wherein the inhibition of the abnormal growth of cells occurs by the inhibition of ras farnesyl protein transferase.

18. The use of Claim 1 wherein the inhibition is of tumor cells wherein the Ras protein is activated as a result of oncogenic mutation in genes other than the Ras gene.

19. Use according to Claim 1 wherein the compound is selected from the following formulae:

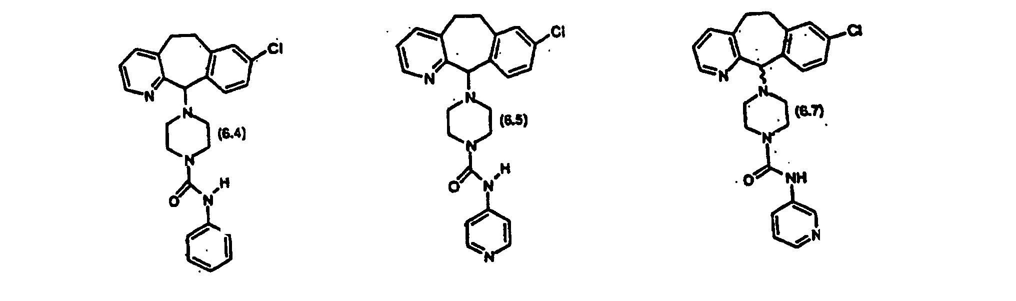

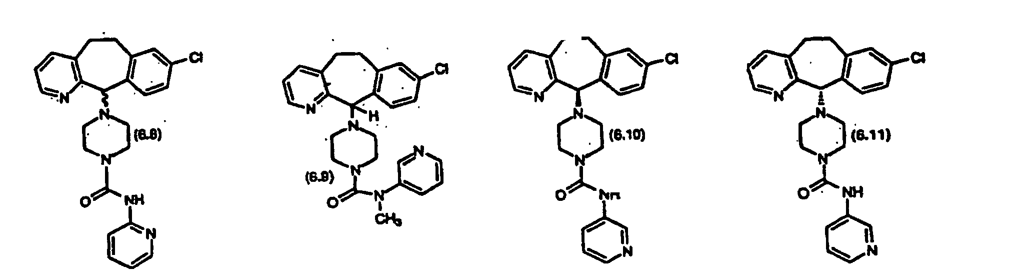

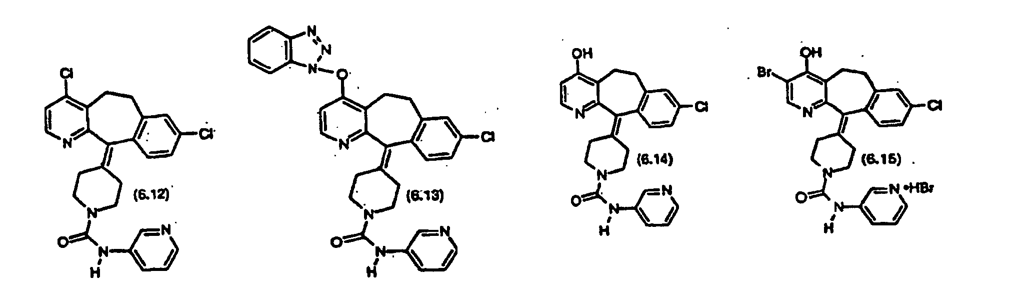

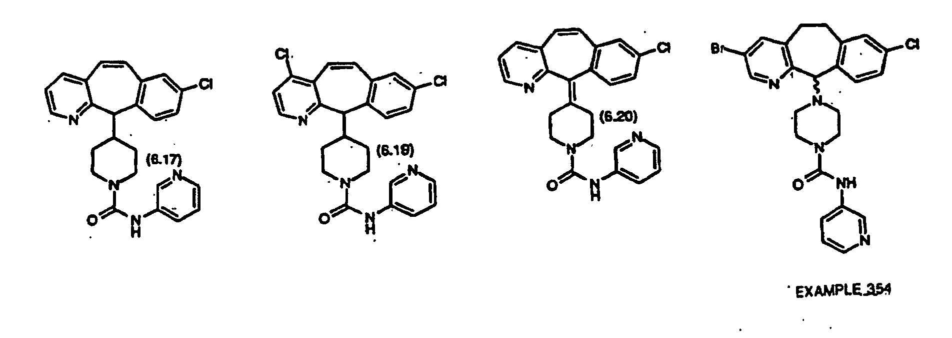

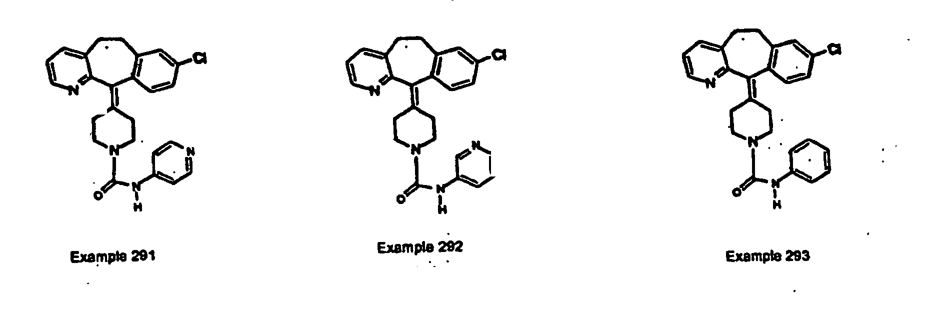

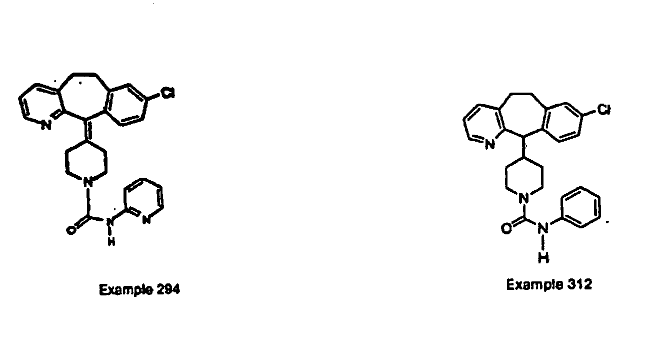

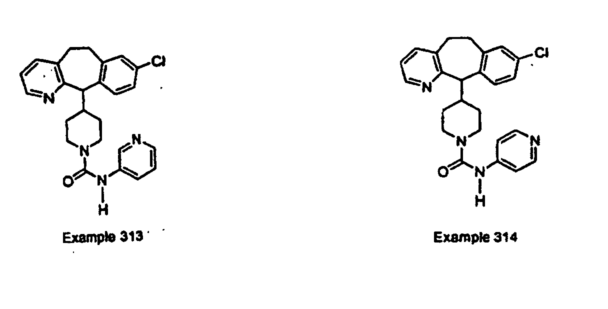

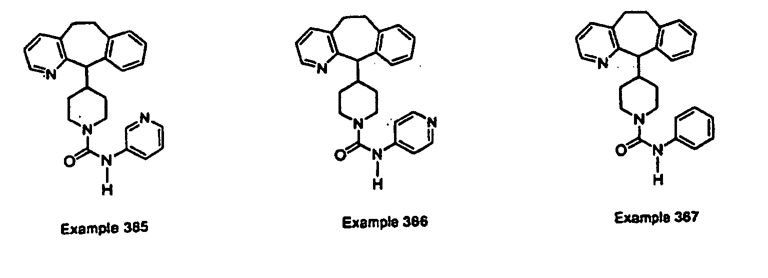

20. A compound selected from a compound of the formula: 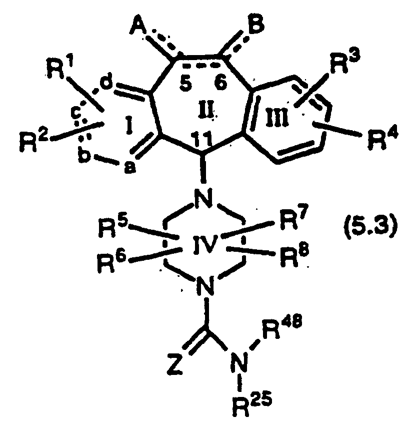 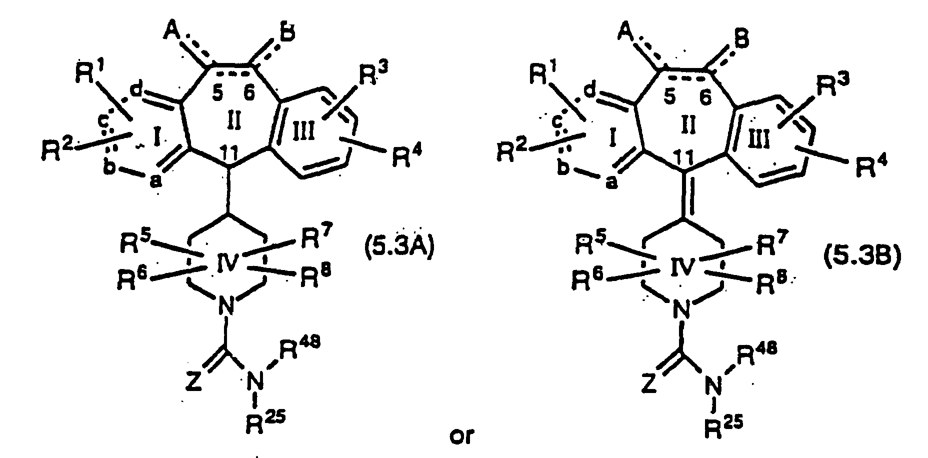 or a pharmaceutically acceptable salt or solvate thereof, wherein all the substituents are as defined in Claim 1.

21. The compound of Claim 20 wherein a is N and b, c, and d are carbon; R¹ and R² are the same or different and each is independently selected from H, halo, -CF₃, lower alkyl, or benzotriazol-1-yloxy, and R¹ is at the C-4 position and R² is at the C-3 position; R3 and R⁴ are the same or different and each is independently selected from H or halo, and R³ is at the C-8 position and R⁴ is at the C-9 position; when the double bond between carbon atoms 5 and 6 is present, A and B independently represent H, lower alkyl or alkyloxy; and when the double bond between carbon atoms 5 and 6 is absent, A and B independently represent H₂, (-H and -OH) or =O; R⁵, R⁶, R⁷, and R⁸ are H; Z is O; and R²⁵ represents phenyl, 3-pyridyl, 3-pyridyl N-oxide, 4-pyridyl, 4-pyridyl N-oxide, 3-N-methylpiperidinyl, 4-N-methylpiperidinyl, 3-N-acetylpiperidinyl or 4-N-acetylpiperidinyl.

22. The compound of Claim 21 wherein R²⁵ represents phenyl, 3-pyridyl, 3-pyridyl N-oxide, 4-pyridyl, 4-pyridyl N-oxide, 3-N-methylpiperidinyl or 4-N-methylpiperidinyl; R³ is Cl; R⁴ is H; R⁴⁸ represents are H or methyl; and R¹ and R² are individually selected from H, benzotriazol-1-yloxy, C₁ to C₄ alkyl or halo.

23. The compound of Claim 22 wherein R¹ and R² are individually selected from H, Br, Cl, methyl or benzotriazol-1-yloxy.

24. A compound of Claim 20 selected from the following formulae:

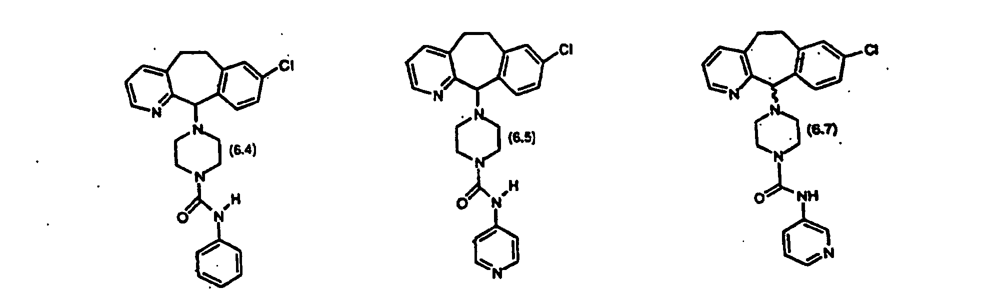

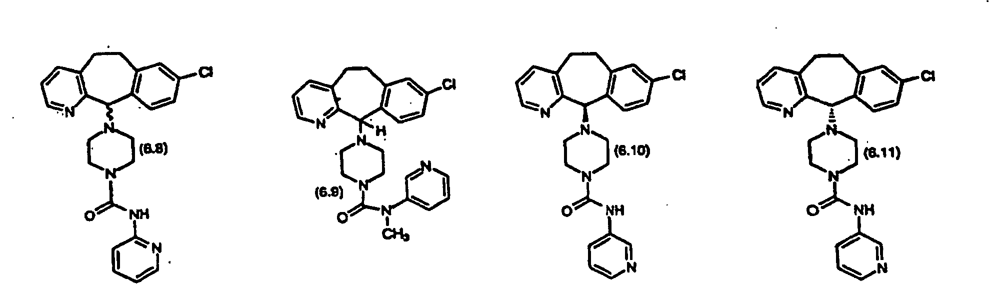

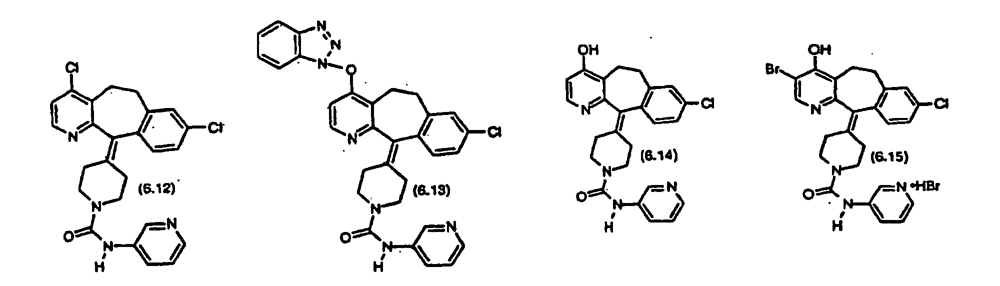

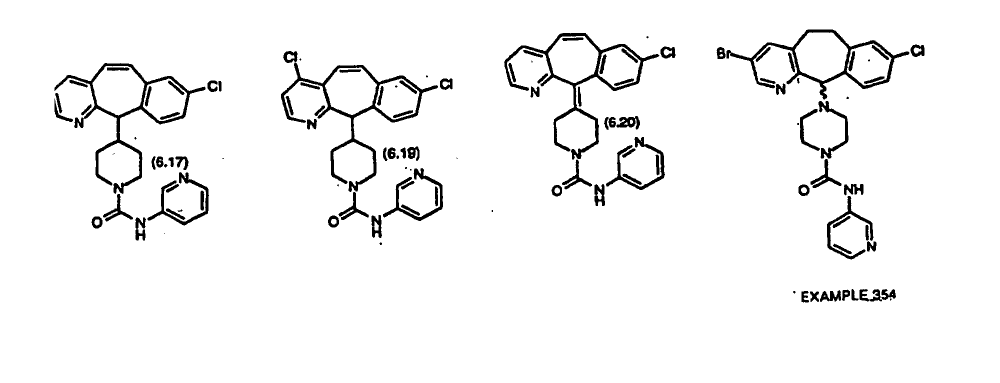

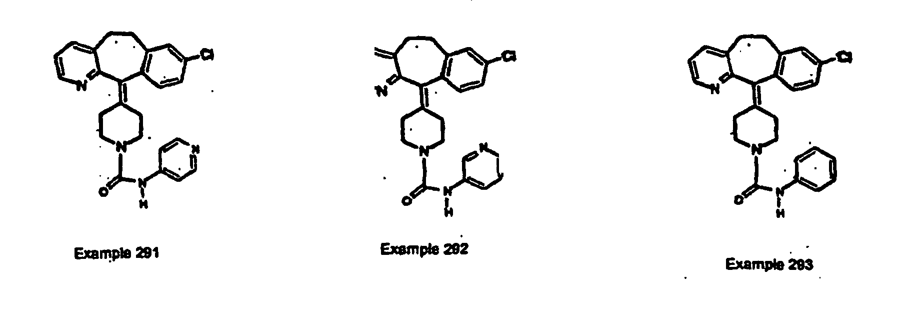

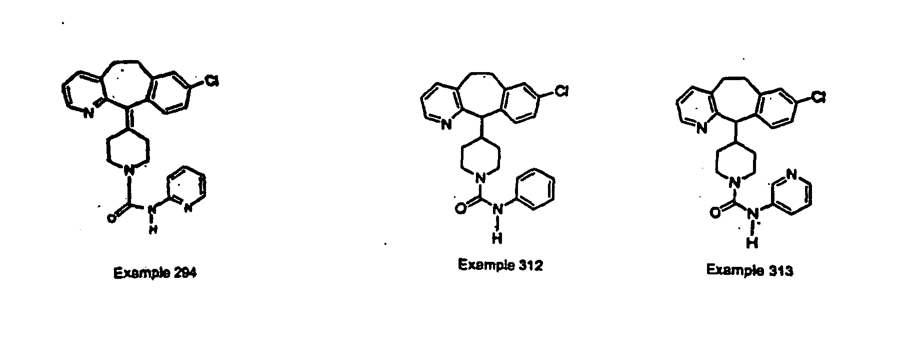

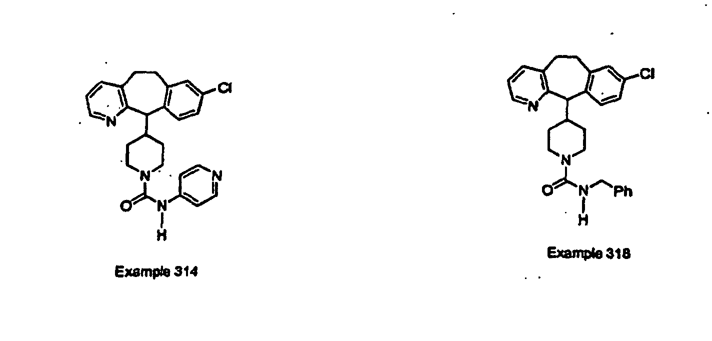

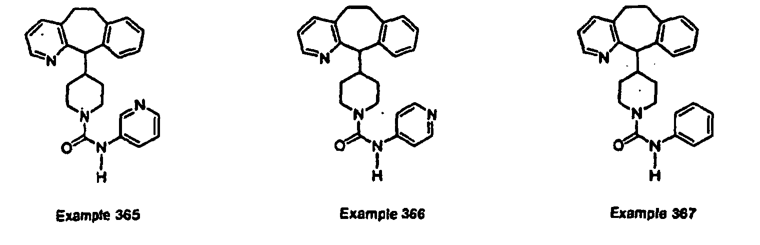

25. A pharmaceutical composition for inhibiting the abnormal growth of cells comprising an effective amount of compound of Claim 20 in combination with a pharmaceutically acceptable carrier.

26. A process for producing 3-nitro substituted compounds of Formula 1.0h as intermediates for the compounds of Formula (1.0): 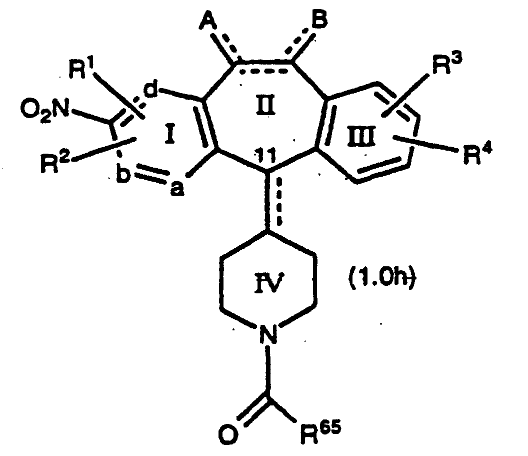 wherein R¹, R², R³, R⁴, A, B, a, b, d, and the dotted lines are as defined for Formula 1.0 in Claim 1, and R⁶⁵ represents H or -OR⁶⁶ wherein R⁶⁶ represents alkyl, said process comprising:
reacting one molar equivalent of a compound of Formula 1.0g: 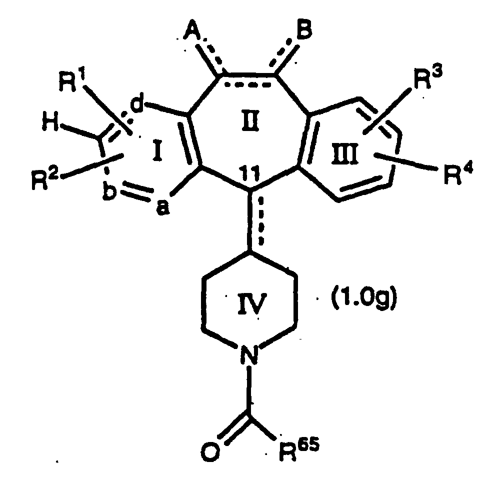
wherein R¹, R², R³, R⁴, A, B, a, b, d, and the dotted lines are as defined for Formula 1.0 in Claim 1, and R⁶⁵ represents H or -OR⁶⁶ wherein R⁶⁶ represents alkyl;
with one molar equivalent of a nitrating reagent, said nitrating reagent being preformed by mixing, at cold temperature, equimolar amounts of tetrabutyl ammonium nitrate with trifluoroacetic anhydride;
the reaction of said nitrating reagent with said compound of Formula 1.0g taking place in a suitable aprotic solvent; and
said reaction with said nitrating reagent being conducted at a temperature and for a period of time sufficient-to allow the reaction to proceed at a reasonable rate to produce the 3-nitro compound of Formula 1.0h.

27. A process for producing 3-nitro compounds of the formula 1.0i as intermediates for the compounds of Formula 1.0: 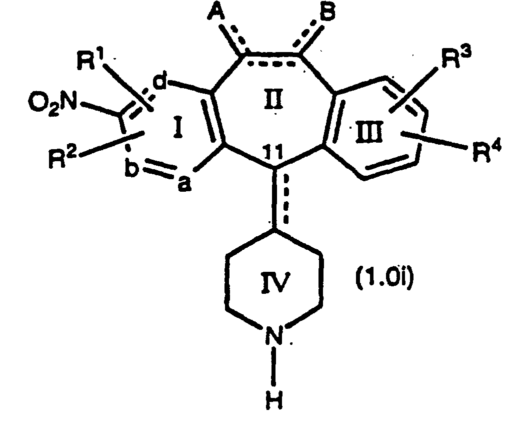 wherein R¹, R², R³, R⁴, A, B, a, b, d, and the dotted lines are as defined for Formula 1.0 in Claim 1, said process comprising:
reacting one molar equivalent of a compound of Formula 1.0g: 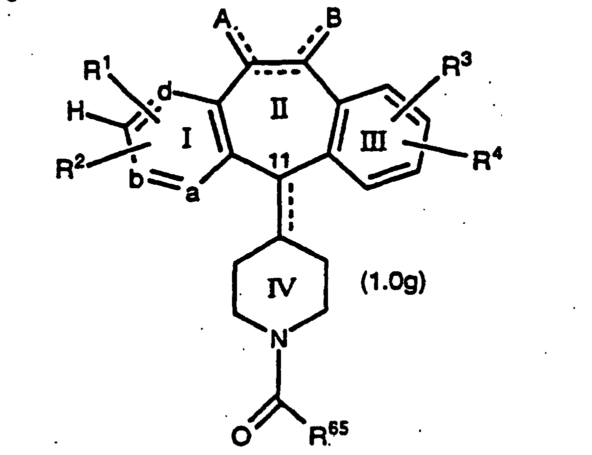
wherein R¹, R², R³, R⁴, A, B, a, b, d, and the dotted lines are as defined for Formula 1.0 in Claim 1, and R⁶⁵ represents H or -OR⁶⁶ wherein R⁶⁶ represents alkyl;
with one molar equivalent of a nitrating reagent, said nitrating reagent being preformed by mixing, at cold temperature, equimolar amounts of tetrabutyl ammonium nitrate with trifluoroacetic anhydride;
the reaction of said nitrating reagent with said compound of Formula 1.0g taking place in a suitable aprotic solvent; and
said reaction with said nitrating reagent being conducted at a temperature and for a period of time sufficient to allow the reaction to proceed at a reasonable rate to produce the 3-nitro compound of Formula 1.0h: 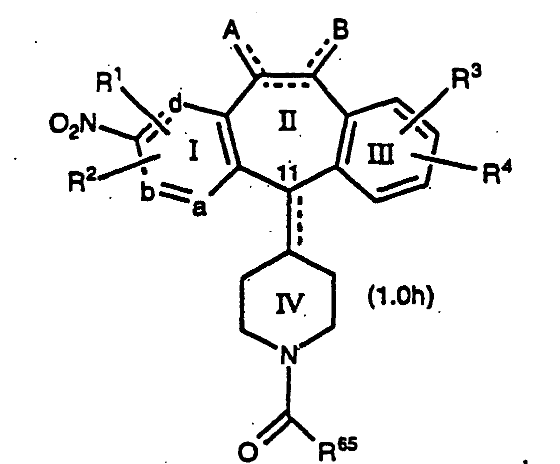 hydrolyzing the compound of Formula 1.0h by dissolving the compound of Formula 1.0h in a sufficient amount of concentrated acid, and heating the resulting mixture to a temperature sufficient to remove the -C(O)R⁶⁵ substituent to produce the compound of Formula 1.0i.

28. A process for producing compounds of the formula 1.0j as intermediates for the compounds of Formula 1.0: 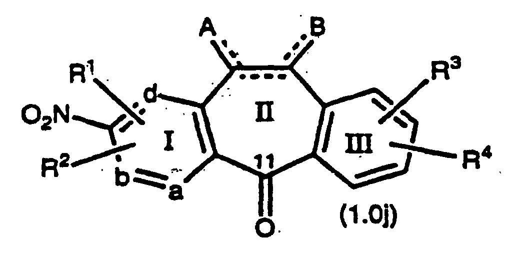 wherein R¹, R², R³, R⁴, A, B, a, b, d, and the dotted lines are as defined for Formula 1.0 in Claim 1, said process comprising:
reacting one molar. equivalent a compound of formula: 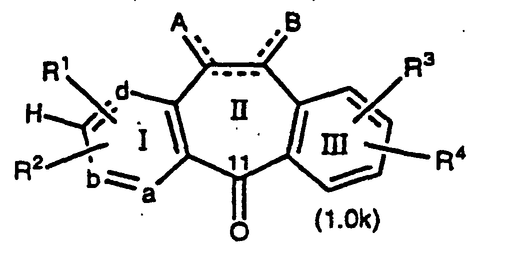
with one molar equivalent of a nitrating reagent;
said nitrating reagent being preformed, by mixing at a cold temperature, equimolar amounts of tetrabutyl ammonium nitrate with trifluoroacetic anhydride;
the reaction of said nitrating reagent with the compound of Formula 1.0k taking place in a suitable aprotic solvent;
said reaction with said nitrating reagent being conducted at a temperature and for a period of time sufficient to allow the reaction to proceed at a reasonable rate to produce the 3-nitro compound of Formula 1.0j.

29. A process for producing a compound of Formula 1.0m as intermediates for the compounds of Formula 1.0: 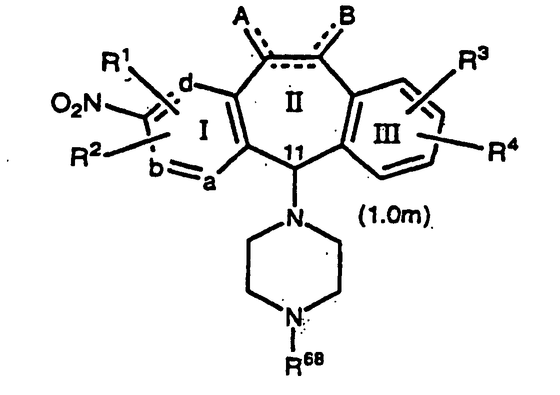 wherein R¹, R², R³, R⁴, A, B, a, b, d, and the dotted lines are as defined for Formula 1.0 in Claim 1, and wherein R⁶⁸ is H or -COOR^{a} wherein R^{a} is a C₁ to C₃ alkyl group, said process comprising:
reacting one molar equivalent a compound of formula: 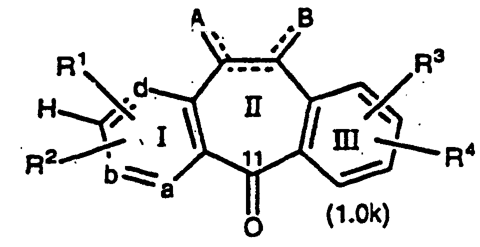
with one molar equivalent of a nitrating reagent;
said nitrating reagent being preformed, by mixing at a cold temperature, equimolar amounts of tetrabutyl ammonium nitrate with trifluoroacetic anhydride;
the reaction of said nitrating reagent with the compound of Formula 1.0k taking place in a suitable aprotic solvent;
said reaction with said nitrating reagent being conducted at a temperature and for a period of time sufficient to allow the reaction to proceed at a reasonable rate to produce the 3-nitro compound of Formula 1.0j: 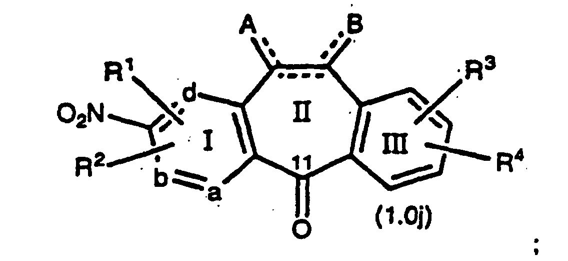 reducing said compound of Formula 1.0j with a suitable reducing agent in a suitable solvent at a suitable temperature to allow the reaction to proceed at a reasonable rate;
reacting the resulting hydroxy product with a chlorinating agent in a suitable organic solvent at a suitable temperature to allow the reaction to proceed at a reasonable rate to produce a compound of Formula 1.0n: 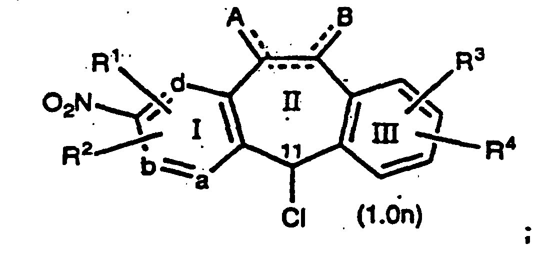 and
reacting said compound of Formula 1.0n with a compound of the formula: 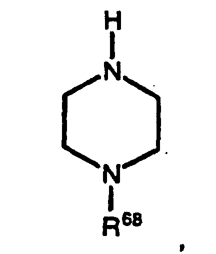 wherein R⁶⁸ is as previously defined, in a suitable organic solvent containing a suitable base at a suitable temperature to allow the reaction to proceed at a reasonable rate to produce the compounds of Formula 1.0m.

30. An intermediate for the preparators of a compound of Formula (1.0) selected from a compound of the formula: 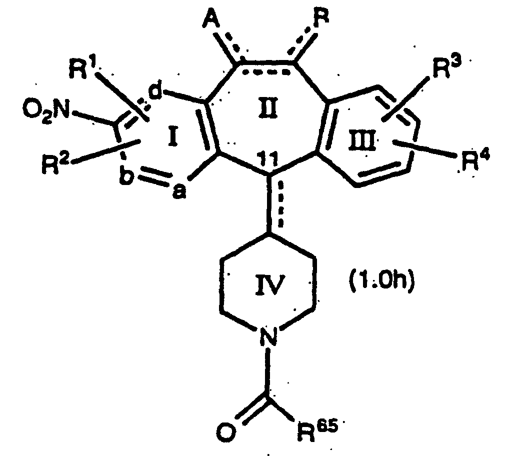 wherein R¹, R², R³, R⁴, A, B, a, b, d, and R⁶⁵ are as defined for Formula 1.0h in Claim 26; 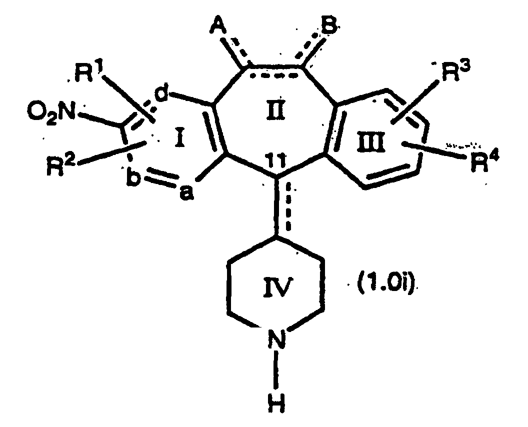 wherein R¹, R2, R³, R⁴, A, B, a, b, and d are as defined for Formula 1.0i in Claim 27; 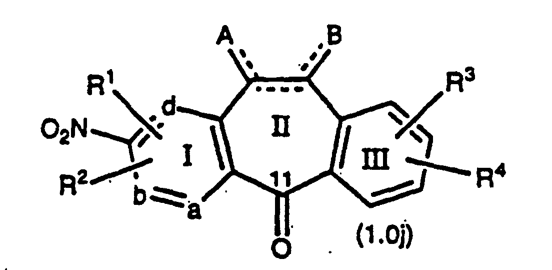 wherein R¹, R², R³, R⁴, A, B, a, b, and d are as defined for Formula 1.0j in Claim 28; 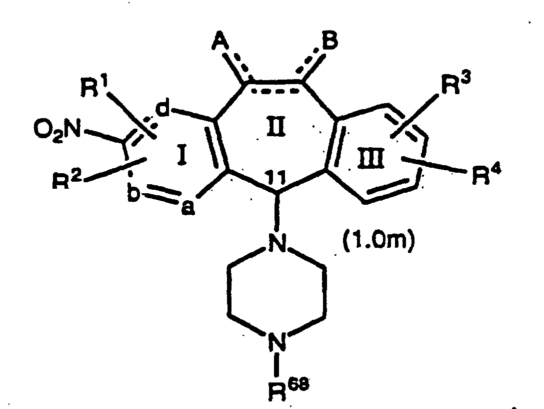 wherein R¹, R², R³, R⁴, A, B, a, b, d and R⁶⁸ are as defined for Formula 1.0m in Claim 29; 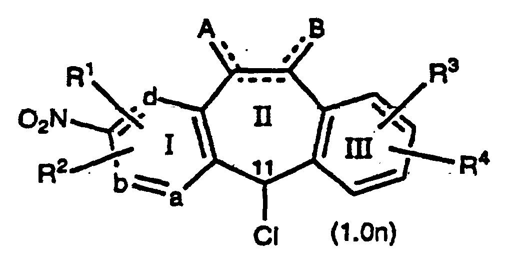 wherein R¹, R², R³, R⁴, A, B, a, b, and d are as defined for Formula 1.0j in Claim 28; or 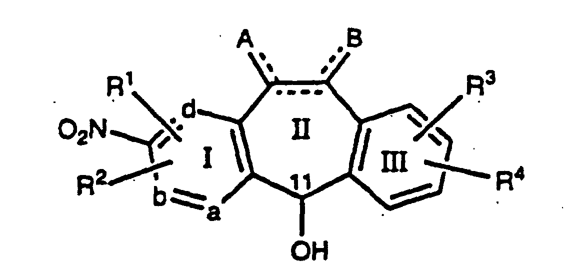 wherein R¹, R², R³, R⁴, A, B, a, b, and d are as defined for Formula 1.0j in Claim 28.

31. A compound according to claim 30 selected from a compound of the formula:

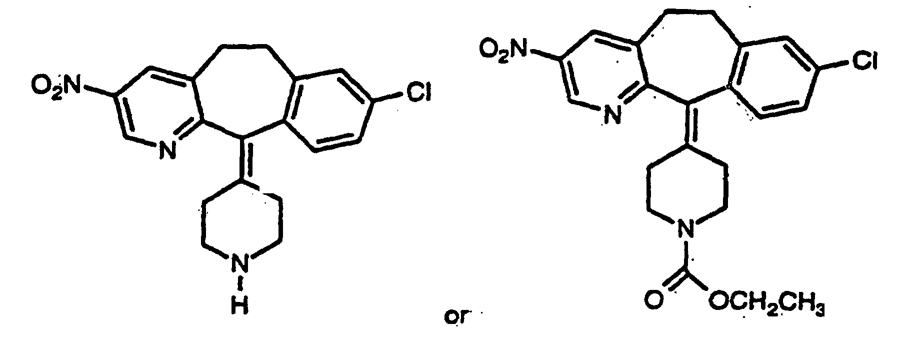

32. The use of a compound of Claim 20 for the manufacture of a medicament for use in inhibiting the abnormal growth of cells.

33. A process for producing a compound of Claim 20, comprising:
(a) reacting a compound of Formula 405.00: 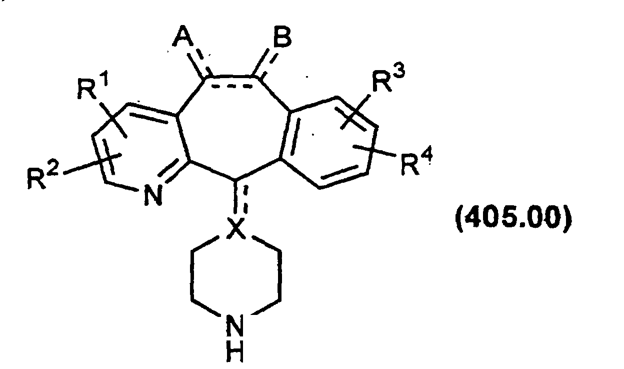 with R⁴⁴COOH at a temperature between 0°C and reflux in an inert solvents;
(b) reacting the compound of Formula 405.00 with R⁴⁴C(O)L, wherein L represents a suitable leaving group, in the presence of base;
(c) reacting a compound of Formula 420.00: 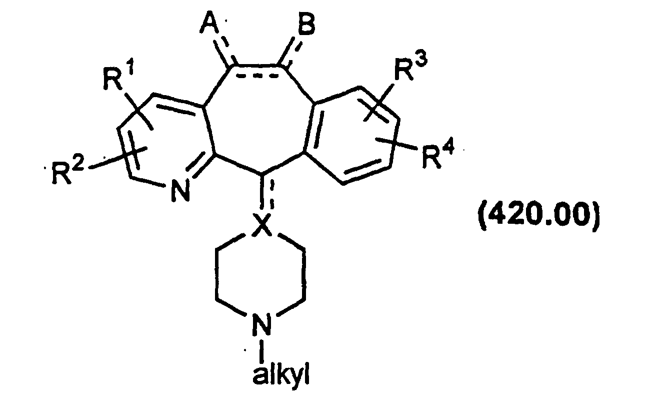 with an appropriate acyl halide or an acyl anhydride;
(d) reducing the compound of Formula 405.00, wherein X is carbon and the bond to C-11 is a double bond, with lithium aluminium hydride in tetrahydrofuran, and then reacting reduced Formula 405.00 with R⁴⁴COOH or R⁴⁴C(O)L to produce compounds of Formula 1.0 in Claim 1 wherein X is carbon and the bond to C-11 is a single bond;
(e) reducing compounds of Formula 470.00d:
with sodium borohydride in methanol or ethanol, converting the resulting alcohol to 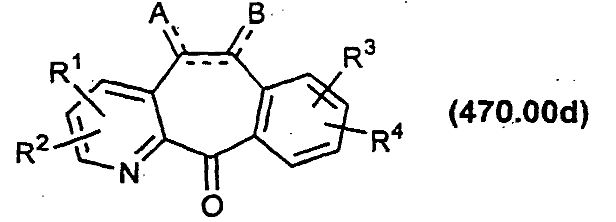
a chloride with thionyl chloride, displacing with piperazine and reacting the resulting compound with R⁴⁴COOH of R⁴⁴C(O)L as described above for the conversion Formula 405.00 to Formula 1.0 wherein the bond to C-11 is a single bond;
(f) cyclizing a compound of Formula 455.00: 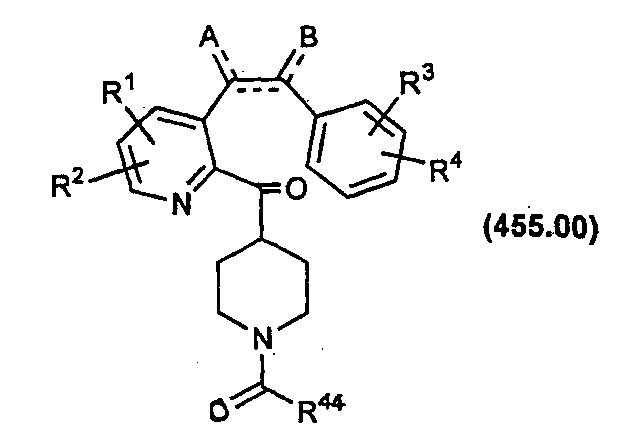 to produce a compound of Formula 1.0 wherein the bond to C-11 is a single bond;
(g) reacting the azaketone of Formula 470.00: 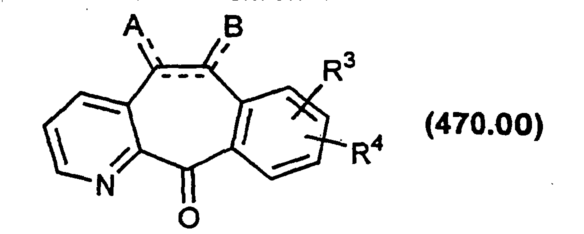 with an oxidizing agent to produce the corresponding compound wherein the nitrogen of the pyridine ring is an N-oxide, reacting the resulting product with a chlorinating agent to produce a compound of Formula 470.00b, optionally repeating the chlorination to produce the disubstituted compound of 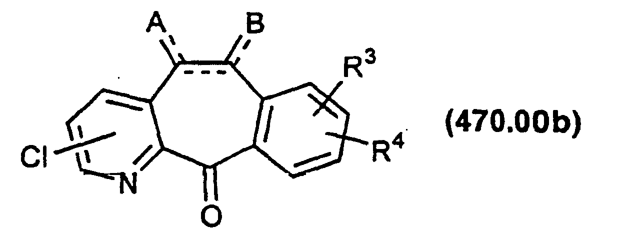 Formula 470.00c, 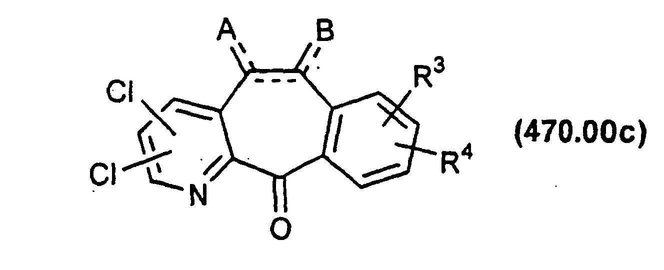 optionally reacting the compound of Formula 470.00b or 470.00c with nucleophiles to obtain the substituted compound of Formula 470.00d above, reacting the compound of Formula 470.00d as described in (f) above to produce compounds of Formula 1.0;
(h) reacting the N-oxide compound of Formula 405.00above with POCl₃ to form a compound of Formula 415.01: 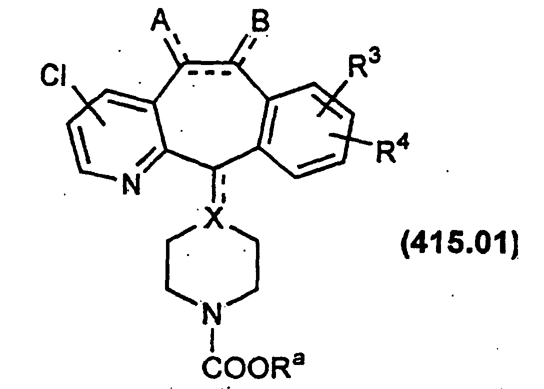
(i) reacting a halo-substituted compound of Formula 405.00 above, wherein R¹ is halo, with an alkyl lithium to provide the lithio derivative and then quenching the lithio derivative with the appropriate electrophile;
(j) reacting a compound of Formula 470.00h: 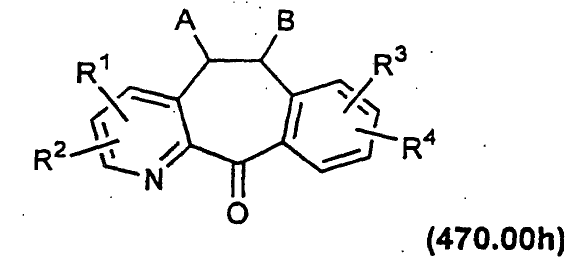 in acetic acid with SeO₂ to produce a compound of Formula 470.00i: 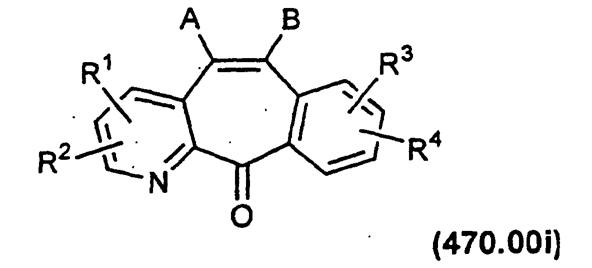 and then converting the compound of 470.00i to a compound of Formula 1.0
(k) alkylating the appropriately substituted piperazine compound of Formula 700.00: 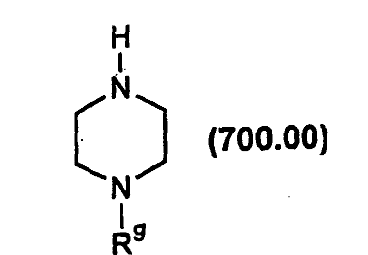 wherein R⁹ is H, C(Z)R⁴⁴ or CO₂R^{a}
with a compound of Formula 705.00: 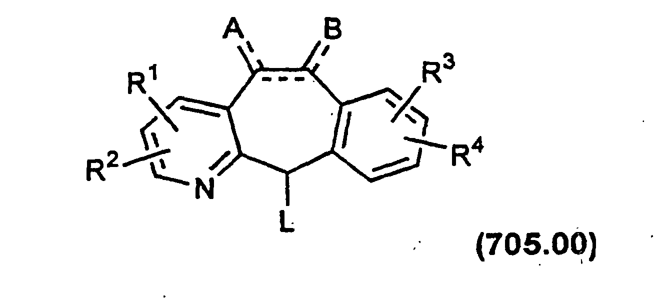 wherein L is a leaving group, to produce a compound of Formula 1.0 wherein X is N when R⁹ is C(Z)R⁴⁴, or when R⁹ is H or CO₂R⁹, the H or a CO₂R^{a} group in the resulting compound is converted to C(Z)R⁴⁴ to produce a compound of formula 1.0 wherein X is N; or
(l) reductive amination of the azaketone of Formula 715.00: 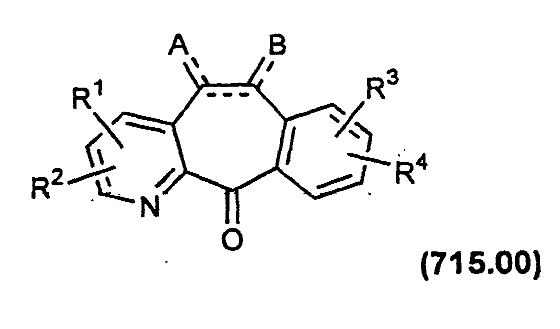 with the piperazine of Formula 700.00 above, wherein R⁹ is H, C(Z)R⁴⁴ or CO₂R^{a} to produce a compound of formula 1.0 when R⁹ is C(Z)R⁴⁴, or when R⁹ is H or CO₂R^{a}, said H or CO₂R^{a} group in the resulting compound is converted to C(Z)R⁴⁴ to produce a compound of formula 1.0.

## Patentansprüche

1. Verwendung einer Verbindung der Formel 1.0: 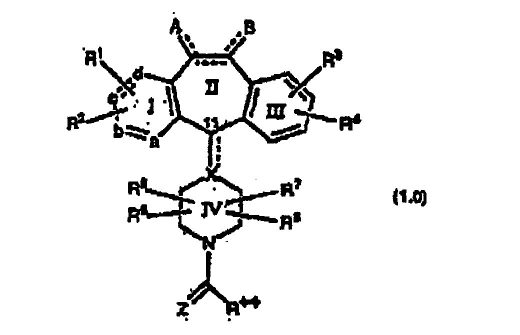 oder eines pharmazeutisch verträglichen Salzes oder Solvats davon, worin:
eines von a, b, c und d für N oder NR⁹ steht, worin R⁹ O, -CH₃ oder -(CH₂)ₙCO₂H, worin n 1 bis 3 ist, ist, und die verbleibenden Gruppen a, b, c und d für CR¹ oder CR² stehen; oder
jedes von a, b, c und d unabhängig aus CR¹ oder CR² ausgewählt wird;
jedes R¹ und jedes R² unabhängig aus H, Halogen, -CF₃, -OR¹⁰, -COR¹⁰, -SR¹⁰, -S(O)ₜR¹¹ (worin t 0, 1 oder 2 ist), -N(R¹⁰)₂, -NO₂, -OC(O)R¹⁰, -CO₂R¹⁰, -OCO₂R¹¹, -CN, -NR¹⁰COOR¹¹, -SR¹¹C(O)OR¹¹, -SR¹¹N(R⁷⁵)₂ (worin jedes R⁷⁵ unabhängig aus H und -C(O)OR¹¹ ausgewählt wird), Benzotriazol-1-yloxy, Tetrazol-5-ylthio oder substituiertem Tetrazol-5-ylthio, Alkinyl, Alkenyl oder Alkyl, wobei die Alkyl- oder Alkenylgruppe gegebenenfalls mit Halogen, -OR¹⁰ oder -CO₂R¹⁰ substituiert ist, ausgewählt wird;
R³ und R⁴ gleich oder verschieden sind und jeweils unabhängig für H, einen beliebigen der Substituenten von R¹ und R² stehen oder R³ und R⁴ zusammengenommen für einen gesättigten oder ungesättigten, an den Benzolring kondensierten C₅-C₇-Ring stehen;
R⁵, R⁶, R⁷ und R⁸ jeweils unabhängig für H, -CF₃, -COR¹⁰, Alkyl oder Aryl stehen, wobei das Alkyl oder Aryl gegebenenfalls mit -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰, OPO₃R¹⁰ substituiert ist, oder eines von R⁵, R⁶, R⁷ und R⁸ mit R⁴⁴, wie nachfolgend definiert, kombiniert werden kann, um -(CH₂)ᵣ, worin r 1 bis 4 ist, welches mit Niederalkyl, Niederalkoxy, -CF₃ oder Aryl substituiert sein kann, zu repräsentieren, oder R⁵ mit R⁶ kombiniert wird, um =O oder =S zu repräsentieren, und/oder R⁷ mit R⁸ kombiniert wird, um =O oder =S zu repräsentieren;
R¹⁰ für H, Alkyl, Aryl oder Arylalkyl steht;
R¹¹ für Alkyl oder Aryl steht;
X für N, CH oder C steht, welches C eine gegebenenfalls vorhandene Doppelbindung, angegeben durch die gestrichelte Linie, zu dem Kohlenstoffatom 11 enthalten kann;
die gestrichelte Linie zwischen den Kohlenstoffatomen 5 und 6 für eine gegebenenfalls vorhandene Doppelbindung steht, so dass, wenn eine Doppelbindung vorhanden ist, A und B unabhängig für -R¹⁰, Halogen, -OR¹¹, -OCO₂R¹¹ oder -OC(O)R¹⁰ stehen, und, wenn keine Doppelbindung zwischen den Kohlenstoffatomen 5 und 6 vorhanden ist, A und B jeweils unabhängig für H₂, -(OR¹¹)₂; H und Halogen, Dihalogen, Alkyl und H, (Alkyl)₂, -H und -OC(O)R¹⁰, H und -OR¹⁰, =O, Aryl und H, =NOR¹⁰ oder -O-(CH₂)ₚ-O-, worin p 2, 3 oder 4 ist, stehen;
R⁴⁴ für 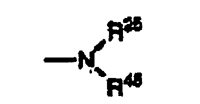
steht wobei R²⁵ für Heteroaryl oder Aryl steht; und R⁴⁸ für H oder Alkyl steht;
oder R⁴⁴ mit R⁵, R⁶, R⁷ oder R⁸, wie oben definiert, kombiniert werden kann, und
Z stellt O oder S dar,
wobei, sofern nicht anders angegeben, die Begriffe "Alkyl", "Alkenyl", "Alkinyl", "Aryl", "Halogen" und "Heteroaryl" die folgenden Bedeutungen haben:
Alkyl (einschließlich der Alkytanteile von Alkoxy, Alkylamino und Dialkylamino) - für gerade und verzweigte Kohlenstoffketten steht und ein bis zwanzig Kohlenstoffatome, vorzugsweise ein bis sechs Kohlenstoffatome enthält;
Alkenyl - für gerade und verzweigte Kohlenstoffketten, welche wenigstens eine Kohlenstoff-Kohlenstoff-Doppelbindung aufweisen und 2 bis 12 Kohlenstoffatome, vorzugsweise 2 bis 6 Kohlenstoffatome und am meisten bevorzugt 3 bis 6 Kohlenstoffatome enthalten, steht;
Alkinyl - für gerade und verzweigte Kohlenstoffketten, welche wenigstens eine Kohlenstoff-Kohlenstoff-Dreifachbindung aufweisen und 2 bis 12 Kohlenstoffatome, vorzugsweise 2 bis 6 Kohlenstoffatome enthalten, steht;
Aryl (einschließlich des Arylanteils von Aryloxy und Alylalkyl) - für eine carbocyclische Gruppe, welche 6 bis 15 Kohlenstoffatome enthält und wenigstens einen aromatischen Ring aufweist (z.B. ist Aryl ein Phenylring) steht, wobei alle verfügbaren substituterbaren Kohlenstoffatome der carbocyclischen Gruppe als mögliche Anheftungspunkte verstanden werden sollen, wobei die carbocyclische Gruppe gegebenenfalls substituiert ist (z.B. 1 bis 3) mit einem oder mehreren von Halogen, Alkyl, Hydroxy, Alkoxy, Phenoxy, CF₃, Amino, Alkylamino, Dialkylamino, -COOR¹⁰ oder -NO₂; und
Halogen - für Fluor, Chlor, Brom und lod steht; und
Heteroaryl - für cyclische Gruppen, die gegebenenfalls mit R³ und R⁴ substituiert sind, steht, die wenigstens ein aus O, S oder N ausgewähltes Heteroatom aufweisen, wobei das Heteroatom eine carbocyclische Ringstruktur unterbricht und eine ausreichende Anzahl von delokalisierten π-Elektronen, um einen aromatischen Charakter zu verleihen, aufweist, wobei die aromatischen heterocyclischen Gruppen vorzugsweise 2 bis 14 Kohlenstoffatome enthalten, z.B. 2-, 3- oder 4-Pyridyl oder Pyridyl-N-oxid (gegebenenfalls substituiert mit R³ und R⁴), wobei Pyridyl-N-oxid angegeben werden kann als: 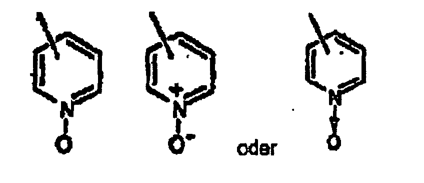
bei der Herstellung einer pharmazeutischen Zusammensetzung zum Hemmen der abnormalen Vermehrung von Zellen.

2. Verwendung nach Anspruch 1, wobei eines von a, b, c und d für N oder NO steht und die verbleibenden Gruppen a, b, c und d für CR¹ oder CR² stehen.

3. Verwendung nach Anspruch 2, wobei a für N steht und b, c und d für CR¹ oder CR² stehen.

4. Verwendung nach einem der vorangegangenen Ansprüche, wobei jedes R¹ und jedes R² unabhängig aus H, Halogen, -CF₃, Niederalkyl oder Benzotriazol-1-yloxy ausgewählt wird.

5. Verwendung nach Anspruch 4, wobei R¹ Cl oder H ist und R² H, Cl oder Br ist

6. Verwendung nach einem der vorangegangenen Ansprüche, wobei R¹ sich an der C-4-Position befindet und R² sich an der C-3-Position befindet

7. Verwendung nach einem der vorangegangenen Ansprüche, wobei R³ und R⁴ gleich oder verschieden sind und jeweils unabhängig für H, Halogen, -CF₃, -OR¹⁰ oder Alkyl stehen.

8. Verwendung nach Anspruch 7, wobei R³ und R⁴ unabhängig für H oder Halogen stehen.

9. Verwendung nach einem der vorangegangenen Ansprüche, wobei R³ sich an der C-8-Position befindet und R⁴ slch an der C-9-Position befindet.

10. Verwendung nach Anspruch 9, wobei R^{a} Cl an der C-8-Position ist und R⁴ H an der C-9-Position ist.

11. Verwendung nach einem der vorangegangenen Ansprüche, wobei R⁵, R⁶, R⁷ und R⁸ jeweils unabhängig für H stehen.

12. Verwendung nach Anspruch 1, wobei a N ist und b, c und d Kohlenstoff sind; R¹ und R² gleich oder verschieden sind und jedes unabhängig aus H, Halogen, -CF₃, Niederalkyl oder Benzotriazol-1-yloxy ausgewählt wird, und R¹ sich an der C-4-Position befindet und R² sich an der C-3-Position befindet R³ und R⁴ gleich oder verschieden sind und jedes unabhängig aus H oder Halogen ausgewählt wird, und R³ sich an der C-8-Position befindet und R⁴ sich an der C-9-Position befindet; wenn die Doppelbindung zwischen den Kohlenstoffatomen 5 und 6 vorhanden ist, A und B unabhängig für H, Niederalkyl oder Alkyloxy stehen; und, wenn die Doppelbindung zwischen den Kohlenstoffatomen 5 und 6 fehlt, A und B unabhängig für H₂, (-H und -OH) oder =O stehen; R⁵, R⁶, R⁷ und R⁸ H sind; Z O ist; und R für R⁴⁴ steht und R²⁵ für Pyridyl oder Phenyl steht.

13. Verwendung nach Anspruch 12, wobei R²⁵ für 3-Pyridyl oder Phenyl steht; R³ Cl ist; R⁴ H ist; R⁴⁸ für H oder Methyl steht; und R¹ und R² individuell aus H, Benzotriazol-1-yloxy, C₁- bis C₄-Alkyl oder Halogen ausgewählt werden.

14. Verwendung nach Anspruch 13, wobei R¹ und R² unabhängig aus H, Br, Cl, Methyl oder Benzotriazol-1-yloxy ausgewählt werden.

15. Verwendung nach einem der vorangegangenen Ansprüche, wobei die gehemmten Zellen Tumorzellen sind, die ein aktiviertes ras-Onkogen exprimieren.

16. Verwendung nach Anspruch 15, wobei die gehemmten Zellen Pankreastumorzellen, Lungenkrebszellen, myeloische Leukämie-Tumorzellen, Schilddrüsenfollikeltumorzellen, myelodysplastische Tumorzellen, Epidermiskarzinomtumorzellen, Blasenkarzinomtumorzellen oder Kolontumorzellen sind.

17. Verwendung nach Anspruch 1, wobei die Hemmung der abnormalen Vermehrung von Zellen durch die Hemmung von ras-Famesylproteintransferase erfolgt.

18. Verwendung nach Anspruch 1, wobei die Hemmung bei Tumorzellen erfolgt, bei welchen das Ras-Protein als Ergebnis einer onkogenen Mutation in anderen Genen als dem Ras-Gen aktiviert ist.

19. Verwendung nach Anspruch 1, wobei die Verbindung ausgewählt wird aus den folgenden Formeln:

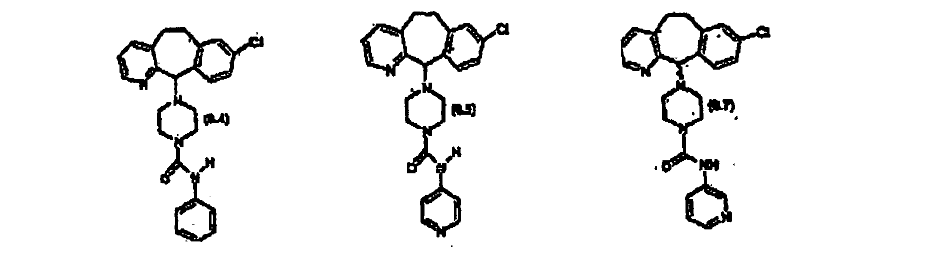

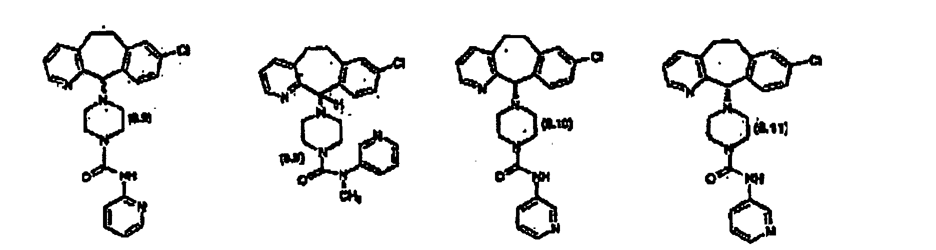

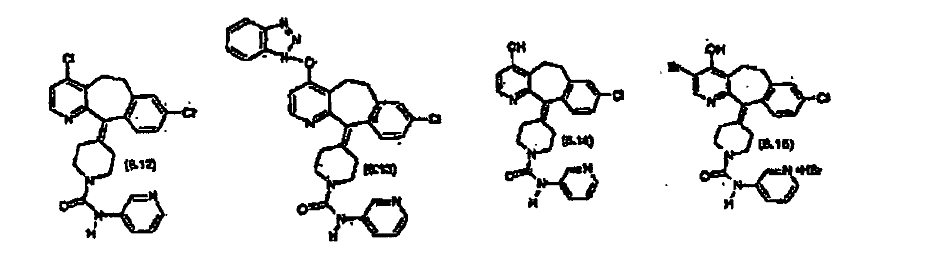

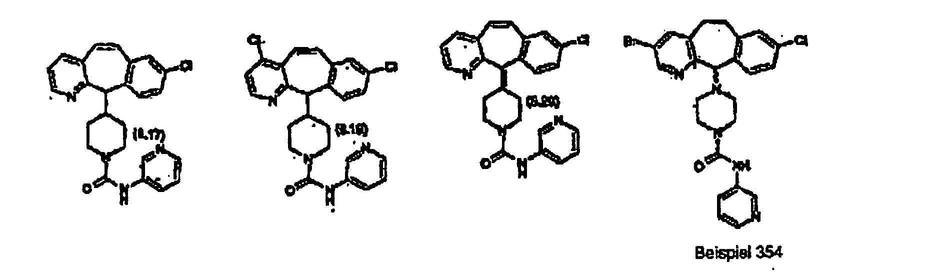

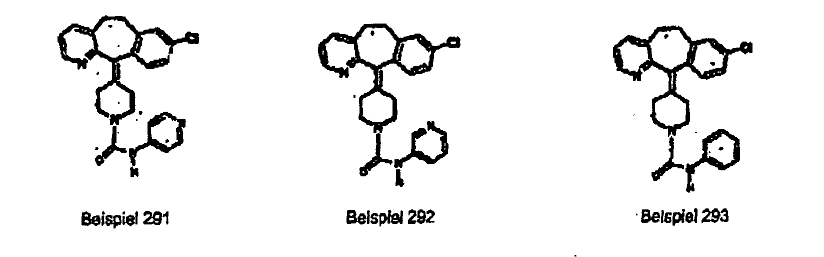

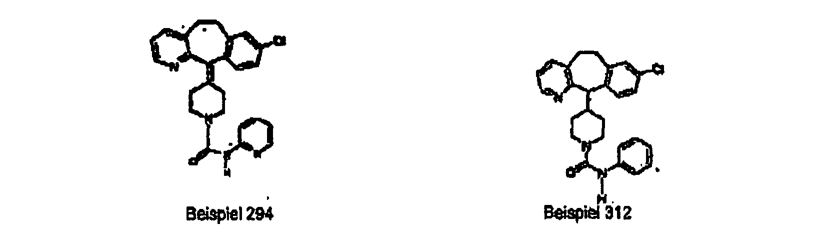

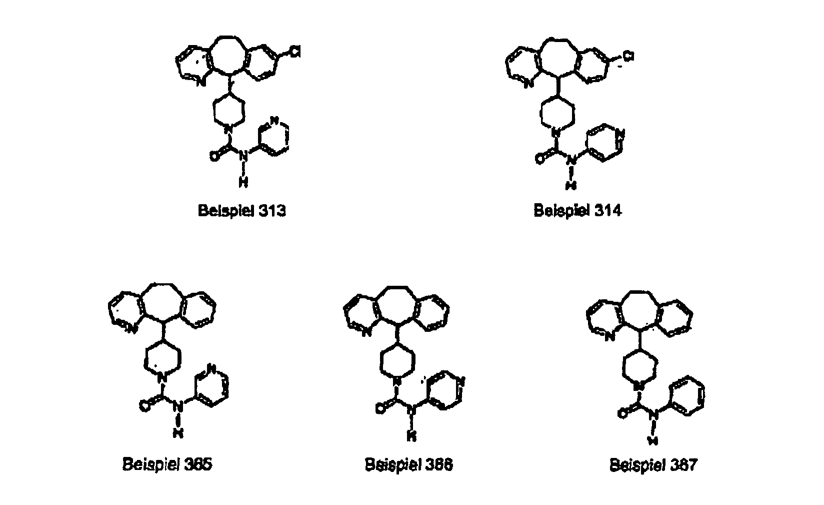

20. Verbindung, ausgewählt aus einer Verbindung der Formel: 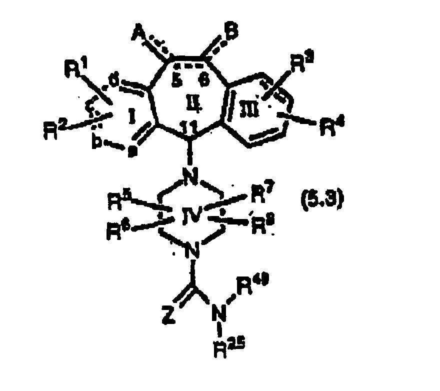 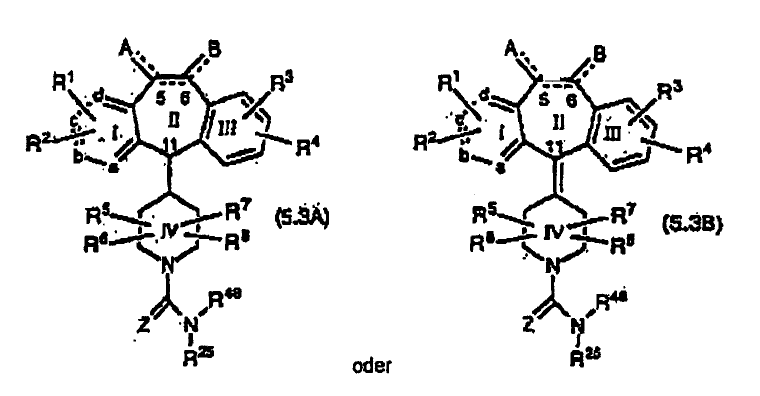 oder ein pharmazeutisch verträgliches Salz oder Solvat davon, wobei alle Substituenten wie in Anspruch 1 definiert sind.

21. Verbindung nach Anspruch 20, wobei a N ist und b, c und d Kohlenstoff sind; R¹ und R² gleich oder verschieden sind und jedes unabhängig aus H, Halogen, -CF₃, Niederalkyl oder Benzotriazol-1-yloxy ausgewählt wird, und R¹ sich an der C-4-Position befindet und R² sich an der C-3-Position befindet; R³ und R⁴ gleich oder verschieden sind und jedes unabhängig aus H oder Halogen ausgewählt wird, und R³ sich an der C-8-Position befindet und R⁴ sich an der C-9-Position befindet; wenn die Doppelbindung zwischen den Kohlenstoffatomen 5 und 6 vorhanden ist, A und B unabhängig für H, Niederalkyl oder Alkyloxy stehen; und, wenn die Doppeibindung zwischen den Kohlenstoffatomen 5 und 6 fehlt A und B unabhängig für H₂, (-H und -OH) oder =O stehen; R⁵, R⁶, R⁷ und R⁸ H sind; Z O ist; und R²⁵ für Phenyl, 3-Pyridyl, 3-Pyridyl-N-oxid, 4-Pyridyl-, 4-Pyridyl-N-oxid, 3-N-Methylpiperidinyl, 4-N-Methylpiperidinyl, 3-N-Acetylpiperidinyl oder 4-N-Acetylpiperidinyl steht.

22. Verbindung nach Anspruch 21, wobei R²⁵ für Phenyl, 3-Pyridyl, 3-Pyridyl-N-oxid, 4-Pyridyl-, 4-Pyridyl-N-oxid, 3-N-Methylpiperidinyl oder 4-N-Methylpiperidinyl steht; R³ Cl ist; R⁴ H ist; R⁴⁸ für H oder Methyl steht; und R¹ und R² individuell aus H, Benzotriazol-1-yloxy, C₁- bis C₄-Alkyl oder Halogen ausgewählt werden.

23. Verbindung nach Anspruch 22, wobei R¹ und R² individuell aus H, Br, Cl, Methyl oder Benzotriazol-1-yloxy ausgewählt werden.

24. Verbindung nach Anspruch 20, ausgewählt aus den folgenden Formeln:

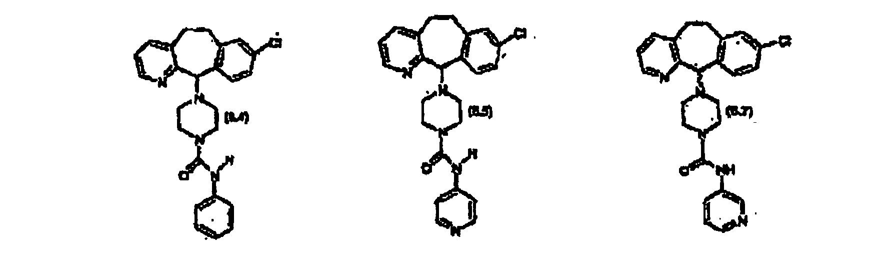

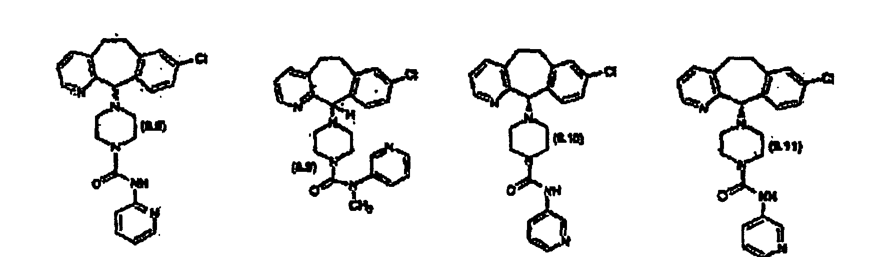

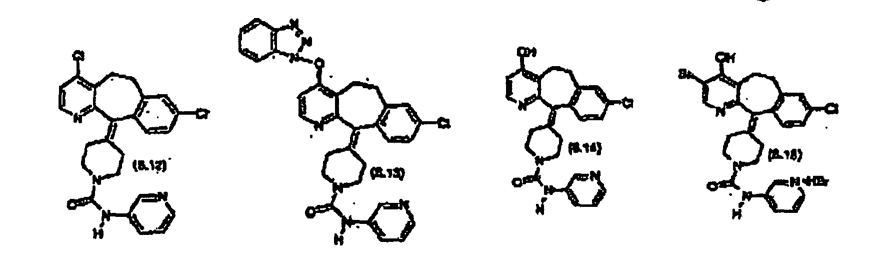

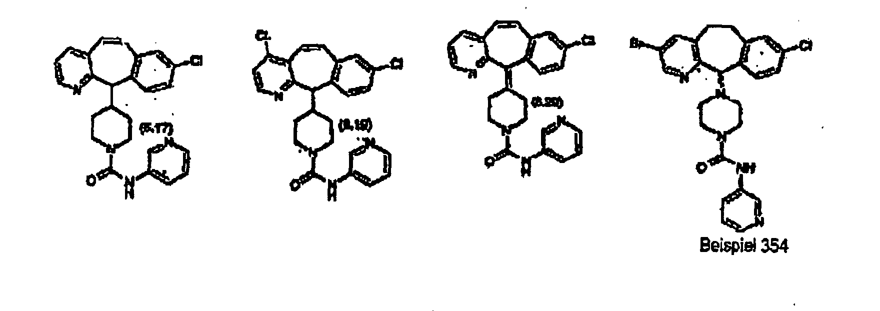

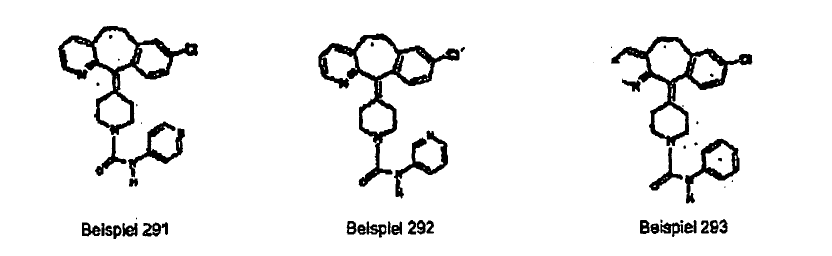

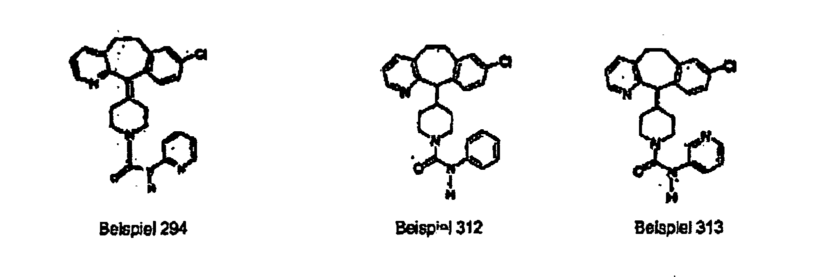

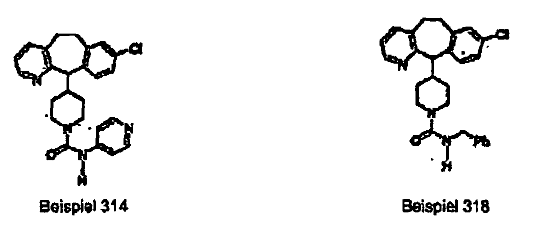

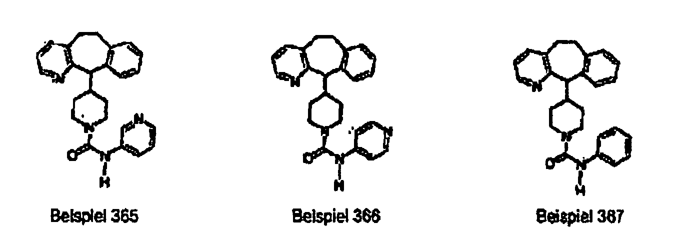

25. Pharmazeutische Zusammensetzung zum Hemmen der abnormalen Vermehrung von Zellen, umfassend eine wirksame Menge einer Verbindung nach Anspruch 20 in Kombination mit einem pharmazeutisch verträglichen Träger.

26. Verfahren zur Herstellung von 3-Nitro-substituierten Verbindungen der Formel 1.0h als Zwischenprodukte für die Verbindungen der Formel (1.0): 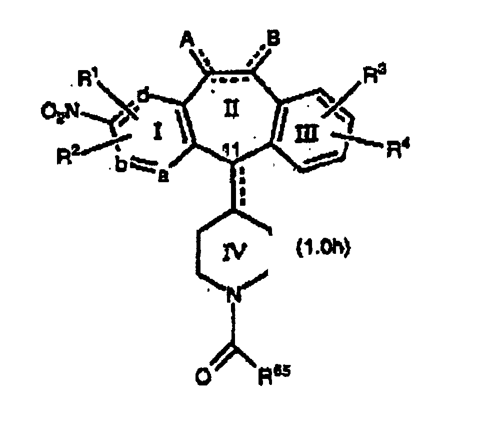 worin R¹, R², R³, R⁴, A, B, a, b, d, und die gestrichelten Linien wie für Formel 1.0 in Anspruch 1 definiert sind und R⁶⁵ für H oder -OR⁶⁶, worin R⁶⁶ für Alkyl steht, steht, wobei das Verfahren umfasst:
ein Moläquivalent einer Verbindung der Formel 1.0g: 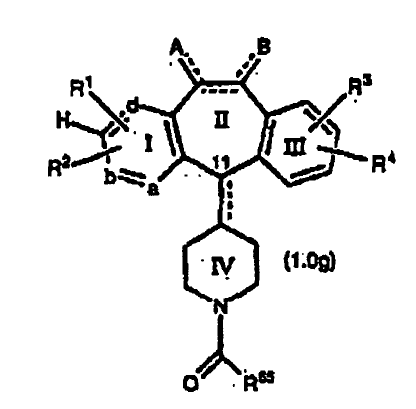
in welcher R¹, R², R³, R⁴, A, B, a, b, d und die gestrichelten Linien wie für Formel 1.0 in Anspruch 1 definiert sind und R⁶⁵ für H oder -OR⁶⁶, worin R⁶⁶ für Alkyl steht, steht;
mit einem Moläquivalent eines Nitrierungsreagens umzusetzen, wobei das Nitrierungsreagens vorab hergestellt wird, indem bei kalter Temperatur äquimolare Mengen von Tetrabutylammoniumnitrat mit Trifluoressigsäureanhydrid gemischt werden;
wobei die Reaktion des Nitrierungsreagens mit der Verbindung der Formel 1.0g in einem geeigneten aprotischen Lösemittel stattfindet; und
wobei die Reaktion mit dem Nitrierungsreagens bel einer Temperatur und für eine Zeitdauer ausgeführt wird, die ausreichen, um zu ermöglichen, dass die Reaktion mit einer angemessenen Geschwindigkeit unter Herstellung der 3-Nitro-Verbindung der Formel 1.0h fortschreitet.

27. Verfahren zur Herstellung von 3-Nitroverbindungen der Formel 1.0i als Zwischenprodukte für die Verbindungen der Formel 1.0: 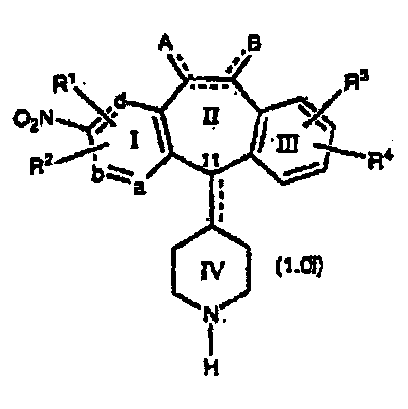 in weicher R¹, R², R³, R⁴, A, B, a, b, d und die gestrichelten Linien wie für Formel 1.0 in Anspruch 1 definiert sind, wobei das Verfahren umfasst:
ein Moläquivalent einer Verbindung der Formel 1.0g: 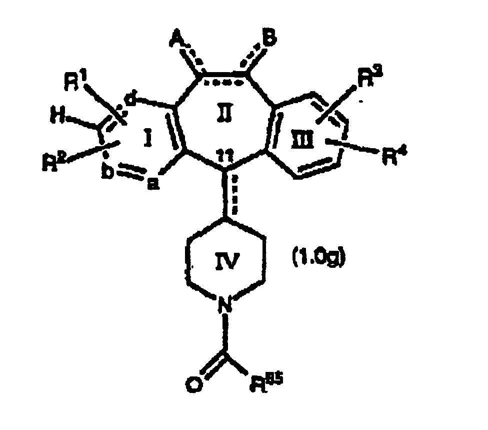
in welcher R¹, R², R³, R⁴, A, B, a, b, d und die gestrichelten Linien wie für Formel 1.0 in Anspruch 1 definiert sind und R⁶⁵ für H oder -OR⁶⁶, worin R⁶⁶ für Alkyl steht, steht;
mit einem Moläquivalent eines Nitrierungsreagens umzusetzen, wobei das Nitrierungsreagens vorab hergestellt wird, indem bei kalter Temperatur äquimolare Mengen von Tetrabutylammoniumnitrat mit Trifluoressigsäureanhydrid gemischt werden;
wobei die Reaktion des Nitrierungsreagens mit der Verbindung der Formel 1.0g in einem geeigneten aprotischen Lösemittel stattfindet; und
wobei die Reaktion mit dem Nitrierungsreagens bei einer Temperatur und für eine Zeitdauer ausgeführt wird, die ausreichen, um zu ermöglichen, dass die Reaktion mit einer angemessenen Geschwindigkeit unter Herstellung der 3-Nitro-Verbindung der Formel 1.0h fortschreitet: 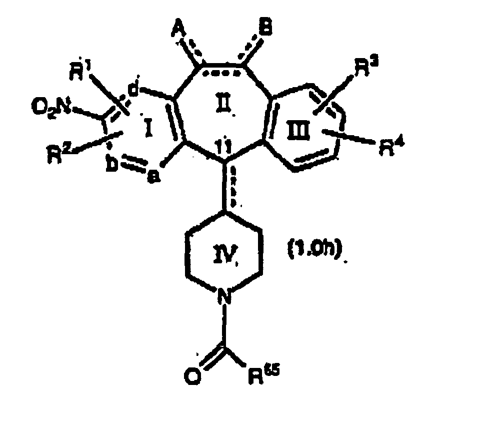 die Verbindung der Formel 1.0h zu hydrolysieren, indem die Verbindung der Formel 1.0h in einer ausreichenden Menge konzentrierter Säure gelöst wird und die resultierende Mischung auf eine ausreichende Temperatur erwärmt wird, um den Substituenten -C(O)R⁶⁵ zu entfernen, um die Verbindung der Formel 1.0i herzustellen.

28. Verfahren zur Herstellung von Verbindungen der Formel 1.0j als Zwischenprodukte für die Verbindungen der Formel 1.0: 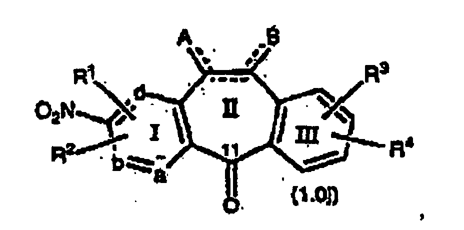 in welcher R¹, R², R³, R⁴, A, B, a, b, d und die gestrichelten Linien wie für Formel 1.0 in Anspruch 1 definiert sind, wobei das Verfahren umfasst:
ein Moläquivalent einer Verbindung der Formel: 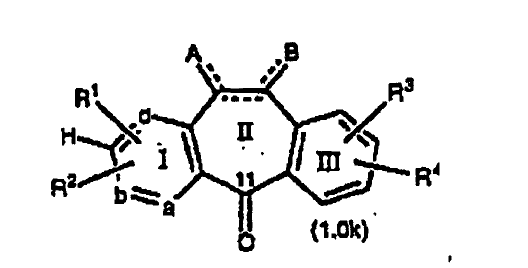
mit einem Moläquivalent eines Nitrierungsnaagens umzusetzen;
wobei das Nilrierungsreagens vorab hergestellt wird, indem bei kalter Temperatur äquimolare Mengen von Tetrabutylammoniumnitrat mit Trifluoressigsäureanhydrid gemischt werden;
wobei die Reaktion des Nitrierungsreagens mit der Verbindung der Formel 1.0k in einem geeigneten aprotischen Lösemittel stattfindet;
wobei die Reaktion mit dem Nitrierungsreagens bei einer Temperatur und für eine Zeitdauer ausgeführt wird, die ausreichen, um zu ermöglichen, dass die Reaktion mit einer angemessenen Geschwindigkeit unter Herstellung der 3-Nitro-Verbindung der Formel 1.0j fortschreitet.

29. Verfahren zur Herstellung einer Verbindung der Formel 1.0m als Zwischenprodukte für die Verbindungen der Formel 1.0: 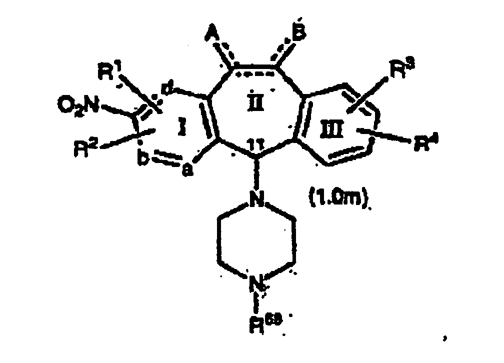 in welcher R¹, R², R³, R⁴, A, B, a, b, d und die gestrichelten Linien wie für Formel 1.0 in Anspruch 1 definiert sind und wobei R⁶⁸ H oder -COOR^{a}, worin R^{a} eine C,- bis C₃-Alkylgruppe ist, ist, wobei das Verfahren umfasst:
ein Moläquivalent einer Verbindung der Formel: 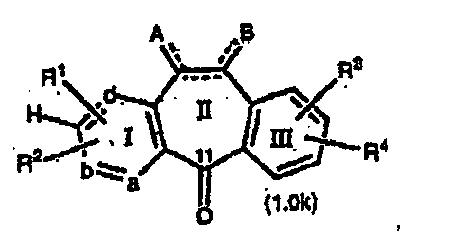
mit einem Moläquivalent eines Nitrierungsreagens umzusetzen;
wobei das Nitrierungsreagens vorab hergestellt wird, Indem bei kalter Temperatur äquimolare Mengen von Tetrabutylammoniumnitrat mit Trifluoressigsäureanhydrid gemischt werden;
wobei die Reaktion des Nitrierungsreagens mit der Verbindung der Formel 1.0k in einem geeigneten aprotischen Lösemittel stattfindet;
wobei die Reaktion mit dem Nitrierungsreagens bei einer Temperatur und für eine Zeitdauer ausgeführt wird, die ausreichen, um zu ermöglichen, dass die Reaktion mit einer angemessenen Geschwindigkeh unter Herstellung der 3-Nitro-Verbindung der Formel 1.0j fortschreitet: 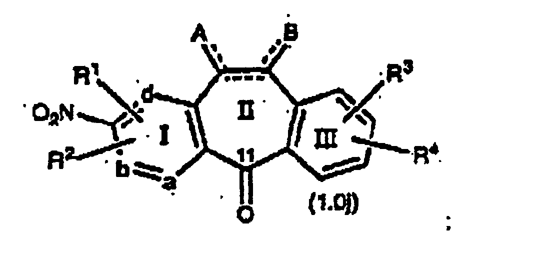 die Verbindung der Formel 1.0j mit einem geeigneten Reduktionsmittel in einem geeigneten Lösemittel bei einer geeigneten Temperatur, um zu ermöglichen, dass die Reaktion mit einer angemessenen Geschwindigkeit fortschreitet, zu reduzieren;
das resultierende Hydroxyprodukt mit einem Chlorierungsmittel in einem geeigneten organischen Lösemittel bei einer geeigneten Temperatur umzusetzen, um zu ermöglichen, dass die Reaktion mit einer angemessenen Geschwindigkeit unter Herstellung einer Verbindung der Formel 1.0n fortschreitet: 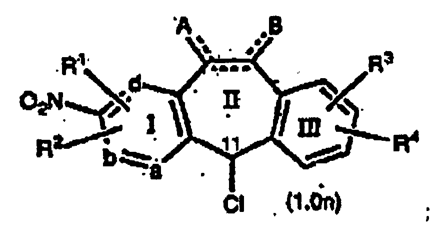 und
die Verbindung der Formel 1.0n mit einer Verbindung der Formel: 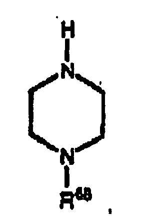 in welcher R⁶⁸ wie zuvor definiert ist, in einem geeigneten organischen Lösemittel, welches eine geeignete Base enthält, bei einer geeigneten Temperatur, um zu ermöglichen, dass die Reaktion mit einer angemessenen Geschwindigkeit fortschreitet, unter Herstellung der Verbindungen der Formel 1.0m umzusetzen.

30. Zwischenprodukt für die Herstellung einer Verbindung der Formel (1.0), ausgewählt aus einer Verbindung der Formel: 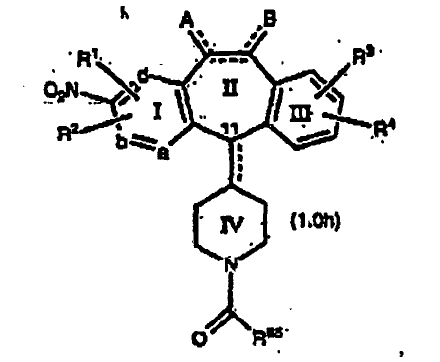 in welcher R¹, R², R³, R⁴, A, B, a, b, d und R⁶⁵ wie für Formel 1.0h in Anspruch 26 definiert sind; 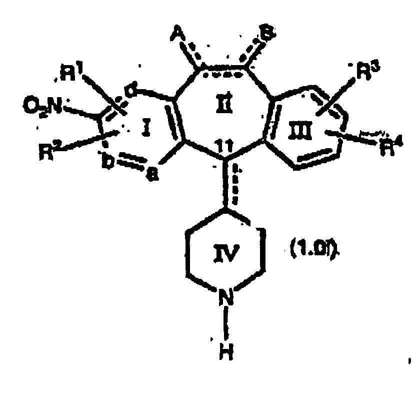 in welcher R¹, R², R³, R⁴, A, B, a, b und d wie für Formel 1.0i in Anspruch 27 definiert sind; 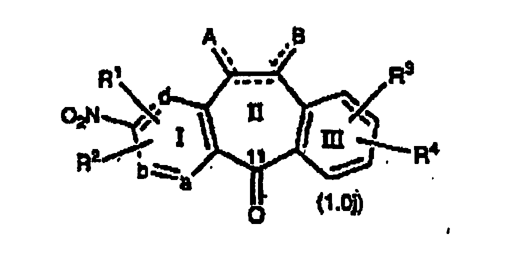 in welcher R¹, R², R³, R⁴, A, B, a, b und d wie für Formel 1.0j in Anspruch 28 definiert sind; 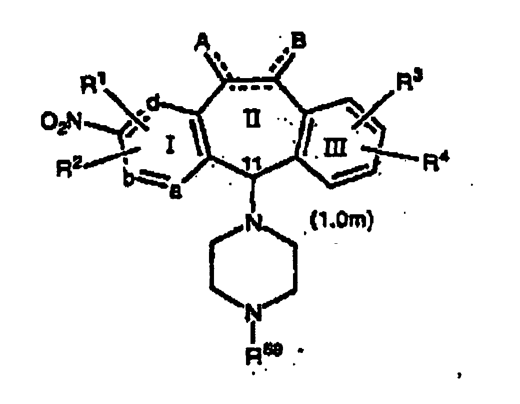 in welcher R¹, R², R³, R⁴, A, B, a, b, d und R⁶⁸ wie für Formel 1.0m in Anspruch 29 definiert sind; 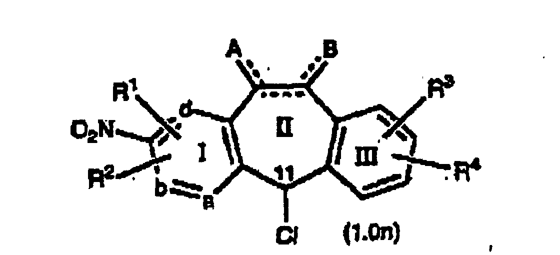 in weicher R¹, R², R³, R⁴, A, B, a, b und d wie für Formel 1.0j in Anspruch 28 definiert sind; oder 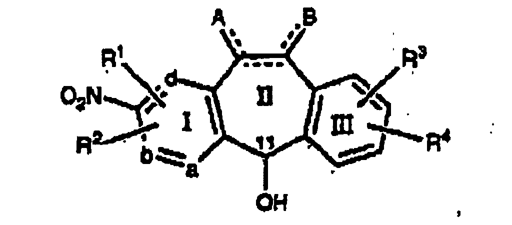 in welcher R¹, R², R³, R⁴, A, B, a, b und d wie für Formel 1.0j in Anspruch 28 definiert sind.

31. Verbindung nach Anspruch 30, ausgewählt aus einer Verbindung der Formel:

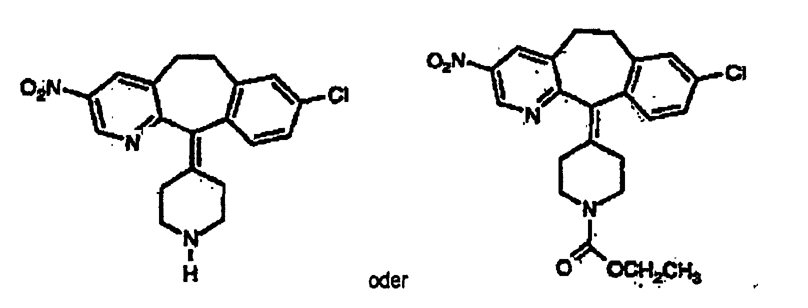

32. Verwendung einer Verbindung nach Anspruch 20 für die Herstellung eines Arzneimittels zur Verwendung bei der Hemmung der abnormalen Vermehrung von Zellen.

33. Verfahren zur Herstellung einer Verbindung nach Anspruch 20, umfassend:
(a) eine Verbindung der Formel 405.00: 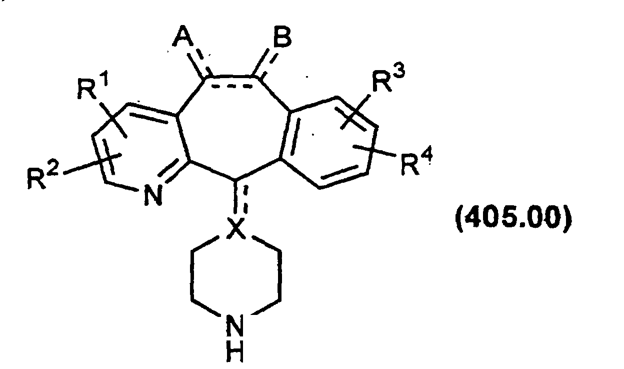 mit R⁴⁴COOH bei einer Temperatur zwischen 0°C und Rückfluss in einem inerten Lösemittel umzusetzen;
(b) die Verbindung der Formel 405.00 mit R⁴⁴C(O)L, worin L für eine geeignete austretende Gruppe steht, in Gegenwart einer Base umzusetzen;
(c) eine Verbindung der Formel 420.00: 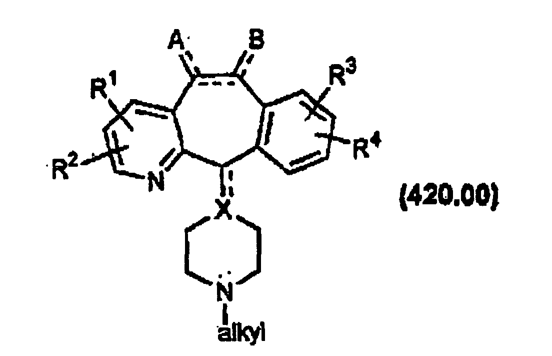 mit einem geeigneten Acylhalogenid oder einem Acylanhydrid umzusetzen;
(d) die Verbindung der Formel 405.00, in welcher X Kohlenstoff ist und die Bindung zu C-11 eine Doppelbindung ist, mit Lithiumaluminiumhydrid in Tetrahydrofuran zu reduzieren und dann die reduzierte Formel 405.00 mit R⁴⁴COOH oder R⁴⁴C(O)L umzusetzen, um Verbindungen der Formel 1.0 in Anspruch 1 herzustellen, in denen X Kohlenstoff ist und die Bindung zu C-11 eine Einfachbindung ist;
(e) Verbindungen der Formel 470.00d: 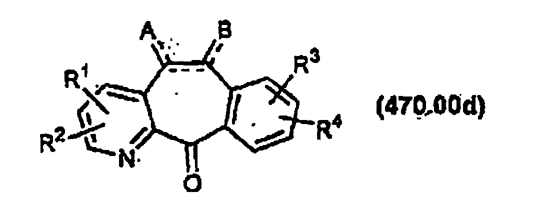 mit Natriumborhydrid in Methanol oder Ethanol zu reduzieren, den resultierenden Alkohol mit Thionylchlorid in ein Chlorid umzuwandeln, mit Piperazin zu verdrängen und die resultierende Verbindung mit R⁴⁴COOH oder R⁴⁴C(O)L umzusetzen, wie oben beschrieben, für die Umwandlung der Formel 405.00 in Formel 1.0. in welcher die Bindung zu C-11 eine Einfachbindung ist;
(f) eine Verbindung der Formel 455.00 zu cyclisieren: 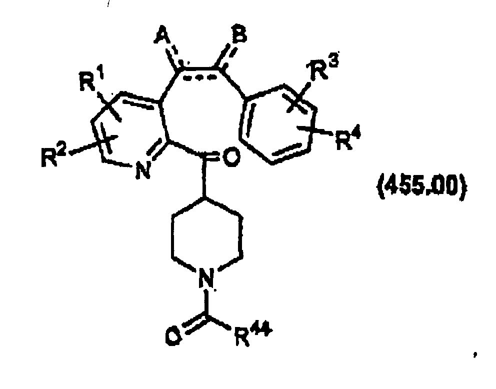 um eine Verbindung der Formel 1.0, in welcher die Bindung zu C-11 eine Einfachbindung ist, herzustellen;
(g) das Azaketon der Formel 470.00: 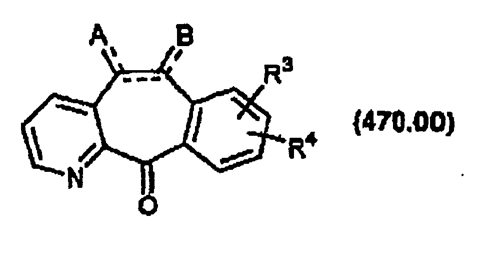 mit einem Oxidationsmittel umzusetzen, um die entsprechende Verbindung, in welcher das Stickstoffatom des Pyridinrings ein N-Oxid ist, herzustellen, das resultierende Produkt mit einem Chlorierungsmittel umzusetzen, um eine Verbindung der Formel 470.00b herzustellen, 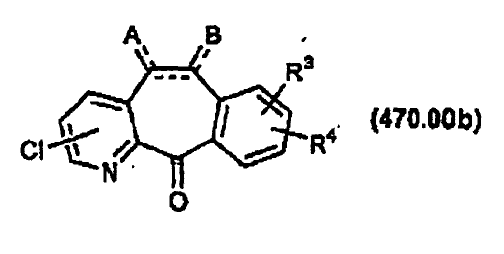 gegebenenfalls die Chlorierung zu wiederholen, um die disubstituierte Verbindung der Formel 470.00c herzustellen, 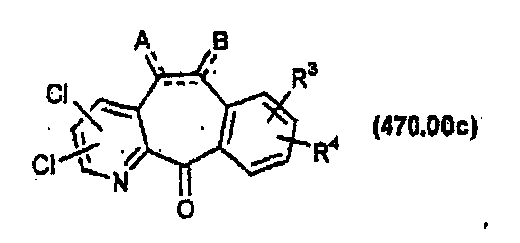 gegebenenfalls die Verbindung der Formel 470.00b oder 470.00c mit Nukleophilen umzusetzen, um die substituierte Verbindung der obigen Formel 470.00d zu erhalten, die Verbindung der Formel 470.00d, wie in (f) oben beschrieben, umzusetzen, um Verbindungen der Formel 1.0 herzustellen;
(h) die N-Oxid-Verbindung der obigen Formel 405.00 mit POCl₃ umzusetzen, um eine Verbindung der Formel 415.01 zu bilden: 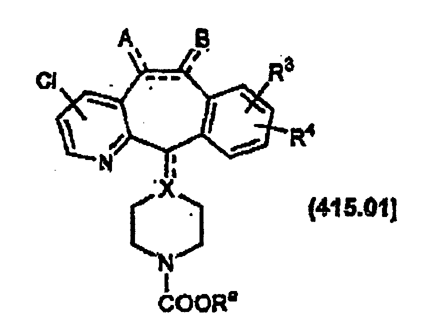
(i) eine Halogen-eubstituierte Verbindung der obigen Formel 405.00, in welcher R¹ Halogen ist, mit einem Alkyllithium umzusetzen, um das Lithiumderivat bereitzustellen, und dann das Lithiumderivat mit dem geeigneten Elektrophil zu quenchen;
(j) eine Verbindung der Formel 470.00h: 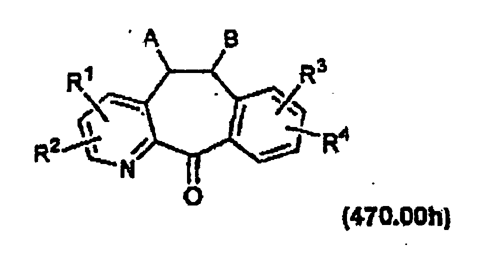 in Essigsäure mit SeO₂ umzusetzen, um eine Verbindung der Formel 470.00i herzustellen, 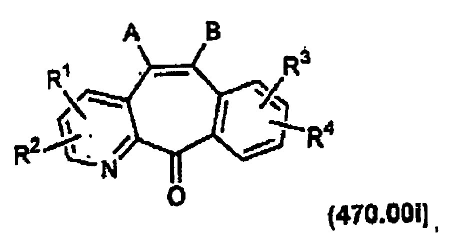 und dann die Verbindung 470.00i in eine Verbindung der Formel 1.0 umzuwandeln;
(k) die geelgnet substituierte Piperazinverbindung der Formel 700.00: 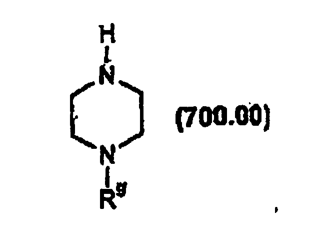 in welcher R⁹ H, C(Z)R⁴⁴ oder CO₂R^{a} ist,
mit einer Verbindung der Formel 705.00: 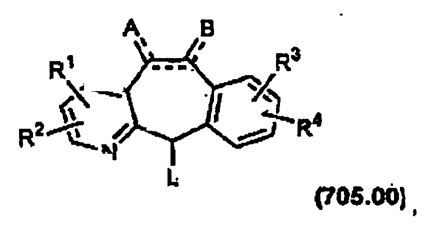 in welcher L eine austretende Gruppe ist, zu alkylieren, um eine Verbindung der Formel 1.0 herzustellen, in welcher X N ist, wenn R⁹ C(Z)R⁴⁴ ist, oder, wenn R⁹ H oder CO₂R^{a} ist, das H oder eine Gruppe CO₂R^{a} in der resultierenden Verbindung in C(Z)R⁴⁴ umgewandelt wird, um eine Verbindung der Formel 1.0 herzustellen, in welcher X N ist; oder
(I) reduktive Aminierung des Azaketons der Formel 715.00: 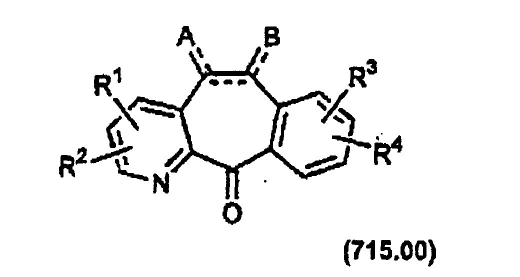 mit dem Piperazin der obigen Formel 700.00, in welcher R⁹ H, C(Z)R⁴⁴ oder CO₂R^{a} ist, um eine Verbindung der Formel 1.0 herzustellen, wenn R⁹ C(Z)R⁴⁴ ist, oder, wenn R⁹ H oder CO₂R^{a} ist, das H oder die Gruppe CO₂R^{a} in der resultierenden Verbindung in C(Z)R⁴⁴ umgewandelt wird, um eine Verbindung der Formel 1.0 herzustellen.

## Revendications

1. Emploi, dans la fabrication d'une composition pharmaceutique devant servir à inhiber la prolifération anormale de cellules, d'un composé de formule 1.0 : 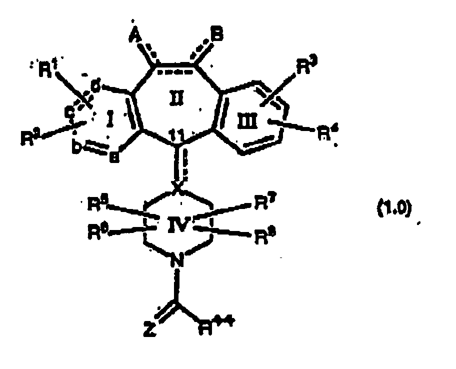 ou d'un sel ou d'un solvat, pharmacologiquement admissible, d'un tel composé,
dans laquelle formule :
l'un des symboles a, b, c et d représente N ou NR⁹ où R⁹ représente O-, -CH₃ ou -(CH₂)ₙCO₂H où n vaut de 1 à 3, et les autres des symboles a, b, c et d représentent CR¹ ou CR²,
ou chacun des symboles a, b, c et d représente indépendamment CR¹ ou CR² ;
chaque symbole R¹ et chaque symbole R² représente indépendamment un atome d'hydrogène ou d'halogène, un groupe de formule -CF₃, -OR¹⁰, -COR¹⁰, -SR¹⁰, -S(O)ₜR¹¹ (où t vaut 0, 1 ou 2), -N(R¹⁰)₂, -NO₂, -OC(O)R¹⁰, -CO₂R¹⁰, -OCO₂R¹¹, -CN, -NR¹⁰COOR¹¹, -SR¹¹C(O)OR¹¹, -SR¹¹N(R⁷⁵)₂ (où chaque symbole R⁷⁵ représente indépendamment un atome d'hydrogène ou un groupe de formule -C(O)OR¹¹), ou un groupe benzotriazol-1-yloxy, tétrazol-5-ylthio ou tétrazol-5-ylthio à substituant, ou encore un groupe alcynyle, alcényle ou alkyle, lequel groupe alkyle ou alcényle peut en option porter un substituant halogéno ou de formule -OR¹⁰ ou -CO₂R¹⁰ ;
R³ et R⁴ représentent des entités identiques ou différentes et représentent chacun, indépendamment, un atome d'hydrogène ou l'un des substituants indiqués à propos de R¹ et R², ou bien R³ et R⁴ représentent conjointement un cycle en C₅₋₇, saturé ou insaturé, condensé avec le cycle benzénique ;
R⁵, R⁶, R⁷ et R⁸ représentent chacun, indépendamment, un atome d'hydrogène, un groupe de formule -CF₃ ou -COR¹⁰, ou un groupe alkyle ou aryle, lequel groupe alkyle ou aryle porte éventuellement un substituant de formule -OR¹⁰, -SR¹⁰, -S(O)ₜR¹¹, -NR¹⁰COOR¹¹, -N(R¹⁰)₂, -NO₂, -COR¹⁰, -OCOR¹⁰, -OCO₂R¹¹, -CO₂R¹⁰ ou -OPO₃R¹⁰, ou l'un des symboles R⁵, R⁶, R⁷ et R⁸ peut représenter, conjointement avec R⁴⁴ dont les significations sont indiquées plus loin, un groupe de formule -(CH₂)ᵣ- où r vaut de 1 à 4, lequel groupe peut porter un substituant alkyle inférieur, alcoxy inférieur, trifluorométhyle ou aryle, ou bien R⁵ et R⁶ représentent conjointement un substituant =O ou =S, et/ou R⁷ et R⁸ représentent conjointement un substituant =O ou =S ;
R¹⁰ représente H ou un groupe alkyle, aryle ou aralkyle ;
R¹¹ représente un groupe alkyle ou aryle ;
X représente N, CH ou C, lequel C peut être lié à l'atome de carbone n° 11 par une éventuelle double liaison qu'indique le trait en pointillé ;
le trait en pointillé entre les atomes de carbone n° 5 et 6 indique une éventuelle double liaison, si bien que, lorsqu'il y a ici une double liaison, A et B représentent chacun, indépendamment, un atome d'halogène ou un groupe symbolisé par -R¹⁰, -OR¹¹, -OCO²R¹¹ ou -OC(O)R¹⁰, et lorsqu'il n'y a pas de double liaison entre les atomes de carbone n° 5 et 6, A et B représentent chacun, indépendamment, une paire H₂, (-OR¹¹)₂, H et halogéno, (halogéno)₂, alkyle et H, (alkyle)₂, H et -OC(O)R¹⁰, H et -OR¹⁰, ou aryle et H, ou un substituant =O, =NOR¹⁰ ou -O-(CH₂)ₚ-O- où p vaut 2, 3 ou 4 ;
R⁴⁴ représente un groupe de formule 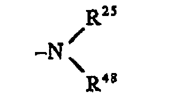 dans laquelle R²⁵ représente un groupe aryle ou hétéroaryle et R⁴⁸ représente un atome d'hydrogène ou un groupe alkyle, ou R⁴⁴, considéré conjointement avec R⁵, R⁶, R⁷ ou R⁸, représente ce qui est indiqué plus haut ;
et Z représente O ou S ;
étant entendu que, sauf indication contraire, les termes "alkyle", "alcényle", "alcynyle", "aryle", "halogène" et "hétéroaryle" ont les significations suivantes :
un groupe "alkyle", y compris les fragments alkyle des groupes alcoxy, alkylamino et dialkylamino, est une chaîne linéaire ou ramifiée d'atomes de carbone, qui en comporte de 1 à 20, et de préférence de 1 à 6 ;
un groupe "alcényle" est une chaîne linéaire ou ramifiée d'atomes de carbone, qui en comporte de 2 à 12, de préférence de 2 à 6 et surtout de 3 à 6, et qui contient au moins une double liaison carbone-carbone ;
un groupe "alcynyle" est une chaîne linéaire ou ramifiée d'atomes de carbone, qui en comporte de 2 à 12 et de préférence de 2 à 6, et qui contient au moins une triple liaison carbone-carbone ;
un groupe "aryle", y compris les fragments aryle des groupes aryloxy et aralkyle, est un groupe carbocyclique qui comporte de 6 à 15 atomes de carbone, ainsi qu'au moins un cycle aromatique (par exemple, un groupe aryle peut être un cycle phényle), et dont tous les atomes de carbone disponibles pour porter un substituant peuvent servir de points de raccordement, ce groupe carbocyclique pouvant en option porter par exemple 1 à 3 substituants choisis parmi les atomes d'halogène et les groupes alkyle, hydroxy, alcoxy, phénoxy, trifluorométhyle, amino, alkylamino, dialkylamino, nitro et -COOR¹⁰ ;
"halogène" signifie fluor, chlore, brome ou iode ;
et "hétéroaryle" est un groupe cyclique, portant éventuellement un substituant R³ ou R⁴, comportant au moins un hétéroatome choisi parmi les atomes d'oxygène, de soufre et d'azote, lequel hétéxoatome est inséré dans une structure carbocylique, et possédant des électrons π délvcalisés en un nombre suffisant pour présenter un caractère aromatique, un tel groupe hétérocyclique aromatique comportant de préférence de 2 à 14 atomes de carbone ; on peut citer comme exemples les groupes 2-pyridyle, 3-pyridyle et 4-pyridyle, ainsi que les groupes pydidyle-N-oxyde, tous portant éventuellement des substituants symbolisés par R³ et R⁴, un groupe pyridyle-N-oxyde pouvant être représenté par l'une des formules suivantes :

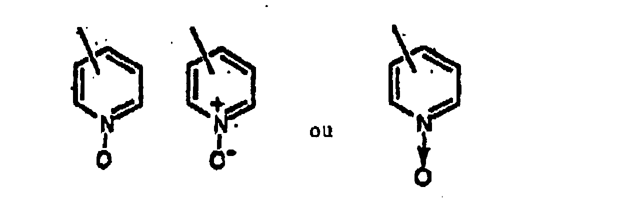

2. Emploi conforme à la revendication 1, dans lequel l'un des symboles a, b, c et d représente N ou NO, et les autres des symboles a, b, c et d représentent CR¹ ou CR².

3. Emploi conforme à la revendication 2, dans lequel le symbole a représente N, et les symboles b, c et d représentent CR¹ ou CR².

4. Emploi conforme à l'une des revendications précédentes, dans lequel chaque symbole R¹ et chaque symbole R² représente indépendamment un atome d'hydrogène ou d'halogène, un groupe de formule -CF₃, un groupe alkyle inférieur ou un groupe benzotriazol-1-yloxy.

5. Emploi conforme à la revendication 4, dans lequel R¹ représente un atome de chlore ou d'hydrogène, et R² représente un atome d'hydrogène, de chlore ou de brome.

6. Emploi conforme à l'une des revendications précédentes, dans lequel le substituant symbolisé par R¹ se situe en position C-4 et le substituant symbolisé par R² se situe en position C-3.

7. Emploi conforme à l'une des revendications précédentes, dans lequel R³ et R⁴ représentent des entités identiques ou différentes et représentent chacun, indépendamment, un atome d'hydrogène ou d'halogène, un groupe de formule -CF₃ ou -OR¹⁰, ou un groupe alkyle.

8. Emploi conforme à la revendication 7, dans lequel R³ et R⁴ représentent chacun, indépendamment, un atome d'hydrogène ou d'halogène.

9. Emploi conforme à l'une des revendications précédentes, dans lequel le substituant symbolisé par R³ se situe on position C-8 et le substituant symbolisé par R⁴ se situe en position C-9.

10. Emploi conforme à la revendication 9, dans lequel R³ représente un atome de chlore situé en position C-8 et R⁴ représente un atome d'hydrogène situé en position C-9.

11. Emploi conforme à l'une des revendications précédentes, dans lequel R⁵, R⁶, R⁷ et R⁸ représentent chacun un atome d'hydrogène.

12. Emploi conforme à la revendication 1, dans lequel :
le symbole a représente un atome d'azote, et les symboles b, c et d représentent des atomes de carbone ;
R¹ et R² représentent des entités identiques ou différentes et représentent chacun, indépendamment, un atome d'hydrogène ou d'halogène, un groupe de formule -CF₃, un groupe alkyle inférieur ou un groupe bcnzotriazol-1-yloxy, le substituant symbolisé par R¹ se situant en position C-4 et le substituant symbolisé par R² se situant en position C-3 ;
R³ et R⁴ représentent des entités identiques ou différentes et représentent chacun, indépendamment, un atome d'hydrogène ou d'halogène, le substituant symbolisé par R³ se situant en position C-8 et le substituant symbolisé par R⁴ se situant en position C-9 ;
dans le cas où il y a une double liaison entre les atomes de carbone n° 5 et 6, A et B représentent chacun, indépendamment, un atome d'hydrogène ou un groupe alkyle inférieur ou alcoxy, et dans le cas où il n'y a pas de double liaison entre les atomes de carbone n° 5 et 6, A et B représentent chacun, indépendamment, une paire d'atomes d'hydrogène, une paire -H et -OH, ou un substituant =O ;
R⁵, R⁶, R⁷ et R⁸ représentent chacun un atome d'hydrogène ;
Z représente un atome d'oxygène O ;
et dans le substituant symbolisé par R⁴⁴, R²⁵ représente un groupe pyridyle ou phényle.

13. Emploi conforme à la revendication 12, dans lequel R²⁵ représente un groupe 3-pyridyle ou phényle, R³ représente un atome de chlore, R⁴ représente un atome d'hydrogène, R⁴⁸ représente un atome d'hydrogène ou un groupe méthyle, et R¹ et R² représentent chacun, indépendamment, un atome d'hydrogène ou d'halogène, un groupe alkyle en C₁₋₄ ou un groupe benzotriazol-1-yloxy.

14. Emploi conforme à la revendication 13, dans lequel R¹ et R² représentent chacun, indépendamment, un atome d'hydrogène, de chlore ou de brome, un groupe méthyle ou un groupe benzotriazol-1-yloxy.

15. Emploi conforme à l'une des revendications précédentes, dans lequel les cellules à inhiber sont des cellules tumorales exprimant un oncogène *ras* activé.

16. Emploi conforme à la revendication 15, dans lequel les cellules à inhiber sont des cellules de tumeur du pancréas, des cellules de cancer du poumon, des cellules tumorales de leucémie myéloïde, des cellules de tumeur des vésicules thyroïdiennes, des cellules tumorales de syndrome myélodysplasique, des cellules tumorales de carcinome épidermoïde, des cellules tumorales de cancer de la vessie ou des cellules de tumeur du côlon.

17. Emploi conforme à la revendication 1, dans lequel l'inhibition de la prolifération anormale des cellules a lieu par inhibition d'une protéine Ras-farnésyl-transférase.

18. Emploi conforme à la revendication 1, dans lequel l'inhibition concerne des cellules tumorales chez lesquelles la protéine Ras est activée en résultat d'une mutation oncogénique survenue dans des gènes autres que le gêne *ras*.

19. Emploi conforme à la revendication 1, dans lequel le composé est choisi parmi ceux qui sont représentés par les formules suivantes :

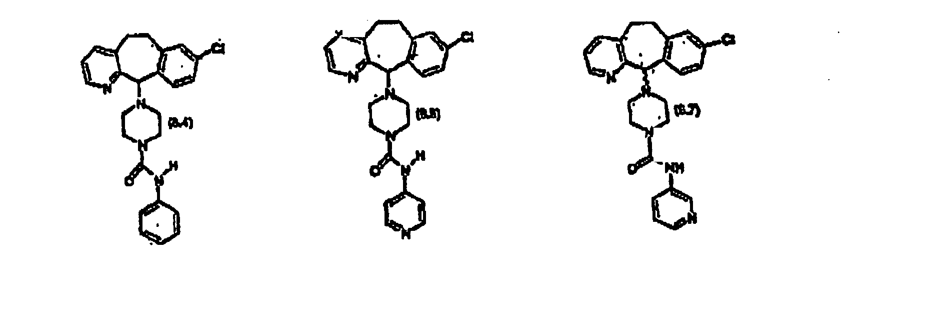

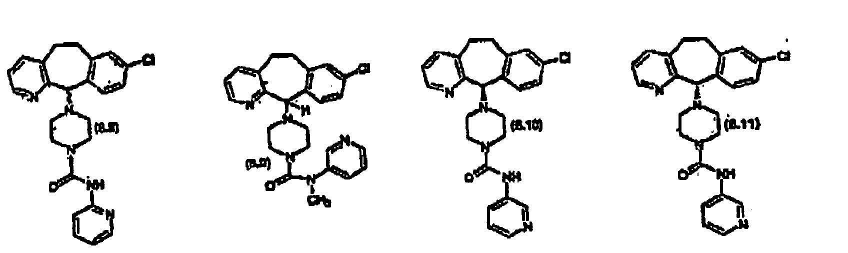

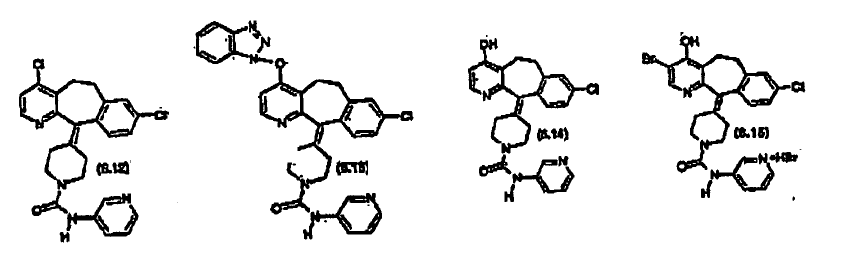

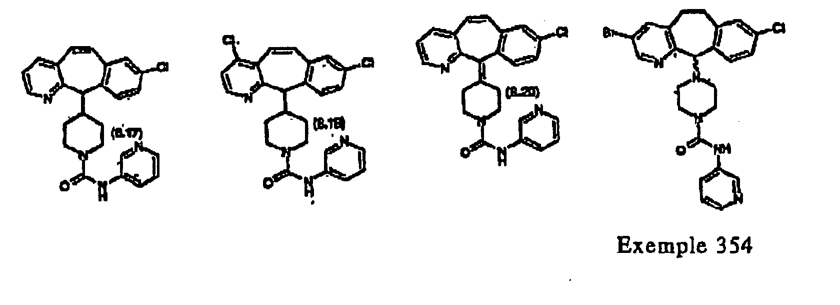

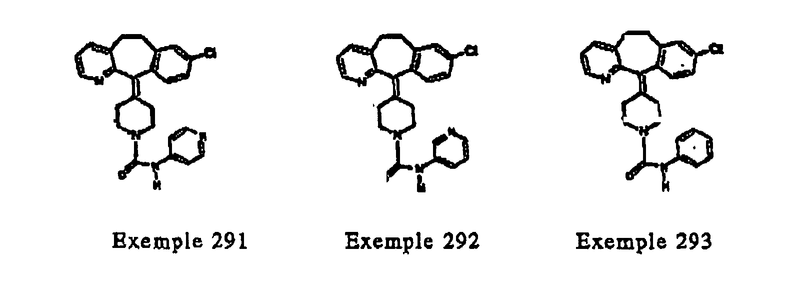

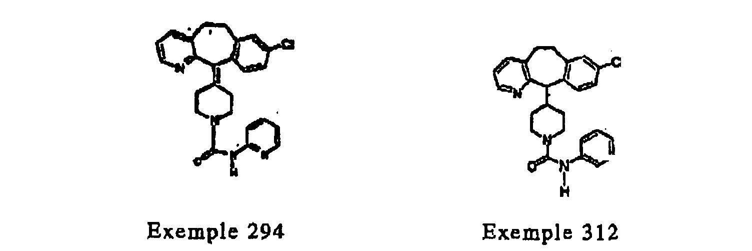

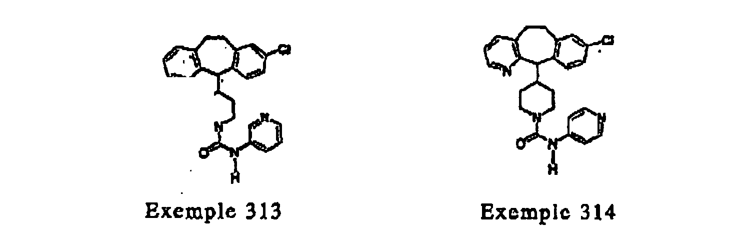

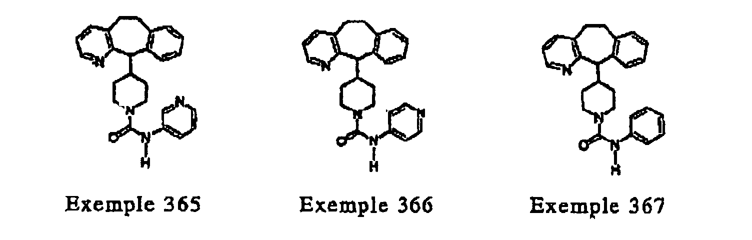

20. Composé choisi parmi ceux correspondant à l'une des formules suivantes : 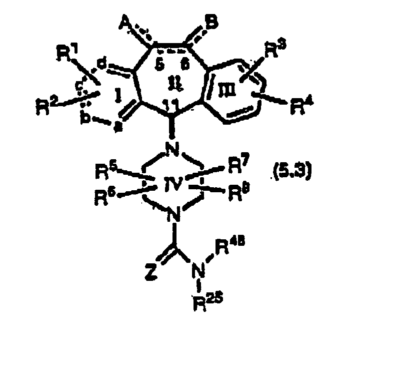 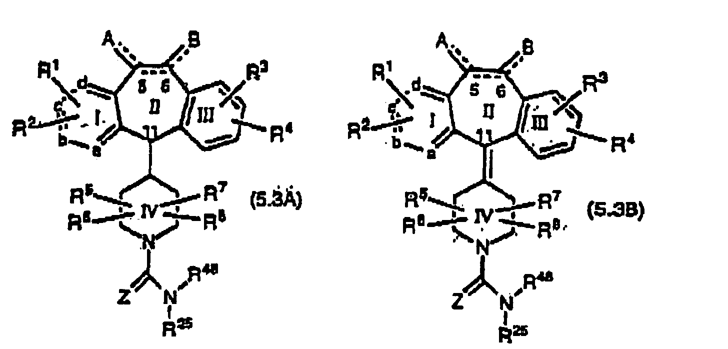 dans lesquelles tous les symboles ont les significations définies dans la revendication 1,
ou sel ou solvat pharmacologiquement admissible d'un tel composé.

21. Composé conforme à la revendication 20, dans lequel :
le symbole a représente un atome d'azote, et les symboles b, c et d représentent des atomes de carbone ;
R¹ et R² représentent des entités identiques ou différentes et représentent chacun, indépendamment, un atome d'hydrogène ou d'halogène, un groupe de formule -CF₃, un groupe alkyle inférieur ou un groupe benzotriazol-1-yloxy, le substituant symbolisé par R¹ se situant en position C-4 et le substituant symbolisé par R² se situant en position C-3 ;
R³ et R⁴ représentent des entités identiques ou différentes et représentent chacun, indépendamment, un atome d'hydrogène ou d'halogène, le substituant symbolisé par R³ se situant en position C-8 et le substituant symbolisé par R⁴ se situant on position C-9 ;
dans le cas où il y a une double liaison entre les atomes de carbone n° 5 et 6, A et B représentent chacun, indépendamment, un atome d'hydrogène ou un groupe alkyle inférieur ou alcoxy, et dans le cas où il n'y a pas de double liaison entre les atomes de carbone n° 5 et 6, A et B représentent chacun, indépendamment, une paire d'atomes d'hydrogène, une paire -H et -OH, ou un substituant =O ;
R⁵, R⁶, R⁷ et R⁸ représentent chacun un atome d'hydrogène ;
Z représente un atome d'oxygène O ;
et R²⁵ représente un groupe phényle, 3-pyridyle, 3-pyridyl-N-oxyde, 4-pyridyle, 4-pyridyl-N-oxyde, N-méthyl-pipéridin-3-yle, N-méthyl-pipéridin-4-yle, N-acétyl-pipéridin-3-yle ou N-acétyl-pipéridin-4-yle.

22. Composé conforme à la revendication 21, dans lequel R²⁵ représente un groupe phényle, 3-pyridyle, 3-pyridyl-N-oxyde, 4-pyridyle, 4-pyridyl-N-oxyde, N-méthyl-pipéridin-3-yle ou N-méthyl-pipéridin-4-yle, R³ représente un atome de chlore, R⁴ représente un atome d'hydrogène, R⁴⁸ représente un atome d'hydrogène ou un groupe méthyle. et R¹ et R² xeprésentent chacun, indépendamment, un atome d'hydrogène ou d'halogène, un groupe alkyle en C₁₋₄ ou un groupe benzotriazol-1-yloxy.

23. Composé conforme à la revendication 22, dans lequel R¹ et R² représentent chacun, indépendamment, un atome d'hydrogène, de chlore ou de brome, un groupe méthyle ou un groupe benzotriazol-1-yloxy.

24. Composé conforme à la revendication 20, choisi parmi ceux qui sont représentés par les formules suivantes :

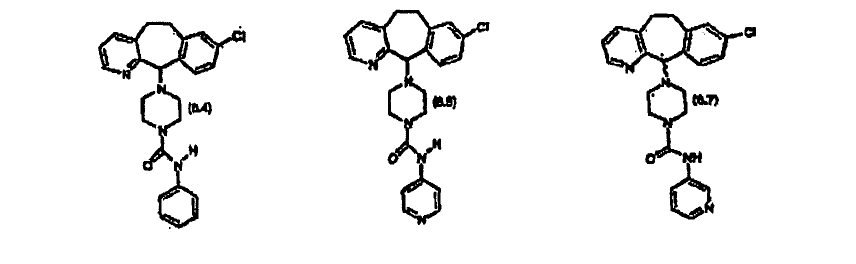

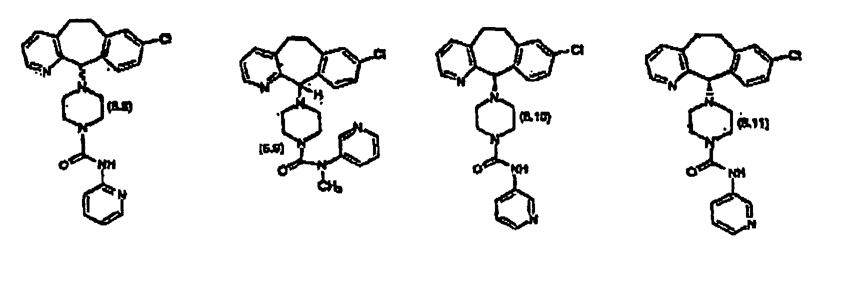

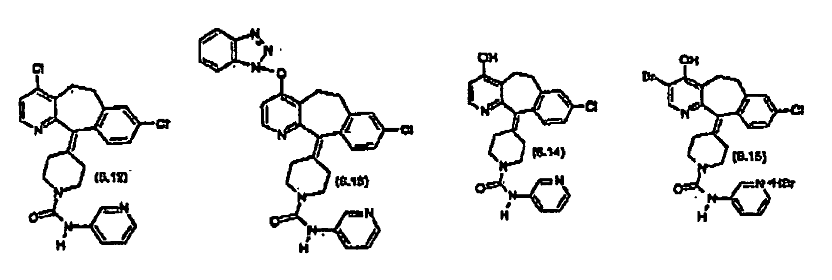

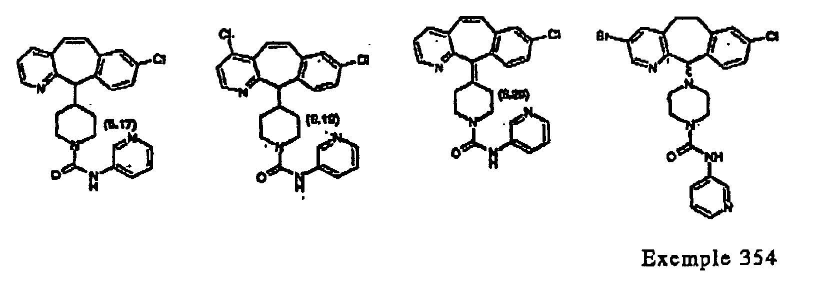

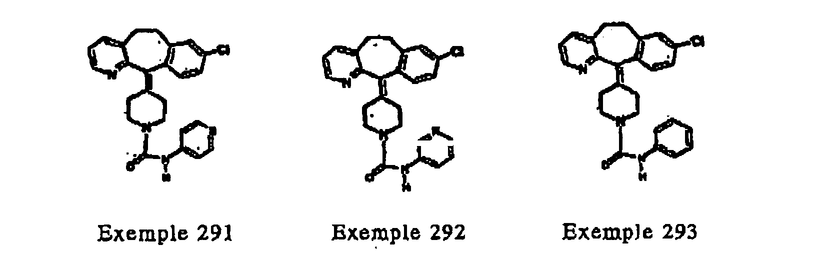

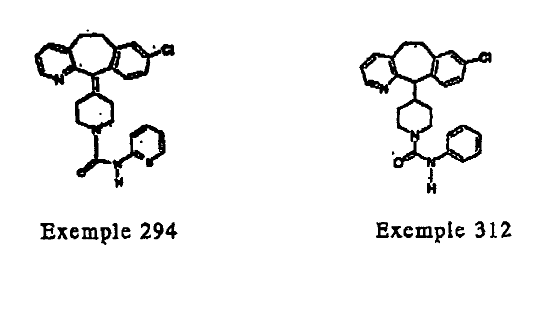

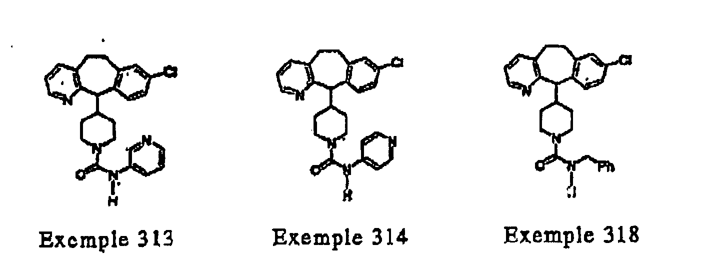

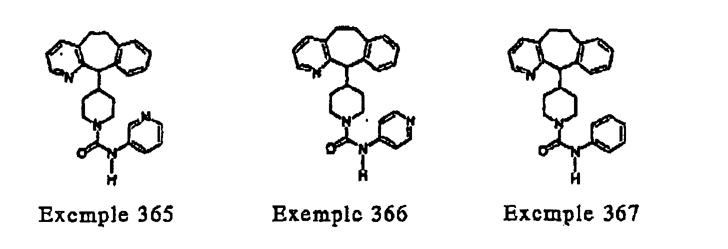

25. Composition pharmaceutique destinée à inhiber la prolifération anormale de cellules, comprenant un composé conforme à la revendication 20 en une quantité suffisante pour qu'il ait un effet, en association avec un véhicule pharmacologiquement admissible.

26. Procédé de préparation de dérivés 3-nitrés de formule 1.0h, qui peuvent servir d'intermédiaires dans la synthèse des composés de formule 1.0 : 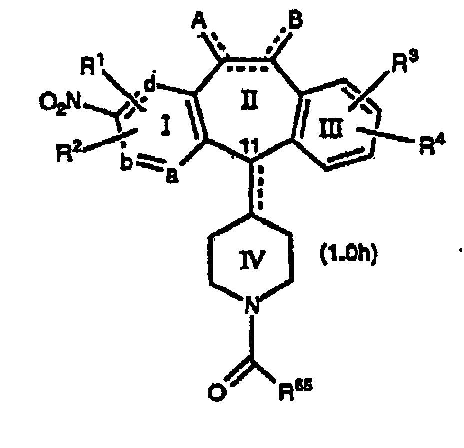 dans laquelle les symboles R¹, R², R³, R⁴, A, B, a, b et d, ainsi que les traits en pointillé, ont les significations indiquées dans la revendication 1 à propos de la formule 1.0, et R⁶⁵ représente un atome d'hydrogène ou un groupe représenté par -OR⁶⁶ où R⁶⁶ représente un groupe alkyle, lequel procédé comporte :
le fait de faire réagir un équivalent molaire d'un composé de formule 1.0g : 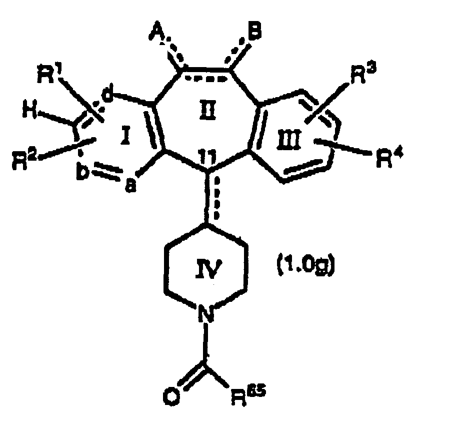
dans laquelle les symboles R¹, R², R³, R⁴, A, B, a, b et d, ainsi que les traits en pointillé, ont les significations indiquées dans la revendication 1 à propos de la formule 1.0, et R⁶⁵ représente un atome d'hydrogène ou un groupe représenté par -OR⁶⁶ où R⁶⁶ représente un groupe alkyle,
avec un équivalent molaire d'un réactif de nitration, lequel réactif de nitration est formé au préalable par mélange à froid de quantités équimolaires de nitrate de tétrabutyl-ammonium et d'anhydride trifluoroacétique,
la réaction de ce réactif de nitration et dudit composé de formule 1,0g se déroulant dans un solvant aprotique approprié,
et cette réaction effectuée avec ledit réactif de nitration étant menée à une température suffisante et durant suffisamment longtemps pour qu'elle puisse avancer à un degré raisonnable afin de donner un dérivé 3-nitré de formule 1.0h.

27. Procédé de préparation de dérivés 3-nitrés de formule 1.0i, qui peuvent servir d'intermédiaires dans la synthèse des composés de formule 1.0 : 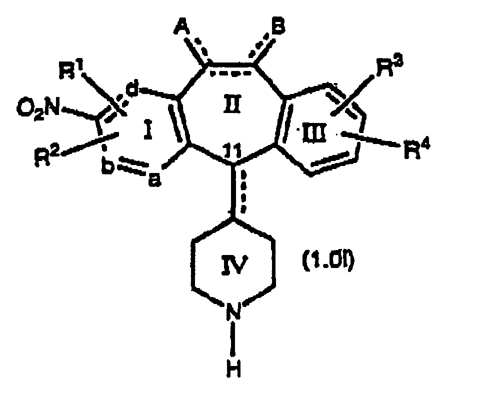 dans laquelle les symboles R¹, R², R³, R⁴, A, B, a, b et d, ainsi que les traits en pointillé, ont les significations indiquées dans la revendication 1 à propos de la formule 1.0, lequel procédé comporte :
le fait de faire réagir un équivalent molaire d'un composé de formule 1.0g : 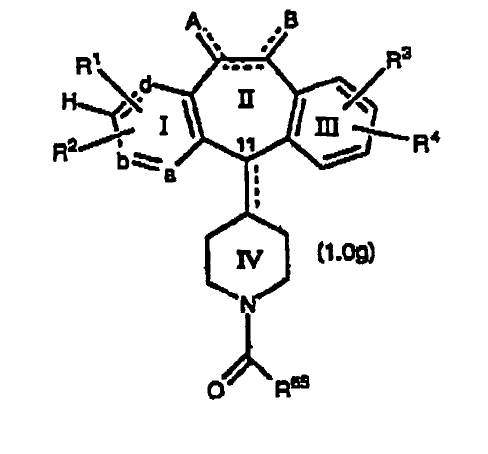
dans laquelle les symboles R¹, R², R³, R⁴, A, B, a, b et d, ainsi que les traits en pointillé, ont les significations indiquées dans la revendication 1 à propos de la formule 1.0, et R⁶⁵ représente un atome d'hydrogène ou un groupe représenté par -OR⁶⁶ où R⁶⁶ représente un groupe alkyle,
avec un équivalent molaire d'un réactif de nitration, lequel réactif de nitration est formé au préalable par mélange à froid de quantités équimolaires de nitrate de tétrabutyl-ammonium et d'anhydride trifluoroacétique,
la réaction de ce réactif de nitration et dudit composé de formule 1.0g se déroulant dans un solvant aprotique approprié,
et cette réaction effectuée avec ledit réactif de nitration étant menée à une température suffisante et durant suffisamment longtemps pour qu'elle puisse avancer à un degré raisonnable afin de donner un dérivé 3-nitré de formule 1.0h : 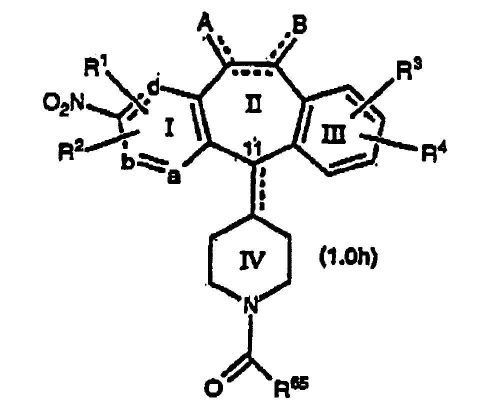 et le fait d'hydrolyser ce composé de formule 1.0h, en dissolvant ce composé de formule 1.0h dans une quantité suffisante d'acide concentré et en chauffant le mélange résultant à une température suffisante pour éliminer le substituant représenté par -C(O)R⁶⁵ et produire ainsi le composé de formule 1.0i.

28. Procédé de préparation de composés de formule 1.0j, qui peuvent servir d'intermédiaires dans la synthèse des composés de formule 1.0 : 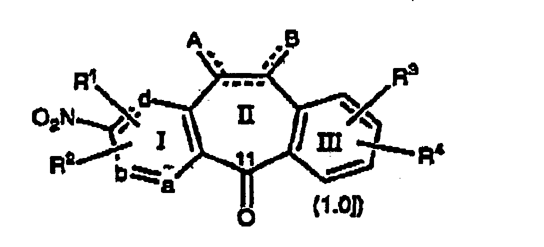 dans laquelle les symboles R¹, R², R³, R⁴, A, B, a, b et d, ainsi que les traits en pointillé, ont les significations indiquées dans la revendication 1 à propos de la formule 1.0,
Jequel procédé comporte :
le fait de faire réagir un équivalent molaire d'un composé de formule : 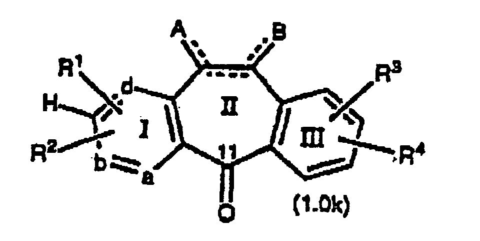
avec un équivalent molaire d'un réactif de nitration,
lequel réactif de nitration est formé au préalable par mélange à froid de quantités équimolaires de nitrate de tétrabutyl-ammonium et d'anhydride trifluoroacétique,
la réaction de ce réactif de nitration et dudit composé de formule 1.0k se déroulant dans un solvant aprotique approprié,
et cette réaction effectuée avec ledit réactif de nitration étant menée à une température suffisante et durant suffisamment longtemps poux qu'elle puisse avancer à un degré raisonnable afin de donner un dérivé 3-nitré de formule 1.0j.

29. Procédé de préparation de composés de formule 1.0m, qui peuvent servir d'intermédiaires dans la synthèse des composés de formule 1.0 : 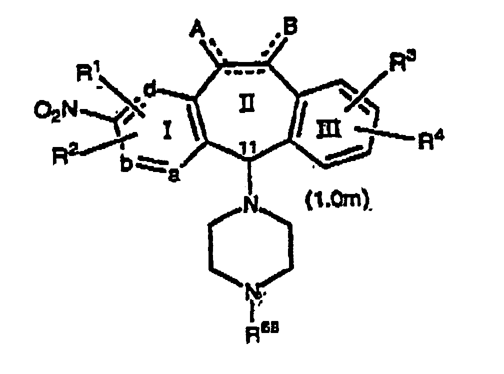 dans laquelle les symboles R¹, R², R³, R⁴, A, B, a, b et d, ainsi que les traits en pointillé, ont les significations indiquées dans la revendication 1 à propos de la formule 1.0, et R⁶⁸ représénte un atome d'hydrogène ou un groupe de formule -COOR^{a} où R^{a} représente un groupe alkyle en C₁₋₃,
lequel procédé comporte :
le fait de faire réagir un équivalent molaire d'un composé de formule : 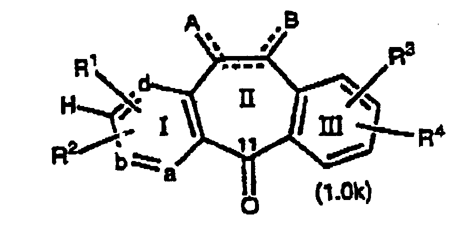
avec un équivalent molaire d'un réactif de nitration,
lequel réactif de nitration est formé au préalable par mélange à froid de quantités équimolaires de nitrate de tétrabutyl-ammonium et d'anhydride trifluoroacétique,
la réaction de ce réactif de nitration et dudit composé de formule 1.0k se déroulant dans un solvant aprotique approprié,
et cette réaction effectuée avec ledit réactif de nitration étant menée à une température suffisante et durant suffisamment longtemps pour qu'elle puisse avancer à un degré raisonnable afin de donner un dérivé 3-nitré de formule 1.0j : 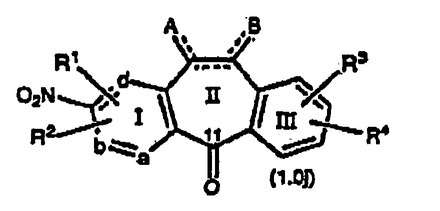 le fait de réduire ce composé de formule 1.0j à l'aide d'un agent réducteur approprié, dans un solvant approprié, et à une température appropriée pour que cette réaction puisse avancer à un degré raisonnable,
le fait de faire réagir le produit hydroxylé ainsi obtenu avec un agent de chloration, dans un solvant organique approprié, et à une température appropriée pour que cette réaction puisse avancer à un degré raisonnable afin de donner un composé de formule 1.0n : 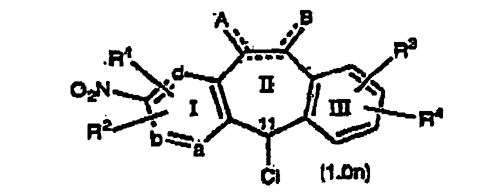 et le fait de faire réagir ce composé de formule 1.0n avec un composé de formule 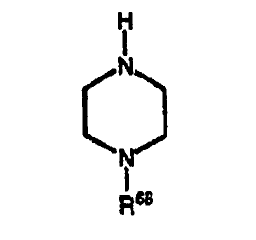 dans laquelle R⁶⁸ a la signification indiquée plus haut,
dans un solvant organique approprié contenant une base appropriée, et à une température appropriée pour que cette réaction puisse avancer à un degré raisonnable afin de donner un composé de formule 1.0m.

30. Composé servant d'intermédiaire pour la préparation d'un composé de formule 1.0, choisi parmi les composés de formules : 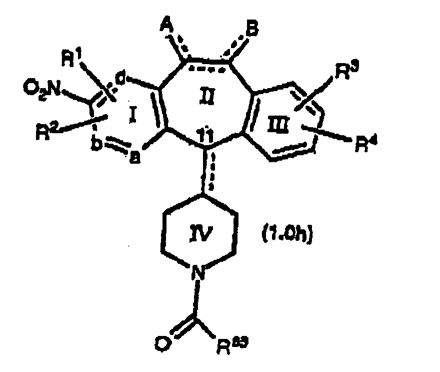 dans laquelle les symboles R¹, R⁵, R³, R⁴, A, B, a, b, d et R⁶⁵ ont les significations indiquées dans la revendication 26 à propos de la formule 1.0h ; 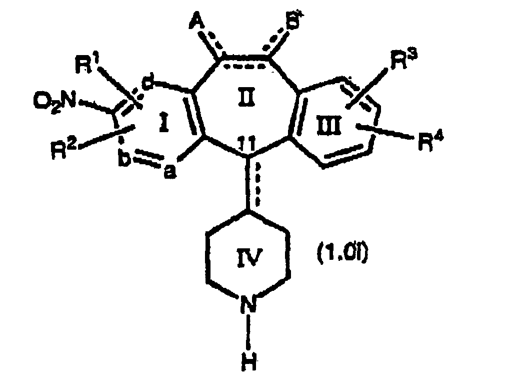 dans laquelle les symboles R¹, R², R³, R⁴, A, B, a, b et d ont les significations indiquées dans la revendication 27 à propos de la formule 1.0i ; 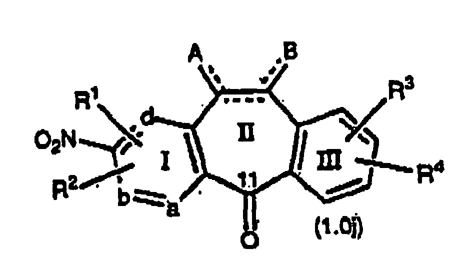 dans laquelle les symboles R¹, R², R³, R⁴, A, B, a, b et d ont les significations indiquées dans la revendication 28 à propos de la formule 1.0j ; 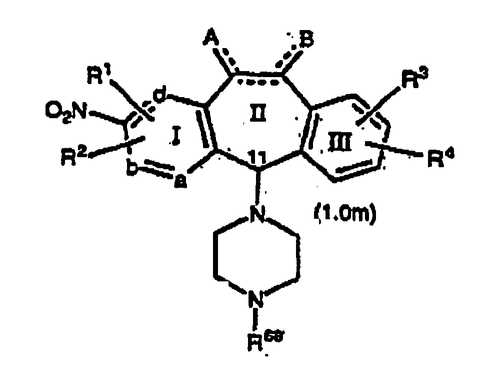 dans laquelle les symboles R¹, R², R³, R⁴, A, B, a, b, d et R⁶⁸ ont les significations indiquées dans la revendication 29 à propos de la formule 1.0m ; 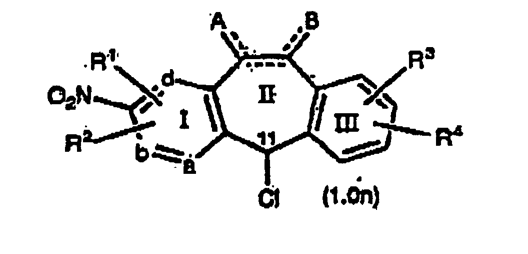 dans laquelle les symboles R¹, R², R³, R⁴, A, B, a, b et d ont les significations indiquées dans la revendication 28 à propos de la formule 1.0j ;
ou 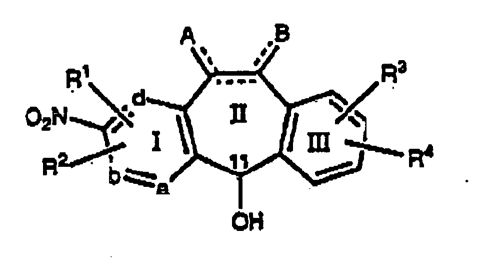 dans laquelle les symboles R¹, R², R³, R⁴, A, B, a, b et d ont les significations indiquées dans la revendication 28 à propos de la formule 1.0j.

31. Composé conforme à la revendication 30, choisi parmi les composés représentés par les formules suivantes :

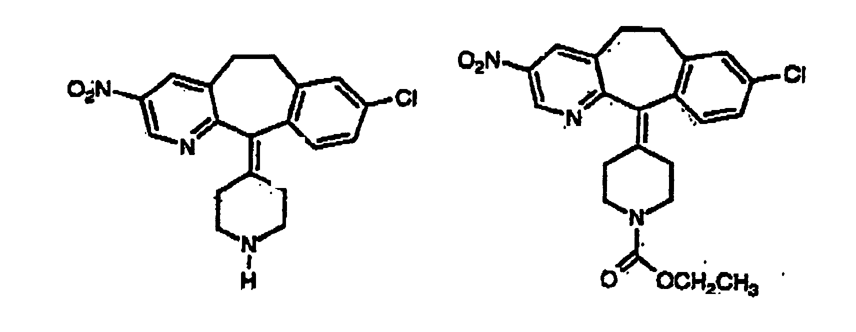

32. Emploi d'un composé conforme à la revendication 20, en vue de la fabrication d'un médicament pouvant servir à inhiber une prolifération anormale de cellules.

33. Procédé de préparation d'un composé conforme à la revendication 20, lequel procédé comporte :
a) le fait de faire réagir un composé de formule 405.00 : 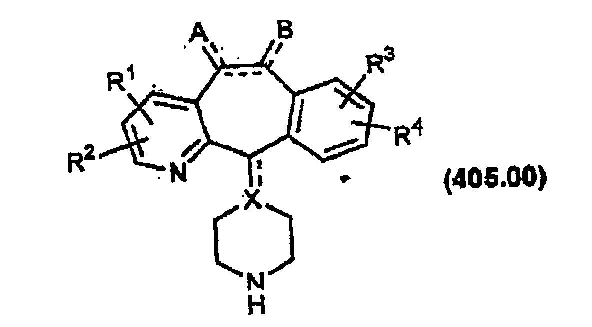 avec un composé de formule R⁴⁴-COOH, dans un solvant inerte et à une température située entre 0 °C et la température de reflux ;
b) le fait de faire réagir un composé de formule 405.00 avec un composé de formule R⁴⁴-C(O)L où L représente un groupe partant approprié, en présence d'une base ;
c) le fait de faire réagir un composé de formule 420.00 : 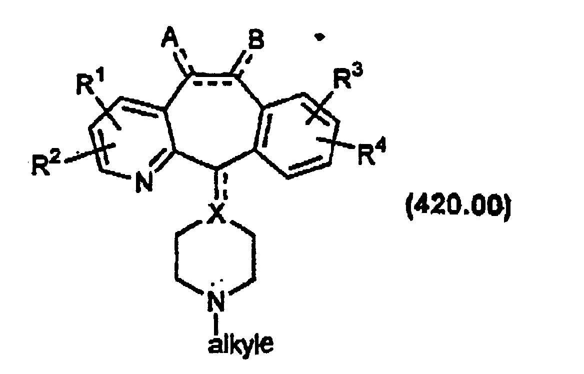 avec un halogénure d'acide ou un anhydride d'acide approprié ;
d) le fait de réduire un composé de formule 405.00 dans laquelle X représente un atome de carbone et la liaison reliant cet atome à l'atome de carbone n° 11 est une liaison double, avec de l'hydrure de lithium et d'aluminium, dans du tétrahydrofurane, et le fait de faire ensuite réagir le produit de cette réduction du composé de formule 405.00 avec un composé de formule R⁴⁴-COOH ou R⁴⁴-C(O)L, ce qui donne un composé de formule 1.0 indiquée dans la revendication 1, dans laquelle X représente un atome de carbone et la liaison reliant cet atome à l'atome de carbone n° 11 est une liaison simple ;
e) le fait de réduire un composé de formule 470.00d : 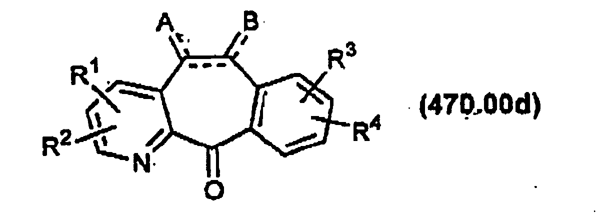 avec du borohydrure de sodium, dans du méthanol ou de l'éthanol, le fait de convertir l'alcool ainsi obtenu en un chlorure, à l'aide de chlorure de thionyle, le fait de déplacer l'atome de chlore avec de la pipérazine et le fait de faire réagir le composé ainsi obtenu avec un composé de formule R⁴⁴-COOH ou R⁴⁴-C(O)L, de la façon décrite ci-dessus à propos de la conversion d'un composé de formule 405.00 en un composé de formule 1.0 dans laquelle la liaison aboutissant à l'atome de carbone n° 11 est une liaison simple ;
f) le fait de cycliser un composé de formule 455.00 : 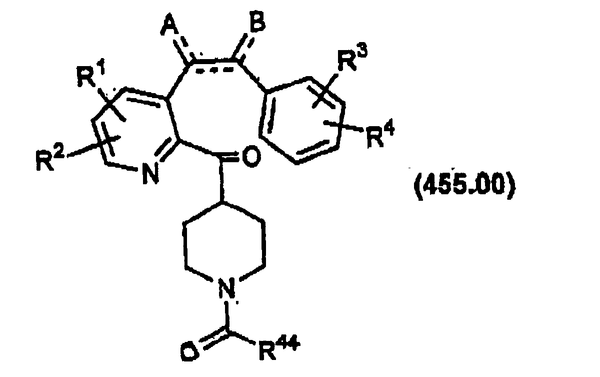 pour obtenir un composé de formule 1.0 dans laquelle la liaison aboutissant à l'atome de carbone n° 11 est une liaison simple ;
g) le fait de faire réagir une azacétone de formule 470.00 : 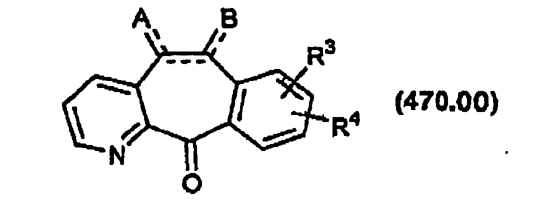 avec un agent oxydant, de manière à obtenir le composé correspondant dans lequel l'atome d'azote du cycle pyridinique aura été converti en N-oxyde, le fait de faire réagir le produit ainsi obtenu avec un agent de chloration, de manière à obtenir un composé de formule 470.00b : 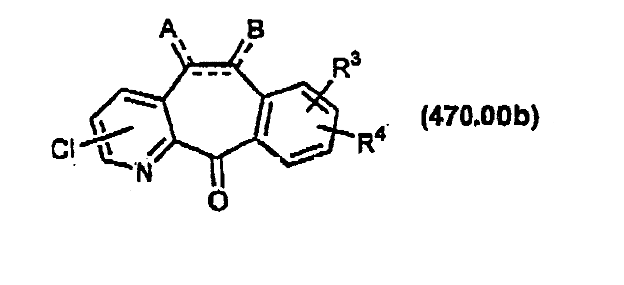 le fait de répéter, en option, l'opération de chloration de manière à obtenir un composé disubstitué de formule 470.00c : 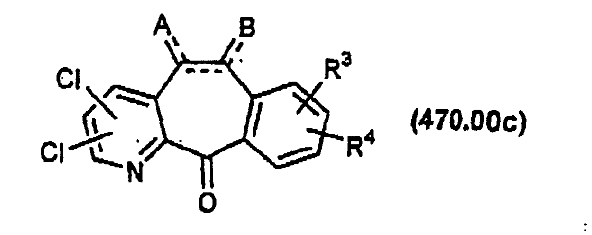 le fait de faire réagir, en option, le composé de formule 470.00b ou 470.00c avec un agent nncléophile, de manière à obtenir un composé substitué de formule 470.00d indiquée ci-dessus, et le fait de faire réagir ce composé de formule 470.00d de la manière décrite ci-dessus en (e), de manière à obtenir un composé de formule 1.0 ;
h) le fait de faire réagir le N-oxyde d'un composé de formule 405.00 indiquée ci-dessus avec de l'oxychlorure de phosphore POCl₃, de manière à obtenir un composé de formule 415.01 : 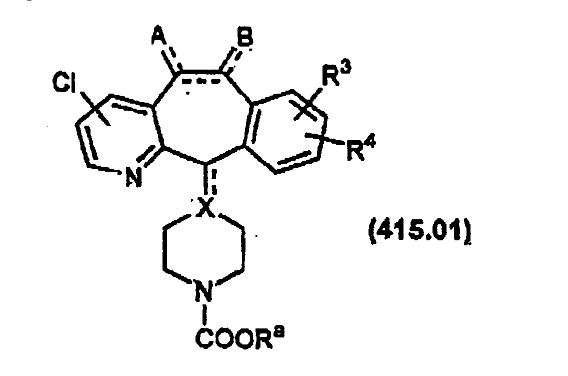
i) le fait de faire réagir un composé halogéné de formule 405.00, indiquée ci-dessus, dans laquelle R¹ représente un atome d'halogène, avec un alkyllithium de manière à obtenir un dérivé lithié, et le fait de faire ensuite réagir ce dérivé lithié avec un agent électrophile approprié ;
j) le fait de faire réagir un composé de formule 470.00h : 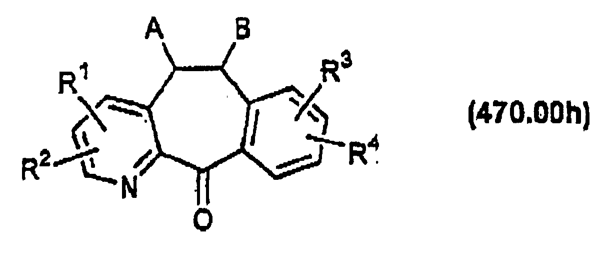 avec du dioxyde de sélénium SeO₂, dans de l'acide acétique, de manière à obtenir un composé de formule 470.00i : 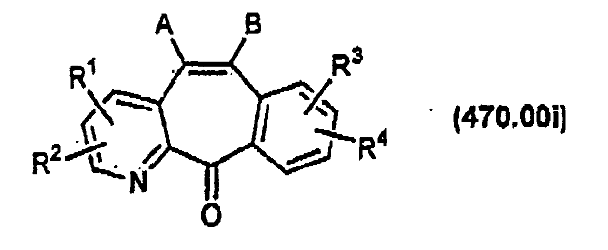 et le fait de convertir ensuite ce composé de formule 470.00i en un composé de formule 1.0 ;
k) le fait d'alkyler un dérivé de pipérazine portant des substituants appropriés, de formule 700.0 : 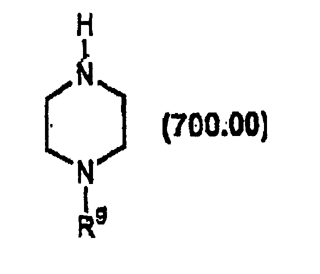 dans laquelle R⁹ représente un atome d'hydrogène ou un groupe de formule -C(Z)R⁴⁴ ou -CO₂R^{a},
avec un composé de formule 705.00 : 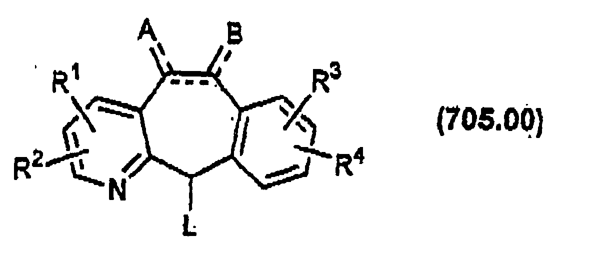 dans laquelle L représente un groupe partant,
de manière à obtenir, dans le cas où R⁹ représente un groupe de formule -C(Z)R⁴⁴, un composé de formule 1.0 dans laquelle X représente N, et dans le cas où R⁹ représente un atome d'hydrogène ou un groupe de formule -CO₂R^{a}, le fait de remplacer, dans le composé obtenu, cet atome d'hydrogène ou ce groupe de formule -CO₂R^{a} par un groupe de formule -C(Z)R⁴⁴, pour obtenir un composé de formule 1.0 dans laquelle X représente N ;
1) ou le fait d'effectuer l'amination réductrice d'une azacétone de formule 715.00 ; 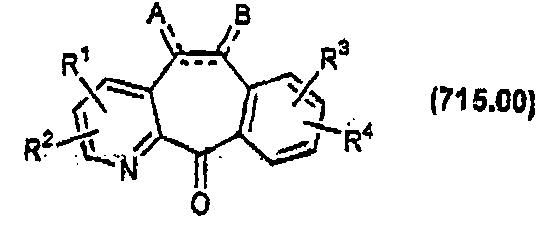 avec une pipérazine de formule 700.00 indiquée ci-dessus, dans laquelle R^{g} représente un atome d'hydrogène ou un groupe de formule -C(Z)R⁴⁴ ou -CO₂R^{a},
de manière à obtenir, dans le cas où R⁹ représente un groupe de formule -C(Z)R⁴⁴, un composé de formule 1.0, et dans le cas où R⁹ représente un atome d'hydrogène ou un groupe de formule -CO₂R^{a}, le fait de remplacer, dans le composé obtenu, cet atome d'hydrogène ou ce groupe de formule -CO₂R^{a} par un groupe de formule -C(Z)R⁴⁴, pour obtenir un composé de formule 1.0.
